# EUROPEAN PATENT APPLICATION

(11) **EP 4 257 155 A2**
(43) Date of publication of application: **11.10.2023**
(21) Application number: 23185569.3
(22) Date of filing: 15.11.2019
(51) Int. Cl.: A61K 48/00

(54) **COMPOSITIONS AND METHODS FOR TREATING WILSON'S DISEASE**

(30) Priority: 16.11.2018 US 201862768744 P
(62) Divisional of application: 19884231.2
(71) Applicant: Encoded Therapeutics, Inc., South San Francisco, CA 94080 (US)
(72) Inventor: RAMAMOORTHI, Kartik, South San Francisco, CA 94080 (US); TAGLIATELA, Stephanie, South San Francisco, CA 94080 (US); TANENHAUS, Anne, South San Francisco, CA 94080 (US); YOUNG, Andrew, South San Francisco, CA 94080 (US); CHEN, Szu-Ying, South San Francisco, CA 94080 (US); ZHANG, Chi, South San Francisco, CA 94080 (US); MARTIN, Stephanie, San Francisco, CA 94134 (US); OBERKOFLER, David, South San Francisco, CA 94080 (US); WONG, Victoria, South San Francisco, CA 94080 (US); LI, Jianmin, South San Francisco, CA 94080 (US)
(74) Representative: Carpmaels & Ransford LLP

(57) **Abstract**

Provided herein are nucleic acid molecules and vectors comprising variant copper-transporting ATPase 2 (ATP7B) nucleic acid sequences. Such sequences have been optimized for expression in mammalian cells, liver cells, and/or from an adeno associated viral vector (AAV), including truncated and/or codon optimized variants. Also provided are viral vectors comprising such ATP7B variant nucleic acid sequences, and methods of use thereof for treating disorders associated with an ATP7B deficiency, such as Wilson's disease.

## Description

### CROSS-REFERENCE

This application claims the benefit of U.S. Provisional Application No. 62/768,744, filed November 16, 2018, which is incorporated herein by reference in its entirety.

### BACKGROUND OF THE DISCLOSURE

Wilson's disease is an autosomal recessively inherited disorder of copper metabolism. Wilson's disease is caused by mutations of the Copper-transporting ATPase 2 (also called ATPase copper transporting beta) or ATP7B gene. ATP7B is expressed mainly in hepatocytes and functions in the transmembrane transport of copper. ATP7B deficiencies lead to decreased hepatocellular excretion of copper into bile and results in copper accumulation in various tissues including the liver, brain and other tissues. Existing therapies for Wilson's disease involve non-curative chelation approaches that seek to reduce copper levels. Therapeutic strategies, such as gene therapy, that can reverse the underlying metabolic defect would be greatly advantageous. However, the ATP7B gene is approximately 4.4 kB, nearing the packaging size limits of many gene therapy vectors and making gene therapy approaches with the full-length gene difficult. There is a need for gene therapy approaches for Wilson's disease that can provide expression of the full-length gene at therapeutic levels.

### SUMMARY OF THE DISCLOSURE

In one aspect, the application provides a nucleic acid molecule comprising a nucleotide sequence having (i) at least 80% sequence identity to SEQ ID NO: 3 or 4, or (ii) at least 92% sequence identity to SEQ ID NO: 5 or 6, wherein said nucleic acid molecule encodes a functional ATP7B protein. In certain embodiments, such nucleic acid molecule comprises a nucleotide sequence having at least 85% sequence identity to SEQ ID NO: 3 or 4, having at least 90% sequence identity to SEQ ID NO: 3 or 4, or having at least 95% sequence identity to any one of SEQ ID NOs: 3-6. In an exemplary embodiment, such nucleic acid molecule comprises a nucleotide sequence having any one of SEQ ID NOs: 3-6.

In certain embodiments, a nucleic acid molecule provided herein further comprises a regulatory element. In certain embodiments, such regulatory element comprises a sequence having at least 85%, 90%, 95% or 100% identity to any one of SEQ ID NOs: 19-43.

In another aspect, the application provides a nucleic acid molecule comprising: (i) a regulatory element comprising a sequence having at least 80% sequence identity to any one of SEQ ID NOs: 19-43, and (ii) a nucleotide sequence encoding a functional ATP7B protein, wherein the nucleotide sequence has been optimized for expression in liver from a viral vector. In certain embodiments, the regulatory element comprises a sequence having at least 85%, 90%, 95% or 100% sequence identity to any one of SEQ ID NOs: 19-43. In certain embodiments, the nucleotide sequence encoding the functional ATP7B protein has been codon optimized for expression in the liver. In certain embodiments, the nucleotide sequence encoding the functional ATP7B protein has been optimized for expression from an AAV vector. In certain embodiments, the nucleotide sequence encoding the functional ATP7B protein has least 80%, 85%, 90%, 95% or 100% sequence identity to any one of SEQ ID NOs: 3-18.

In certain embodiments, a nucleic acid molecule provided herein encodes a functional fragment of ATP7B. In other embodiments, a nucleic acid molecule provided herein encodes a full length ATP7B protein. In other embodiments, a nucleic acid molecule provided herein encodes an ATP7B protein having SEQ ID NO: 2.

In certain embodiments, a nucleic acid molecule provided herein results in a level of expression of functional ATP7B protein from the nucleic acid molecule in the liver that is at least 3-fold greater relative to the level of expression of ATP7B protein in the liver from a nucleic acid molecule comprising SEQ ID NO: 1. In other embodiments, the level of expression of functional ATP7B protein from the nucleic acid molecule in the liver is at least 5-fold or 7-fold greater relative to the level of expression of ATP7B protein in the liver from a nucleic acid molecule comprising SEQ ID NO: 1.

In certain embodiments, a nucleic acid molecule provided herein comprises a regulatory element wherein the regulatory element comprises a promoter sequence. In certain embodiments, the promoter sequence produces at least 10-fold or at least 50-fold greater expression in a mammalian cell relative to the CMV promoter. In certain embodiments, the regulatory element comprises an enhancer sequence. In certain embodiments, the regulatory element has less than 150 bp, 120 bp, or 105 bp.

In certain embodiments, a nucleic acid molecule provided herein further comprises a 5' ITR and 3' ITR sequences of a virus. In certain embodiments, the 5' ITR and 3' ITR sequences are adeno-associated virus (AAV) sequences. In certain embodiments, the AAV is an AAV2, AAV5, AAV8 or AAV9.

In another aspect, the application provides an expression vector comprising any of the nucleic acid molecules provided herein. In certain embodiments, the expression vector is a viral vector. In certain embodiments, the viral vector is an AAV vector.

In another aspect, the application provides a viral particle comprising any of the nucleic acid molecules or any of the expression vectors provided herein. In certain embodiments, the viral particle comprises capsid proteins of an AAV. In certain embodiments, the AAV is an AAV5, AAV8 or AAV9.

In another aspect, the application provides a host cell comprising any of the nucleic acid molecules or any of the expression vectors provided herein.

In another aspect, the application provides a pharmaceutical composition comprising any of the nucleic acid molecules, any of the expression vectors, or any of the viral particles described herein, and one or more pharmaceutically acceptable excipients.

In another aspect, the application provides a method for increasing expression of functional ATP7B protein in a subject comprising administering to said subject any of the nucleic acid molecules, any of the expression vectors, any of the viral particles, or any of the pharmaceutical compositions described herein.

In another aspect, the application provides, a method for treating a disorder associated with an ATP7B deficiency comprising administering to a subject in need thereof a therapeutically effective amount of any of the nucleic acid molecules, any of the expression vectors, any of the viral particles, or any of the pharmaceutical compositions described herein.

In another aspect, the application provides a method for treating Wilson's disease comprising administering to a subject in need thereof a therapeutically effective amount of any of the nucleic acid molecules, any of the expression vectors, any of the viral particles, or any of the pharmaceutical compositions described herein.

In another aspect, the application provides any of the nucleic acid molecules, any of the expression vectors, any of the viral particles, or any of the pharmaceutical compositions described herein for use in increasing the expression of functional ATP7B in a subject.

In another aspect, the application provides any of the nucleic acid molecules, any of the expression vectors, any of the viral particles, or any of the pharmaceutical compositions described herein for use in treating a disorder associated with an ATP7B deficiency.

In another aspect, the application provides any of the nucleic acid molecules, any of the expression vectors, any of the viral particles, or any of the pharmaceutical compositions described herein for use in treating Wilson's disease.

In another aspect, the application provides a process of producing a viral particle as provided herein, comprising: (i) culturing a host cell provided herein in a culture medium, and (ii) harvesting the viral particles from the cell culture supernatant or the host cells. In certain embodiments, the host cell further comprises (a) a nucleic acid molecule encoding AAV rep and/or cap genes, and/or (b) a nucleic acid molecule comprising viral helper genes.

In another aspect, the application provides use of any of the nucleic acid molecules or any of the expression vectors described herein for the production of viral particles.

In another aspect, the application provides a nucleic acid molecule comprising: (i) a regulatory element comprising a sequence having at least 80% sequence identity to any one of SEQ ID NOs: 66-68, and (ii) a nucleotide sequence encoding a therapeutic transgene, wherein the therapeutic transgene is operably linked to the regulatory element.

In certain embodiments, the regulatory element comprises a sequence having at least 85% sequence identity to any one of SEQ ID NOs: 66-68. In certain embodiments, the regulatory element comprises a sequence having at least 90% sequence identity to any one of SEQ ID NOs: 66-68. In certain embodiments, the regulatory element comprises a sequence having at least 95% sequence identity to any one of SEQ ID NOs: 66-68. In certain embodiments, the regulatory element comprises a sequence having any one of SEQ ID NOs: 66-68. In certain embodiments, the regulatory element comprises SEQ ID NO: 67.

In certain embodiments, the therapeutic transgene encodes any one of ATP7A, ATP7B, ATP8B1, ABCB4, ABCB11, CDKL5, CNTNAP2, ZEB2, Factor V, Factor VII, Factor VIII, Factor IX, Factor X, Factor XI or Factor XII, or a variant or functional fragment thereof. In certain embodiments, the therapeutic transgene encodes Factor VIII or a variant or functional fragment thereof. In certain embodiments, the therapeutic transgene encodes ATP7B or a variant or functional fragment thereof. In certain embodiments, the therapeutic transgene comprises a variant nucleotide sequence has been codon optimized for expression in the liver. In certain embodiments, the therapeutic transgene comprises a variant nucleotide sequence that has been optimized for expression from an AAV vector. In certain embodiments, the therapeutic transgene comprises a variant nucleotide sequence having least 80% sequence identity to any one of SEQ ID NOs: 3-18. In certain embodiments, the therapeutic transgene comprises a variant nucleotide sequence having at least 85% sequence identity to any one of SEQ ID NOs: 3-18. In certain embodiments, the therapeutic transgene comprises a variant nucleotide sequence having at least 90% sequence identity to any one of SEQ ID NOs: 3-18. In certain embodiments, the therapeutic transgene comprises a variant nucleotide sequence having at least 95% sequence identity to any one of SEQ ID NOs: 3-18. In certain embodiments, the therapeutic transgene comprises any one of SEQ ID NOs: 3-18. In certain embodiments, the therapeutic transgene comprises SEQ ID NO: 5.

In certain embodiments, the regulatory element is a promoter sequence. In certain embodiments, the nucleic acid molecule further comprises an enhancer sequence.

In certain embodiments, the regulatory element has less than 150 bp. In certain embodiments, the regulatory element has less than 120 bp. In certain embodiments, the regulatory element has less than 105 bp.

In certain embodiments, the regulatory element produces at least 10-fold greater expression in a mammalian cell relative to the CMV promoter. In certain embodiments, the regulatory element produces at least 50-fold greater expression in a mammalian cell relative to the CMV promoter.

In certain embodiments, the therapeutic transgene encodes a functional fragment of ATP7B. In certain embodiments, the therapeutic transgene encodes an ATP7B protein having SEQ ID NO: 2.

In certain embodiments, the level of expression of the functional ATP7B protein from the variant nucleotide sequence in the liver is at least 5-fold greater relative to the level of expression of ATP7B protein in the liver from a nucleic acid molecule comprising SEQ ID NO: 1. In certain embodiments, the level of expression of the functional ATP7B protein from the variant nucleotide sequence in the liver is at least 7-fold greater relative to the level of expression of ATP7B protein in the liver from a nucleic acid molecule comprising SEQ ID NO: 1.

In certain embodiments, the nucleic acid molecule further comprising a 5' ITR and 3' ITR sequences of a virus. In certain embodiments, the 5' ITR and 3' ITR sequences are adeno-associated virus (AAV) sequences. In certain embodiments, the 5' ITR and 3' ITR AAV sequences are AAV2, AAV5, AAV8 or AAV9 sequences.

In another aspect, the application provides an expression vector comprising any of the nucleic acid molecules described herein that comprise (i) a regulatory element comprising a sequence having at least 80% sequence identity to any one of SEQ ID NOs: 66-68, and (ii) a nucleotide sequence encoding a therapeutic transgene, wherein the therapeutic transgene is operably linked to the regulatory element. In certain embodiments, the expression vector is a viral vector. In certain embodiments, the viral vector is an AAV vector.

In another aspect, the application provides a viral particle comprising any of the nucleic acid molecules or the expression vectors described herein that comprise (i) a regulatory element comprising a sequence having at least 80% sequence identity to any one of SEQ ID NOs: 66-68, and (ii) a nucleotide sequence encoding a therapeutic transgene, wherein the therapeutic transgene is operably linked to the regulatory element. In certain embodiments, the viral particle comprises capsid proteins of an AAV. In certain embodiments, the viral particle comprises capsid proteins of an AAV5, AAV8 or AAV9.

In another aspect, the application provides a host cell comprising any of the nucleic acid molecule described herein that comprise (i) a regulatory element comprising a sequence having at least 80% sequence identity to any one of SEQ ID NOs: 66-68, and (ii) a nucleotide sequence encoding a therapeutic transgene, wherein the therapeutic transgene is operably linked to the regulatory element.

In another aspect, the application provides a pharmaceutical composition comprising a nucleic acid molecule, expression vector, or the viral particle that comprises (i) a regulatory element comprising a sequence having at least 80% sequence identity to any one of SEQ ID NOs: 66-68, and (ii) a nucleotide sequence encoding a therapeutic transgene, wherein the therapeutic transgene is operably linked to the regulatory element, and one or more pharmaceutically acceptable excipients.

In another aspect, the application provides a method for increasing expression of functional ATP7B protein in a subject comprising administering to said subject a nucleic acid molecule, expression vector, viral particle, or the pharmaceutical composition that comprises (i) a regulatory element comprising a sequence having at least 80% sequence identity to any one of SEQ ID NOs: 66-68, and (ii) a nucleotide sequence encoding a therapeutic transgene, wherein the therapeutic transgene is operably linked to the regulatory element.

In another aspect, the application provides a method for treating a disorder associated with an ATP7B deficiency comprising administering to a subject in need thereof a therapeutically effective amount of a nucleic acid molecule, expression vector, viral particle, or the pharmaceutical composition that comprises (i) a regulatory element comprising a sequence having at least 80% sequence identity to any one of SEQ ID NOs: 66-68, and (ii) a nucleotide sequence encoding a therapeutic transgene, wherein the therapeutic transgene is operably linked to the regulatory element.

In another aspect, the application provides a method for treating Wilson's disease comprising administering to a subject in need thereof a therapeutically effective amount of a nucleic acid molecule, expression vector, viral particle, or the pharmaceutical composition that comprises (i) a regulatory element comprising a sequence having at least 80% sequence identity to any one of SEQ ID NOs: 66-68, and (ii) a nucleotide sequence encoding a therapeutic transgene, wherein the therapeutic transgene is operably linked to the regulatory element.

In another aspect the application provides a nucleic acid molecule, expression vector, viral particle, or the pharmaceutical composition that comprises (i) a regulatory element comprising a sequence having at least 80% sequence identity to any one of SEQ ID NOs: 66-68, and (ii) a nucleotide sequence encoding a therapeutic transgene, wherein the therapeutic transgene is operably linked to the regulatory element, for use in increasing the expression of functional ATP7B in a subject.

In another aspect, the application provides a nucleic acid molecule, expression vector, viral particle, or the pharmaceutical composition that comprises (i) a regulatory element comprising a sequence having at least 80% sequence identity to any one of SEQ ID NOs: 66-68, and (ii) a nucleotide sequence encoding a therapeutic transgene, wherein the therapeutic transgene is operably linked to the regulatory element, for use in increasing treating a disorder associated with an ATP7B deficiency.

In another aspect, the application provides a nucleic acid molecule, expression vector, viral particle, or the pharmaceutical composition that comprises (i) a regulatory element comprising a sequence having at least 80% sequence identity to any one of SEQ ID NOs: 66-68, and (ii) a nucleotide sequence encoding a therapeutic transgene, wherein the therapeutic transgene is operably linked to the regulatory element, for use in treating Wilson's disease.

In another aspect, the application provides a process of producing a viral particle comprising (i) culturing a host cell in a culture medium, wherein the host cell comprises an expression vector comprising (a) a regulatory element comprising a sequence having at least 80% sequence identity to any one of SEQ ID NOs: 66-68, and (b) a nucleotide sequence encoding a therapeutic transgene, wherein the therapeutic transgene is operably linked to the regulatory element, and (ii) harvesting the viral particles from the cell culture supernatant or the host cells. In certain embodiments, the host cell further comprises (a) a nucleic acid molecule encoding AAV rep and/or cap genes, and/or (b) a nucleic acid molecule comprising viral helper genes.

In another aspect, the application provides use of a nucleic acid molecule or expression vector that comprises (i) a regulatory element comprising a sequence having at least 80% sequence identity to any one of SEQ ID NOs: 66-68, and (ii) a nucleotide sequence encoding a therapeutic transgene, wherein the therapeutic transgene is operably linked to the regulatory element, for the production of viral particles.

In another aspect, the application provides a nucleic acid molecule comprising: (i) a regulatory element comprising a sequence having at least 80% sequence identity to SEQ ID NO: 24, and (ii) a variant nucleotide sequence having at least 80% sequence identity to SEQ ID NO: 5, wherein said variant nucleotide sequence encodes a functional ATP7B protein. In certain embodiments, the regulatory element comprises a sequence having at least 85% sequence identity to SEQ ID NO: 24. In certain embodiments, the regulatory element comprises a sequence having at least 90% sequence identity to SEQ ID NO: 24. In certain embodiments, the regulatory element comprises a sequence having at least 95% sequence identity to SEQ ID NOs: 24. In certain embodiments, wherein the regulatory element comprises a sequence having SEQ ID NO: 24. In certain embodiments, the variant nucleotide sequence comprises a sequence having at least 85% sequence identity to SEQ ID NO: 5. In certain embodiments, the variant nucleotide sequence comprises a sequence having at least 90% sequence identity to SEQ ID NO: 5. In certain embodiments, the variant nucleotide sequence comprises a sequence having at least 95% sequence identity to SEQ ID NOs: 5. In certain embodiments, the variant nucleotide sequence comprises a sequence having SEQ ID NO: 5.

In another aspect, the application provides a nucleic acid molecule comprising: (i) a regulatory element comprising a sequence having at least 80% sequence identity to SEQ ID NO: 67, and (ii) a variant nucleotide sequence having at least 80% sequence identity to SEQ ID NO: 5, wherein said variant nucleotide sequence encodes a functional ATP7B protein. In certain embodiments, the regulatory element comprises a sequence having at least 85% sequence identity to SEQ ID NO: 67. In certain embodiments, the regulatory element comprises a sequence having at least 90% sequence identity to SEQ ID NO: 67. In certain embodiments, the regulatory element comprises a sequence having at least 95% sequence identity to SEQ ID NOs: 67. In certain embodiments, the regulatory element comprises a sequence having SEQ ID NO: 67. In certain embodiments, the variant nucleotide sequence comprises a sequence having at least 85% sequence identity to SEQ ID NO: 5. In certain embodiments, the variant nucleotide sequence comprises a sequence having at least 90% sequence identity to SEQ ID NO: 5. In certain embodiments, the variant nucleotide sequence comprises a sequence having at least 95% sequence identity to SEQ ID NOs: 5. In certain embodiments, the variant nucleotide sequence comprises a sequence having SEQ ID NO: 5.

In certain embodiments, the variant nucleotide sequence encodes a functional fragment of ATP7B. In certain embodiments, the variant nucleotide sequence encodes an ATP7B protein having SEQ ID NO: 2.

In certain embodiments, the nucleic acid molecule further comprises a 5' ITR and 3' ITR sequences of a virus. In certain embodiments, the 5' ITR and 3' ITR sequences are adeno-associated virus (AAV) sequences. In certain embodiments, the 5' ITR and 3' ITR sequences are AAV2, AAV5, AAV8 or AAV9 sequences.

In another aspect, the application provides an expression vector comprising a nucleic acid molecule comprising (i) a regulatory element comprising a sequence having at least 80% sequence identity to SEQ ID NO: 24 or SEQ ID NO: 67, and (ii) a variant nucleotide sequence having at least 80% sequence identity to SEQ ID NO: 5, wherein said variant nucleotide sequence encodes a functional ATP7B protein. In certain embodiments, the expression vector is a viral vector. In certain embodiments, the viral vector is an AAV vector.

In another aspect, the application provides a viral particle comprising a nucleic acid molecule or the expression vector comprising (i) a regulatory element comprising a sequence having at least 80% sequence identity to SEQ ID NO: 24 or SEQ ID NO: 67, and (ii) a variant nucleotide sequence having at least 80% sequence identity to SEQ ID NO: 5, wherein said variant nucleotide sequence encodes a functional ATP7B protein. In certain embodiments, the viral particle comprises capsid proteins of an AAV. In certain embodiments, the AAV is an AAV5, AAV8 or AAV9.

In another aspect, the application provides a host cell comprising a nucleic acid molecule or the expression vector comprising (i) a regulatory element comprising a sequence having at least 80% sequence identity to SEQ ID NO: 24 or SEQ ID NO: 67, and (ii) a variant nucleotide sequence having at least 80% sequence identity to SEQ ID NO: 5, wherein said variant nucleotide sequence encodes a functional ATP7B protein.

In another aspect, the application provides a pharmaceutical composition comprising a nucleic acid molecule, expression vector, or the viral particle comprising (i) a regulatory element comprising a sequence having at least 80% sequence identity to SEQ ID NO: 24 or SEQ ID NO: 67, and (ii) a variant nucleotide sequence having at least 80% sequence identity to SEQ ID NO: 5, wherein said variant nucleotide sequence encodes a functional ATP7B protein, and one or more pharmaceutically acceptable excipients.

In another aspect, the application provides a method for increasing expression of functional ATP7B protein in a subject comprising administering to said subject a nucleic acid molecule, expression vector, viral particle, or the pharmaceutical composition comprising (i) a regulatory element comprising a sequence having at least 80% sequence identity to SEQ ID NO: 24 or SEQ ID NO: 67, and (ii) a variant nucleotide sequence having at least 80% sequence identity to SEQ ID NO: 5, wherein said variant nucleotide sequence encodes a functional ATP7B protein.

In another aspect, the application provides a method for treating a disorder associated with an ATP7B deficiency comprising administering to a subject in need thereof a therapeutically effective amount of a nucleic acid molecule, expression vector, viral particle, or the pharmaceutical composition comprising (i) a regulatory element comprising a sequence having at least 80% sequence identity to SEQ ID NO: 24 or SEQ ID NO: 67, and (ii) a variant nucleotide sequence having at least 80% sequence identity to SEQ ID NO: 5, wherein said variant nucleotide sequence encodes a functional ATP7B protein.

In another aspect, the application provides a method for treating Wilson's disease comprising administering to a subject in need thereof a therapeutically effective amount of a nucleic acid molecule, expression vector, viral particle, or the pharmaceutical composition comprising (i) a regulatory element comprising a sequence having at least 80% sequence identity to SEQ ID NO: 24 or SEQ ID NO: 67, and (ii) a variant nucleotide sequence having at least 80% sequence identity to SEQ ID NO: 5, wherein said variant nucleotide sequence encodes a functional ATP7B protein.

In another aspect, the application provides a nucleic acid molecule, expression vector, viral particle, or the pharmaceutical composition comprising (i) a regulatory element comprising a sequence having at least 80% sequence identity to SEQ ID NO: 24 or SEQ ID NO: 67, and (ii) a variant nucleotide sequence having at least 80% sequence identity to SEQ ID NO: 5, wherein said variant nucleotide sequence encodes a functional ATP7B protein, for use in increasing the expression of functional ATP7B in a subject.

In another aspect, the application provides a nucleic acid molecule, expression vector, viral particle, or the pharmaceutical composition comprising (i) a regulatory element comprising a sequence having at least 80% sequence identity to SEQ ID NO: 24 or SEQ ID NO: 67, and (ii) a variant nucleotide sequence having at least 80% sequence identity to SEQ ID NO: 5, wherein said variant nucleotide sequence encodes a functional ATP7B protein, for use in increasing treating a disorder associated with an ATP7B deficiency.

In another aspect, the application provides a nucleic acid molecule, expression vector, viral particle, or the pharmaceutical composition comprising (i) a regulatory element comprising a sequence having at least 80% sequence identity to SEQ ID NO: 24 or SEQ ID NO: 67, and (ii) a variant nucleotide sequence having at least 80% sequence identity to SEQ ID NO: 5, wherein said variant nucleotide sequence encodes a functional ATP7B protein, for use in treating Wilson's disease.

In another aspect, the application provides a process of producing a viral particle comprising: (i) culturing a host cell in a culture medium, wherein the host cell comprises a nucleic acid molecule or expression vector comprising (a) a regulatory element comprising a sequence having at least 80% sequence identity to SEQ ID NO: 24 or SEQ ID NO: 67, and (b) a variant nucleotide sequence having at least 80% sequence identity to SEQ ID NO: 5, wherein said variant nucleotide sequence encodes a functional ATP7B protein, and (ii) harvesting the viral particles from the cell culture supernatant or the host cells. In certain embodiments, the host cell further comprises (a) a nucleic acid molecule encoding AAV rep and/or cap genes, and/or (b) a nucleic acid molecule comprising viral helper genes.

In another aspect, the application provides use of a nucleic acid molecule or expression vector comprising (i) a regulatory element comprising a sequence having at least 80% sequence identity to SEQ ID NO: 24 or SEQ ID NO: 67, and (ii) a variant nucleotide sequence having at least 80% sequence identity to SEQ ID NO: 5, wherein said variant nucleotide sequence encodes a functional ATP7B protein, for the production of viral particles.

### INCORPORATION BY REFERENCE

All publications, patents, and patent applications mentioned in this specification are herein incorporated by reference to the same extent as if each individual publication, patent, or patent application was specifically and individually indicated to be incorporated by reference. In the case of conflict, the specification, including definitions, will control.

### BRIEF DESCRIPTION OF THE DRAWINGS

The novel features of the invention are set forth with particularity in the appended claims. A better understanding of the features and advantages of the present invention will be obtained by reference to the following detailed description that sets forth illustrative embodiments, in which the principles of the invention are utilized, and the accompanying drawings of which:
**FIG. 1** shows a comparison of the expression levels of wild-type ATP7B (SEQ ID NO: 1) as compared to two codon optimized variants (variant 2 (SEQ ID NO: 4) and variant 3 (SEQ ID NO: 5)) in HEK293 cells.
**FIG. 2** shows a comparison of the expression levels of wild-type ATP7B (SEQ ID NO: 1) as compared to two codon optimized variants (variant 2 (SEQ ID NO: 4) and variant 3 (SEQ ID NO: 5)) in mouse liver.
**FIG. 3A** illustrates the normalized luciferase values of plasmids comprising different regulatory elements (e.g., SEQ ID NO: 19 and SEQ ID NO: 20), demonstrating the effect the regulatory elements had on expression of luciferase in HEK293T cells. For example, SEQ ID NO: 21 (comprising SEQ ID NO: 19 combined with a minimal CMV (minCMV) promoter) drove expression of luciferase at a level about 1.4 fold higher than the expression driven by the minCMV promoter alone, and about 60 fold higher than the expression driven by a SCP promoter.
**FIG. 3B** illustrates the size-normalized activity (calculated by dividing the normalized luciferase activity by the length of the regulatory element in base pairs) of each regulatory element (e.g., SEQ ID NO: 21 and SEQ ID NO: 22) as compared to SCP, minCMV, and CAG in HEK293T cells. SEQ ID NO: 22 (comprising SEQ ID NO: 20 linked to a minCMV promoter) resulted in size-normalized activity at a level about 3.5 fold higher than the minCMV promoter alone and about 140 fold higher than a SCP promoter.
**FIG. 3C** illustrates normalized luciferase expression of regulatory elements SEQ ID NO: 28, SEQ ID NO: 29, SEQ ID NO: 30, SEQ ID NO: 31, SEQ ID NO: 32, SEQ ID NO: 34, SEQ ID NO: 35, SEQ ID NO: 36, SEQ ID NO: 37, SEQ ID NO: 38 and SEQ ID NO: 39 as compared to negative controls and a positive control (SEQ ID NO: 22). All the regulatory elements resulted in high levels of normalized luciferase expression that were comparable to the level of expression drove by SEQ ID NO: 22.
**FIG. 3D** illustrates normalized luciferase expression of regulatory elements SEQ ID NO: 34, SEQ ID NO: 36, SEQ ID NO: 32, or SEQ ID NO: 33 as compared to a negative control, positive control (SEQ ID NO: 22), CMV+CMVe, and CMV alone in HEK293T cells. Each regulatory element drove higher luciferase expression than CMV alone and CMV+CMVe.
**FIG. 3E** illustrates normalized luciferase expression of regulatory elements SEQ ID NO: 28, SEQ ID NO: 29, SEQ ID NO: 40, SEQ ID NO: 41, SEQ ID NO: 42, and SEQ ID NO: 43 as compared to a negative control in HEK293T cells. Each regulatory element tested drove higher luciferase expression than the negative control.
**FIG. 4** illustrates the relative expression of ATP7B (Log2) in mice treated with an expression cassette comprising a regulatory element having a sequence of SEQ ID NO: 23 at doses of 1E10 (or 10¹⁰) genomic copies per mouse (gc/mouse), 1E11 (or 10¹¹) gc/mouse, or 1E12 (or 10¹²) gc/mouse. The data showed a positive correlation between the dose of the expression cassette comprising SEQ ID NO: 23 and the expression of ATP7B *in vivo.*
**FIG. 5** illustrates the relative expression of EGFP (Log10) in mice treated with an expression cassette comprising a regulatory element having a sequence of SEQ ID NO: 26, 27, 23, 25, 22, 33, or 24 and administered at a dose of 5E11 (or 5×10¹¹) gc/mouse. The data show that the regulatory elements resulted in elevated levels of EGFP expression, as measured by RNA transcripts, in the liver of the mice *in vivo.*
**FIG. 6** illustrates the percentage of hepatocytes expressing GFP in mice treated with an expression cassette comprising a regulatory element having a sequence of SEQ ID NO: 26, 27, 23, 25, 22, 33, or 24 and administered at a dose of 5E11 (or 5×10¹¹) gc/mouse. The data show that many of the regulatory elements tested were expressed in a high percentage of hepatocytes in mice, with SEQ ID NO: 24 being expressed in greater than 80% of hepatocytes.

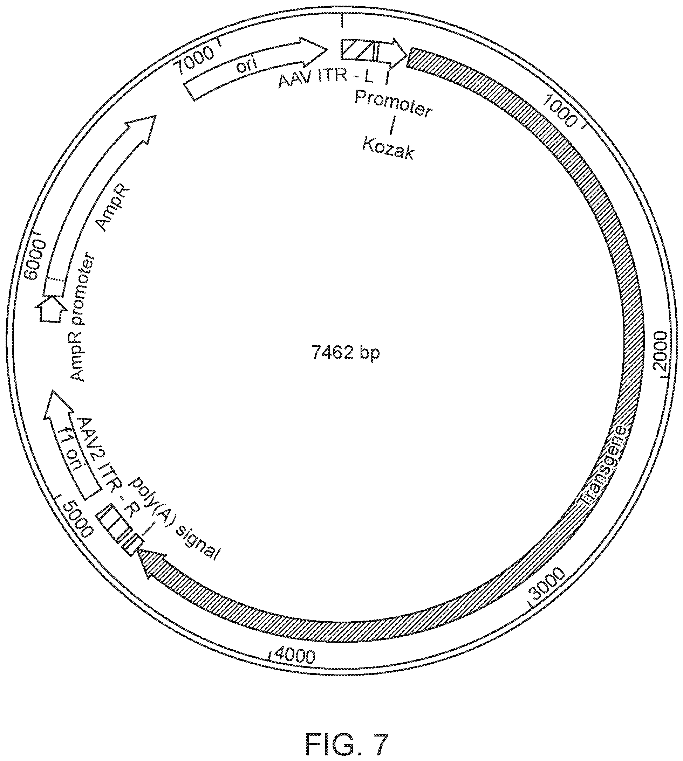

**FIG. 7** illustrates an exemplary expression cassette, vector, or plasmid of this disclosure, comprising AAV ITR-L and ITR-R and one or more relatively short human-derived regulatory elements of this disclosure (e.g., SEQ ID NOs: 23-43) operably linked to a large transgene, e.g., a codon optimized variant of ATP7B. Such rAAV vector can be used for a gene therapy treatment.
**FIG. 8** is a graph showing relative mRNA expression in the liver of mice treated with AAV8 virus or PBS control. AAV8 virus contained ATP7B variant 3 (SEQ ID NO: 5) under the control of several promoter elements at the labeled concentrations.
**FIGs. 9A** and **9B** are a Western Blot analysis showing relative protein expression in the liver of male (panel A) and female (panel B) wild-type and ATP7B KO mice treated with AAV8 virus or PBS control. AAV8 virus contained ATP7B variant 3 (SEQ ID NO: 5) under the control of the promoter having SEQ ID NO: 24.
**FIGs. 10A** and **10B** are graphs showing MSD-ELISA analyses showing relative protein expression in the liver of wild-type and ATP7B KO mice treated with AAV8 virus or PBS control in male (panel A) and female (panel B) mice. AAV8 virus contained ATP7B variant 3 (SEQ ID NO: 5) under the control of the promoter having SEQ ID NO: 24.
**FIG. 11** is a graph showing the vector copy number per diploid genome in wild-type and ATP7B KO mice treated with AAV8 virus or PBS control. AAV8 virus contained ATP7B variant 3 (SEQ ID NO: 5) under the control of the promoter having SEQ ID NO: 24.
**FIG. 12** is a graph showing alanine transaminase (ALT) activity in wild-type and ATP7B KO mice treated with AAV8 virus or PBS control. AAV8 virus contained ATP7B variant 3 (SEQ ID NO: 5) under the control of the promoter having SEQ ID NO: 24. Statistical tests were performed between ATP7B KO + PBS and ATP7B KO + virus groups (^{∗} = p<0.05, ^{∗∗∗∗} = p<1E-5).
**FIG. 13** is a graph showing aspartate aminotransferase (AST) activity in wild-type and ATP7B KO mice treated with AAV8 virus or PBS control. AAV8 virus contained ATP7B variant 3 (SEQ ID NO: 5) under the control of the promoter having SEQ ID NO: 24. Statistical tests were performed between ATP7B KO + PBS and ATP7B KO + virus groups (^{∗∗} = p<0.01, ^{∗∗∗} = p<0.001).
**FIGs. 14A-14C** are a series of graphs showing serum chemistry analysis of liver injury and metabolic function in WT and ATP7B KO mice treated with AAV8 virus or PBS control. (A) ALP, ALT, and AST; (B) ALB/Glob, albumin, conjugated bilirubin, and total bilirubin; and (C) cholesterol and glucose. AAV8 virus contained ATP7B variant 3 (SEQ ID NO: 5) under the control of the promoter having SEQ ID NO: 24.
**FIG. 15** is a graph showing TIMP1 protein levels in WT and ATP7B KO mice treated with AAV8 virus or PBS control. AAV8 virus contained ATP7B variant 3 (SEQ ID NO: 5) under the control of the promoter having SEQ ID NO: 24.
**FIG. 16** is a graph showing urine copper concentrations in wild-type and ATP7B KO mice 21 weeks after treatment with AAV8 virus or PBS control. AAV8 virus contained ATP7B variant 3 (SEQ ID NO: 5) under the control of the promoter having SEQ ID NO: 24.
**FIG. 17** is a graph showing terminal serum copper levels in WT and ATP7B KO mice treated with AAV8 virus or PBS control. AAV8 virus contained ATP7B variant 3 (SEQ ID NO: 5) under the control of the promoter having SEQ ID NO: 24.
**FIG. 18** is a graph showing brain copper levels (dry weight) in wild-type and ATP7B KO mice treated with AAV8 virus or PBS control. AAV8 virus contained ATP7B variant 3 (SEQ ID NO: 5) under the control of the promoter having SEQ ID NO: 24.
**FIG. 19** is a graph showing liver rhodamine staining in wild-type and ATP7B KO mice treated with AAV8 virus or PBS control. AAV8 virus contained ATP7B variant 3 (SEQ ID NO: 5) under the control of the promoter having SEQ ID NO: 24.
**FIGs. 20A-20C** are a series of graphs showing inflammation, fibrosis, and large cell change scores in wild-type and ATP7B KO mice treated with AAV8 virus or PBS control. (A): inflammation score; (B): fibrosis score, and (C): large cell change score. AAV8 virus contained ATP7B variant 3 (SEQ ID NO: 5) under the control of the promoter having SEQ ID NO: 24.
**FIGs. 21A** and **21B** show immune cell infiltration in wild-type and ATP7B KO mice treated with AAV8 virus or PBS control. AAV8 virus contained ATP7B variant 3 (SEQ ID NO: 5) under the control of the promoter having SEQ ID NO: 24. White arrows show immune cell infiltration ATP7B KO mice. Black arrows show collagen fiber as a result of fibrosis in liver tissue of PBS-treated ATP7B KO mice.
**FIG. 22** shows alpha-SMA staining in WT and ATP7B KO mice treated with AAV8 virus or PBS control. AAV8 virus contained ATP7B variant 3 (SEQ ID NO: 5) under the control of the promoter having SEQ ID NO: 24. White arrows show activation of hepatic stellate cells observed in PBS-treated ATP7B KO mice.
**FIG. 23A** (male) and **FIG. 23B** (female) are graphs showing differential gene expression of genes in WT and ATP7B KO mice treated with AAV8 virus or PBS control. AAV8 virus contained ATP7B variant 3 (SEQ ID NO: 5) under the control of the promoter having SEQ ID NO: 24.
**FIG. 24** is a graph showing ATP7B expression levels in mice treated with different concentrations of AAV5, AAV8, or PBS control. AAV5 and AAV8 viruses contained ATP7B variant 3 (SEQ ID NO: 5) under the control of the promoter having SEQ ID NO: 67.
**FIG. 25** is a graph showing the vector copy number per diploid genome in wild-type mice treated with different concentrations of AAV5 or AAV8 virus, or PBS control. AAV5 and AAV8 viruses contained ATP7B variant 3 (SEQ ID NO: 5) under the control of the promoter having SEQ ID NO: 67.
**FIG. 26** is a graph showing ATP7B protein concentration in wild-type mice treated with different concentrations of AAV5 or AAV8 virus, or PBS control. AAV5 and AAV8 viruses contained ATP7B variant 3 (SEQ ID NO: 5) under the control of the promoter having SEQ ID NO: 67.

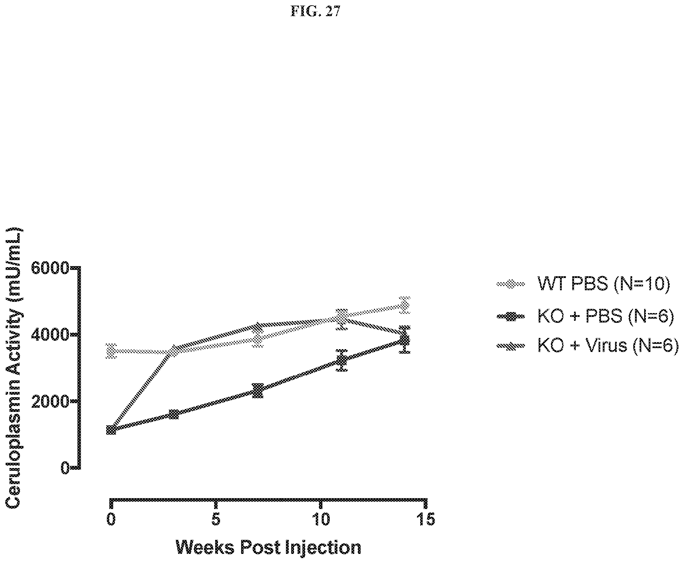

**FIG. 27** is a graph showing ceruloplasmin activity in wild-type and ATP7B KO mice treated with AAV8 virus or PBS control. AAV8 virus contained ATP7B variant 3 (SEQ ID NO: 5) under the control of the promoter having SEQ ID NO: 67.
**FIG. 28** is a graph showing ceruloplasmin activity in wild-type and ATP7B KO mice treated with AAV5 virus or PBS control. AAV5 virus contained ATP7B variant 3 (SEQ ID NO: 5) under the control of the promoter having SEQ ID NO: 67 or SEQ ID NO: 24, as labeled.
**FIG. 29** is a graph showing alanine transaminase (ALT) activity in wild-type and ATP7B KO mice treated with AAV8 virus or PBS control. AAV8 virus contained ATP7B variant 3 (SEQ ID NO: 5) under the control of the promoter having SEQ ID NO: 67.
**FIG. 30** is a graph showing aspartate aminotransferase (AST) activity in wild-type and ATP7B KO mice treated with AAV8 virus or PBS control. AAV8 virus contained ATP7B variant 3 (SEQ ID NO: 5) under the control of the promoter having SEQ ID NO: 67.
**FIG. 31** is a graph showing alanine transaminase (ALT) activity in wild-type and ATP7B KO mice treated with AAV5 virus or PBS control. AAV5 virus contained ATP7B variant 3 (SEQ ID NO: 5) under the control of the promoter having SEQ ID NO: 67 or SEQ ID NO: 24, as labeled.
**FIG. 32** is a graph showing aspartate aminotransferase (AST) activity in wild-type and ATP7B KO mice treated with AAV5 virus or PBS control. AAV5 virus contained ATP7B variant 3 (SEQ ID NO: 5) under the control of the promoter having SEQ ID NO: 67 or SEQ ID NO: 24, as labeled.
**FIG. 33** is a graph showing alanine transaminase (ALT) activity in wild-type and ATP7B KO mice treated with different concentrations of AAV5 virus, or PBS control. AAV5 virus contained ATP7B variant 3 (SEQ ID NO: 5) under the control of the promoter having SEQ ID NO: 67.
**FIG. 34** is a graph showing aspartate aminotransferase (AST) activity in wild-type and ATP7B KO mice treated with different concentrations of AAV5 virus, or PBS control. AAV5 virus contained ATP7B variant 3 (SEQ ID NO: 5) under the control of the promoter having SEQ ID NO: 67.
**FIG. 35** is a graph showing TIMP1 protein levels in WT and ATP7B KO mice treated with AAV8 virus or PBS control. AAV8 virus contained ATP7B variant 3 (SEQ ID NO: 5) under the control of the promoter having SEQ ID NO: 67.
**FIG. 36** is a graph showing urine copper levels in WT and ATP7B KO mice treated with AAV8 virus or PBS control. Urine samples were collected three weeks after virus was injected. AAV8 virus contained ATP7B variant 3 (SEQ ID NO: 5) under the control of the promoter having SEQ ID NO: 67.
**FIG. 37** is a graph showing urine copper levels in WT and ATP7B KO mice treated with AAV5 virus or PBS control. Urine samples were collected sixteen weeks after virus was injected. AAV5 virus contained ATP7B variant 3 (SEQ ID NO: 5) under the control of the promoter having SEQ ID NO: 67 or SEQ ID NO: 24, as labeled.
**FIG. 38** is a graph showing urine copper levels in WT and ATP7B KO mice treated with different concentrations of AAV5 virus or PBS control. Urine samples were collected four weeks after virus was injected. AAV5 virus contained ATP7B variant 3 (SEQ ID NO: 5) under the control of the promoter having SEQ ID NO: 67.
**FIG. 39** is a graph showing dose-dependent ATP7B expression in wild-type and ATP7B KO mice treated with different concentrations of AAV5 virus or PBS control. RNA was measured eight weeks after virus was injected. AAV5 virus contained ATP7B variant 3 (SEQ ID NO: 5) under the control of the promoter having SEQ ID NO: 67.
**FIG. 40** is a graph showing ATP7B protein concentration in wild-type and ATP7B KO mice treated with AAV5 virus or PBS control. AAV5 virus contained ATP7B variant 3 (SEQ ID NO: 5) under the control of the promoter having SEQ ID NO: 67 or SEQ ID NO: 24, as labeled.
**FIG. 41** is a graph showing ATP7B protein concentration in wild-type and ATP7B KO mice treated with different concentrations of AAV5 virus, or PBS control. Protein was measured four or eight weeks after virus was injected. AAV5 virus contained ATP7B variant 3 (SEQ ID NO: 5) under the control of the promoter having SEQ ID NO: 67.
**FIGs. 42A** and **42B** are a Western Blot analysis showing relative protein expression in the liver of female (panel A) and male (panel B) wild-type and ATP7B KO mice treated with AAV5 virus or PBS control. AAV5 virus contained ATP7B variant 3 (SEQ ID NO: 5) under the control of the promoter having SEQ ID NO: 67 or SEQ ID NO: 24, as labeled.

### DETAILED DESCRIPTION OF THE DISCLOSURE

Provided herein are ATP7B variant nucleotide sequences, vectors comprising such sequences, and uses thereof for treatment of a variety of diseases and disorders, particularly Wilson's disease.

### Definitions

As used herein, the singular forms "a", "an" and "the" are intended to include the plural forms as well, unless the context clearly indicates otherwise. Furthermore, to the extent that the terms "including", "includes", "having", "has", "with", or variants thereof are used in either the detailed description and/or the claims, such terms are intended to be inclusive in a manner similar to the term "comprising". In any of the embodiments described herein, "comprising" may be replaced with "consisting essentially of" or "consisting of."

The term "AAV" is an abbreviation for adeno-associated virus, and may be used to refer to the virus itself or a derivative thereof. The term covers all serotypes, subtypes, and both naturally occurring and recombinant forms, except where required otherwise. The abbreviation "rAAV" refers to recombinant adeno-associated virus. The term "AAV" includes AAV1, AAV2, AAV3, AAV4, AAV5, AAV6, AAV7, AAV8, AAV9, AAV10, AAV11, AAV12, rh10, and hybrids thereof, avian AAV, bovine AAV, canine AAV, equine AAV, primate AAV, non-primate AAV, and ovine AAV. The genomic sequences of various serotypes of AAV, as well as the sequences of the native terminal repeats (TRs), Rep proteins, and capsid subunits are known in the art. Such sequences may be found in the literature or in public databases such as GenBank. A "rAAV vector" as used herein refers to an AAV vector comprising a polynucleotide sequence not of AAV origin (i.e., a polynucleotide heterologous to AAV), typically a sequence of interest for the genetic transformation of a cell. In general, the heterologous polynucleotide is flanked by at least one, and generally by two, AAV inverted terminal repeat sequences (ITRs). An rAAV vector may either be single-stranded (ssAAV) or self-complementary (scAAV). An "AAV virus" or "AAV viral particle" refers to a viral particle composed of at least one AAV capsid protein and an encapsidated polynucleotide rAAV vector. If the particle comprises a heterologous polynucleotide (i.e., a polynucleotide other than a wild-type AAV genome such as a transgene to be delivered to a mammalian cell), it is typically referred to as an "rAAV viral particle" or simply an "rAAV particle".

The term "about" or "approximately" means within an acceptable error range for the particular value as determined by one of ordinary skill in the art, which will depend in part on how the value is measured or determined, i.e., the limitations of the measurement system. For example, "about" can mean within one or more than one standard deviation, per the practice in the art. Alternatively, "about" can mean a range of up to 20%, up to 15%, up to 10%, up to 5%, or up to 1%) of a given value.

The terms "determining", "measuring", "evaluating", "assessing", "assaying", "analyzing", and their grammatical equivalents can be used interchangeably herein to refer to any form of measurement, and include determining if an element is present or not (for example, detection). These terms can include both quantitative and/or qualitative determinations. Assessing may be relative or absolute.

The term "expression" refers to the process by which a nucleic acid sequence or a polynucleotide is transcribed from a DNA template (such as into mRNA or other RNA transcript) and/or the process by which a transcribed mRNA is subsequently translated into peptides, polypeptides, or proteins. Transcripts and encoded polypeptides may be collectively referred to as "gene product." If the polynucleotide is derived from genomic DNA, expression may include splicing of the mRNA in a eukaryotic cell.

An "expression cassette" refers to a nucleic molecule comprising one or more regulatory elements operably linked to a coding sequence (e.g., a gene or genes) for expression.

The term "effective amount" or "therapeutically effective amount" refers to that amount of a composition described herein that is sufficient to affect the intended application, including but not limited to disease treatment, as defined below. The therapeutically effective amount may vary depending upon the intended treatment application (in a cell or *in vivo*), or the subject and disease condition being treated, e.g., the weight and age of the subject, the severity of the disease condition, the manner of administration and the like, which can readily be determined by one of ordinary skill in the art. The term also applies to a dose that will induce a particular response in a target cell. The specific dose will vary depending on the particular composition chosen, the dosing regimen to be followed, whether it is administered in combination with other compounds, timing of administration, the tissue to which it is administered, and the physical delivery system in which it is carried.

A "fragment" of a nucleotide or peptide sequence is meant to refer to a sequence that is less than that believed to be the "full-length" sequence.

A "functional fragment" of a DNA or protein sequence refers to a biologically active fragment of the sequence that is shorter than the full-length or reference DNA or protein sequence, but which retains at least one biological activity (either functional or structural) that is substantially similar to a biological activity of the full-length or reference DNA or protein sequence.

The terms "host cell," "host cell line," and "host cell culture" are used interchangeably and refer to cells into which exogenous nucleic acid has been introduced, including the progeny of such cells. Host cells include "transformants" and "transformed cells," which include the primary transformed cell and progeny derived therefrom without regard to the number of passages. Progeny may not be completely identical in nucleic acid content to a parent cell, but may contain mutations. Mutant progeny that have the same function or biological activity as screened or selected for in the originally transformed cell are included herein.

The term "*in vitro*" refers to an event that takes places outside of a subject's body. For example, an *in vitro* assay encompasses any assay run outside of a subject. *In vitro* assays encompass cell-based assays in which cells alive or dead are employed. *In vitro* assays also encompass a cell-free assay in which no intact cells are employed.

The term "*in vivo*" refers to an event that takes place in a subject's body.

An "isolated" nucleic acid refers to a nucleic acid molecule that has been separated from a component of its natural environment. An isolated nucleic acid includes a nucleic acid molecule contained in cells that ordinarily contain the nucleic acid molecule, but the nucleic acid molecule is present extrachromosomally, at a chromosomal location that is different from its natural chromosomal location, or contains only coding sequences.

As used herein, "operably linked", "operable linkage", "operatively linked", or grammatical equivalents thereof refer to juxtaposition of genetic elements, e.g., a promoter, an enhancer, a polyadenylation sequence, etc., wherein the elements are in a relationship permitting them to operate in the expected manner. For instance, a regulatory element, which can comprise promoter and/or enhancer sequences, is operatively linked to a coding region if the regulatory element helps initiate transcription of the coding sequence. There may be intervening residues between the regulatory element and coding region so long as this functional relationship is maintained.

A "pharmaceutically acceptable carrier" refers to an ingredient in a pharmaceutical formulation or composition, other than an active ingredient, which is nontoxic to a subject. A pharmaceutically acceptable carrier includes, but is not limited to, a buffer, excipient, stabilizer, or preservative.

The terms "pharmaceutical formulation" or "pharmaceutical composition" refer to a preparation which is in such form as to permit the biological activity of an active ingredient contained therein to be effective, and which contains no additional components which are unacceptably toxic to a subject to which the formulation would be administered.

In general, "sequence identity" or "sequence homology", which can be used interchangeably, refer to an exact nucleotide-to-nucleotide or amino acid-to-amino acid correspondence of two polynucleotides or polypeptide sequences, respectively. Two or more sequences (polynucleotide or amino acid) can be compared by determining their "percent identity", also referred to as "percent homology". The percent identity to a reference sequence (e.g., nucleic acid or amino acid sequence) may be calculated as the number of exact matches between two optimally aligned sequences divided by the length of the reference sequence and multiplied by 100. Conservative substitutions are not considered as matches when determining the number of matches for sequence identity. It will be appreciated that where the length of a first sequence (A) is not equal to the length of a second sequence (B), the percent identity of A:B sequence will be different than the percent identity of B:A sequence. Sequence alignments, such as for the purpose of assessing percent identity, may be performed by any suitable alignment algorithm or program, including but not limited to the Needleman-Wunsch algorithm (see, e.g., the EMBOSS Needle aligner available on the world wide web at ebi.ac.uk/Tools/psa/emboss needle/), the BLAST algorithm (see, e.g., the BLAST alignment tool available on the world wide web at blast.ncbi.nlm.nih.gov/Blast.cgi), the Smith-Waterman algorithm (see, e.g., the EMBOSS Water aligner available on the world wide web at ebi.ac.uk/Tools/psa/emboss_water/), and Clustal Omega alignment program (see e.g., the world wide web at clustal.org/omega/ and F. Sievers et al., Mol Sys Biol. 7: 539 (2011)). Optimal alignment may be assessed using any suitable parameters of a chosen algorithm, including default parameters. The BLAST program is based on the alignment method of Karlin and Altschul, Proc. Natl. Acad. Sci. USA 87:2264-2268 (1990) and as discussed in Altschul, et al., J. Mol. Biol. 215:403-410 (1990); Karlin and Altschul, Proc. Natl. Acad. Sci. USA 90:5873-5877 (1993); and Altschul et al., Nucleic Acids Res. 25:3389-3402 (1997).

The terms "subject" and "individual" are used interchangeably herein to refer to a vertebrate, preferably a mammal, more preferably a human. The methods described herein can be useful in human therapeutics, veterinary applications, and/or preclinical studies in animal models of a disease or condition.

As used herein, the terms "treat", "treatment", "therapy" and the like refer to obtaining a desired pharmacologic and/or physiologic effect, including, but not limited to, alleviating, delaying or slowing progression, reducing effects or symptoms, preventing onset, preventing reoccurrence, inhibiting, ameliorating onset of a diseases or disorder, obtaining a beneficial or desired result with respect to a disease, disorder, or medical condition, such as a therapeutic benefit and/or a prophylactic benefit. "Treatment," as used herein, covers any treatment of a disease in a mammal, particularly in a human, and includes: (a) preventing the disease from occurring in a subject which may be predisposed to the disease or at risk of acquiring the disease but has not yet been diagnosed as having it; (b) inhibiting the disease, i.e., arresting its development; and (c) relieving the disease, i.e., causing regression of the disease. A therapeutic benefit includes eradication or amelioration of the underlying disorder being treated. Also, a therapeutic benefit is achieved with the eradication or amelioration of one or more of the physiological symptoms associated with the underlying disorder such that an improvement is observed in the subject, notwithstanding that the subject may still be afflicted with the underlying disorder. In some cases, for prophylactic benefit, the compositions are administered to a subject at risk of developing a particular disease, or to a subject reporting one or more of the physiological symptoms of a disease, even though a diagnosis of this disease may not have been made. The methods of the present disclosure may be used with any mammal. In some cases, the treatment can result in a decrease or cessation of symptoms. A prophylactic effect includes delaying or eliminating the appearance of a disease or condition, delaying or eliminating the onset of symptoms of a disease or condition, slowing, halting, or reversing the progression of a disease or condition, or any combination thereof.

A "variant" of a nucleotide sequence refers to a sequence having a genetic alteration or a mutation as compared to the most common wild-type DNA sequence (e.g., cDNA or a sequence referenced by its GenBank accession number) or a specified reference sequence.

A "vector" as used herein refers to a nucleic acid molecule that can be used to mediate delivery of another nucleic acid molecule to which it is linked into a cell where it can be replicated or expressed. The term includes the vector as a self-replicating nucleic acid structure as well as the vector incorporated into the genome of a host cell into which it has been introduced. Certain vectors are capable of directing the expression of nucleic acids to which they are operatively linked. Such vectors are referred to herein as "expression vectors." Other examples of vectors include plasmids and viral vectors.

Unless otherwise indicated, all terms used herein have the same meaning as they would to one skilled in the art and the practice of the present invention will employ conventional techniques of molecular biology, microbiology, and recombinant DNA technology, which are within the knowledge of those of skill of the art.

### ATP7B Variants

Copper-transporting ATPase 2 or ATPase copper transporting beta (ATP7B) is a P-type cation transport ATPase that functions by exporting copper out of the cells. ATP7B is responsible for transporting copper from intracellular chaperone proteins into the secretory pathway, both for excretion into bile and for incorporation into apo-ceruloplasmin for the synthesis of functional ceruloplasmin. ATP7B-mediated transport of copper involves binding copper via its cytosolic N-terminal domain and ATP via the nucleotide-binding domain. ATP is then hydrolyzed and ATP7B becomes transiently phosphorylated at residue D1027 located in the P-domain. Subsequent dephosphorylation releases energy necessary to transport copper across the membrane. The gene that encodes the human enzyme is located at chromosome 13 (chromosome location 13ql4.3; gene name ATP7B). Five isoforms produced by alternative splicing have been described for ATP7B. Isoform 1 (1465 amino acids long) is the longest isoform and is referred to herein as the canonical or wild-type sequence (NCBI Reference Sequence: NP_000044.2; SEQ ID NO: 1 is the wild-type nucleotide sequence and SEQ ID NO: 2 is the wild-type amino acid sequence as used herein). The four additional isoforms are: NCBI Reference Sequences NP_001005918.1, NP_001230111.1, NP_001317507.1, NP_001317508.1.

ATP7B has eight transmembrane domains that form a path through cell membranes for copper translocation. Several conserved motifs are present in ATP7B that are characteristic for the P-type ATPase protein family. These motifs are required for ATP catalysis and include the nucleotide binding domain (N-domain) containing the ATP-binding site, the phosphorylation domain (P-domain) containing the conserved aspartic acid residue, and the actuator domain (A-domain) comprising the phosphatase domain. Highly conserved signature residues are present in these motifs; SEHPL in the N-domain, DKTG in the P-domain, and TGE in the A-domain. The protein also contains the intramembrane CPC motif that is required for copper translocation through the membrane. ATP7B also has a large N-terminus with six metal-binding domains (MBDs) (also called metal-binding sites (MBS) or heavy metal associated (HMA) sites), each comprising approximately 70 amino acids and the highly conserved metal-binding motif GMxCxxC (where x is any amino acid). These MBDs bind Cu(I) in a stoichiometry of one atom of Cu(I) per MBD. These amino-terminal MBDs of ATP7B are associated with several aspects of its function, including copper translocation, incorporation of copper in cuproenzymes, ATPase activity, localization and trafficking, and protein-protein interactions. The MDB sites are identified starting at the amino end, as domains MBD 1 (amino acids 59 - 125 in the wild-type sequence), MBD 2 (amino acids 144 - 210), MBD 3 (258 - 327), MBD 4 (360 - 426), MBD 5 (489 - 555), and MBD 6 (565 - 631).

In one aspect, the application provides variant ATP7B nucleotide sequences that encode a functional ATP7B protein or a functional fragment of an ATP7B protein. Any suitable method may be used to determine whether an ATP7B protein or fragment thereof is functional. In certain embodiments, a functional ATP7B protein or functional fragment thereof, is one that is capable of exhibiting one or more activities of the wild-type protein, such as, for example, catalytic activity and/or copper transport activity in an *in vitro* or *in vivo* assay. In certain embodiments, a functional ATP7B protein or functional fragment thereof has at least 25%, 30%, 35%, 40%, 45%, 50%, 55%, 60%, 65%, 70%, 75%, 80%, 85%, 90%, 95%, 98% or 99% of the activity as compared to the wild-type ATP7B. One example of an *in vitro* assay for measuring ATP7B activity involves a yeast strain in which the endogenous copper-transporting ATPase CCC2 has been deleted thereby disrupting copper delivery to the secretory compartment and rendering the copper dependent ferroxidase Fet3 inactive. Expression of a functional ATP7B protein or functional fragment in such a strain restores Fet3 activity. See R. Tsivkovskii et al., J. Biol. Chem 77(2): 976-983 (2002), J.R. Forbes et al., Am J Hum Genet. 63: 1663-1674 (1998), G. Hsi et al., Hum Mutat 29: 491-501 (2008), and L.M. Luoma et al., Hum Mutat 31: 569-577 (2010). Another exemplary *in vitro* assay for measuring ATP7B activity involves expressing ATP7B or variants thereof using a baculovirus expression system in Sf9 cells and then measuring catalytic activity and copper (⁶⁴Cu) transport into vesicles. See e.g., R. Tsivkovskii et al., J. Biol. Chem 77(2): 976-983 (2002), and D. Huster et al., Gastroenterology 142(4): 947-956 (2012). In an exemplary embodiment, a functional ATP7B protein or functional fragment thereof, is one that is capable of substituting for the wild-type protein in an *in vivo* assay (e.g., a mouse model of Wilson's disease as described below). Various endpoints may be used to determine whether a variant ATP7B is functional including for example, measurement of non-ceruloplasmin-bound copper (NCC or "free copper" or copper index), serum transaminase levels (ALT), serum copper levels, 24 hour urine copper, hepatic copper levels, and clinical assessment of various factors such as dietary copper tolerance, liver inflammation, bile duct proliferation and liver fibrosis. Exemplary *in vivo* assays for assessing whether an ATP7B variant is functional include various animal models for Wilson's disease including, for example, the Long Evans Cinnamon rat, which has a large deletion in the ATP7B gene (K. Terada et al. Pediatr Int. 41(4):414-8(1999)); the Jackson's toxic milk mouse, which has a point mutation in the ATP7B coding sequence (E.A. Roberts et al. Mol Genet Metab. 93(l):54-65 (2008)); and an ATP7B mouse (D. Huster D et al. Am J Pathol. 168(2):423-34 (2006)). Standard methods may be used to analyze ATP7B activity in the mouse, for example, (i) serum transaminases (ALT) levels may be determined by the DGKC method (Roche Diagnostics, Mannheim, Germany) using a Hitachi 747 Clinical Analyzer (Hitachi, Tokyo, Japan ), (ii) serum ceruloplasmin activity may be determined using o-dianisidine dihydrochloridc (4, 4'-diamino-3,3'-dimethoxy-biphenyl) as substrate (Sigma-Aldrich, San Louis, MO, United States) as described by Schosinsky et al., Clinical Chemistry 20(12): 1556-1563 (1974)) and measuring absorbance at 540 nm in a spectrophotometer, and (iii) urine copper content may be determined by atomic absorption spectroscopy (SIMAA 6000, from Perkin-Eimer GmbH, Bodenseewerk). After sacrifice, the liver may be excised for histological determination and analysis of copper content, inflammation, biliary duct proliferation and fibrosis: (i) hepatic copper content may be determined in dry liver tissue by atomic absorption spectroscopy (SIMAA 6000, from Perkin-Elmer GmbH, Bodenseewerk), and by Timm's sulphide silver staining (G. Danscher and J. Zimmer, Histochemistry 55(1): 27-40 (1978)), (ii) liver structure may be assessed in sections stained with hematozylin and eosin, (iii) immunohistochemistry with anti-mouse CD45 antibody (e.g., BioLegend, San Diego, USA; Catalog umber 103102) may be used to detect inflammatory infiltration in the liver, (iv) immunohistochemistry with anti-mouse PanCk antibody (e.g., Invitrogen/Life Technologies, 18-0132, clone AE1/AE3) may be used to detect biliary duct cells, and (v) fibrosis may be determined using Sirius Red staining for detecting collagen. See e.g., WO 2016/097218, WO 2017/103624, and WO 2018/126116. In general, ATP7B knock-out mice models of Wilson's disease involve severe copper accumulation in the liver leading to liver disease, rising levels of serum copper levels, and eventually an increase in urine copper levels above that observed in heterozygous mice. The effects of ATP7B variants in these *in vitro* and *in vivo* assays can be compared against a wild-type ATP7B control to look for desired effects and thus functional activity.

In certain embodiments, the variant ATP7B nucleotide sequences described herein have been codon optimized to increase expression. In an exemplary embodiment, such variant ATP7B nucleotide sequences have been codon optimized to increase expression in a particular cell or tissue type, such as, for example liver or a liver cell. In certain embodiments, variant ATP7B sequences that have been codon optimized to increase expression of a functional ATP7B sequence by at least about 2-fold, 5-fold, 10-fold, 15-fold, 20-fold, 25-fold, 30-fold, 40-fold, 50-fold, 100-fold or more relative to expression of the same functional ATP7B protein from a nucleotide sequence that has not been codon optimized. In one embodiment, a variant ATP7B sequence that has been codon optimized increases expression of the full length ATP7B sequence (e.g., SEQ ID NO: 2) by at least about 2-fold, 5-fold, 10-fold, 15-fold, 20-fold, 25-fold, 30-fold, 40-fold, 50-fold, 100-fold or more relative to expression of the full length ATP7B protein from a nucleotide sequence that has not been codon optimized (e.g., SEQ ID NO: 1). In an exemplary embodiment, a variant ATP7B sequence that has been codon optimized increases expression of a functional ATP7B sequence in the liver or a liver cell. When comparing expression levels of codon optimized and non-codon optimized sequences, the comparison should be done using standard conditions, e.g., the same or similar expression vectors, cell type, culture conditions, etc. In certain embodiments, a variant ATP7B nucleotide sequence as disclosed herein that has been codon optimized comprises a sequence having less than 99%, 98%, 97%, 96%, 95%, 94%, 93%, 92%, 91%, 90%, 89%, 88%, 87%, 86%, 85%, 84%, 83%, 82%, 81%, 80%, 79%, 78%, 77%, 76%, 75%, 74%, 73%, 72%, 71%, or 70% sequence identity to the wild type ATP7B nucleotide sequence (e.g., SEQ ID NO: 1) and encodes a protein having a sequence that is at least 90%, 90.5%, 91%, 91.5%, 92%, 92.5%, 93%, 93.5%, 94%, 94.5%, 95%, 95.5%, 96%, 96.5%, 97%, 97.5%, 98%, 98.5%, 99%, 99.5%, or 100% identical to the wild type ATP7B protein sequence (e.g., SEQ ID NO: 2). In an exemplary embodiment, a variant ATP7B nucleotide sequence as disclosed herein that has been codon optimized comprises a sequence having less than 80% sequence identity to the wild type ATP7B nucleotide sequence (e.g., SEQ ID NO: 1) and encodes an ATP7B protein that is 100% identical to the wild type ATP7B protein sequence (e.g., SEQ ID NO: 2). In certain embodiments, a variant ATP7B nucleotide sequence as disclosed herein that has been codon optimized comprises a sequence having from about 60-70%, 60-75%, 60-80%, 60-85%, 60-90%, 60-95%, 65-70%, 65-75%, 65-80%, 65-85%, 65-90%, 65-95%, 70-75%, 70-80%, 70-85%, 70-90%, 70-95%, 75-80%, 75-85%, 75-90%, 75-95% sequence identity to the wild type ATP7B nucleotide sequence (e.g., SEQ ID NO: 1) and encodes a protein having a sequence that is at least 90%, 90.5%, 91%, 91.5%, 92%, 92.5%, 93%, 93.5%, 94%, 94.5%, 95%, 95.5%, 96%, 96.5%, 97%, 97.5%, 98%, 98.5%, 99%, 99.5%, or 100% identical to the wild type ATP7B protein sequence (e.g., SEQ ID NO: 2). In an exemplary embodiment, a variant ATP7B nucleotide sequence as disclosed herein that has been codon optimized comprises a sequence having from 70-80% sequence identity to the wild type ATP7B nucleotide sequence (e.g., SEQ ID NO: 1) and encodes an ATP7B protein that is 100% identical to the wild type ATP7B protein sequence (e.g., SEQ ID NO: 2).

In certain embodiments, a variant ATP7B nucleotide sequence provided herein has at least 350, 375, 400, 425, 450, 475, 500, 525, 550, 575, 600, 625, 650, 675, 700, 725, 750, 775, 800, 825, 859, 875, 900, 925, 950, 975, 1000, 025, 1050, 1075, 1100, 1125, 1150, 1175, 1200, 1225, 1250, 1275, 1300, 1325, 1350, 1375, 1400, 1425, or 1450, or from about 350-1200, 350-1100, 350-900, 350-750, 450-1200, 450-1100, 450-900, 450-750, 750-1100, 750-1200, 750-900, 900-1200, or 900-1100 codons that have been modified as compared to the codon present at the same location in the wild-type sequence (e.g., SEQ ID NO: 1). In certain embodiments, a variant ATP7B nucleotide sequence provided herein has at least 20%, 25%, 30%, 35%, 40%, 45%, 50%, 55%, 60%, 65%, 70%, 75%, 80%, 85%, 90%, or 95%, or from about 20-90%, 20-80%, 20-70%, 20-60%, 20-50%, 20-40%, 30-90%, 30-80%, 30-70%, 30-60%, 30-50%, 40-90%, 40-80%, 40-70%, 40-60%, 40-50%, 50-90%, 50-80%, 50-70%, 50-60%, 60-90%, 60-80%, or 60-79% of codons that have been modified as compared to the codon present at the same location in the wild-type sequence (e.g., SEQ ID NO: 1). In an exemplary embodiment, a variant ATP7B nucleotide sequence as disclosed herein that has been codon optimized comprises a sequence having from about 500-1100 codons that have been modified as compared to the codon present at the same location in the wild-type sequence (e.g., SEQ ID NO: 1) and encodes an ATP7B protein that is 100% identical to the wild type ATP7B protein sequence (e.g., SEQ ID NO: 2). In an exemplary embodiment, a variant ATP7B nucleotide sequence as disclosed herein that has been codon optimized comprises a sequence having from about 40-70% codons that have been modified as compared to the codon present at the same location in the wild-type sequence (e.g., SEQ ID NO: 1) and encodes an ATP7B protein that is 100% identical to the wild type ATP7B protein sequence (e.g., SEQ ID NO: 2).

ATP7B sequences may be codon optimized using any method known in the art. For example, codon optimized ATP7B sequences may be produced using methods available online (see e.g., Integrated DNA Technologies (IDT)) codon optimization tool available on the world wide web at idtdna.com or Codon Optimizer software available on the world wide web at cs.ubc.ca), using published methods (see e.g., SM Richardson et al., Genome Research 16:550-556 (2006), A. Villalobos et al., BMC Bioinformatics 7:285 (2006), W. Gao et al., Biotechnology Progress 20:443-448 (2004), S. Jayaraj et al., Nucleic Acids Research 33:3011-3016 (2005), G. Wu et al., Protein Expr Purif. 47:441-445 (2006), M. Bode et al., Nucleic Acids Research. 37:W214-221 (2009), D. Raab et al., Systems and Synthetic Biology 4:215-225 (2010), P. Gaspar et al., Bioinformatics 28:2683-2684 (2012), E. Angov et al., PloS One 3:e2189 (2008), A. Fuglsang, Protein Expr Purif. 31:247-249 (2003), W. Qian et al., PLoS Genetics. 8:e1002603 (2012), GW Hatfield and DA Roth, Biotechnology Annual Review 13:27-42 (2007), VP Mauro and SA Chappell, Trends Mol. Med. 20(11): 604-613 (2014), WO 2015/-12924, US 2014/0032186, and US 2006/0136184), or using a company which provides codon optimization services (see e.g., ATUM on the world wide web at atum.bio or GenScript on the world wide web at genscript.com). In certain embodiments, a portion of the nucleic acid sequence encoding ATP7B is codon optimized. In exemplary embodiments, the entire sequence encoding ATP7B is codon optimized.

In certain embodiments, the variant ATP7B nucleotide sequences described herein have been truncated so as to encode a functional fragment or domain of ATP7B. Such truncated ATP7B variant nucleotide sequences are useful for constructing gene therapy vectors having a packing capacity of less than about 5 kb, such that the full length ATP7B gene nears or exceeds the packing capacity of the vector. For example, AAV vectors have a packing capacity of ~4.7 kb and the full length ATP7B gene is ~4.4 kb thereby approaching the packaging capacity of the AAV vector. In certain embodiments, ATP7B variant nucleotide sequences are truncated by at least 100 bp, 200 bp, 300 bp, 400 bp, 500 bp, 600 bp, 700 bp, 800 bp, 900 bp, 1000 bp, 1100 bp, 1200 bp, 1300 bp, 1400 bp, 1500 bp, 1600 bp, 1700 bp, 1800 bp, 1900 bp, or 2000 bp, or by about 100-2000 bp, 100-1500 bp, 100-1000 bp, 100-500 bp, 200-2000 bp, 200-1500 bp, 200-100 bp, 200-500 bp, 500-2000 bp, 500-1500 bp, 500-1000 bp, 750-2000 bp, 750-1500 bp, 750-1000 bp, 1000 -1500 bp, or 1200-1500 bp. In certain embodiments, such variant ATP7B nucleotide sequences encode a functional ATP7B protein that has been truncated at the N-terminus, the C-terminus, in a position within the ATP7B protein that does not encompass the N-terminus or C-terminus, or partially at the N-terminus and the C-terminus. In exemplary embodiments, the variant ATP7B nucleotide sequence encodes a functional ATP7B protein that has been truncated at the N-terminus.

In certain embodiments, the variant ATP7B nucleotide sequences disclosed herein have been both codon optimized and truncated and encode a functional fragment of ATP7B protein. In one embodiment, a variant ATP7B sequence has been truncated by at least 100 bp, 200 bp, 300 bp, 400 bp, 500 bp, 600 bp, 700 bp, 800 bp, 900 bp, 1000 bp, 1100 bp, 1200 bp, 1300 bp, 1400 bp, 1500 bp, 1600 bp, 1700 bp, 1800 bp, 1900 bp, or 2000 bp, or by about 100-2000 bp, 100-1500 bp, 100-1000 bp, 100-500 bp, 200-2000 bp, 200-1500 bp, 200-1000 bp, 200-500 bp, 500-2000 bp, 500-1500 bp, 500-1000 bp, 750-2000 bp, 750-1500 bp, 750-1000 bp, 1000 -1500 bp, or 1200-1500 bp and encodes a functional fragment of ATP7B and such variant sequence has been codon optimized so as to increase expression of the functional ATP7B fragment by at least about 2-fold, 5-fold, 10-fold, 15-fold, 20-fold, 25-fold, 30-fold, 40-fold, 50-fold, 100-fold or more relative to expression of the equivalent ATP7B protein fragment from a nucleotide sequence that has not been codon optimized (e.g., a fragment of SEQ ID NO: 1). In an exemplary embodiment, a variant ATP7B sequence disclosed herein has been truncated by 1-1000 bp at the N-terminus, encodes a functional ATP7B fragment, and has been codon optimized to increase expression in the liver or a liver cell by at least 5-fold relative to expression of the equivalent ATP7B fragment that has not been codon optimized.

In certain embodiments, the variant ATP7B nucleotide sequences disclosed herein comprise a sequence that has at least 60%, 65%, 70%, 75%, 80%, 85%, 90%, 91%, 92%, 93%, 94%, 95%, 96%, 97%, 98%, 99% or 100% identity to any of one of SEQ ID NOs: 1-18. In exemplary embodiments, such variant ATP7B nucleotide sequences encode a functional ATP7B protein or a fragment thereof. In exemplary embodiments, a variant ATP7B nucleotide sequence provided herein is a non-naturally occurring sequence.

In one embodiment, a variant ATP7B nucleotide sequence disclosed herein comprises a sequence that has at least 60%, 65%, 70%, 75%, 80%, 85%, 90%, 91%, 92%, 93%, 94%, 95%, 96%, 97%, 98%, 99% or 100% identity to SEQ ID NO: 3. In an exemplary embodiment, such sequence is codon optimized and encodes a full length ATP7B protein (e.g., having SEQ ID NO: 2). In certain embodiments, such codon optimized sequence increases expression of the ATP7B protein by at least about 2-fold, 3-fold, 4-fold, 5-fold, 6-fold, 7-fold, 8-fold, 9-fold, 10-fold, 11-fold, 12-fold, 13-fold, 14-fold, 15-fold, 20-fold, 25-fold, 30-fold, 40-fold, 50-fold, 100-fold or more relative to expression of the equivalent ATP7B protein from a nucleotide sequence that has not been codon optimized (e.g., SEQ ID NO: 1). In another embodiment, a variant ATP7B nucleotide sequence disclosed herein comprises a truncation of SEQ ID NO: 3 that encodes a functional fragment of ATP7B. For example, in certain embodiments, SEQ ID NO: 3 may be truncated by about 100 bp, 200 bp, 300 bp, 400 bp, 500 bp, 600 bp, 700 bp, 800 bp, 900 bp, 1000 bp, 1100 bp, 1200 bp, 1300 bp, 1400 bp, 1500 bp, 1600 bp, 1700 bp, 1800 bp, 1900 bp, or 2000 bp, or by about 100-2000 bp, 100-1500 bp, 100-1000 bp, 100-500 bp, 200-2000 bp, 200-1500 bp, 200-100 bp, 200-500 bp, 500-2000 bp, 500-1500 bp, 500-1000 bp, 750-2000 bp, 750-1500 bp, 750-1000 bp, 1000-1500 bp, or 1200-1500 bp and the resulting truncated sequence encodes a functional fragment of ATP7B. In one embodiment, a variant ATP7B sequence comprises SEQ ID NO: 3 that has been truncated by 1-1000 bp at the N-terminus, encodes a functional ATP7B fragment, and has been codon optimized to increase expression in the liver or a liver cell by at least 5-fold relative to expression of the equivalent ATP7B fragment that has not been codon optimized. In an exemplary embodiment, a variant ATP7B sequence disclosed herein comprises SEQ ID NO: 3.

In one embodiment, a variant ATP7B nucleotide sequence disclosed herein comprises a sequence that has at least 60%, 65%, 70%, 75%, 80%, 85%, 90%, 91%, 92%, 93%, 94%, 95%, 96%, 97%, 98%, 99% or 100% identity to SEQ ID NO: 4. In an exemplary embodiment, such sequence is codon optimized and encodes a full length ATP7B protein (e.g., having SEQ ID NO: 2). In certain embodiments, such codon optimized sequence increases expression of the ATP7B protein by at least about 2-fold, 3-fold, 4-fold, 5-fold, 6-fold, 7-fold, 8-fold, 9-fold, 10-fold, 11-fold, 12-fold, 13-fold, 14-fold, 15-fold, 20-fold, 25-fold, 30-fold, 40-fold, 50-fold, 100-fold or more relative to expression of the equivalent ATP7B protein from a nucleotide sequence that has not been codon optimized (e.g., SEQ ID NO: 1). In another embodiment, a variant ATP7B nucleotide sequence disclosed herein comprises a truncation of SEQ ID NO: 4 that encodes a functional fragment of ATP7B. For example, in certain embodiments, SEQ ID NO: 4 may be truncated by about 100 bp, 200 bp, 300 bp, 400 bp, 500 bp, 600 bp, 700 bp, 800 bp, 900 bp, 1000 bp, 1100 bp, 1200 bp, 1300 bp, 1400 bp, 1500 bp, 1600 bp, 1700 bp, 1800 bp, 1900 bp, or 2000 bp, or by about 100-2000 bp, 100-1500 bp, 100-1000 bp, 100-500 bp, 200-2000 bp, 200-1500 bp, 200-100 bp, 200-500 bp, 500-2000 bp, 500-1500 bp, 500-1000 bp, 750-2000 bp, 750-1500 bp, 750-1000 bp, 1000-1500 bp, or 1200-1500 bp and the resulting truncated sequence encodes a functional fragment of ATP7B. In one embodiment, a variant ATP7B sequence comprises SEQ ID NO: 4 that has been truncated by 1-1000 bp at the N-terminus, encodes a functional ATP7B fragment, and has been codon optimized to increase expression in the liver or a liver cell by at least 5-fold relative to expression of the equivalent ATP7B fragment that has not been codon optimized. In an exemplary embodiment, a variant ATP7B sequence disclosed herein comprises SEQ ID NO: 4.

In one embodiment, a variant ATP7B nucleotide sequence disclosed herein comprises a sequence that has at least 60%, 65%, 70%, 75%, 80%, 85%, 90%, 91%, 92%, 93%, 94%, 95%, 96%, 97%, 98%, 99% or 100% identity to SEQ ID NO: 5. In an exemplary embodiment, such sequence is codon optimized and encodes a full length ATP7B protein (e.g., having SEQ ID NO: 2). In certain embodiments, such codon optimized sequence increases expression of the ATP7B protein by at least about 2-fold, 3-fold, 4-fold, 5-fold, 6-fold, 7-fold, 8-fold, 9-fold, 10-fold, 11-fold, 12-fold, 13-fold, 14-fold, 15-fold, 20-fold, 25-fold, 30-fold, 40-fold, 50-fold, 100-fold or more relative to expression of the equivalent ATP7B protein from a nucleotide sequence that has not been codon optimized (e.g., SEQ ID NO: 1). In another embodiment, a variant ATP7B nucleotide sequence disclosed herein comprises a truncation of SEQ ID NO: 5 that encodes a functional fragment of ATP7B. For example, in certain embodiments, SEQ ID NO: 5 may be truncated by about 100 bp, 200 bp, 300 bp, 400 bp, 500 bp, 600 bp, 700 bp, 800 bp, 900 bp, 1000 bp, 1100 bp, 1200 bp, 1300 bp, 1400 bp, 1500 bp, 1600 bp, 1700 bp, 1800 bp, 1900 bp, or 2000 bp, or by about 100-2000 bp, 100-1500 bp, 100-1000 bp, 100-500 bp, 200-2000 bp, 200-1500 bp, 200-100 bp, 200-500 bp, 500-2000 bp, 500-1500 bp, 500-1000 bp, 750-2000 bp, 750-1500 bp, 750-1000 bp, 1000-1500 bp, or 1200-1500 bp and the resulting truncated sequence encodes a functional fragment of ATP7B. In one embodiment, a variant ATP7B sequence comprises SEQ ID NO: 5 that has been truncated by 1-1000 bp at the N-terminus, encodes a functional ATP7B fragment, and has been codon optimized to increase expression in the liver or a liver cell by at least 5-fold relative to expression of the equivalent ATP7B fragment that has not been codon optimized. In an exemplary embodiment, a variant ATP7B sequence disclosed herein comprises SEQ ID NO: 5.

In one embodiment, a variant ATP7B nucleotide sequence disclosed herein comprises a sequence that has at least 60%, 65%, 70%, 75%, 80%, 85%, 90%, 91%, 92%, 93%, 94%, 95%, 96%, 97%, 98%, 99% or 100% identity to SEQ ID NO: 6. In an exemplary embodiment, such sequence is codon optimized and encodes a full length ATP7B protein (e.g., having SEQ ID NO: 2). In certain embodiments, such codon optimized sequence increases expression of the ATP7B protein by at least about 2-fold, 3-fold, 4-fold, 5-fold, 6-fold, 7-fold, 8-fold, 9-fold, 10-fold, 11-fold, 12-fold, 13-fold, 14-fold, 15-fold, 20-fold, 25-fold, 30-fold, 40-fold, 50-fold, 100-fold or more relative to expression of the equivalent ATP7B protein from a nucleotide sequence that has not been codon optimized (e.g., SEQ ID NO: 1). In another embodiment, a variant ATP7B nucleotide sequence disclosed herein comprises a truncation of SEQ ID NO: 6 that encodes a functional fragment of ATP7B. For example, in certain embodiments, SEQ ID NO: 3 may be truncated by about 100 bp, 200 bp, 300 bp, 400 bp, 500 bp, 600 bp, 700 bp, 800 bp, 900 bp, 1000 bp, 1100 bp, 1200 bp, 1300 bp, 1400 bp, 1500 bp, 1600 bp, 1700 bp, 1800 bp, 1900 bp, or 2000 bp, or by about 100-2000 bp, 100-1500 bp, 100-1000 bp, 100-500 bp, 200-2000 bp, 200-1500 bp, 200-100 bp, 200-500 bp, 500-2000 bp, 500-1500 bp, 500-1000 bp, 750-2000 bp, 750-1500 bp, 750-1000 bp, 1000-1500 bp, or 1200-1500 bp and the resulting truncated sequence encodes a functional fragment of ATP7B. In one embodiment, a variant ATP7B sequence comprises SEQ ID NO: 6 that has been truncated by 1-1000 bp at the N-terminus, encodes a functional ATP7B fragment, and has been codon optimized to increase expression in the liver or a liver cell by at least 5-fold relative to expression of the equivalent ATP7B fragment that has not been codon optimized. In an exemplary embodiment, a variant ATP7B sequence disclosed herein comprises SEQ ID NO: 6.

In certain embodiments, a variant ATP7B sequence that may be used in connection with the regulatory elements disclosed herein comprises a codon optimized ATP7B sequence as disclosed in WO 2017/103624. In such embodiments, a variant ATP7B nucleotide sequence comprises a sequence that has at least 60%, 65%, 70%, 75%, 80%, 85%, 90%, 91%, 92%, 93%, 94%, 95%, 96%, 97%, 98%, 99% or 100% identity to SEQ ID NO: 7. In an exemplary embodiment, such sequence is codon optimized and encodes a full length ATP7B protein. In another embodiment, a variant ATP7B nucleotide sequence disclosed herein comprises a truncation of SEQ ID NO: 7 that encodes a functional fragment of ATP7B. For example, in certain embodiments, SEQ ID NO: 7 may be truncated by about 100 bp, 200 bp, 300 bp, 400 bp, 500 bp, 600 bp, 700 bp, 800 bp, 900 bp, 1000 bp, 1100 bp, 1200 bp, 1300 bp, 1400 bp, 1500 bp, 1600 bp, 1700 bp, 1800 bp, 1900 bp, or 2000 bp, or by about 100-2000 bp, 100-1500 bp, 100-1000 bp, 100-500 bp, 200-2000 bp, 200-1500 bp, 200-100 bp, 200-500 bp, 500-2000 bp, 500-1500 bp, 500-1000 bp, 750-2000 bp, 750-1500 bp, 750-1000 bp, 1000-1500 bp, or 1200-1500 bp and the resulting truncated sequence encodes a functional fragment of ATP7B. In one embodiment, a variant ATP7B sequence comprises SEQ ID NO: 7 that has been truncated by 1-1000 bp at the N-terminus and encodes a functional ATP7B fragment. In an exemplary embodiment, a codon optimized variant ATP7B sequence that can be used in connection with the regulatory sequences disclosed herein comprises SEQ ID NO: 7.

In certain embodiments, a variant ATP7B sequence that may be used in connection with the regulatory elements disclosed herein comprises a codon optimized ATP7B sequence as disclosed in WO 2018/126116. In such embodiments, a variant ATP7B nucleotide sequence comprises a sequence that has at least 60%, 65%, 70%, 75%, 80%, 85%, 90%, 91%, 92%, 93%, 94%, 95%, 96%, 97%, 98%, 99% or 100% identity to SEQ ID NO: 8. In an exemplary embodiment, such sequence is codon optimized and encodes a full length ATP7B protein. In another embodiment, a variant ATP7B nucleotide sequence disclosed herein comprises a truncation of SEQ ID NO: 8 that encodes a functional fragment of ATP7B. For example, in certain embodiments, SEQ ID NO: 8 may be truncated by about 100 bp, 200 bp, 300 bp, 400 bp, 500 bp, 600 bp, 700 bp, 800 bp, 900 bp, 1000 bp, 1100 bp, 1200 bp, 1300 bp, 1400 bp, 1500 bp, 1600 bp, 1700 bp, 1800 bp, 1900 bp, or 2000 bp, or by about 100-2000 bp, 100-1500 bp, 100-1000 bp, 100-500 bp, 200-2000 bp, 200-1500 bp, 200-100 bp, 200-500 bp, 500-2000 bp, 500-1500 bp, 500-1000 bp, 750-2000 bp, 750-1500 bp, 750-1000 bp, 1000-1500 bp, or 1200-1500 bp and the resulting truncated sequence encodes a functional fragment of ATP7B. In one embodiment, a variant ATP7B sequence comprises SEQ ID NO: 8 that has been truncated by 1-1000 bp at the N-terminus and encodes a functional ATP7B fragment. Exemplary codon optimized, truncated variant ATP7B sequences as described in WO 2018/126116 include: a codon optimized variant ATP7B sequence that has been truncated so as to remove the first two metal-binding domains (SEQ ID NO: 9), a codon optimized variant ATP7B sequence that has been truncated so as to remove the first four metal-binding domains (SEQ ID NO: 10), a codon optimized variant ATP7B sequence that has been truncated so as to remove the first five metal-binding domains (SEQ ID NO: 11), a codon optimized variant ATP7B sequence that has been truncated so as to remove the first metal-binding domain (SEQ ID NO: 12), a codon optimized variant ATP7B sequence that has been truncated so as to remove the second metal-binding domain (SEQ ID NO: 13), and a codon optimized variant ATP7B sequence that has been truncated so as to remove the third metal-binding domain (SEQ ID NO: 14). In an exemplary embodiment, a codon optimized variant ATP7B sequence that can be used in connection with the regulatory sequences disclosed herein comprises SEQ ID NO: 8. In an exemplary embodiment, a codon optimized, truncated variant ATP7B sequence that can be used in connection with the regulatory sequences disclosed herein comprises any one of SEQ ID NOs: 9-14.

In other embodiments, a codon optimized, truncated variant ATP7B sequence that can be used in connection with the regulatory sequences disclosed herein comprises an ATP7B sequence that has been truncated so as to remove the first three metal-binding domains (see WO 2018/126116) or metal-binding domains 1-4 and 6 (see e.g., Carter et al., Biochem J. 380: 805-813 (2004), Gourdon et al., Biol. Chem. 393(4): 205-216 (2012), Lutsenko et al., Physiological Reviews 87(3): 1011-1046 (2013), and US 2015/0045284).

In certain embodiments, a variant ATP7B sequence that may be used in connection with the regulatory elements disclosed herein comprises a codon optimized ATP7B sequence as disclosed in WO 2016/097218 or WO 2016/097219. In such embodiments, a variant ATP7B nucleotide sequence comprises a sequence that has at least 60%, 65%, 70%, 75%, 80%, 85%, 90%, 91%, 92%, 93%, 94%, 95%, 96%, 97%, 98%, 99% or 100% identity to SEQ ID NO: 15. In an exemplary embodiment, such sequence is codon optimized and encodes a full length ATP7B protein. In another embodiment, a variant ATP7B nucleotide sequence disclosed herein comprises a truncation of SEQ ID NO: 15 that encodes a functional fragment of ATP7B. For example, in certain embodiments, SEQ ID NO: 15 may be truncated by about 100 bp, 200 bp, 300 bp, 400 bp, 500 bp, 600 bp, 700 bp, 800 bp, 900 bp, 1000 bp, 1100 bp, 1200 bp, 1300 bp, 1400 bp, 1500 bp, 1600 bp, 1700 bp, 1800 bp, 1900 bp, or 2000 bp, or by about 100-2000 bp, 100-1500 bp, 100-1000 bp, 100-500 bp, 200-2000 bp, 200-1500 bp, 200-100 bp, 200-500 bp, 500-2000 bp, 500-1500 bp, 500-1000 bp, 750-2000 bp, 750-1500 bp, 750-1000 bp, 1000-1500 bp, or 1200-1500 bp and the resulting truncated sequence encodes a functional fragment of ATP7B. In one embodiment, a variant ATP7B sequence comprises SEQ ID NO: 15 that has been truncated by 1-1000 bp at the N-terminus and encodes a functional ATP7B fragment. Exemplary codon optimized, truncated variant ATP7B sequences as described in WO 2016/097219 include codon optimized, truncated variants in which one or more of the metal-binding sites or heavy metal associated (HMA) domains have been removed (e.g., HMA1 comprises amino acids 59-125 of the wild-type ATP7B sequence, HMA2 comprises amino acids 144-210 of the wild-type ATP7B sequence, HMA3 comprises amino acids 258-327 of the wild-type ATP7B sequence, HMA4 comprises amino acids 360-426 of the wild-type ATP7B sequence, HMA5 comprises amino acids 489-555 of the wild-type ATP7B sequence, and HMA6 comprises amino acids 565-631 of the wild-type ATP7B sequence). In an exemplary embodiment, the variant ATP7B sequence has been truncated so as to encode a protein wherein the first 4 metal-binding domains have been removed. In an exemplary embodiment, a codon optimized variant ATP7B sequence that can be used in connection with the regulatory sequences disclosed herein comprises SEQ ID NO: 15. In an exemplary embodiment, a codon optimized, truncated variant ATP7B sequence that can be used in connection with the regulatory sequences disclosed herein comprises SEQ ID NO: 17. In another embodiment, a variant ATP7B sequence that can be used in connection with the regulatory sequences disclosed herein is a truncated, non-codon optimized sequence comprising SEQ ID NO: 16 or 18.

In certain embodiments, a variant ATP7B nucleic acid molecule provided herein does not contain any modified bases. In certain embodiments, a variant ATP7B nucleic acid molecule provided herein is a DNA molecule. In certain embodiments, a variant ATP7B nucleic acid molecule provided herein is a DNA molecule that does not contain any modified bases.

### Nucleic Acid Constructs

In certain embodiments, the variant ATP7B constructs disclosed herein are part of a nucleic acid construct comprising one or more regulatory elements in addition to the variant ATP7B sequence. In exemplary embodiments, the variant ATP7B constructs disclosed herein are part of a nucleic acid construct comprising a promoter situated upstream of the ATP7B construct so as to be capable of driving expression of the variant ATP7B sequence in a cell.

In one embodiment, a nucleic acid construct disclosed herein comprises (1) a promoter having (i) any one of SEQ ID NOs: 23-43, 48-55 or 66-70 (as shown below in Tables 2 and 3), (ii) a sequence having at least 80%, 85%, 90%, 91%, 92%, 93%, 94%, 95%, 96%, 97%, 98%, 99% or 100% identity to any one of SEQ ID NOs: 23-43, 48-55 or 66-70, or (iii) a functional fragment of any of the foregoing, operably linked to (2) any one of the variant ATP7B sequences disclosed herein, e.g., a variant ATP7B sequence comprising (i) any one of SEQ ID NOs: 1-18 (as shown below in Table 1), (ii) a sequence having at least 80%, 85%, 90%, 91%, 92%, 93%, 94%, 95%, 96%, 97%, 98%, 99% or 100% identity to any one of SEQ ID NOs: 1-18, or (iii) a functional fragment or variant thereof.

In one embodiment, a nucleic acid construct disclosed herein comprises a promoter having any one of SEQ ID NOs: 23-43, 48-55 or 66-70 (as shown below in Tables 2 and 3) operably linked to any one of the variant ATP7B sequences disclosed herein, e.g., a variant ATP7B sequence comprising any one of SEQ ID NOs: 1-18 (as shown below in Table 1), or a functional fragment thereof. In another embodiment, a nucleic acid construct disclosed herein comprises a regulatory element having a combination of two or more (e.g., two or more, three or more, four or more, five or more, or 2, 3, 4, or 5) of any one of SEQ ID NOs: 19-43 or 66-68 (as shown below in Table 2) operably linked to any one of the variant ATP7B sequences disclosed herein, e.g., a variant ATP7B sequence comprising any one of SEQ ID NOs: 1-18 (as shown below in Table 1), or a functional fragment thereof.

In certain embodiments, a nucleic acid construct disclosed herein comprises a promoter having any one of SEQ ID NOs: 23-43 or 66-68 (as shown below in Table 2) operably linked to any one of the variant ATP7B sequences disclosed herein, e.g., a variant ATP7B sequence comprising any one of SEQ ID NOs: 1-18 (as shown below in Table 1), or a functional fragment thereof. In certain embodiments, the promoter sequence produces at least 5-fold, 10-fold, 15-fold, 20-fold, 25-fold, 30-fold, 35-fold, 40-fold, 45-fold, 50-fold, 55-fold, 60-fold, 65-fold, 70-fold, or 75-fold, or at least 20-90 fold, 20-80 fold, 20-70 fold, 20-60 fold, 30-90 fold, 30-80 fold, 30-70 fold, 30-60 fold, 40-90 fold, 40-80 fold, 40-70 fold, 40-60 fold, 50-90 fold, 50-80 fold, 50-70 fold, 50-60 fold, 60-90 fold, 60-80 fold, 60-70 fold, 70-90 fold, 70-80 fold, 80-90 fold greater expression of the variant ATP7B sequence in a mammalian cell relative to the level of expression of the same variant ATP7B sequence from the CMV promoter. In certain embodiments, the promoter sequence drives expression of the variant ATP7B sequence in a high percentage of hepatocyte cells, e.g., at least 20%, 25%, 30%, 40%, 45%, 50%, 55%, 60%, 65%, 70%, 75%, 80%, 81%, 82%, 83%, 84%, 85%, 86%, 87%, 88%, 89%, 90%, 91%, 92%, 93%, 94%, 95%, 96%, 97%, 98% or greater, or at least 20-90%, 20-80%, 20-70%, 30-90%, 30-80%, 30-70%, 40-90%, 40-80%, 40-70%, 50-90%, 50-80%, 50-70%, 60-90%, 60-80%, 60-70%, 70-90%, 70-80%, 80-100%, 80-95%, 80-90%, 90-100%, or 90-95% of hepatocytes containing the nucleic acid construct express the variant ATP7B construct.

In one embodiment, a nucleic acid construct disclosed herein comprises a promoter having any one of SEQ ID NOs: 23-43, 48-55 or 66-70 operably linked to a codon optimized variant ATP7B sequence comprising (i) any one of SEQ ID NOs: 3-6, (ii) a sequence having at least 60%, 65%, 70%, 75%, 80%, 85%, 90%, 91%, 92%, 93%, 94%, 95%, 96%, 97%, 98%, 99% or 100% identity to any one of SEQ ID NOs: 3-6, or (iii) a functional fragment of any of the foregoing. In certain embodiments, such codon optimized variant ATP7B nucleotide sequence encodes a protein having a sequence that is at least 90%, 95%, 98%, 99% or 100% identical to the wild type ATP7B protein sequence (e.g., SEQ ID NO: 2). In certain embodiments, such codon optimized variant ATP7B sequence encodes a full length ATP7B protein, e.g., having SEQ ID NO: 2.

In one embodiment, a nucleic acid construct of the disclosure comprises a promoter having SEQ ID NO: 22 operably linked to a codon optimized variant ATP7B sequence comprising (i) any one of SEQ ID NOs: 3-6, (ii) a sequence having at least 60%, 65%, 70%, 75%, 80%, 85%, 90%, 91%, 92%, 93%, 94%, 95%, 96%, 97%, 98%, 99% or 100% identity to any one of SEQ ID NOs: 3-6, or (iii) a functional fragment of any of the foregoing. In certain embodiments, such codon optimized variant ATP7B nucleotide sequence encodes a protein having a sequence that is at least 90%, 95%, 98%, 99% or 100% identical to the wild type ATP7B protein sequence (e.g., SEQ ID NO: 2). In certain embodiments, such codon optimized variant ATP7B sequence encodes a full length ATP7B protein, e.g., having SEQ ID NO: 2.

In one embodiment, a nucleic acid construct of the disclosure comprises a promoter having SEQ ID NO: 33 operably linked to a codon optimized variant ATP7B sequence comprising (i) any one of SEQ ID NOs: 3-6, (ii) a sequence having at least 60%, 65%, 70%, 75%, 80%, 85%, 90%, 91%, 92%, 93%, 94%, 95%, 96%, 97%, 98%, 99% or 100% identity to any one of SEQ ID NOs: 3-6, or (iii) a functional fragment of any of the foregoing. In certain embodiments, such codon optimized variant ATP7B nucleotide sequence encodes a protein having a sequence that is at least 90%, 95%, 98%, 99% or 100% identical to the wild type ATP7B protein sequence (e.g., SEQ ID NO: 2). In certain embodiments, such codon optimized variant ATP7B sequence encodes a full length ATP7B protein, e.g., having SEQ ID NO: 2.

In one embodiment, a nucleic acid construct of the disclosure comprises a promoter having SEQ ID NO: 24 operably linked to a codon optimized variant ATP7B sequence comprising (i) any one of SEQ ID NOs: 3-6, (ii) a sequence having at least 60%, 65%, 70%, 75%, 80%, 85%, 90%, 91%, 92%, 93%, 94%, 95%, 96%, 97%, 98%, 99% or 100% identity to any one of SEQ ID NOs: 3-6, or (iii) a functional fragment of any of the foregoing. In certain embodiments, such codon optimized variant ATP7B nucleotide sequence encodes a protein having a sequence that is at least 90%, 95%, 98%, 99% or 100% identical to the wild type ATP7B protein sequence (e.g., SEQ ID NO: 2). In certain embodiments, such codon optimized variant ATP7B sequence encodes a full length ATP7B protein, e.g., having SEQ ID NO: 2.

In an exemplary embodiment, a nucleic acid construct of the disclosure comprises a promoter having SEQ ID NO: 33 operably linked to a codon optimized variant ATP7B sequence comprising (i) SEQ ID NO: 4, (ii) a sequence having at least 80%, 85%, 90%, 91%, 92%, 93%, 94%, 95%, 96%, 97%, 98%, 99% or 100% identity to SEQ ID NO: 4, or (iii) a functional fragment of any of the foregoing. In certain embodiments, such codon optimized variant ATP7B nucleotide sequence encodes a protein having a sequence that is at least 90%, 95%, 98%, 99% or 100% identical to the wild type ATP7B protein sequence (e.g., SEQ ID NO: 2). In certain embodiments, such codon optimized variant ATP7B sequence encodes a full length ATP7B protein, e.g., having SEQ ID NO: 2.

In an exemplary embodiment, a nucleic acid construct of the disclosure comprises a promoter having SEQ ID NO: 33 operably linked to a codon optimized variant ATP7B sequence comprising (i) SEQ ID NO: 5, (ii) a sequence having at least 80%, 85%, 90%, 91%, 92%, 93%, 94%, 95%, 96%, 97%, 98%, 99% or 100% identity to SEQ ID NO: 5, or (iii) a functional fragment of any of the foregoing. In certain embodiments, such codon optimized variant ATP7B nucleotide sequence encodes a protein having a sequence that is at least 90%, 95%, 98%, 99% or 100% identical to the wild type ATP7B protein sequence (e.g., SEQ ID NO: 2). In certain embodiments, such codon optimized variant ATP7B sequence encodes a full length ATP7B protein, e.g., having SEQ ID NO: 2.

In an exemplary embodiment, a nucleic acid construct of the disclosure comprises (1) a promoter comprising (i) SEQ ID NO: 24, (ii) a sequence having at least 80%, 85%, 90%, 91%, 92%, 93%, 94%, 95%, 96%, 97%, 98%, 99% or 100% identity to SEQ ID NO: 24, or (iii) a functional fragment of any of the foregoing, operably linked to (2) a codon optimized variant ATP7B sequence comprising (i) SEQ ID NO: 5, (ii) a sequence having at least 80%, 85%, 90%, 91%, 92%, 93%, 94%, 95%, 96%, 97%, 98%, 99% or 100% identity to SEQ ID NO: 5, or (iii) a functional fragment of any of the foregoing. In certain embodiments, such codon optimized variant ATP7B nucleotide sequence encodes a protein having a sequence that is at least 90%, 95%, 98%, 99% or 100% identical to the wild type ATP7B protein sequence (e.g., SEQ ID NO: 2). In certain embodiments, such codon optimized variant ATP7B sequence encodes a full length ATP7B protein, e.g., having SEQ ID NO: 2.

In an exemplary embodiment, a nucleic acid construct of the disclosure comprises a promoter having SEQ ID NO: 24 operably linked to a codon optimized variant ATP7B sequence comprising (i) SEQ ID NO: 4, (ii) a sequence having at least 80%, 85%, 90%, 91%, 92%, 93%, 94%, 95%, 96%, 97%, 98%, 99% or 100% identity to SEQ ID NO: 4, or (iii) a functional fragment of any of the foregoing. In certain embodiments, such codon optimized variant ATP7B nucleotide sequence encodes a protein having a sequence that is at least 90%, 95%, 98%, 99% or 100% identical to the wild type ATP7B protein sequence (e.g., SEQ ID NO: 2). In certain embodiments, such codon optimized variant ATP7B sequence encodes a full length ATP7B protein, e.g., having SEQ ID NO: 2.

In an exemplary embodiment, a nucleic acid construct of the disclosure comprises a promoter having SEQ ID NO: 24 operably linked to a codon optimized variant ATP7B sequence comprising (i) SEQ ID NO: 5, (ii) a sequence having at least 80%, 85%, 90%, 91%, 92%, 93%, 94%, 95%, 96%, 97%, 98%, 99% or 100% identity to SEQ ID NO: 5, or (iii) a functional fragment of any of the foregoing. In certain embodiments, such codon optimized variant ATP7B nucleotide sequence encodes a protein having a sequence that is at least 90%, 95%, 98%, 99% or 100% identical to the wild type ATP7B protein sequence (e.g., SEQ ID NO: 2). In certain embodiments, such codon optimized variant ATP7B sequence encodes a full length ATP7B protein, e.g., having SEQ ID NO: 2.

In an exemplary embodiment, a nucleic acid construct of the disclosure comprises (1) a promoter comprising (i) any one of SEQ ID NOs: 66-68, (ii) a sequence having at least 80%, 85%, 90%, 91%, 92%, 93%, 94%, 95%, 96%, 97%, 98%, 99% or 100% identity to any one of SEQ ID NOs: 66-68, or (iii) a functional fragment of any of the foregoing, operably linked to (2) a codon optimized variant ATP7B sequence comprising (i) any one of SEQ ID NOs: 1-18, (ii) a sequence having at least 80%, 85%, 90%, 91%, 92%, 93%, 94%, 95%, 96%, 97%, 98%, 99% or 100% identity to any one of SEQ ID NOs: 1-18, or (iii) a functional fragment of any of the foregoing. In certain embodiments, such codon optimized variant ATP7B nucleotide sequence encodes a protein having a sequence that is at least 90%, 95%, 98%, 99% or 100% identical to the wild type ATP7B protein sequence (e.g., SEQ ID NO: 2). In certain embodiments, such codon optimized variant ATP7B sequence encodes a full length ATP7B protein, e.g., having SEQ ID NO: 2.

In an exemplary embodiment, a nucleic acid construct of the disclosure comprises (1) a promoter comprising (i) SEQ ID NO: 66, (ii) a sequence having at least 80%, 85%, 90%, 91%, 92%, 93%, 94%, 95%, 96%, 97%, 98%, 99% or 100% identity to SEQ ID NO: 66, or (iii) a functional fragment of any of the foregoing, operably linked to (2) a codon optimized variant ATP7B sequence comprising (i) SEQ ID NO: 4, (ii) a sequence having at least 80%, 85%, 90%, 91%, 92%, 93%, 94%, 95%, 96%, 97%, 98%, 99% or 100% identity to SEQ ID NO: 4, or (iii) a functional fragment of any of the foregoing. In certain embodiments, such codon optimized variant ATP7B nucleotide sequence encodes a protein having a sequence that is at least 90%, 95%, 98%, 99% or 100% identical to the wild type ATP7B protein sequence (e.g., SEQ ID NO: 2). In certain embodiments, such codon optimized variant ATP7B sequence encodes a full length ATP7B protein, e.g., having SEQ ID NO: 2.

In an exemplary embodiment, a nucleic acid construct of the disclosure comprises (1) a promoter comprising (i) SEQ ID NO: 66, (ii) a sequence having at least 80%, 85%, 90%, 91%, 92%, 93%, 94%, 95%, 96%, 97%, 98%, 99% or 100% identity to SEQ ID NO: 66, or (iii) a functional fragment of any of the foregoing, operably linked to (2) a codon optimized variant ATP7B sequence comprising (i) SEQ ID NO: 5, (ii) a sequence having at least 80%, 85%, 90%, 91%, 92%, 93%, 94%, 95%, 96%, 97%, 98%, 99% or 100% identity to SEQ ID NO: 5, or (iii) a functional fragment of any of the foregoing. In certain embodiments, such codon optimized variant ATP7B nucleotide sequence encodes a protein having a sequence that is at least 90%, 95%, 98%, 99% or 100% identical to the wild type ATP7B protein sequence (e.g., SEQ ID NO: 2). In certain embodiments, such codon optimized variant ATP7B sequence encodes a full length ATP7B protein, e.g., having SEQ ID NO: 2.

In an exemplary embodiment, a nucleic acid construct of the disclosure comprises (1) a promoter comprising (i) SEQ ID NO: 67, (ii) a sequence having at least 80%, 85%, 90%, 91%, 92%, 93%, 94%, 95%, 96%, 97%, 98%, 99% or 100% identity to SEQ ID NO: 67, or (iii) a functional fragment of any of the foregoing, operably linked to (2) a codon optimized variant ATP7B sequence comprising (i) SEQ ID NO: 4, (ii) a sequence having at least 80%, 85%, 90%, 91%, 92%, 93%, 94%, 95%, 96%, 97%, 98%, 99% or 100% identity to SEQ ID NO: 4, or (iii) a functional fragment of any of the foregoing. In certain embodiments, such codon optimized variant ATP7B nucleotide sequence encodes a protein having a sequence that is at least 90%, 95%, 98%, 99% or 100% identical to the wild type ATP7B protein sequence (e.g., SEQ ID NO: 2). In certain embodiments, such codon optimized variant ATP7B sequence encodes a full length ATP7B protein, e.g., having SEQ ID NO: 2.

In an exemplary embodiment, a nucleic acid construct of the disclosure comprises (1) a promoter comprising (i) SEQ ID NO: 67, (ii) a sequence having at least 80%, 85%, 90%, 91%, 92%, 93%, 94%, 95%, 96%, 97%, 98%, 99% or 100% identity to SEQ ID NO: 67, or (iii) a functional fragment of any of the foregoing, operably linked to (2) a codon optimized variant ATP7B sequence comprising (i) SEQ ID NO: 5, (ii) a sequence having at least 80%, 85%, 90%, 91%, 92%, 93%, 94%, 95%, 96%, 97%, 98%, 99% or 100% identity to SEQ ID NO: 5, or (iii) a functional fragment of any of the foregoing. In certain embodiments, such codon optimized variant ATP7B nucleotide sequence encodes a protein having a sequence that is at least 90%, 95%, 98%, 99% or 100% identical to the wild type ATP7B protein sequence (e.g., SEQ ID NO: 2). In certain embodiments, such codon optimized variant ATP7B sequence encodes a full length ATP7B protein, e.g., having SEQ ID NO: 2.

In an exemplary embodiment, a nucleic acid construct of the disclosure comprises (1) a promoter comprising (i) SEQ ID NO: 68, (ii) a sequence having at least 80%, 85%, 90%, 91%, 92%, 93%, 94%, 95%, 96%, 97%, 98%, 99% or 100% identity to SEQ ID NO: 68, or (iii) a functional fragment of any of the foregoing, operably linked to (2) a codon optimized variant ATP7B sequence comprising (i) SEQ ID NO: 4, (ii) a sequence having at least 80%, 85%, 90%, 91%, 92%, 93%, 94%, 95%, 96%, 97%, 98%, 99% or 100% identity to SEQ ID NO: 4, or (iii) a functional fragment of any of the foregoing. In certain embodiments, such codon optimized variant ATP7B nucleotide sequence encodes a protein having a sequence that is at least 90%, 95%, 98%, 99% or 100% identical to the wild type ATP7B protein sequence (e.g., SEQ ID NO: 2). In certain embodiments, such codon optimized variant ATP7B sequence encodes a full length ATP7B protein, e.g., having SEQ ID NO: 2.

In an exemplary embodiment, a nucleic acid construct of the disclosure comprises (1) a promoter comprising (i) SEQ ID NO: 68, (ii) a sequence having at least 80%, 85%, 90%, 91%, 92%, 93%, 94%, 95%, 96%, 97%, 98%, 99% or 100% identity to SEQ ID NO: 68, or (iii) a functional fragment of any of the foregoing, operably linked to (2) a codon optimized variant ATP7B sequence comprising (i) SEQ ID NO: 5, (ii) a sequence having at least 80%, 85%, 90%, 91%, 92%, 93%, 94%, 95%, 96%, 97%, 98%, 99% or 100% identity to SEQ ID NO: 5, or (iii) a functional fragment of any of the foregoing. In certain embodiments, such codon optimized variant ATP7B nucleotide sequence encodes a protein having a sequence that is at least 90%, 95%, 98%, 99% or 100% identical to the wild type ATP7B protein sequence (e.g., SEQ ID NO: 2). In certain embodiments, such codon optimized variant ATP7B sequence encodes a full length ATP7B protein, e.g., having SEQ ID NO: 2.

In one embodiment, a nucleic acid construct disclosed herein comprises a promoter selected from the group consisting of: the thyroxine binding globulin (TBG) promoter, the modified thyroxine binding globulin (TBG-S1) promoter, the liver specific transthyretin (TTR) promoter, a modified transthyretin (mTTR) promoter, the alpha 1 anti-trypsin (A1AT) promoter, human albumin (humAlb) promoter, the liver specific promoter (LSP), and the hepatitis B core promoter (see e.g., WO 2018/126116, Miyatake et al., J. Virol. 71: 5124-32 (1997), and Sandig et al., Gene Ther. 3: 1002-9 (1996)), operably linked to a codon optimized variant ATP7B sequence comprising (i) any one of SEQ ID NOs: 3-6, (ii) a sequence having at least 60%, 65%, 70%, 75%, 80%, 85%, 90%, 91%, 92%, 93%, 94%, 95%, 96%, 97%, 98%, 99% or 100% identity to any one of SEQ ID NOs: 3-6, or (iii) a functional fragment of any of the foregoing. In certain embodiments, such codon optimized variant ATP7B nucleotide sequence encodes a protein having a sequence that is at least 90%, 95%, 98%, 99% or 100% identical to the wild type ATP7B protein sequence (e.g., SEQ ID NO: 2). In certain embodiments, such codon optimized variant ATP7B sequence encodes a full length ATP7B protein, e.g., having SEQ ID NO: 2.

In one embodiment, a nucleic acid construct disclosed herein comprises a promoter having any one of SEQ ID NOs: 21-43 or 66-68 operably linked to a variant ATP7B sequence comprising (i) any one of SEQ ID NOs: 7-18, (ii) a sequence having at least 60%, 65%, 70%, 75%, 80%, 85%, 90%, 91%, 92%, 93%, 94%, 95%, 96%, 97%, 98%, 99% or 100% identity to any one of SEQ ID NOs: 7-18, or (iii) a functional fragment of any of the foregoing. In certain embodiments, such variant ATP7B nucleotide sequence are codon optimized sequences that encode a protein having a sequence that is at least 90%, 95%, 98%, 99% or 100% identical to the wild type ATP7B protein sequence (e.g., SEQ ID NO: 2). In certain embodiments, such codon optimized variant ATP7B sequences may comprise (i) any one of SEQ ID NOs: 7, 8 or 15, or (ii) a sequence having at least 60%, 65%, 70%, 75%, 80%, 85%, 90%, 91%, 92%, 93%, 94%, 95%, 96%, 97%, 98%, 99% or 100% identity to any one of SEQ ID NOs: 7, 8 or 15, wherein such codon optimized variant ATP7B sequence encodes a full length ATP7B protein, e.g., having SEQ ID NO: 2. In certain embodiments, a nucleic acid construct disclosed herein comprises a codon optimized variant ATP7B nucleotide sequence that has been truncated so as to encode a functional fragment of an ATP7B protein. Exemplary codon optimized, truncated ATP7B nucleotide sequences may comprise (i) any one of SEQ ID NOs: 9-14 or 17, or (ii) a sequence having at least 60%, 65%, 70%, 75%, 80%, 85%, 90%, 91%, 92%, 93%, 94%, 95%, 96%, 97%, 98%, 99% or 100% identity to any one of SEQ ID NOs: 9-14, or 17, wherein such codon optimized, truncated variant ATP7B nucleotide sequence encodes a functional fragment of ATP7B. In certain embodiments, a nucleic acid construct disclosed herein comprises a variant ATP7B nucleotide sequence that has been truncated so as to encode a functional fragment of an ATP7B protein. Exemplary truncated ATP7B nucleotide sequences may comprise (i) any one of SEQ ID NOs: 16 or 18, or (ii) a sequence having at least 60%, 65%, 70%, 75%, 80%, 85%, 90%, 91%, 92%, 93%, 94%, 95%, 96%, 97%, 98%, 99% or 100% identity to any one of SEQ ID NOs: 16 or 18, wherein such truncated variant ATP7B nucleotide sequence encodes a functional fragment of ATP7B .

In another aspect, the application provides a nucleic acid construct comprising a regulatory element comprising a sequence having at least 80% sequence identity to any one of SEQ ID NOs: 66-68. In certain embodiments, such regulatory elements have at least 80%, 85%, 90%, 91%, 92%, 93%, 94%, 95%, 96%, 97%, 98%, 99% or 100% sequence identity to any one of SEQ ID NOs: 66-68. In certain embodiments, the regulatory element has less than 150 bp, 140 bp, 130 bp, 125 bp, 120 bp, 119 bp, 118 bp, 117 bp, 116 bp, 115 bp, 114 bp, 113 bp, 112 bp, 111 bp, 110 bp, 105 bp, 100 bp, 99 bp, 98 bp, 97 bp, 96 bp, 95 bp, 94 bp, 93 bp, 92 bp, 91 bp, 90 bp, 85 bp, 80 bp, or 75 bp. In certain embodiments, the regulatory element has from about 75-150 bp, 75-140 bp, 75-130 bp, 75-120 bp, 75-110 bp, 75-100 bp, 75-90 bp, 80-150 bp, 80-140 bp, 80-130 bp, 80-120 bp, 80-110 bp, 80-100 bp, 80-90 bp, 85-150 bp, 85-140 bp, 85-130 bp, 85-120 bp, 85-110 bp, 85-100 bp, 85-90 bp, 90-150 bp, 90-140 bp, 90-130 bp, 90-120 bp, 90-110 bp, 90-100 bp, 95-150 bp, 95-140 bp, 95-130 bp, 95-120 bp, 95-110 bp, 95-100 bp, 100-150 bp, 100-140 bp, 100-130 bp, 100-120 bp, or 100-110 bp. In certain embodiments, such regulatory element produces at least 5-fold, 10-fold, 15-fold, 20-fold, 25-fold, 30-fold, 35-fold, 40-fold, 45-fold, 50-fold, 55-fold, 60-fold, 65-fold, 70-fold, or 75-fold, or at least 20-90 fold, 20-80 fold, 20-70 fold, 20-60 fold, 30-90 fold, 30-80 fold, 30-70 fold, 30-60 fold, 40-90 fold, 40-80 fold, 40-70 fold, 40-60 fold, 50-90 fold, 50-80 fold, 50-70 fold, 50-60 fold, 60-90 fold, 60-80 fold, 60-70 fold, 70-90 fold, 70-80 fold, 80-90 fold greater expression of a transgene in a mammalian cell relative to the level of expression of the same transgene sequence from a control promoter, such as, for example, a CMV, mTTR or alpha 1 anti-trypsin promoter. In certain embodiments, such regulatory elements drive expression of a transgene sequence in a high percentage of hepatocyte cells, e.g., at least 20%, 25%, 30%, 40%, 45%, 50%, 55%, 60%, 65%, 70%, 75%, 80%, 81%, 82%, 83%, 84%, 85%, 86%, 87%, 88%, 89%, 90%, 91%, 92%, 93%, 94%, 95%, 96%, 97%, 98% or greater, or at least 20-90%, 20-80%, 20-70%, 30-90%, 30-80%, 30-70%, 40-90%, 40-80%, 40-70%, 50-90%, 50-80%, 50-70%, 60-90%, 60-80%, 60-70%, 70-90%, 70-80%, 80-100%, 80-95%, 80-90%, 90-100%, or 90-95% of hepatocytes containing the nucleic acid construct express the transgene sequence. In certain embodiments, such regulatory elements are operably linked to a therapeutic transgene, such as, for example, ATPase Copper Transporting Alpha (ATP7A), ATPase Copper Transporting Beta (ATP7B), ATPase Phospholipid Transporting 8B1 (ATP8B1), ATP Binding Cassette Subfamily B Member 4 (ABCB4), ATP Binding Cassette Subfamily B Member 4 (ABCB11), Cyclin Dependent Kinase Like 5 (CDKL5), Contactin Associated Protein Like 2 (CNTNAP2), Zinc Finger E-Box Binding Homeobox 2 (ZEB2), Factor V, Factor VII, Factor VIII, Factor IX, Factor X, Factor XI or Factor XII, or a variant or functional fragment thereof. In certain embodiments, the therapeutic transgene encodes Factor VIII or a variant or functional fragment thereof. In certain embodiments, the therapeutic transgene encodes ATP7B or a variant or functional fragment thereof. In certain embodiments, the therapeutic transgene has been codon optimized for expression in a particular cell or tissue type, such as, for example, liver.

In certain embodiments, the nucleic acid constructs described herein comprise another regulatory element in an addition to a promoter, such as, for example, sequences associated with transcription initiation or termination, enhancer sequences, and efficient RNA processing signals. Exemplary regulatory elements include, for example, an intron, an enhancer, UTR, stability element, WPRE sequence, a Kozak consensus sequence, posttranslational response element, or a polyadenylation (polyA) sequence, or a combination thereof. Regulatory elements can function to modulate gene expression at the transcriptional phase, post-transcriptional phase, or at the translational phase of gene expression. At the RNA level, regulation can occur at the level of translation (e.g., stability elements that stabilize mRNA for translation), RNA cleavage, RNA splicing, and/or transcriptional termination. In various embodiments, regulatory elements can recruit transcription factors to a coding region that increase gene expression selectivity in a cell type of interest, increase the rate at which RNA transcripts are produced, increase the stability of RNA produced, and/or increase the rate of protein synthesis from RNA transcripts.

In one embodiment, the nucleic acid constructs described herein further comprise an enhancer sequence. Exemplary enhancer sequences include, for example, the En34 enhancer (34 bp core enhancer from the human apolipoprotein hepative control region, the EnTTR enhancer (100 bp enhancer sequence from transthyretin), the α1-microglobulin/bikunin precursor enhancer, the ABPS enhancer (shortened version of the 100 bp distal enhancer from the α1-microglobulin/bikunin precursor to 42 bp), or the ApoE enhancer. See e.g., WO 2018/126116 and Wu et al., Mol Therapy 16(2): 280-289 (2008)). In another embodiment, a suitable enhancer sequence is an intronic sequence comprising SEQ ID NO: 19 or SEQ ID NO: 20. In certain embodiments, an enhancer sequence is positioned upstream of the transgene and the promoter, or between the promoter and the transgene in the nucleic acid constructs described herein.

In certain embodiments, the nucleic acid constructs described herein further comprise a polyA sequence. Suitable polyA sequences include, for example, an artificial polyA that is about 75 bp in length (PA75) (see e.g., WO 2018/126116), the bovine growth hormone polyA, SV40 early polyA signal, SV40 late polyA signal, rabbit beta globin polyA, HSV thymidine kinase polyA, protamine gene polyA, adenovirus 5 EIb polyA, growth hormone polyA, or a PBGD polyA. In certain embodiments, the polyA sequence is positioned downstream of the transgene in the nucleic acid constructs described herein.

In certain embodiments, a regulatory element suitable for use in accordance with the nucleic acid molecules described herein comprises less than 500 bp, 450 bp, 400 bp, 350 bp, 300 bp, 250 bp, 225 bp, 200 bp, 175 bp, 150 bp, 145 bp, 140 bp, 135 bp, 130 bp, 125 bp, 120 bp, 115 bp, 110 bp, 105 bp, 100 bp, 95 bp, 90 bp, 85 bp, 80 bp or 75 bp, or from about 80-300 bp, 80-275 bp, 80-250 bp, 80-200 bp, 80-150 bp, 80-125 bp, 80-120 bp, 80-115 bp, 80-110 bp, 80-105 bp, 80-100 bp, 85-300 bp, 85-275 bp, 85-250 bp, 85-200 bp, 85-150 bp, 85-125 bp, 85-120 bp, 85-115 bp, 85-110 bp, 85-105 bp, 85-100 bp, 90-300 bp, 90-275 bp, 90-250 bp, 90-200 bp, 90-150 bp, 90-125 bp, 90-120 bp, 90-115 bp, 90-110 bp, 90-105 bp, 90-100 bp, 95-300 bp, 95-275 bp, 95-250 bp, 95-200 bp, 95-150 bp, 95-125 bp, 95-120 bp, 95-115 bp, 95-110 bp, 95-105 bp, 95-100 bp, 100-300 bp, 100-275 bp, 100-250 bp, 100-200 bp, 100-150 bp, 100-125 bp, 100-120 bp, 100-115 bp, 100-110 bp, or 100-105 bp. In exemplary embodiments, a regulatory element suitable for use in accordance with the nucleic acid molecules described herein comprises from about 100-120 bp, about 117 bp, or about 100 bp.

In certain embodiments, a nucleic acid construct described herein comprising an ATP7B nucleic acid sequence and a regulatory element is suitable for packaging in an AAV vector, e.g., comprising less than ~4.7 Kb. In certain embodiments, a nucleic acid construct described herein comprising an ATP7B nucleic acid sequence and a regulatory element comprising from about 4,450-4,550 bp, 4,450-4,540 bp, 4,450-4,530 bp, 4,450-4,520 bp, 4,450-4,510 bp, 4,450-4,500 bp, 4,460-4,550 bp, 4,460-4,540 bp, 4,460-4,530 bp, 4,460-4,520 bp, 4,460-4,510 bp, 4,460-4,500 bp, 4,470-4,550 bp, 4,470-4,540 bp, 4,470-4,530 bp, 4,470-4,520 bp, 4,470-4,510 bp, 4,470-4,500 bp, 4,480-4,550 bp, 4,480-4,540 bp, 4,480-4,530 bp, 4,480-4,520 bp, 4,480-4,510 bp, 4,480-4,500 bp, 4,490-4,550 bp, 4,490-4,540 bp, 4,490-4,530 bp, 4,490-4,520 bp, 4,490-4,510 bp, or 4,490-4,500 bp, or comprises about 4,498 bp or about 4,515 bp. In exemplary embodiments, such nucleic acid constructs encode a full length ATP7B protein, e.g., an ATP7B protein having SEQ ID NO: 2.

### Expression Vectors

In certain embodiments, the variant ATP7B nucleotide sequences or expression constructs described herein may be incorporated into an expression vector.

Expression vectors may be used to deliver the nucleic acid molecule to a target cell via transfection or transduction. A vector may be an integrating or non-integrating vector, referring to the ability of the vector to integrate the expression cassette or transgene into the genome of the host cell. Examples of expression vectors include, but are not limited to, (a) non-viral vectors such as nucleic acid vectors including linear oligonucleotides and circular plasmids; artificial chromosomes such as human artificial chromosomes (HACs), yeast artificial chromosomes (YACs), and bacterial artificial chromosomes (BACs or PACs)); episomal vectors; transposons (e.g., PiggyBac); and (b) viral vectors such as retroviral vectors, lentiviral vectors, adenoviral vectors, and adeno-associated viral vectors.

Expression vectors may be linear oligonucleotides or circular plasmids and can be delivered to a cell via various transfection methods, including physical and chemical methods. Physical methods generally refer to methods of delivery employing a physical force to counteract the cell membrane barrier in facilitating intracellular delivery of genetic material. Examples of physical methods include the use of a needle, ballistic DNA, electroporation, sonoporation, photoporation, magnetofection, and hydroporation. Chemical methods generally refer to methods in which chemical carriers deliver a nucleic acid molecule to a cell and may include inorganic particles, lipid-based vectors, polymer-based vectors and peptide-based vectors.

In some embodiments, an expression vector is administered to a target cell using an inorganic particle. Inorganic particles may refer to nanoparticles, such as nanoparticles that are engineered for various sizes, shapes, and/or porosity to escape from the reticuloendothelial system or to protect an entrapped molecule from degradation. Inorganic nanoparticles can be prepared from metals (e.g., iron, gold, and silver), inorganic salts, or ceramics (e.g, phosphate or carbonate salts of calcium, magnesium, or silicon). The surface of these nanoparticles can be coated to facilitate DNA binding or targeted gene delivery. Magnetic nanoparticles (e.g., supermagnetic iron oxide), fullerenes (e.g., soluble carbon molecules), carbon nanotubes (e.g., cylindrical fullerenes), quantum dots and supramolecular systems may also be used.

In some embodiments, an expression vector is administered to a target cell using a cationic lipid (e.g., cationic liposome). Various types of lipids have been investigated for gene delivery, such as, for example, a lipid nano emulsion (e.g., which is a dispersion of one immiscible liquid in another stabilized by emulsifying agent) or a solid lipid nanoparticle.

In some embodiments, an expression vector is administered to a target cell using a peptide based delivery vehicle. Peptide based delivery vehicles can have advantages of protecting the genetic material to be delivered, targeting specific cell receptors, disrupting endosomal membranes and delivering genetic material into a nucleus. In some embodiments, an expression vector is administered to a target cell using a polymer based delivery vehicle. Polymer based delivery vehicles may comprise natural proteins, peptides and/or polysaccharides or synthetic polymers. In one embodiment, a polymer based delivery vehicle comprises polyethylenimine (PEI). PEI can condense DNA into positively charged particles which bind to anionic cell surface residues and are brought into the cell via endocytosis. In other embodiments, a polymer based delivery vehicle may comprise poly-L-lysine (PLL), poly (DL-lactic acid) (PLA), poly (DL-lactide-co-glycoside) (PLGA), polyornithine, polyarginine, histones, protamines, dendrimers, chitosans, synthetic amino derivatives of dextran, and/or cationic acrylic polymers. In certain embodiments, polymer based delivery vehicles may comprise a mixture of polymers, such as, for example PEG and PLL.

In certain embodiments, an expression vector may be a viral vector suitable for gene therapy. Preferred characteristics of viral gene therapy vectors or gene delivery vectors may include the ability to be reproducibly and stably propagated and purified to high titres; to mediate targeted delivery (e.g., to deliver the transgene specifically to the tissue or organ of interest without widespread vector dissemination elsewhere); and to mediate gene delivery and transgene expression without inducing harmful side effects.

Several types of viruses, for example the non-pathogenic parvovirus referred to as adeno-associated virus, have been engineered for the purposes of gene therapy by harnessing the viral infection pathway but avoiding the subsequent expression of viral genes that can lead to replication and toxicity. Such viral vectors can be obtained by deleting all, or some, of the coding regions from the viral genome, but leaving intact those sequences (e.g., terminal repeat sequences) that may be necessary for functions such as packaging the vector genome into the virus capsid or the integration of vector nucleic acid (e.g., DNA) into the host chromatin.

In various embodiments, suitable viral vectors include retroviruses (e.g., A-type, B-type, C-type, and D-type viruses), adenovirus, parvovirus (e.g. adeno-associated viruses or AAV), coronavirus, negative strand RNA viruses such as orthomyxovirus (e.g., influenza virus), rhabdovirus (e.g., rabies and vesicular stomatitis virus), paramyxovirus (e. g. measles and Sendai), positive strand RNA viruses such as picornavirus and alphavirus, and doublestranded DNA viruses including adenovirus, herpesvirus (e.g., Herpes Simplex virus types 1 and 2, Epstein-Barr virus, cytomegalovirus), and poxvirus (e.g., vaccinia, fowlpox and canarypox). Examples of retroviruses include avian leukosis-sarcoma virus, human T-lymphotrophic virus type 1 (HTLV-1), bovine leukemia virus (BLV), lentivirus, and spumavirus. Other viruses include Norwalk virus, togavirus, flavivirus, reoviruses, papovavirus, hepadnavirus, and hepatitis virus, for example. Viral vectors may be classified into two groups according to their ability to integrate into the host genome - integrating and non-integrating. Oncoretroviruses and lentiviruses can integrate into host cellular chromatin while adenoviruses, adeno-associated viruses, and herpes viruses predominantly persist in the cell nucleus as extrachromosomal episomes.

In certain embodiments, a suitable viral vector is a retroviral vector. Retroviruses refer to viruses of the family Retroviridae. Examples of retroviruses include oncoretroviruses, such as murine leukemia virus (MLV), and lentiviruses, such as human immunodeficiency virus 1 (HIV-1). Retroviral genomes are single-stranded (ss) RNAs and comprise various genes that may be provided in cis or trans. For example, retroviral genome may contain cis-acting sequences such as two long terminal repeats (LTR), with elements for gene expression, reverse transcription and integration into the host chromosomes. Other components include the packaging signal (psi or ψ), for the specific RNA packaging into newly formed virions and the polypurine tract (PPT), the site of the initiation of the positive strand DNA synthesis during reverse transcription. In addition, the retroviral genome may comprise gag, pol and env genes. The gag gene encodes the structural proteins, the pol gene encodes the enzymes that accompany the ssRNA and carry out reverse transcription of the viral RNA to DNA, and the env gene encodes the viral envelope. Generally, the gag, pol and env are provided in trans for viral replication and packaging.

In certain embodiments, a retroviral vector provided herein may be a lentiviral vector. At least five serogroups or serotypes of lentiviruses are recognized. Viruses of the different serotypes may differentially infect certain cell types and/or hosts. Lentiviruses, for example, include primate retroviruses and non-primate retroviruses. Primate retroviruses include HIV and simian immunodeficiency virus (SIV). Non-primate retroviruses include feline immunodeficiency virus (FIV), bovine immunodeficiency virus (BIV), caprine arthritis-encephalitis virus (CAEV), equine infectious anemia virus (EIAV) and visnavirus. Lentiviruses or lentivectors may be capable of transducing quiescent cells. As with oncoretrovirus vectors, the design of lentivectors may be based on the separation of cis- and trans-acting sequences.

In certain embodiments, the application provides expression vectors that have been designed for delivery by an optimized therapeutic retroviral vector. The retroviral vector can be a lentivirus comprising a left (5') LTR; sequences which aid packaging and/or nuclear import of the virus; a promoter; optionally one or more additional regulatory elements (such as, for example, an enhancer or polyA sequence); optionally a lentiviral reverse response element (RRE); a variant ATP7B transgene; optionally an insulator; and a right (3') retroviral LTR.

In exemplary embodiments, a viral vector provided herein is an adeno-associated virus (AAV). AAV is a small, replication-defective, non-enveloped animal virus that infects humans and some other primate species. AAV is not known to cause human disease and induces a mild immune response. AAV vectors can also infect both dividing and quiescent cells without integrating into the host cell genome.

The AAV genome consists of a linear single stranded DNA which is ~4.7kb in length. The genome consists of two open reading frames (ORF) flanked by an inverted terminal repeat (ITR) sequence that is about 145bp in length. The ITR consists of a nucleotide sequence at the 5' end (5' ITR) and a nucleotide sequence located at the 3' end (3' ITR) that contain palindromic sequences. The ITRs function in cis by folding over to form T-shaped hairpin structures by complementary base pairing that function as primers during initiation of DNA replication for second strand synthesis. The two open reading frames encode for rep and cap genes that are involved in replication and packaging of the virion. In an exemplary embodiment, an AAV vector provided herein does not contain the rep or cap genes. Such genes may be provided in trans for producing virons as described further below.

In certain embodiments, an AAV vector may include a stuffer nucleic acid. In some embodiments, the stuffer nucleic acid may encode a green fluorescent protein or antibiotic resistance gene such as kanamycin or ampicillin. In certain embodiments, the stuffer nucleic acid may be located outside of the ITR sequences (e.g., as compared to the variant ATPB transgene sequence and regulatory sequences, which are located between the 5' and 3' ITR sequences).

Various serotypes of AAV exist, including AAV1, AAV2, AAV3, AAV4, AAV5, AAV6, AAV7, AAV8, AAV9, AAV10, AAV11, AAV12, and AAV13. These serotypes differ in their tropism, or the types of cells they infect. AAVs may comprise the genome and capsids from multiple serotypes (e.g., pseudotypes). For example, an AAV may comprise the genome of serotype 2 (e.g., ITRs) packaged in the capsid from serotype 5 or serotype 8. Pseudotypes may improve transduction efficiency as well as alter tropism.

In some embodiments, an AAV vector or an AAV viral particle, or virion, may be used to deliver a variant ATP7B transgene into a cell, cell type, or tissue, and may done either *in vivo*, *ex vivo*, or *in vitro.* In exemplary embodiments, such an AAV vector is replication-deficient. In some embodiments, an AAV virus is engineered or genetically modified so that it can replicate and generate virions only in the presence of helper factors.

In exemplary embodiments, the application provides expression vectors that have been designed for delivery by an AAV. The AAV can be any serotype, for examples, AAV1, AAV2, AAV3, AAV3b, AAV4, AAV5, AAV6, AAV7, AAV8, AAV9, AAV10, AAV11, AAV12, AAV-DJ, or a chimeric, hybrid, or variant AAV. The AAV can also be a self-complementary AAV (scAAV). In certain embodiments, an expression vector designed for delivery by an AAV comprises a 5' ITR and a 3' ITR. In certain embodiments, an expression vector designed for delivery by an AAV comprises a 5' ITR, a promoter, an ATP7B transgene, and a 3' ITR. In certain embodiments, an expression vector designed for delivery by an AAV comprises a 5' ITR, an enhancer, a promoter, an ATP7B transgene, a polyA sequence, and a 3' ITR. In exemplary embodiments, an expression vector designed for delivery by an AAV comprises a 5' ITR, a promoter comprising any one of SEQ ID NOs: 21-55 or a variant or functional fragment thereof, an ATP7B transgene comprising any one of SEQ ID NOs: 1-18 or a variant or functional fragment thereof, and a 3' ITR. In one embodiment, an expression vector designed for delivery by an AAV comprises a 5' ITR, a promoter comprising any one of SEQ ID NOs: 21-55 or a variant or functional fragment thereof, an ATP7B transgene comprising any one of SEQ ID NOs: 3-6 or a variant or functional fragment thereof, and a 3' ITR. In another embodiment, an expression vector designed for delivery by an AAV comprises a 5' ITR, a promoter comprising any one of SEQ ID NOs: 15-17 or a variant or functional fragment thereof, an ATP7B transgene comprising any one of SEQ ID NOs: 4 or 5, and a 3' ITR. In another embodiment, an expression vector designed for delivery by an AAV comprises a 5' ITR, a promoter comprising any one of SEQ ID NOs: 21-47 or a variant or functional fragment thereof, an ATP7B transgene comprising any one of SEQ ID NOs: 7-18, and a 3' ITR. In another embodiment, an expression vector designed for delivery by an AAV comprises a 5' ITR, a promoter comprising any one of SEQ ID NOs: 23-43 or a variant or functional fragment thereof, an ATP7B transgene comprising any one of SEQ ID NOs: 7, 8 or 15, and a 3' ITR.

### Host Cells

In another aspect, the invention relates to a host cell comprising a nucleic acid molecule or expression vector comprising a variant ATP7B sequence of the invention. Host cells may be a bacterial cell, a yeast cell, an insect cell or a mammalian cell. In an exemplary embodiment, a host cell refers to any cell line that is susceptible to infection by a virus of interest, and amenable to culture *in vitro.*

In certain embodiments, a host cell provided herein may be used for *ex vivo* gene therapy purposes. In such embodiments, the cells are transfected with a nucleic acid molecule or expression vector comprising a variant ATP7B sequence of the invention and subsequently transplanted into the patient or subject. Transplanted cells can have an autologous, allogenic or heterologous origin. For clinical use, cell isolation will generally be carried out under Good Manufacturing Practices (GMP) conditions. Before transplantation, cell quality and absence of microbial or other contaminants is typically checked and liver preconditioning, such as with radiation and/or an immunosuppressive treatment, may be carried out. Furthermore, the host cells may be transplanted together with growth factors to stimulate cell proliferation and/or differentiation, such as Hepatocyte Growth Factor (HGF).

In certain embodiments, a host cell may be used for *ex vivo* gene therapy into the liver. Preferably, said cells are eukaryotic cells such as mammalian cells, these include, but are not limited to, humans, non-human primates such as apes; chimpanzees; monkeys, and orangutans, domesticated animals, including dogs and cats, as well as livestock such as horses, cattle, pigs, sheep, and goats, or other mammalian species including, without limitation, mice, rats, guinea pigs, rabbits, hamsters, and the like. A person skilled in the art will choose the more appropriate cells according to the patient or subject to be transplanted.

In certain embodiments, a host cell provided herein may be a cell with self-renewal and pluripotency properties, such as stem cells or induced pluripotent stem cells. Stem cells are preferably mesenchymal stem cells. Mesenchymal stem cells (MSCs) are capable of differentiating into at least one of an osteoblast, a chondrocyte, an adipocyte, or a myocyte and may be isolated from any type of tissue. Generally MSCs will be isolated from bone marrow, adipose tissue, umbilical cord, or peripheral blood. Methods for obtaining thereof are well known to a person skilled in the art. Induced pluripotent stem cells (also known as iPS cells or iPSCs) are a type of pluripotent stem cell that can be generated directly from adult cells. Yamanaka et al. induced iPS cells by transferring the Oct3/4, Sox2, Klf4 and c-Myc genes into mouse and human fibroblasts, and forcing the cells to express the genes (WO 2007/069666). Thomson et al. subsequently produced human iPS cells using Nanog and Lin28 in place of Klf4 and c-Myc (WO 2008/118820).

In certain embodiments, a host cell provided herein may be a hepatocyte. Hepatocyte transplantation procedures, including cell isolation and subsequent transplantation into a human or mice recipient is described for instance in Filippi and Dhawan, Ann NY Acad Sci. 2014, 1315 50-55; Yoshida et al., Gastroenterology 1996, 111: 1654-1660; Irani et al. Molecular Therapy 2001, 3:3, 302-309; and Vogel et al. J Inherit Metab Dis 2014, 37:165-176. A method for *ex vivo* transduction of a viral vector into hepatocytes is described for instance in Merle et al., Scandinavian Journal of Gastroenterology 2006, 41:8, 974-982.

In an exemplary embodiment, a host cell provided herein is a packaging cell. Said cells can be adherent or suspension cells. The packaging cell, and helper vector or virus or DNA construct(s) provide together in trans all the missing functions which are required for the complete replication and packaging of the viral vector.

Preferably, said packaging cells are eukaryotic cells such as mammalian cells, including simian, human, dog and rodent cells. Examples of human cells are PER.C6 cells (WO01/38362), MRC-5 (ATCC CCL-171), WI-38 (ATCC CCL-75), HEK-293 cells (ATCC CRL-1573), HeLa cells (ATCC CCL2), and fetal rhesus lung cells (ATCC CL-160). Examples of non-human primate cells are Vero cells (ATCC CCL81), COS-1 cells (ATCC CRL-1650) or COS-7 cells (ATCC CRL-1651). Examples of dog cells are MDCK cells (ATCC CCL-34). Examples of rodent cells are hamster cells, such as BHK21-F, HKCC cells, or CHO cells.

As an alternative to mammalian sources, cell lines for use in the invention may be derived from avian sources such as chicken, duck, goose, quail or pheasant. Examples of avian cell lines include avian embryonic stem cells (WO01/85938 and WO03/076601), immortalized duck retina cells (WO2005/042728), and avian embryonic stem cell derived cells, including chicken cells (WO2006/108846) or duck cells, such as EB66 cell line (WO2008/129058 & WO2008/142124).

In another embodiment, said host cell are insect cells, such as SF9 cells (ATCC CRL-1711), Sf21 cells (IPLB-Sf21), MG1 cells (BTI-TN-MG1) or High Five^{™} cells (BTI-TN-5B1-4).

In certain embodiments, the host cells provided herein comprising a nucleic acid construct (e.g., a plasmid) carrying the recombinant AAV vector/genome of the invention (e.g., comprising a variant ATP7B nucleic acid sequence) may further comprise one or more additional nucleic acid constructs, such as, for example (i) a nucleic acid construct (e.g., an AAV helper plasmid) that encodes rep and cap genes, but does not carry ITR sequences; and/or (ii) a nucleic acid construct (e.g., a plasmid) providing the adenoviral functions necessary for AAV replication. In an exemplary embodiment, a host cell provided herein comprises: i) a nucleic acid construct or an expression vector comprising a variant ATP7B sequence of the invention (i.e., the recombinant AAV genome); ii) a nucleic acid construct encoding AAV rep and cap genes which does not carry the ITR sequences; and iii) a nucleic acid construct comprising adenoviral helper genes (as described further below).

In certain embodiments, the rep, cap, and adenoviral helper genes can be combined on a single plasmid (Blouin V et al. J Gene Med. 2004; 6(suppl): S223-S228; Grimm D. et al. Hum. Gene Ther. 2003; 7: 839-850). Thus, in another exemplary embodiment, a host cell provided herein comprises: i) a nucleic acid molecule or an expression vector comprising a variant ATP7B sequence of the invention (i.e., the recombinant AAV genome); and ii) a plasmid encoding AAV rep and cap genes which does not carry the ITR sequences and further comprising adenoviral helper genes.

In another embodiment, a host cell provided herein comprises: a) a nucleic acid construct or an expression vector comprising a variant ATP7B sequence of the invention (i.e., the recombinant AAV genome); b) a plasmid encoding AAV rep and cap genes which does not carry the ITR sequences; and c) a plasmid comprising adenoviral helper genes E2a, E4, and VA RNAs; wherein co-transfection is performed in cells, preferably mammalian cells, that constitutively express and transcomplement the adenoviral E1 gene, like HEK-293 cells (ATCC CRL-1573).

In certain embodiments, a host cell suitable for large-scale production of AAV vectors is an insect cells that can be infected with a combination of recombinant baculoviruses (Urabe et al. Hum. Gene Ther. 2002; 13: 1935-1943). For example, SF9 cells may be co-infected with three baculovirus vectors respectively expressing AAV rep, AAV cap and the AAV vector to be packaged. The recombinant baculovirus vectors will provide the viral helper gene functions required for virus replication and/or packaging.

Further guidance for the construction and production of virions for gene therapy according to the invention can be found in: Viral Vectors for Gene Therapy, Methods and Protocols. Series: Methods in Molecular Biology, Vol. 737. Merten and Al-Rubeai (Eds.); 2011 Humana Press (Springer); Gene Therapy. M. Giacca. 2010 Springer-Verlag; Heilbronn R. and Weger S. Viral Vectors for Gene Transfer: Current Status of Gene Therapeutics. In: Drug Delivery, Handbook of Experimental Pharmacology 197; M. Schafer-Korting (Ed.). 2010 Springer-Verlag; pp. 143-170; Adeno-Associated Virus: Methods and Protocols. R. O. Snyder and P. Moulllier (Eds). 2011 Humana Press (Springer); Bunning H. et al. Recent developments in adeno-associated virus technology. J. Gene Med. 2008; 10:717-733; and Adenovirus: Methods and Protocols. M. Chillon and A. Bosch (Eds.); Third. Edition. 2014 Humana Press (Springer).

### Virions & Methods of Producing Virions

In certain embodiments, the application provides viral particles comprising a viral vector comprising a variant ATP7B sequence of the invention. The terms "viral particle", and "virion" are used herein interchangeably and relate to an infectious and typically replication-defective virus particle comprising the viral genome (e.g., the viral expression vector) packaged within a capsid and, as the case may be e.g., for retroviruses, a lipidic envelope surrounding the capsid. A "capsid" refers to the structure in which the viral genome is packaged. A capsid consists of several oligomeric structural subunits made of proteins. For example, AAV have an icosahedral capsid formed by the interaction of three capsid proteins: VP1, VP2 and VP3. In one embodiment, a virion provided herein is a recombinant AAV virion or rAAV virion obtained by packaging an AAV vector comprising a variant ATP7B sequence as described herein in a protein shell.

In certain embodiments, a recombinant AAV virion provided herein may be prepared by encapsidating an AAV genome derived from a particular AAV serotype in a viral particle formed by natural Cap proteins corresponding to an AAV of the same particular serotype. In other embodiments, an AAV viral particle provided herein comprises a viral vector comprising ITR(s) of a given AAV serotype packaged into proteins from a different serotype. See e.g., Bunning H et al. J Gene Med 2008; 10: 717-733. For example, a viral vector having ITRs from a given AAV serotype may be package into: a) a viral particle constituted of capsid proteins derived from a same or different AAV serotype (e.g. AAV2 ITRs and AAV5 capsid proteins; AAV2 ITRs and AAV8 capsid proteins; etc); b) a mosaic viral particle constituted of a mixture of capsid proteins from different AAV serotypes or mutants (e.g. AAV2 ITRs with AAV1 and AAV5 capsid proteins); c) a chimeric viral particle constituted of capsid proteins that have been truncated by domain swapping between different AAV serotypes or variants (e.g. AAV2 ITRs with AAV5 capsid proteins with AAV3 domains); or d) a targeted viral particle engineered to display selective binding domains, enabling stringent interaction with target cell specific receptors (e.g. AAV4 ITRs with AAV2 capsid proteins genetically truncated by insertion of a peptide ligand; or AAV2 capsid proteins non-genetically modified by coupling of a peptide ligand to the capsid surface).

The skilled person will appreciate that an AAV virion provided herein may comprise capsid proteins of any AAV serotype. In one embodiment, the viral particle comprises capsid proteins from an AAV serotype selected from the group consisting of an AAV1, an AAV5, an AAV7, an AAV8, and an AAV9, which are more suitable for delivery to the liver cells (Nathwani et al. Blood 2007; 109: 1414-1421; Kitajima et al. Atherosclerosis 2006; 186:65-73). In a particular embodiment, the viral particle comprises a nucleic acid construct of the invention wherein the 5'ITR and 3'ITR sequences of the nucleic acid construct are of an AAV2 serotype and the capsid proteins are of an AAV8 serotype.

Numerous methods are known in the art for production of rAAV virions, including transfection, stable cell line production, and infectious hybrid virus production systems which include adenovirus-AAV hybrids, herpesvirus-AAV hybrids (Conway, J E et al., (1997) J. Virology 71(11):8780-8789) and baculovirus-AAV hybrids. rAAV production cultures for the production of rAAV virus particles all require; 1) suitable host cells, including, for example, human-derived cell lines such as HeLa, A549, or 293 cells, or insect-derived cell lines such as SF-9, in the case of baculovirus production systems; 2) suitable helper virus function, provided by wild-type or mutant adenovirus (such as temperature sensitive adenovirus), herpes virus, baculovirus, or a plasmid construct providing helper functions; 3) AAV rep and cap genes and gene products; 4) a transgene (e.g., a variant ATP7B sequence as described herein) flanked by AAV ITR sequences; and 5) suitable media and media components to support rAAV production.

In various embodiments, the host cells described herein comprise the following three components: (1) a rep gene and a cap gene, (2) genes providing helper functions, and (3) a transgene (e.g., a variant ATP7B sequence disclosed herein under the control of a promoter and flanked by ITRs). The AAV rep gene, AAV cap gene, and genes providing helper functions can be introduced into the cell by incorporating said genes into a vector such as, for example, a plasmid, and introducing said vector into the host cell. The rep, cap and helper function genes can be incorporated into the same plasmid or into different plasmids. In a preferred embodiment, the AAV rep and cap genes are incorporated into one plasmid and the genes providing helper functions are incorporated into another plasmid. The various plasmids for creation of a host cell for virion production (e.g., comprising AAV rep and cap genes, helper functions, or a transgene) can be introduced into the cell by using any suitable method well known in the art. Examples of transfection methods include, but are not limited to, co-precipitation with calcium phosphate, DEAE-dextran, polybrene, electroporation, microinjection, liposome-mediated fusion, lipofection, retrovirus infection and biolistic transfection. In certain embodiments, the plasmids providing the rep and cap genes, the helper functions and the transgene (e.g., a variant ATP7B sequence disclosed herein under the control of a promoter and flanked by ITRs) can be introduced into the cell simultaneously. In another embodiment, the plasmids providing the rep and cap genes and the helper functions can be introduced in the cell before or after the introduction of plasmid comprising the transgene. In an exemplary embodiment, the cells are transfected simultaneously with three plasmids (e.g., a triple transfection method): (1) a plasmid comprising the transgene (e.g., a variant ATP7B sequence disclosed herein under the control of a promoter and flanked by ITRs), (2) a plasmid comprising the AAV rep and cap genes, and (3) a plasmid comprising the genes providing the helper functions. Exemplary host cells may be 293, A549 or HeLa cells.

In other embodiments, one or more of (1) the AAV rep and cap genes, (2) genes providing helper functions, and (3) the transgene, may be carried by the packaging cell, either episomally and/or integrated into the genome of the packaging cell. In one embodiment, host cells may be packaging cells in which the AAV rep and cap genes and helper functions are stably maintained in the host cell and the host cell is transiently transfected with a plasmid containing a transgene (e.g., a variant ATP7B sequence disclosed herein under the control of a promoter and flanked by ITRs). In another embodiment, host cells are packaging cells in which the AAV rep and cap genes are stably maintained in the host cell and the host cell is transiently transfected with a plasmid containing a transgene (e.g., a variant ATP7B sequence disclosed herein under the control of a promoter and flanked by ITRs) and a plasmid containing the helper functions. In another embodiment, host cells may be packaging cells in which the helper functions are stably maintained in the host cell and the host cell is transiently transfected with a plasmid containing a transgene (e.g., a variant ATP7B sequence disclosed herein under the control of a promoter and flanked by ITRs) and a plasmid containing rep and cap genes. In another embodiment, host cells may be producer cell lines that are stably transfected with rep and cap genes, helper functions and the transgene sequence (e.g., a variant ATP7B sequence disclosed herein under the control of a promoter and flanked by ITRs). Exemplary packaging and producer cells may be derived from 293, A549 or HeLa cells.

In another embodiment, the producer cell line is an insect cell line (typically Sf9 cells) that is infected with baculovirus expression vectors that provide Rep and Cap proteins. This system does not require adenovirus helper genes (Ayuso E, et al., Curr. Gene Ther. 2010, 10:423-436).

The term "cap protein", as used herein, refers to a polypeptide having at least one functional activity of a native AAV Cap protein (e.g. VP1, VP2, VP3). Examples of functional activities of cap proteins include the ability to induce formation of a capsid, facilitate accumulation of single-stranded DNA, facilitate AAV DNA packaging into capsids (i.e. encapsidation), bind to cellular receptors, and facilitate entry of the virion into host cells. In principle, any Cap protein can be used in the context of the present invention.

Cap proteins have been reported to have effects on host tropism, cell, tissue, or organ specificity, receptor usage, infection efficiency, and immunogenicity of AAV viruses. Accordingly, an AAV cap for use in an rAAV may be selected taking into consideration, for example, the subject's species (e.g. human or non-human), the subject's immunological state, the subject's suitability for long or short-term treatment, or a particular therapeutic application (e.g. treatment of a particular disease or disorder, or delivery to particular cells, tissues, or organs). In certain embodiments, the cap protein is derived from the AAV of the group consisting of AAV2, AAV5, AAV7, AAV8, AAV9, AAV10 and AAVrh10 serotypes. In an exemplary embodiment, the cap protein is derived from AAV8.

In some embodiments, an AAV Cap for use in the method of the invention can be generated by mutagenesis (i.e. by insertions, deletions, or substitutions) of one of the aforementioned AAV caps or its encoding nucleic acid. In some embodiments, the AAV cap is at least 70%, 75%, 80%, 85%, 90%, 95%, 98%, or 99% or more similar to one or more of the aforementioned AAV caps.

In some embodiments, the AAV cap is chimeric, comprising domains from two, three, four, or more of the aforementioned AAV caps. In some embodiments, the AAV cap is a mosaic of VP1, VP2, and VP3 monomers originating from two or three different AAV or a recombinant AAV. In some embodiments, a rAAV composition comprises more than one of the aforementioned caps.

In some embodiments, an AAV cap for use in a rAAV virion is engineered to contain a heterologous sequence or other modification. For example, a peptide or protein sequence that confers selective targeting or immune evasion may be engineered into a cap protein. Alternatively or in addition, the cap may be chemically modified so that the surface of the rAAV is polyethylene glycolated (i.e. pegylated), which may facilitate immune evasion. The cap protein may also be mutagenized (e.g. to remove its natural receptor binding, or to mask an immunogenic epitope).

The term "rep protein", as used herein, refers to a polypeptide having at least one functional activity of a native AAV rep protein (e.g. rep 40, 52, 68, 78). Examples of functional activities of a rep protein include any activity associated with the physiological function of the protein, including facilitating replication of DNA through recognition, binding and nicking of the AAV origin of DNA replication as well as DNA helicase activity. Additional functions include modulation of transcription from AAV (or other heterologous) promoters and site-specific integration of AAV DNA into a host chromosome. In a particular embodiment, AAV rep genes may be from the serotypes AAV1, AAV2, AAV4, AAV5, AAV6, AAV7, AAV8, AAV9, AAV10 or AAVrh10; more preferably from an AAV serotype selected from the group consisting of AAV2, AAV5, AAV7, AAV8, AAV9, AAV10 and AAVrh10.

In some embodiments, an AAV rep protein for use in the method of the invention can be generated by mutagenesis (i.e. by insertions, deletions, or substitutions) of one of the aforementioned AAV reps or its encoding nucleic acid. In some embodiments, the AAV rep is at least 70%, 75%, 80%, 85%, 90%, 95%, 98%, or 99% or more similar to one or more of the aforementioned AAV reps.

The expressions "helper functions" or "helper genes", as used herein, refer to viral proteins upon which AAV is dependent for replication. The helper functions include those proteins required for AAV replication including, without limitation, those proteins involved in activation of AAV gene transcription, stage specific AAV mRNA splicing, AAV DNA replication, synthesis of cap expression products, and AAV capsid assembly. Viral-based accessory functions can be derived from any of the known helper viruses such as adenovirus, herpesvirus (other than herpes simplex virus type-1), and vaccinia virus. Helper functions include, without limitation, adenovirus E1, E2a, VA, and E4 or herpesvirus UL5, ULB, UL52, and UL29, and herpesvirus polymerase. In a preferred embodiment, the proteins upon which AAV is dependent for replication are derived from adenovirus.

In some embodiments, a viral protein upon which AAV is dependent for replication for use in the method of the invention can be generated by mutagenesis (i.e. by insertions, deletions, or substitutions) of one of the aforementioned viral proteins or its encoding nucleic acid. In some embodiments, the viral protein is at least 70%, 75%, 80%, 85%, 90%, 95%, 98%, or 99% or more similar to one or more of the aforementioned viral proteins.

Methods for assaying the functions of cap proteins, rep proteins and viral proteins upon which AAV is dependent for replication are well known in the art.

Host cells for expressing a transgene of interest (e.g., a variant ATP7B sequence) may be grown under conditions adequate for assembly of the AAV virions. In certain embodiments, host cells are grown for a suitable period of time in order to promote the assembly of the AAV virions and the release of virions into the media. Generally, cells may be grown for about 24 hours, about 36 hours, about 48 hours, about 72 hours, about 4 days, about 5 days, about 6 days, about 7 days, about 8 days, about 9 days, or up to about 10 days. After about 10 days (or sooner, depending on the culture conditions and the particular host cell used), the level of production generally decreases significantly. Generally, time of culture is measured from the point of viral production. For example, in the case of AAV, viral production generally begins upon supplying helper virus function in an appropriate host cell as described herein. Generally, cells are harvested about 48 to about 100, preferably about 48 to about 96, preferably about 72 to about 96, preferably about 68 to about 72 hours after helper virus infection (or after viral production begins).

rAAV production cultures can be grown under a variety of conditions (over a wide temperature range, for varying lengths of time, and the like) suitable to the particular host cell being utilized. rAAV production cultures include attachment-dependent cultures which can be cultured in suitable attachment-dependent vessels such as, for example, roller bottles, hollow fiber filters, microcarriers, and packed-bed or fluidized-bed bioreactors. rAAV vector production cultures may also include suspension-adapted host cells such as HeLa, 293, and SF-9 cells which can be cultured in a variety of ways including, for example, spinner flasks, stirred tank bioreactors, and disposable systems such as the Wave bag system.

Suitable media known in the art may be used for the production of rAAV virions. These media include, without limitation, media produced by Hyclone Laboratories and JRH including Modified Eagle Medium (MEM), Dulbecco's Modified Eagle Medium (DMEM), each of which is incorporated herein by reference in its entirety. In certain embodiments, rAAV production culture media may be supplemented with serum or serum-derived recombinant proteins at a level of 0.5%-20% (v/v or w/v). Alternatively, rAAV vectors may be produced in serum-free conditions which may also be referred to as media with no animal-derived products.

After culturing the host cells to allow AAV virion production, the resulting virions may be then be harvested and purified. In certain embodiments, the AAV virions can be obtained from (1) the host cells of the production culture by lysis of the host cells, and/or (2) the culture medium of said cells after a period of time post-transfection, preferably 72 hours. The rAAV virions may be harvested from the spent media from the production culture, provided the cells are cultured under conditions that cause release of rAAV virions into the media from intact cells (see e.g., U.S. Pat. No. 6,566,118). Suitable methods of lysing cells are also known in the art and include for example multiple freeze/thaw cycles, sonication, microfluidization, and treatment with chemicals, such as detergents and/or proteases.

After harvesting, the rAAV virions may be purified. The term "purified" as used herein includes a preparation of rAAV virions devoid of at least some of the other components that may also be present where the rAAV virions naturally occur or are initially prepared from. Thus, for example, purified rAAV virions may be prepared using an isolation technique to enrich it from a source mixture, such as a culture lysate or production culture supernatant. Enrichment can be measured in a variety of ways, such as, for example, by the proportion of DNase-resistant particles (DRPs) or genome copies (gc) present in a solution, or by infectivity, or it can be measured in relation to a second, potentially interfering substance present in the source mixture, such as contaminants, including production culture contaminants or in-process contaminants, including helper virus, media components, and the like.

In certain embodiments, the rAAV production culture harvest may be clarified to remove host cell debris. In some embodiments, the production culture harvest may be clarified using a variety of standard techniques, such as, centrifugation or filtration through a filter of 0.2 µm or greater pore size (e.g., a cellulose acetate filter or a series of depth filters).

In certain embodiments, the rAAV production culture harvest is further treated with Benzonase^{™} to digest any high molecular weight DNA present in the production culture. In some embodiments, the Benzonase^{™} digestion is performed under standard conditions, for example, a final concentration of 1-2.5 units/ml of Benzonase^{™} at a temperature ranging from ambient to 37°C for a period of 30 minutes to several hours.

In certain embodiments, the rAAV virions may be isolated or purified using one or more of the following purification steps: equilibrium centrifugation; flow-through anionic exchange filtration; tangential flow filtration (TFF) for concentrating the rAAV particles; rAAV capture by apatite chromatography; heat inactivation of helper virus; rAAV capture by hydrophobic interaction chromatography; buffer exchange by size exclusion chromatography (SEC); nanofiltration; and rAAV capture by anionic exchange chromatography, cationic exchange chromatography, or affinity chromatography. These steps may be used alone, in various combinations, or in different orders. Methods to purify rAAV particles are found, for example, in Xiao et al., (1998) Journal of Virology 72:2224-2232; U.S. Pat. Nos. 6,989,264 and 8,137,948; and WO 2010/148143.

In certain embodiments, purified AAV virions can be dialyzed against PBS, filtered and stored at -80°C. Titers of viral genomes can be determined by quantitative PCR using linearized plasmid DNA as standard curve (see e.g., Lock M, et al., Hum. Gene Ther. 2010; 21:1273-1285).

### Pharmaceutical Compositions

In certain embodiments, the application provides compositions comprising a variant ATP7B sequence and a pharmaceutically acceptable carrier. In other embodiments, the application provides virions comprising a variant ATP7B sequence and a pharmaceutically acceptable carrier. In exemplary embodiments, such compositions are suitable for gene therapy applications. Pharmaceutical compositions are preferably sterile and stable under conditions of manufacture and storage. Sterile solutions may be accomplished, for example, by filtration through sterile filtration membranes.

Acceptable carriers and excipients in the pharmaceutical compositions are preferably nontoxic to recipients at the dosages and concentrations employed. Acceptable carriers and excipients may include buffers such as phosphate, citrate, HEPES, and TAE, antioxidants such as ascorbic acid and methionine, preservatives such as hexamethonium chloride, octadecyldimethylbenzyl ammonium chloride, resorcinol, and benzalkonium chloride, proteins such as human serum albumin, gelatin, dextran, and immunoglobulins, hydrophilic polymers such as polyvinylpyrrolidone, amino acids such as glycine, glutamine, histidine, and lysine, and carbohydrates such as glucose, mannose, sucrose, and sorbitol. Pharmaceutical compositions of the disclosure can be administered parenterally in the form of an injectable formulation. Pharmaceutical compositions for injection can be formulated using a sterile solution or any pharmaceutically acceptable liquid as a vehicle. Pharmaceutically acceptable vehicles include, but are not limited to, sterile water and physiological saline.

The pharmaceutical compositions of the disclosure may be prepared in microcapsules, such as hydroxylmethylcellulose or gelatin-microcapsules and polymethylmethacrylate microcapsules. The pharmaceutical compositions of the disclosure may also be prepared in other drug delivery systems such as liposomes, albumin microspheres, microemulsions, nano-particles, and nanocapsules. The pharmaceutical composition for gene therapy can be in an acceptable diluent, or can comprise a slow release matrix in which the gene delivery vehicle is imbedded.

Pharmaceutical compositions provided herein may be formulated for parenteral administration, subcutaneous administration, intravenous administration, intramuscular administration, intra-arterial administration, intrathecal administration, or intraperitoneal administration. The pharmaceutical composition may also be formulated for, or administered via, nasal, spray, oral, aerosol, rectal, or vaginal administration. In one embodiment, a pharmaceutical composition provided herein is administered by interstitial route, i.e. by injection to or into the interstices of a tissue. The tissue target may be specific, for example the liver tissue, or it may be a combination of several tissues, for example the muscle and liver tissues. Exemplary tissue targets may include liver, skeletal muscle, heart muscle, adipose deposits, kidney, lung, vascular endothelium, epithelial and/or hematopoietic cells. In a preferred embodiment, a pharmaceutical composition provided herein is administered by intrahepatic injection, i.e. injection into the interstitial space of hepatic tissue. One or more of these methods may be used to administer a pharmaceutical composition of the disclosure.

In certain embodiments, a pharmaceutical composition provided herein comprises an "effective amount" or a "therapeutically effective amount." As used herein, such amounts refer to an amount effective, at dosages and for periods of time necessary to achieve the desired therapeutic result, such as increasing the level of ATP7B expression and/or an elevation of copper translocation activity, thus increasing copper in bile and reducing copper in serum, liver, brain and urine.

The dosage of the pharmaceutical compositions of the disclosure depends on factors including the route of administration, the disease to be treated, and physical characteristics (e.g., age, weight, general health) of the subject. Dosage may be adjusted to provide the optimum therapeutic response. Typically, a dosage may be an amount that effectively treats the disease without inducing significant toxicity. In one embodiment, an AAV vector provided herein can be administered to the patient for the treatment of an ATP7B deficiency (including for example, Wilson's disease) in an amount or dose within a range of 5x10¹¹ to 1x10¹⁴ gc/kg (genome copies per kilogram of patient body weight (gc/kg)). In a more particular embodiment, the AAV vector is administered in an amount comprised within a range of about 5×10¹¹ gc/kg to about 3×10¹³ gc/kg, or about 1×10¹² to about 1×10¹⁴ gc/kg, or about 1×10¹² to about 1×10¹³ gc/kg, or about 5×10¹¹ gc/kg, 1×10¹² gc/kg, 1.5×10¹² gc/kg, 2.0×10¹² gc/kg, 2.5×10¹² gc/kg, 3×10¹² gc/kg, 3.5×10¹² gc/kg, 4×10¹² gc/kg, 4.5×10¹² gc/kg, 5×10¹² gc/kg, 5.5×10¹² gc/kg, 6×10¹² gc/kg, 6.5×10¹² gc/kg, 7×10¹² gc/kg, 7.5×10¹² gc/kg, 8×10¹² gc/kg, 8.5×10¹² gc/kg, 9×10¹² gc/kg or 9.5×10¹² gc/kg. The gc/kg may be determined, for example, by qPCR or digital droplet PCR (ddPCR) (see e.g., M. Lock et al, Hum Gene Ther Methods. 2014 Apr;25(2): 115-25). In another embodiment, an AAV vector provided herein can be administered to the patient for the treatment of an ATP7B deficiency (including for example, Wilson's disease) in an amount or dose within a range of 1×10⁹ to 1×10¹¹ iu/kg (infective units of the vector (iu)/subject's or patient's body weight (kg)). In certain embodiments, the pharmaceutical composition may be formed in a unit dose as needed. Such single dosage units may contain about 1×10⁹ gc to about 1×10¹⁵ gc.

Pharmaceutical compositions of the disclosure may be administered to a subject in need thereof, for example, one or more times (e.g., 1 -10 times or more) daily, weekly, monthly, biannually, annually, or as medically necessary. In an exemplary embodiment, a single administration is sufficient. In one embodiment, the pharmaceutical composition is suitable for use in human subjects and is administered intravenously. In one embodiment, the pharmaceutical composition is delivered via a peripheral vein by bolus injection. In other embodiments, the pharmaceutical composition is delivered via a peripheral vein by infusion over about 10 minutes (±5 minutes), over about 20 minutes (±5 minutes), over about 30 minutes (±5 minutes), over about 60 minutes (±5 minutes), or over about 90 minutes (±10 minutes).

In another aspect, the application further provides a kit comprising a nucleic acid molecule, vector, host cell, virion or pharmaceutical composition as described herein in one or more containers. A kit may include instructions or packaging materials that describe how to administer a nucleic acid molecule, vector, host cell or virion contained within the kit to a patient. Containers of the kit can be of any suitable material, e.g., glass, plastic, metal, etc., and of any suitable size, shape, or configuration. In certain embodiments, the kits may include one or more ampoules or syringes that contain a nucleic acid molecule, vector, host cell, virion or pharmaceutical composition in a suitable liquid or solution form.

### Methods of Treatment

In another aspect, the application provides methods of increasing ATP7B expression in a cell, tissue or subject, treating a disease or disorder associated with a decrease in ATP7B expression or deficiency in ATP7B function in a subject, or treating Wilson's disease by administering a nucleic acid molecule, expression vector, host cell, pharmaceutical composition, or virion comprising a variant ATP7B nucleic acid sequence as described herein. Any known technique can be used to deliver the variant ATP7B nucleic acid sequence to cells of interest to confer or induce *in vitro, in vivo,* or *ex vivo* expression of the variant ATP7B transgene in a cell, tissue or subject of interest.

In one embodiment, the application provides a method for increasing the level of ATP7B expression in a cell, tissue or subject using a nucleic acid molecule, expression vector, host cell, pharmaceutical composition, or virion comprising a variant ATP7B nucleic acid sequence as described herein. In certain embodiments, the level of ATP7B expression in the cell, tissue or subject is increased by at least about 1-fold, 1.5 fold, 2-fold, 5-fold, 10-fold, 15-fold, 20-fold, 25-fold, 30-fold, or 50-fold, or by at least about 2%, 5%, 10%, 20%, 25%, 50%, 75%, 80%, 90%, 100%, 125%, 150%, or 200%, relative to the level of expression of ATP7B in the cell, tissue or subject prior to administration of the nucleic acid molecule, expression vector, host cell, pharmaceutical composition, or virion comprising the variant ATP7B nucleic acid sequence. Various methods may be used for determining the level of ATP7B expression in a cell, tissue, or sample from a subject, including, for example, qPCR, northern blots, western blots, ELISA assays, etc. In certain embodiments, the cell, tissue or subject to be treated comprises a mutation in the endogenous genomic ATP7B such that the level of expression of ATP7B is decreased and/or the level of expression of functional ATP7B is decreased. In certain embodiments, the cell, tissue or subject is haploinsufficient for ATP7B. In an exemplary embodiment, the application provides a method for increasing ATP7B expression in a subject, comprising administering to a subject in need thereof an AAV virion comprising a variant ATP7B nucleic acid sequence as described herein operably linked to a promoter.

In another embodiment, the application provides a method for treating a disease or disorder associated with a decrease in ATP7B expression, a deficiency in ATP7B function, or any other disorder in which an upregulation of ATP7B expression and/or activity may produce a therapeutic benefit, in a subject comprising administering to a subject in need thereof a nucleic acid molecule, expression vector, host cell, pharmaceutical composition, or virion comprising a variant ATP7B nucleic acid sequence as described herein. Examples of disorders in which an increase in ATP7B expression and/or function may be beneficial include, for example, a disorder associated with a decrease of ATP7B-dependent lysosomal exocytosis and accumulation of copper in lysosomes, such as choleostatic disorders, Alzheimer disease and/or cancer (see e.g., Polishchuck et al., Dev Cell. 29(6), 686-700 (2014); Gupta and Lutsenko, Future Med. Chem. 1:1125-1142 (2009)). In certain embodiments, the subject comprises a mutation in the endogenous genomic ATP7B such that the level of expression of ATP7B is decreased and/or the level of expression of functional ATP7B is decreased. In certain embodiments, the subject is haploinsufficient for ATP7B. In certain embodiment, the subject has an ATPB variant protein which causes disease. In an exemplary embodiment, the application provides a method for treating a disease or disorder associated with a decrease in ATP7B expression, a deficiency in ATP7B function, or any other disorder in which an upregulation of ATP7B expression and/or activity may produce a therapeutic benefit, in a subject comprising administering to a subject in need thereof an AAV virion comprising a variant ATP7B nucleic acid sequence as described herein operably linked to a promoter.

In another embodiment, the application provides a method for treating Wilson's Disease, comprising administering to a subject in need thereof a nucleic acid molecule, expression vector, host cell, pharmaceutical composition, or virion comprising a variant ATP7B nucleic acid sequence as described herein. Wilson's disease is an autosomal recessive disorder of copper metabolism in which copper cannot be incorporated into ceruloplasmin in liver, and cannot be excreted from the liver into the bile. Copper accumulates in the liver and subsequently in the brain and kidney. The disease is characterized by neurologic manifestations and signs of cirrhosis. Wilson's Disease is an inherited error of metabolism caused predominantly by mutations in the ATP7B gene, which encodes a copper-transporting P-type ATPase. ATP7B is responsible for transporting copper from intracellular chaperone proteins into the secretory pathway, both for excretion into bile and for incorporation into apo-ceruloplasmin for the synthesis of functional ceruloplasmin. The development of Wilson's disease is due to the accumulation of copper in affected tissues (see e.g., EASL Clinical Practice Guidelines: Wilson's disease, EASL Journal of Hepatology, 2012, 56(671-85) and WD, Online Mendelian Inheritance in Man catalog accession number OMIN 277900; available on the world wide web at omim.org/entry/277900). Various mutations in the hATP7B gene and/or resulting protein are known which are present in some or all patients with Wilson's Disease. A complete listing of the known mutations contributing to Wilson's disease can be found on the world wide web at uniprot.org/uniprot/P35670.

The clinical hallmark of Wilson's disease is the Kayser-Fleischer ring, which is present in 95% of patients with neurologic symptoms and somewhat over half of those without neurologic symptoms. Neurologic signs are variable, most often tremor, ataxia, and dystonia. Any type of liver disease may be encountered in patients with Wilson's disease. Clinically evident liver disease may precede neurologic manifestations by as much as 10 years and most patients with neurologic symptoms have some degree of liver disease at presentation. Presenting symptoms of liver disease can be highly variable, ranging from asymptomatic, with only biochemical abnormalities, to overt cirrhosis with all its complications. Wilson's disease may also present as acute hepatic failure sometimes associated with Coombs-negative hemolytic anemia and acute renal failure. See e.g., EASL Clinical Practice Guidelines: Wilson's disease, EASL Journal of Hepatology, 2012, 56(671-85).

In various embodiments, a subject having any severity of Wilson's disease may be treated in accordance with the methods provided herein. In certain embodiment, a subject having Wilson's disease may have one or more of the following characteristics: elevated serum bilirubin level (e.g., greater than about 100 µmol/L), elevated aspartate aminotransferase (AST) level (e.g., greater than about 100 U/L), elevated international normalization ratio (INR) level (e.g., greater than about 1.3), elevated white blood cell (WBC) count (e.g., greater than about 6.8×10⁹/L), and/or decreased albumin level (e.g., less than about 44 g/L). Other suitable tests that can be used to identify patients with Wilson's disease include, for example, non-ceruloplasmin-bound copper (NCC; also called the "free copper" or copper index), 24-h urine copper, hepatic copper, and genetic mutation testing (see e.g., McMillin et al, Am J Clin Pathol. 2009; 131(2): 160-165 (2009) for exemplary methods for measurement of copper levels).

In various embodiments, the methods provided herein comprising administration of a variant ATP7B sequence may alleviate, ameliorate, or reduce the severity of one or more symptoms of Wilson's disease, including, for example, increasing and/or restoring holoceruplasmin synthesis, ceruloplasmin oxidase activity, and/or copper excretion in the bile (thus reducing copper accumulation in serum, liver, brain and urine), and/or may alleviate, ameliorate, or reduce the severity of abdominal pain, fatigue, jaundice, frequency of uncontrolled movements, muscle stiffness, problems with speech, swallowing or physical coordination. In certain embodiments, the methods provided herein comprising administration of a variant ATP7B sequence may result in one or more of the following outcomes: a reduction of serum copper levels of 25% or more, urinary copper excretion of 3-8 µmol or less per 24 hours, normalization of serum non-ceruloplasmin bound copper (NCC) (<150 µg/L), normalization of serum aminotransferase (liver biochemistries, ALT/AST), normalization of urinary Cu (<40 µg/24 hours (0.6 µmol/24 hours) upper limit of normal, normalization of serum ceruloplasmin (>200 mg/L), improvement of Clinician Global Impression (CGI) scale. In various embodiments, assessment of outcome may be evaluated by measuring non-ceruloplasmin-bound copper, 24-h urine copper, or hepatic copper levels, and/or by clinical assessment of dietary copper tolerance. In certain embodiments, the methods provided herein comprising administration of a variant ATP7B sequence may result in a reduction of serum copper levels of 5%, 10%, 15%, 20%, 25%, 30%, 40%, 50% or more.

In an exemplary embodiment, the application provides a method for treating Wilson's disease comprising administering to a subject in need thereof an AAV virion comprising a variant ATP7B nucleic acid sequence as described herein operably linked to a promoter.

In certain embodiments, the methods provided herein that involve administering a nucleic acid molecule, expression vector, host cell, pharmaceutical composition, or virion comprising a variant ATP7B nucleic acid sequence as described herein, may further comprise administration of one or more additional therapeutic agents. For example, in certain embodiments, the methods provided herein may involve treatment with chelating agents such as D-penicillamine and trientine, and/or treatment with other agents such as sodium dimercaptosuccinate, dimercaptosuccinic acid, zinc, and tetrathiomolybdate. In other embodiments, the methods provided herein may involve treating a subject that is on a diet low in copper. In various embodiments, the additional therapeutic agents may be administered concurrently or sequentially with the variant ATP7B nucleic acids described herein. For example, the additional therapeutic agent may be administered before, after and/or before and after administration of a nucleic acid molecule, expression vector, host cell, pharmaceutical composition, or virion comprising a variant ATP7B nucleic acid sequence as described herein. In other embodiments, an additional therapeutic agent may be administered concurrently with a nucleic acid molecule, expression vector, host cell, pharmaceutical composition, or virion comprising a variant ATP7B nucleic acid sequence as described herein.

In various embodiments, a subject that can be treated in accordance with the methods described herein is a mammal, such as, for example, a mouse, rat, hamster, guinea pig, gerbil, cow, sheep, pig, goat, donkey, horse, dog, cat, llama, monkey (e.g., a macaque such as a Rhesus or cynomolgus), or human. In an exemplary embodiment, a subject is a human.

### SEQUENCES

**TABLE 1: ATP7B Sequences**

| | | |
|---|---|---|
| **SEQ ID NO** | **SEQUENCE** | **NAME** |
| **1** | ATGCCTGAGCAGGAGAGACAGATCACAGCCAGAGAAGGGGCCAGTCGGAAAA TCTTATCTAAGCTTTCTTTGCCTACCCGTGCCTGGGAACCAGCAATGAAGAA GAGTTTTGCTTTTGACAATGTTGGCTATGAAGGTGGTCTGGATGGCCTGGGC CCTTCTTCTCAGGTGGCCACCAGCACAGTCAGGATCTTGGGCATGACTTGCC AGTCATGTGTGAAGTCCATTGAGGACAGGATTTCCAATTTGAAAGGCATCAT CAGCATGAAGGTTTCCCTGGAACAAGGCAGTGCCACTGTGAAATATGTGCCA TCGGTTGTGTGCCTGCAACAGGTTTGCCATCAAATTGGGGACATGGGCTTCG AGGCCAGCATTGCAGAAGGAAAGGCAGCCTCCTGGCCCTCAAGGTCCTTGCC TGCCCAGGAGGCTGTGGTCAAGCTCCGGGTGGAGGGCATGACCTGCCAGTCC TGTGTCAGCTCCATTGAAGGCAAGGTCCGGAAACTGCAAGGAGTAGTGAGAG TCAAAGTCTCACTCAGCAACCAAGAGGCCGTCATCACTTATCAGCCTTATCT CATTCAGCCCGAAGACCTCAGGGACCATGTAAATGACATGGGATTTGAAGCT GCCATCAAGAGCAAAGTGGCTCCCTTAAGCCTGGGACCAATTGATATTGAGC GGTTACAAAGCACTAACCCAAAGAGACCTTTATCTTCTGCTAACCAGAATTT TAATAATTCTGAGACCTTGGGGCACCAAGGAAGCCATGTGGTCACCCTCCAA CTGAGAATAGATGGAATGCATTGTAAGTCTTGCGTCTTGAATATTGAAGAAA ATATTGGCCAGCTCCTAGGGGTTCAAAGTATTCAAGTGTCCTTGGAGAACAA AACTGCCCAAGTAAAGTATGACCCTTCTTGTACCAGCCCAGTGGCTCTGCAG AGGGCTATCGAGGCACTTCCACCTGGGAATTTTAAAGTTTCTCTTCCTGATG GAGCCGAAGGGAGTGGGACAGATCACAGGTCTTCCAGTTCTCATTCCCCTGG CTCCCCACCGAGAAACCAGGTCCAGGGCACATGCAGTACCACTCTGATTGCC ATTGCCGGCATGACCTGTGCATCCTGTGTCCATTCCATTGAAGGCATGATCT CCCAACTGGAAGGGGTGCAGCAAATATCGGTGTCTTTGGCCGAAGGGACTGC AACAGTTCTTTATAATCCCTCTGTAATTAGCCCAGAAGAACTCAGAGCTGCT ATAGAAGACATGGGATTTGAGGCTTCAGTCGTTTCTGAAAGCTGTTCTACTA ACCCTCTTGGAAACCACAGTGCTGGGAACTCCATGGTGCAAACTACAGATGG TACACCTACATCTGTGCAGGAAGTGGCTCCCCACACTGGGAGGCTCCCTGCA AACCATGCCCCGGACATCTTGGCAAAGTCCCCACAATCAACCAGAGCAGTGG CACCGCAGAAGTGCTTCTTACAGATCAAAGGCATGACCTGTGCATCCTGTGT GTCTAACATAGAAAGGAATCTGCAGAAAGAAGCTGGTGTTCTCTCCGTGTTG GTTGCCTTGATGGCAGGAAAGGCAGAGATCAAGTATGACCCAGAGGTCATCC AGCCCCTCGAGATAGCTCAGTTCATCCAGGACCTGGGTTTTGAGGCAGCAGT CATGGAGGACTACGCAGGCTCCGATGGCAACATTGAGCTGACAATCACAGGG ATGACCTGCGCGTCCTGTGTCCACAACATAGAGTCCAAACTCACGAGGACAA ATGGCATCACTTATGCCTCCGTTGCCCTTGCCACCAGCAAAGCCCTTGTTAA GTTTGACCCGGAAATTATCGGTCCACGGGATATTATCAAAATTATTGAGGAA ATTGGCTTTCATGCTTCCCTGGCCCAGAGAAACCCCAACGCTCATCACTTGG ACCACAAGATGGAAATAAAGCAGTGGAAGAAGTCTTTCCTGTGCAGCCTGGT GTTTGGCATCCCTGTCATGGCCTTAATGATCTATATGCTGATACCCAGCAAC GAGCCCCACCAGTCCATGGTCCTGGACCACAACATCATTCCAGGACTGTCCA TTCTAAATCTCATCTTCTTTATCTTGTGTACCTTTGTCCAGCTCCTCGGTGG GTGGTACTTCTACGTTCAGGCCTACAAATCTCTGAGACACAGGTCAGCCAAC ATGGACGTGCTCATCGTCCTGGCCACAAGCATTGCTTATGTTTATTCTCTGG TCATCCTGGTGGTTGCTGTGGCTGAGAAGGCGGAGAGGAGCCCTGTGACATT CTTCGACACGCCCCCCATGCTCTTTGTGTTCATTGCCCTGGGCCGGTGGCTG GAACACTTGGCAAAGAGCAAAACCTCAGAAGCCCTGGCTAAACTCATGTCTC TCCAAGCCACAGAAGCCACCGTTGTGACCCTTGGTGAGGACAATTTAATCAT CAGGGAGGAGCAAGTCCCCATGGAGCTGGTGCAGCGGGGCGATATCGTCAAG | **WT ATP7B nt** |

| **SEQ ID NO** | **SEQUENCE** | **NAME** |
|---|---|---|
| | GTGGTCCCTGGGGGAAAGTTTCCAGTGGATGGGAAAGTCCTGGAAGGCAATA CCATGGCTGATGAGTCCCTCATCACAGGAGAAGCCATGCCAGTCACTAAGAA ACCCGGAAGCACTGTAATTGCGGGGTCTATAAATGCACATGGCTCTGTGCTC ATTAAAGCTACCCACGTGGGCAATGACACCACTTTGGCTCAGATTGTGAAAC TGGTGGAAGAGGCTCAGATGTCAAAGGCACCCATTCAGCAGCTGGCTGACCG GTTTAGTGGATATTTTGTCCCATTTATCATCATCATGTCAACTTTGACGTTG GTGGTATGGATTGTAATCGGTTTTATCGATTTTGGTGTTGTTCAGAGATACT TTCCTAACCCCAACAAGCACATCTCCCAGACAGAGGTGATCATCCGGTTTGC TTTCCAGACGTCCATCACGGTGCTGTGCATTGCCTGCCCCTGCTCCCTGGGG CTGGCCACGCCCACGGCTGTCATGGTGGGCACCGGGGTGGCCGCGCAGAACG GCATCCTCATCAAGGGAGGCAAGCCCCTGGAGATGGCGCACAAGATAAAGAC TGTGATGTTTGACAAGACTGGCACCATTACCCATGGCGTCCCCAGGGTCATG CGGGTGCTCCTGCTGGGGGATGTGGCCACACTGCCCCTCAGGAAGGTTCTGG CTGTGGTGGGGACTGCGGAGGCCAGCAGTGAACACCCCTTGGGCGTGGCAGT CACCAAATACTGTAAAGAGGAACTTGGAACAGAGACCTTGGGATACTGCACG GACTTCCAGGCAGTGCCAGGCTGTGGAATTGGGTGCAAAGTCAGCAACGTGG AAGGCATCCTGGCCCACAGTGAGCGCCCTTTGAGTGCACCGGCCAGTCACCT GAATGAGGCTGGCAGCCTTCCCGCAGAAAAAGATGCAGTCCCCCAGACCTTC TCTGTGCTGATTGGAAACCGTGAGTGGCTGAGGCGCAACGGTTTAACCATTT CTAGCGATGTCAGTGACGCTATGACAGACCACGAGATGAAAGGACAGACAGC CATCCTGGTGGCTATTGACGGTGTGCTCTGTGGGATGATCGCAATCGCAGAC GCTGTCAAGCAGGAGGCTGCCCTGGCTGTGCACACGCTGCAGAGCATGGGTG TGGACGTGGTTCTGATCACGGGGGACAACCGGAAGACAGCCAGAGCTATTGC CACCCAGGTTGGCATCAACAAAGTCTTTGCAGAGGTGCTGCCTTCGCACAAG GTGGCCAAGGTCCAGGAGCTCCAGAATAAAGGGAAGAAAGTCGCCATGGTGG GGGATGGGGTCAATGACTCCCCGGCCTTGGCCCAGGCAGACATGGGTGTGGC CATTGGCACCGGCACGGATGTGGCCATCGAGGCAGCCGACGTCGTCCTTATC AGAAATGATTTGCTGGATGTGGTGGCTAGCATTCACCTTTCCAAGAGGACTG TCCGAAGGATACGCATCAACCTGGTCCTGGCACTGATTTATAACCTGGTTGG GATACCCATTGCAGCAGGTGTCTTCATGCCCATCGGCATTGTGCTGCAGCCC TGGATGGGCTCAGCGGCCATGGCAGCCTCCTCTGTGTCTGTGGTGCTCTCAT CCCTGCAGCTCAAGTGCTATAAGAAGCCTGACCTGGAGAGGTATGAGGCACA GGCGCATGGCCACATGAAGCCCCTGACGGCATCCCAGGTCAGTGTGCACATA GGCATGGATGACAGGTGGCGGGACTCCCCCAGGGCCACACCATGGGACCAGG TCAGCTATGTCAGCCAGGTGTCGCTGTCCTCCCTGACGTCCGACAAGCCATC TCGGCACAGCGCTGCAGCAGACGATGATGGGGACAAGTGGTCTCTGCTCCTG AATGGCAGGGATGAGGAGCAGTACATCTGA | |
| **2** | | **WT ATP7B aa NCBI Accession No.** NP_00004 4.2 |
| | | |
| **3** | ATGCCTGAGCAGGAAAGGCAAATCACGGCCCGCGAAGGGGCTTCTCGCAAGA TACTTAGCAAGCTTTCACTTCCTACGCGAGCCTGGGAACCTGCCATGAAGAA AAGCTTTGCCTTTGATAATGTTGGCTACGAGGGAGGGCTTGATGGTTTGGGC CCATCCAGCCAGGTAGCAACGTCAACTGTTAGGATCTTGGGGATGACATGCC AAAGCTGCGTGAAGTCCATTGAAGACCGGATATCCAATCTCAAAGGTATCAT TAGTATGAAAGTGTCCCTGGAGCAGGGATCTGCGACAGTTAAGTACGTTCCT TCAGTCGTGTGCCTGCAACAAGTCTGCCATCAGATAGGCGACATGGGGTTCG AGGCCTCAATTGCGGAGGGTAAGGCGGCCAGCTGGCCATCTAGAAGCCTGCC CGCACAAGAGGCGGTTGTGAAACTGAGGGTTGAGGGCATGACTTGTCAATCT TGCGTAAGTAGTATCGAGGGAAAGGTTCGGAAGCTCCAGGGAGTTGTGAGAG TTAAAGTGAGCCTCTCAAATCAAGAAGCCGTTATAACGTACCAGCCCTACTT GATTCAGCCTGAGGATTTGCGCGACCATGTCAATGATATGGGTTTTGAAGCG GCTATTAAGAGTAAGGTGGCCCCTCTGAGTCTCGGGCCGATTGACATCGAAA GGCTTCAATCCACGAACCCTAAGAGACCTCTTAGTAGTGCGAATCAGAATTT TAACAATAGTGAGACGCTGGGTCATCAAGGATCTCATGTAGTTACTTTGCAA CTGAGAATTGACGGCATGCATTGTAAATCCTGTGTGCTGAACATTGAGGAAA ATATTGGGCAACTGCTTGGAGTGCAAAGCATACAAGTCTCCCTCGAGAACAA AACGGCCCAGGTGAAGTACGACCCGTCATGCACTTCACCGGTGGCCCTCCAA AGAGCGATTGAGGCTCTGCCCCCAGGTAATTTCAAAGTAAGCCTGCCGGATG GGGCTGAGGGGTCTGGAACTGACCACAGATCAAGTAGCTCACACAGCCCAGG AAGCCCACCGCGGAACCAGGTTCAGGGGACGTGCTCAACAACTCTTATCGCT ATAGCGGGTATGACTTGTGCGTCCTGTGTTCATTCAATCGAGGGAATGATAA GTCAGCTTGAGGGAGTCCAGCAGATAAGTGTTTCCCTTGCCGAGGGGACAGC TACCGTACTTTACAATCCGAGCGTGATTAGTCCTGAAGAACTTCGGGCAGCA ATTGAGGACATGGGGTTTGAGGCGAGCGTCGTGAGCGAGTCATGCTCAACTA ACCCTCTTGGAAATCACAGTGCCGGGAACTCAATGGTGCAAACGACAGATGG GACCCCGACTTCTGTACAGGAAGTCGCTCCCCACACCGGACGGCTTCCCGCA AACCATGCCCCAGACATTCTCGCGAAATCACCGCAGTCAACACGCGCGGTGG CGCCGCAGAAATGTTTTCTTCAGATTAAAGGCATGACGTGCGCCAGTTGTGT ATCTAACATCGAACGGAACTTGCAGAAAGAAGCTGGTGTACTGAGCGTGTTG GTAGCGCTTATGGCCGGAAAGGCGGAAATCAAGTATGATCCAGAGGTTATTC AGCCACTTGAGATTGCGCAGTTCATCCAGGACTTGGGCTTTGAGGCAGCAGT GATGGAAGACTACGCCGGTAGTGATGGTAACATTGAGCTTACCATCACAGGC ATGACCTGTGCAAGCTGTGTCCACAATATCGAGTCTAAGCTTACCCGAACAA ACGGGATCACGTATGCCAGCGTCGCGCTTGCAACGTCCAAAGCATTGGTAAA ATTCGACCCAGAAATCATAGGACCCCGCGATATTATTAAGATTATCGAGGAA ATTGGCTTCCACGCCTCTCTGGCCCAGCGCAACCCAAACGCTCATCATCTTG | **ATP7B variant 1** |
| | ATCATAAAATGGAAATCAAACAGTGGAAGAAGTCTTTCCTTTGTAGTCTGGT CTTCGGGATACCTGTTATGGCACTTATGATCTACATGCTCATTCCGTCTAAC GAGCCACACCAAAGTATGGTTTTGGACCATAATATAATACCTGGGCTGAGCA TTCTTAACCTGATCTTCTTTATACTGTGCACGTTTGTACAATTGCTTGGTGG ATGGTATTTTTATGTACAGGCGTATAAAAGCCTGCGGCATCGATCTGCCAAT ATGGACGTACTCATTGTACTGGCGACATCAATTGCCTACGTGTACAGTCTTG TCATTTTGGTAGTAGCGGTCGCTGAAAAAGCAGAACGGAGCCCTGTTACTTT CTTCGATACCCCGCCGATGCTTTTTGTTTTCATTGCTCTTGGCCGGTGGTTG GAGCATCTGGCTAAATCAAAAACGAGCGAGGCCCTTGCAAAGCTCATGTCAT TGCAAGCGACTGAAGCCACGGTGGTCACCCTGGGCGAAGATAACTTGATTAT CAGAGAAGAGCAAGTGCCCATGGAACTGGTTCAAAGAGGTGATATAGTGAAG GTTGTGCCGGGGGGGAAGTTCCCCGTTGATGGGAAGGTACTTGAGGGTAATA CAATGGCAGATGAATCATTGATAACGGGAGAGGCTATGCCCGTCACTAAGAA ACCTGGGTCAACTGTCATCGCAGGGTCTATCAACGCCCATGGGAGCGTATTG ATAAAAGCTACGCATGTCGGGAATGATACTACGTTGGCTCAGATAGTTAAGC TTGTCGAGGAAGCTCAAATGAGCAAGGCTCCTATACAGCAACTGGCCGATAG GTTCAGTGGGTATTTCGTGCCGTTCATTATAATAATGAGTACTTTGACATTG GTAGTATGGATTGTAATCGGGTTCATAGACTTTGGAGTAGTACAGCGATACT TTCCAAATCCCAACAAGCATATCTCCCAAACCGAGGTTATAATCAGATTCGC TTTCCAAACGTCCATAACGGTCCTCTGTATAGCCTGTCCGTGTAGCTTGGGT CTTGCAACTCCAACGGCGGTCATGGTTGGAACCGGAGTTGCGGCCCAAAACG GCATACTCATTAAGGGTGGGAAACCTCTGGAAATGGCACATAAGATAAAAAC AGTTATGTTTGATAAGACAGGGACCATTACCCATGGCGTGCCGAGAGTTATG AGGGTCCTCCTTTTGGGTGATGTTGCGACTCTGCCCCTTAGAAAGGTATTGG CAGTGGTTGGGACGGCGGAAGCGTCCTCAGAACACCCGCTGGGGGTAGCCGT AACTAAGTACTGTAAAGAAGAGCTCGGTACAGAGACTCTTGGGTACTGTACA GATTTCCAAGCTGTCCCAGGTTGCGGAATCGGATGTAAGGTCAGCAATGTCG AAGGAATCTTGGCTCATTCAGAAAGACCTTTGTCAGCACCTGCATCTCATTT GAATGAGGCTGGCAGTTTGCCAGCTGAAAAAGACGCTGTACCCCAAACTTTT AGTGTCTTGATTGGCAACCGAGAGTGGCTGCGCCGCAATGGGCTCACTATAA GCTCCGATGTATCAGATGCCATGACAGACCACGAAATGAAAGGTCAGACTGC GATACTGGTGGCAATCGATGGTGTACTCTGTGGTATGATAGCCATAGCCGAT GCAGTTAAACAGGAAGCAGCACTTGCCGTACACACTTTGCAATCTATGGGTG TCGACGTAGTCCTGATTACCGGTGACAACAGAAAAACCGCGAGAGCTATTGC CACACAAGTTGGCATTAACAAAGTGTTTGCTGAGGTTCTGCCTTCCCATAAA GTCGCTAAAGTTCAGGAACTGCAAAATAAGGGAAAAAAAGTCGCTATGGTTG GCGACGGCGTCAATGACTCACCGGCATTGGCGCAAGCGGATATGGGCGTGGC GATCGGCACTGGCACAGACGTCGCGATAGAGGCCGCGGACGTAGTTTTGATC AGAAATGATCTCCTTGATGTTGTTGCCTCTATCCATTTGAGCAAACGAACTG TGCGAAGAATCCGAATAAATCTTGTCCTCGCACTTATTTATAATCTGGTTGG CATTCCAATTGCCGCTGGTGTCTTCATGCCTATCGGAATCGTCCTTCAGCCA TGGATGGGCAGCGCGGCGATGGCAGCTAGTTCAGTCTCTGTGGTCCTTAGTT CCCTCCAGCTGAAATGCTATAAAAAACCTGATCTTGAGAGGTATGAGGCCCA AGCACATGGCCATATGAAACCACTTACGGCAAGTCAGGTGTCAGTTCACATC GGGATGGATGACAGGTGGAGGGATTCCCCGCGCGCCACTCCGTGGGACCAGG TAAGCTATGTATCTCAGGTTTCTCTCAGTAGTTTGACCAGTGACAAACCTTC TCGACATTCTGCTGCCGCAGATGACGATGGTGATAAGTGGTCTCTTCTTCTT AACGGGAGGGACGAAGAGCAATACATTTGA | |
| 4 | ATGCCAGAACAGGAACGGCAAATAACAGCTCGAGAAGGTGCTTCACGGAAGA TACTGAGTAAACTTTCATTGCCAACCCGCGCCTGGGAGCCCGCTATGAAGAA ATCCTTTGCCTTCGACAACGTGGGATACGAGGGGGGTTTGGACGGACTTGGA | **ATP7B variant 2** |
| | CCCTCCTCTCAAGTAGCTACATCTACTGTTCGAATTTTGGGAATGACATGTC AAAGTTGTGTCAAATCTATAGAGGATAGAATCAGCAACCTGAAGGGCATCAT ATCAATGAAGGTCTCTTTGGAACAAGGATCCGCAACAGTAAAGTACGTACCC AGTGTGGTATGCCTTCAACAGGTCTGCCACCAGATAGGGGACATGGGATTTG AAGCATCCATCGCCGAGGGGAAAGCAGCTTCTTGGCCTAGCCGAAGCTTGCC AGCCCAGGAAGCAGTAGTGAAGCTCAGAGTTGAGGGTATGACTTGCCAGTCT TGCGTTTCCTCTATAGAGGGCAAAGTCCGCAAACTGCAGGGGGTTGTGCGCG TGAAAGTAAGTCTGAGCAACCAAGAGGCAGTAATCACATATCAACCATATCT CATTCAGCCTGAGGACTTGCGAGACCATGTTAACGATATGGGATTTGAGGCT GCCATTAAATCCAAAGTGGCACCCCTCTCTCTTGGGCCAATAGACATAGAGA GGCTTCAGAGTACTAACCCAAAACGGCCATTGTCTTCAGCTAATCAAAACTT CAACAATTCCGAAACCCTCGGTCATCAGGGCTCACATGTTGTAACTCTGCAG TTGCGCATAGACGGAATGCATTGCAAGTCCTGCGTTCTGAACATCGAAGAAA ACATAGGTCAGCTTCTGGGGGTCCAGTCTATACAAGTGAGTCTCGAAAACAA GACTGCCCAAGTTAAATATGATCCAAGCTGTACCAGCCCAGTTGCCCTGCAA AGAGCCATAGAAGCACTGCCTCCTGGAAATTTCAAGGTTTCTCTTCCAGACG GAGCTGAAGGAAGCGGCACTGACCATCGATCCTCAAGTAGCCATAGCCCTGG ATCTCCCCCCAGAAATCAAGTCCAGGGTACATGCAGTACCACCCTCATAGCC ATAGCCGGCATGACCTGTGCTTCATGCGTACACAGTATTGAAGGAATGATCT CCCAGTTGGAGGGAGTACAGCAAATTTCCGTATCCTTGGCCGAAGGTACTGC AACTGTACTCTACAATCCATCCGTTATAAGCCCCGAGGAACTTAGAGCCGCA ATTGAGGACATGGGATTCGAGGCAAGTGTAGTTTCAGAATCATGTAGTACTA ATCCACTTGGTAACCATTCAGCAGGTAACAGCATGGTACAAACCACAGACGG CACTCCAACATCCGTTCAGGAAGTGGCCCCCCATACCGGCCGCCTCCCCGCC AACCACGCACCTGACATCCTCGCTAAGTCCCCCCAATCAACACGGGCTGTAG CCCCACAGAAGTGCTTTCTGCAGATAAAGGGCATGACTTGCGCAAGCTGCGT TTCAAACATCGAGCGAAACCTTCAGAAAGAGGCCGGCGTGCTGAGTGTCTTG GTTGCTCTTATGGCAGGTAAAGCTGAAATCAAATACGATCCCGAAGTCATAC AACCTCTTGAAATCGCACAATTTATCCAAGACTTGGGCTTCGAGGCTGCAGT CATGGAAGACTACGCTGGCTCAGACGGCAACATCGAATTGACAATAACCGGG ATGACATGTGCTTCCTGCGTTCATAATATAGAGTCTAAGCTCACTAGAACCA ATGGTATTACCTATGCTTCAGTGGCACTCGCTACCTCCAAAGCACTTGTCAA ATTTGACCCAGAGATCATAGGGCCACGGGATATTATCAAAATTATTGAGGAA ATAGGTTTCCATGCATCACTCGCTCAGAGGAACCCCAACGCCCATCATTTGG ATCACAAAATGGAAATAAAACAGTGGAAGAAAAGTTTCCTGTGTAGCCTTGT GTTCGGCATCCCAGTGATGGCACTGATGATCTACATGTTGATACCCAGCAAT GAACCACACCAGAGCATGGTTCTTGATCATAATATTATCCCCGGACTCAGCA TTTTGAATCTTATATTCTTTATTCTCTGCACTTTTGTCCAACTCCTCGGAGG CTGGTATTTTTACGTCCAAGCCTACAAGAGCTTGAGGCATAGATCAGCTAAC ATGGATGTTTTGATTGTTTTGGCAACAAGTATCGCCTACGTCTACAGTCTCG TGATACTGGTCGTGGCAGTTGCTGAAAAAGCAGAGCGCAGTCCAGTAACATT TTTTGATACACCCCCAATGCTCTTCGTATTTATCGCCTTGGGACGGTGGCTT GAGCACCTTGCTAAATCAAAGACTAGCGAAGCTCTTGCCAAGCTCATGAGTC TCCAAGCAACAGAAGCAACTGTGGTAACTCTGGGGGAAGATAACCTCATAAT ACGGGAGGAACAAGTACCTATGGAATTGGTACAGCGAGGGGATATTGTAAAA GTTGTCCCCGGCGGGAAGTTTCCAGTGGACGGCAAGGTACTGGAGGGCAACA CTATGGCAGATGAGAGTTTGATCACCGGCGAAGCAATGCCTGTGACTAAAAA ACCAGGAAGTACCGTCATTGCCGGGAGCATTAATGCACATGGTAGTGTCCTG ATCAAGGCCACACATGTAGGCAACGATACTACTCTGGCACAGATCGTGAAGC TCGTAGAAGAAGCACAGATGTCTAAGGCCCCCATTCAGCAACTCGCAGACCG ATTTAGTGGATACTTCGTTCCATTCATAATCATTATGTCTACATTGACCCTC GTTGTTTGGATCGTAATAGGCTTCATAGATTTTGGAGTAGTTCAGCGATACT | |
| | TCCCCAACCCTAATAAACATATTAGTCAGACCGAGGTTATAATAAGATTTGC TTTCCAGACTTCAATAACCGTACTCTGTATAGCATGTCCCTGCTCCCTGGGC CTCGCCACCCCTACAGCTGTTATGGTTGGCACCGGAGTAGCAGCCCAGAACG GCATTTTGATTAAAGGTGGCAAGCCTCTTGAAATGGCTCATAAAATTAAAAC AGTAATGTTCGACAAAACTGGGACCATCACTCACGGCGTGCCCCGCGTTATG CGAGTTCTCTTGTTGGGCGATGTTGCAACCTTGCCTCTGAGAAAAGTACTCG CTGTAGTTGGTACAGCTGAAGCTTCTTCCGAGCATCCACTTGGCGTGGCAGT GACTAAGTATTGCAAGGAAGAACTGGGTACCGAGACCCTTGGCTACTGTACC GATTTTCAGGCCGTGCCCGGCTGTGGGATAGGTTGTAAGGTTTCAAACGTGG AGGGGATTCTGGCACACTCAGAACGCCCACTCTCTGCTCCAGCTAGCCATCT CAACGAAGCAGGGTCACTTCCCGCTGAAAAGGACGCAGTTCCTCAAACTTTC AGTGTGCTGATCGGTAACCGCGAGTGGTTGCGGAGGAATGGCTTGACAATCA GTTCCGACGTCAGTGATGCTATGACCGATCACGAAATGAAGGGGCAAACTGC AATATTGGTGGCTATTGACGGAGTGCTGTGTGGAATGATAGCTATCGCTGAT GCTGTCAAACAAGAAGCTGCATTGGCCGTGCACACTTTGCAATCTATGGGCG TAGATGTAGTCTTGATCACCGGGGATAATCGCAAGACCGCCCGCGCTATTGC CACACAGGTGGGAATCAACAAAGTCTTCGCAGAGGTTCTGCCAAGTCATAAA GTCGCTAAGGTACAGGAGCTCCAAAATAAGGGCAAGAAGGTTGCCATGGTTG GTGACGGCGTCAATGATTCTCCCGCCCTCGCTCAGGCTGATATGGGGGTCGC CATCGGAACCGGAACTGATGTTGCCATCGAAGCCGCCGACGTGGTTCTCATA CGGAACGACTTGTTGGATGTCGTGGCCTCAATACACCTCTCCAAACGGACAG TACGCCGGATAAGGATTAATTTGGTTCTTGCCCTGATCTACAATCTGGTAGG AATCCCTATTGCTGCTGGTGTGTTTATGCCAATAGGGATTGTCCTCCAACCT TGGATGGGATCCGCAGCTATGGCCGCCTCCTCAGTGTCAGTGGTGTTGTCAT CTCTCCAACTCAAGTGTTATAAAAAACCAGATCTTGAGCGATATGAGGCACA AGCTCATGGCCACATGAAGCCTCTCACAGCCTCTCAAGTTTCAGTGCACATA GGTATGGATGATAGATGGCGGGACAGTCCCAGGGCTACACCTTGGGACCAGG TTAGCTATGTGTCACAGGTGTCACTGTCCTCCCTTACCAGTGATAAGCCAAG TAGGCATTCCGCCGCAGCTGACGATGATGGCGATAAGTGGTCTCTTCTCCTC AACGGACGAGATGAAGAACAATACATTTGA | |
| 5 | ATGCCAGAACAGGAACGCCAGATTACCGCACGAGAGGGAGCCAGCCGGAAGA TCCTGAGCAAACTGAGCCTGCCCACCAGAGCTTGGGAGCCCGCCATGAAGAA GAGCTTCGCCTTTGACAACGTGGGATACGAGGGAGGCCTGGATGGCCTGGGA CCTAGCTCCCAGGTGGCCACCAGCACCGTGAGAATCCTGGGCATGACATGCC AGAGCTGCGTGAAGTCCATCGAGGACAGAATCTCTAATCTGAAGGGCATCAT CTCTATGAAGGTGAGCCTGGAGCAGGGCTCCGCCACCGTGAAGTATGTGCCT TCTGTGGTGTGCCTGCAGCAGGTGTGCCACCAGATCGGCGATATGGGCTTCG AGGCCAGCATCGCAGAGGGCAAGGCAGCCTCCTGGCCTTCTCGGAGCCTGCC AGCACAGGAGGCAGTGGTGAAGCTGAGAGTGGAAGGAATGACCTGTCAGAGC TGCGTGAGCAGCATCGAGGGCAAGGTGAGGAAGCTGCAGGGCGTGGTGCGCG TGAAGGTGTCCCTGTCTAACCAGGAGGCCGTGATCACCTACCAGCCCTATCT GATCCAGCCTGAGGACCTGAGGGATCACGTGAATGACATGGGCTTCGAGGCC GCCATCAAGAGCAAGGTGGCACCACTGTCCCTGGGACCAATCGACATCGAGC GGCTGCAGTCCACCAACCCAAAGCGGCCCCTGAGCAGCGCCAACCAGAACTT CAACAATTCTGAGACACTGGGACACCAGGGCAGCCACGTGGTGACCCTGCAG CTGAGGATCGACGGCATGCACTGCAAGAGCTGCGTGCTGAACATCGAGGAGA ATATCGGCCAGCTGCTGGGCGTGCAGTCCATCCAGGTGTCTCTGGAGAACAA GACAGCCCAGGTGAAGTACGATCCTTCTTGCACCAGCCCAGTGGCCCTGCAG AGGGCAATCGAGGCCCTGCCCCCTGGCAATTTCAAGGTGTCCCTGCCTGACG GAGCAGAGGGCTCTGGCACCGATCACCGGAGCAGCAGCAGCCACTCCCCAGG CTCTCCACCCAGAAACCAGGTGCAGGGCACATGTTCTACCACACTGATCGCA | **ATP7B variant 3** |
| | ATCGCAGGAATGACCTGCGCCAGCTGCGTGCACTCCATCGAGGGCATGATCA GCCAGCTGGAGGGCGTGCAGCAGATCAGCGTGTCCCTGGCCGAGGGCACCGC AACAGTGCTGTACAATCCCAGCGTGATCTCCCCTGAGGAGCTGAGGGCAGCA ATCGAGGATATGGGATTTGAGGCCTCCGTGGTGTCTGAGAGCTGCTCCACAA ACCCCCTGGGCAATCACTCTGCCGGCAACAGCATGGTGCAGACCACAGACGG CACCCCTACAAGCGTGCAGGAGGTGGCACCACACACCGGCCGGCTGCCAGCA AATCACGCACCAGACATCCTGGCCAAGTCTCCCCAGAGCACAAGAGCCGTGG CCCCTCAGAAGTGTTTTCTGCAGATCAAGGGCATGACCTGCGCCTCCTGCGT GAGCAACATCGAGCGGAATCTGCAGAAGGAGGCAGGCGTGCTGTCCGTGCTG GTGGCCCTGATGGCAGGCAAGGCCGAGATCAAGTACGACCCTGAAGTGATCC AGCCACTGGAGATCGCCCAGTTCATCCAGGATCTGGGCTTTGAGGCCGCCGT GATGGAGGACTATGCCGGCAGCGATGGCAACATCGAGCTGACCATCACAGGC ATGACCTGCGCCTCTTGCGTGCACAACATCGAGAGCAAGCTGACCAGAACAA ATGGCATCACATACGCCTCTGTGGCCCTGGCCACCAGCAAGGCCCTGGTGAA GTTCGACCCCGAGATCATCGGCCCCCGGGACATCATCAAGATCATCGAGGAG ATCGGCTTTCACGCCTCCCTGGCCCAGCGCAACCCAAATGCCCACCACCTGG ACCACAAGATGGAGATCAAGCAGTGGAAGAAGTCCTTCCTGTGCTCTCTGGT GTTTGGCATCCCCGTGATGGCCCTGATGATCTACATGCTGATCCCTTCCAAC GAGCCACACCAGTCTATGGTGCTGGATCACAACATCATCCCTGGCCTGAGCA TCCTGAATCTGATCTTCTTTATCCTGTGCACATTCGTGCAGCTGCTGGGCGG CTGGTACTTTTATGTGCAGGCCTATAAGTCCCTGCGGCACCGGAGCGCCAAT ATGGACGTGCTGATCGTGCTGGCCACCAGCATCGCCTACGTGTATAGCCTGG TCATCCTGGTGGTGGCAGTGGCAGAGAAGGCAGAGCGGTCCCCCGTGACCTT CTTTGATACACCTCCAATGCTGTTCGTGTTTATCGCCCTGGGCAGATGGCTG GAGCACCTGGCCAAGAGCAAGACCTCCGAGGCCCTGGCCAAGCTGATGAGCC TGCAGGCCACAGAGGCCACCGTGGTGACACTGGGCGAGGACAACCTGATCAT CAGGGAGGAGCAGGTGCCTATGGAGCTGGTGCAGCGCGGCGACATCGTGAAG GTGGTGCCAGGCGGCAAGTTCCCAGTGGACGGCAAGGTGCTGGAGGGCAATA CAATGGCCGATGAGAGCCTGATCACCGGCGAGGCCATGCCTGTGACCAAGAA GCCAGGCTCTACAGTGATCGCAGGCAGCATCAACGCCCACGGCTCCGTGCTG ATCAAGGCCACCCACGTGGGCAATGACACCACACTGGCCCAGATCGTGAAGC TGGTGGAGGAGGCCCAGATGTCCAAGGCCCCTATCCAGCAGCTGGCCGATCG GTTCTCCGGCTACTTCGTGCCCTTCATCATCATCATGTCTACCCTGACACTG GTGGTGTGGATCGTGATCGGCTTCATCGACTTTGGCGTGGTGCAGAGGTATT TTCCCAACCCTAATAAGCACATCAGCCAGACCGAAGTGATCATCCGCTTCGC CTTTCAGACCAGCATCACAGTGCTGTGCATCGCATGCCCATGTTCCCTGGGC CTGGCCACCCCAACAGCCGTGATGGTGGGCACAGGCGTGGCAGCACAGAACG GCATCCTGATCAAGGGCGGCAAGCCCCTGGAGATGGCCCACAAGATCAAGAC CGTGATGTTTGACAAGACCGGCACAATCACCCACGGCGTGCCCAGAGTGATG AGAGTGCTGCTGCTGGGCGATGTGGCCACACTGCCTCTGAGAAAGGTGCTGG CCGTGGTGGGCACCGCCGAGGCCTCCTCTGAGCACCCACTGGGAGTGGCCGT GACAAAGTACTGCAAGGAGGAGCTGGGCACAGAGACACTGGGCTATTGTACC GACTTCCAGGCCGTGCCCGGATGCGGAATCGGCTGTAAGGTGAGCAACGTGG AGGGCATCCTGGCACACTCCGAGCGGCCCCTGAGCGCCCCTGCCTCCCACCT GAATGAGGCAGGCTCTCTGCCCGCCGAGAAGGACGCCGTGCCTCAGACCTTC AGCGTGCTGATCGGCAACAGAGAGTGGCTGCGGAGAAATGGCCTGACCATCA GCTCCGACGTGTCCGATGCCATGACAGATCACGAGATGAAGGGCCAGACCGC AATCCTGGTGGCAATCGACGGCGTGCTGTGCGGCATGATCGCCATCGCCGAT GCAGTGAAGCAGGAGGCCGCCCTGGCCGTGCACACCCTGCAGAGCATGGGCG TGGACGTGGTGCTGATCACCGGCGATAACAGGAAGACAGCCAGAGCCATCGC CACCCAAGTGGGCATCAATAAGGTGTTCGCCGAGGTGCTGCCTTCCCACAAG GTGGCCAAGGTGCAGGAGCTGCAGAACAAGGGCAAGAAGGTGGCTATGGTGG | |
| | GCGACGGCGTGAATGATTCTCCAGCCCTGGCCCAGGCAGACATGGGAGTGGC AATCGGCACAGGCACCGACGTGGCAATCGAGGCCGCCGATGTGGTGCTGATC AGGAATGACCTGCTGGATGTGGTGGCCTCTATCCACCTGAGCAAGCGGACCG TGAGGCGCATCAGAATCAACCTGGTGCTGGCCCTGATCTACAATCTGGTGGG CATCCCAATCGCCGCCGGCGTGTTTATGCCAATCGGCATCGTGCTGCAGCCC TGGATGGGCAGCGCCGCTATGGCCGCCTCTAGCGTGAGCGTGGTGCTGTCCT CTCTGCAGCTGAAGTGCTACAAGAAGCCCGACCTGGAGCGGTACGAGGCACA GGCCCACGGCCACATGAAGCCACTGACCGCCTCTCAGGTGAGCGTGCACATC GGCATGGACGATAGGTGGAGGGACAGCCCAAGGGCCACACCCTGGGATCAGG TGAGCTACGTGAGCCAGGTGAGCCTGAGCAGCCTGACCAGCGACAAGCCCTC CCGCCACAGCGCCGCCGCCGACGACGACGGGGACAAGTGGAGCCTGCTGCTG AACGGGAGGGATGAAGAACAGTACATTTGA | |
| 6 | ATGCCCGAACAGGAAAGACAGATTACTGCCAGGGAAGGAGCCTCACGGAAGA TTCTCTCAAAACTCTCACTGCCTACACGAGCTTGGGAGCCCGCTATGAAGAA GTCCTTCGCCTTTGACAACGTGGGATACGAGGGCGGACTGGATGGCCTGGGA CCTAGCTCCCAGGTGGCCACCAGCACCGTGAGAATCCTGGGCATGACATGCC AGAGCTGCGTGAAGTCCATCGAGGACAGAATCTCTAACCTGAAGGGAATCAT CTCTATGAAGGTGAGCCTGGAGCAGGGCTCCGCCACCGTGAAGTACGTGCCT TCTGTGGTGTGCCTGCAGCAGGTGTGCCACCAGATCGGAGATATGGGCTTCG AGGCCAGCATCGCTGAGGGAAAGGCCGCTTCCTGGCCTTCTAGGAGCCTGCC AGCCCAGGAGGCTGTGGTGAAGCTGAGAGTGGAAGGTATGACCTGTCAGAGC TGCGTGAGCAGCATCGAGGGAAAGGTGCGGAAGCTGCAGGGAGTGGTGCGCG TGAAGGTGTCCCTGTCTAACCAGGAGGCCGTGATCACCTACCAGCCCTACCT GATCCAGCCTGAGGACCTGAGGGATCACGTGAACGACATGGGATTCGAGGCC GCTATCAAGAGCAAGGTGGCTCCACTGTCCCTGGGACCAATCGACATCGAGA GGCTGCAGTCCACCAACCCAAAGAGGCCCCTGAGCAGCGCCAACCAGAACTT TAACAACTCTGAGACACTGGGACACCAGGGCAGCCACGTGGTGACCCTGCAG CTGCGGATCGACGGCATGCACTGCAAGTCTTGCGTGCTGAACATCGAGGAGA ACATCGGACAGCTGCTGGGCGTGCAGTCCATCCAGGTGTCTCTGGAGAACAA GACAGCTCAGGTGAAGTACGATCCTTCTTGCACCAGCCCAGTGGCCCTGCAG AGGGCTATCGAGGCTCTGCCCCCTGGAAACTTCAAGGTGTCCCTGCCTGACG GAGCTGAGGGCTCTGGAACCGATCACAGGAGCAGCAGCAGCCACTCCCCAGG ATCTCCACCCAGGAACCAGGTGCAGGGCACATGTTCTACCACACTGATCGCT ATCGCTGGAATGACCTGCGCCAGCTGCGTGCACTCCATCGAGGGAATGATCA GCCAGCTGGAGGGCGTGCAGCAGATCAGCGTGTCCCTGGCTGAGGGAACCGC CACAGTGCTGTACAACCCCAGCGTGATCTCCCCTGAGGAGCTGAGAGCCGCT ATCGAGGACATGGGTTTTGAGGCCAGCGTGGTGTCTGAGAGCTGCTCCACAA ACCCACTGGGAAACCACTCTGCCGGCAACAGCATGGTGCAGACCACAGACGG AACCCCTACATCCGTGCAGGAGGTGGCTCCACACACCGGCAGGCTGCCAGCT AACCACGCTCCAGACATCCTGGCCAAGTCTCCCCAGAGCACAAGAGCTGTGG CCCCTCAGAAGTGTTTTCTGCAGATCAAGGGCATGACCTGCGCTTCCTGCGT GAGCAACATCGAGAGGAACCTGCAGAAGGAGGCTGGAGTGCTGTCCGTGCTG GTGGCCCTGATGGCTGGCAAGGCCGAGATCAAGTACGACCCTGAAGTGATCC AGCCACTGGAGATCGCCCAGTTCATCCAGGATCTGGGCTTTGAGGCCGCTGT GATGGAGGACTACGCTGGAAGCGATGGCAACATCGAGCTGACCATCACAGGA ATGACCTGCGCCTCTTGCGTGCACAACATCGAGAGCAAGCTGACCAGAACAA ACGGCATCACATACGCTTCTGTGGCTCTGGCCACCAGCAAGGCCCTGGTGAA GTTCGACCCCGAGATCATCGGACCCAGGGACATCATCAAGATCATCGAGGAG ATCGGCTTTCACGCTTCCCTGGCCCAGAGGAACCCAAACGCTCACCACCTGG ACCACAAGATGGAGATCAAGCAGTGGAAGAAGTCCTTCCTGTGCTCTCTGGT GTTTGGAATCCCCGTGATGGCCCTGATGATCTACATGCTGATCCCTTCCAAC | **ATP7B variant 4** |
| | GAGCCACACCAGTCTATGGTGCTGGATCACAACATCATCCCTGGCCTGAGCA TCCTGAACCTGATCTTCTTTATCCTGTGCACATTCGTGCAGCTGCTGGGCGG ATGGTACTTTTACGTGCAGGCTTACAAGTCCCTGAGGCACAGGAGCGCCAAC ATGGACGTGCTGATCGTGCTGGCTACCAGCATCGCCTACGTGTACAGCCTGG TCATCCTGGTGGTGGCTGTGGCTGAGAAGGCTGAGAGGAGCCCCGTGACCTT CTTCGATACACCTCCAATGCTGTTCGTGTTTATCGCTCTGGGCAGATGGCTG GAGCACCTGGCCAAGAGCAAGACCTCCGAGGCTCTGGCCAAGCTGATGAGCC TGCAGGCTACAGAGGCCACCGTGGTGACACTGGGAGAGGACAACCTGATCAT CCGGGAGGAGCAGGTGCCTATGGAGCTGGTGCAGCGCGGCGACATCGTGAAG GTGGTGCCAGGCGGAAAGTTCCCCGTGGACGGAAAGGTGCTGGAGGGCAACA CAATGGCTGATGAGAGCCTGATCACCGGCGAGGCCATGCCTGTGACCAAGAA GCCAGGATCTACAGTGATCGCTGGCAGCATCAACGCCCACGGCAGCGTGCTG ATCAAGGCTACACACGTGGGCAACGACACCACACTGGCCCAGATCGTGAAGC TGGTGGAGGAGGCTCAGATGTCCAAGGCCCCTATCCAGCAGCTGGCCGATCG GTTCTCCGGCTACTTCGTGCCCTTCATCATCATCATGTCTACCCTGACACTG GTGGTGTGGATCGTGATCGGATTCATCGACTTTGGCGTGGTGCAGCGGTACT TTCCCAACCCTAACAAGCACATCAGCCAGACCGAAGTGATCATCCGCTTCGC TTTTCAGACCAGCATCACAGTGCTGTGCATCGCCTGCCCATGTTCCCTGGGA CTGGCTACCCCCACAGCCGTGATGGTGGGCACAGGAGTGGCCGCTCAGAACG GCATCCTGATCAAGGGCGGAAAGCCTCTGGAGATGGCCCACAAGATCAAGAC CGTGATGTTCGACAAGACCGGAACAATCACCCACGGCGTGCCCAGAGTGATG AGAGTGCTGCTGCTGGGCGATGTGGCTACACTGCCTCTGAGAAAGGTGCTGG CCGTGGTGGGAACCGCTGAGGCCTCCTCTGAGCACCCACTGGGAGTGGCTGT GACAAAGTACTGCAAGGAGGAGCTGGGAACAGAGACACTGGGCTACTGTACC GACTTCCAGGCCGTGCCCGGATGCGGAATCGGCTGTAAGGTGAGCAACGTGG AGGGAATCCTGGCTCACTCCGAGAGGCCCCTGAGCGCCCCTGCCTCCCACCT GAACGAGGCTGGCTCTCTGCCCGCTGAGAAGGACGCCGTGCCTCAGACCTTC AGCGTGCTGATCGGAAACAGAGAGTGGCTGAGGAGAAACGGCCTGACCATCA GCTCCGACGTGTCCGATGCCATGACAGATCACGAGATGAAGGGCCAGACCGC TATCCTGGTGGCCATCGACGGAGTGCTGTGCGGCATGATCGCTATCGCCGAT GCTGTGAAGCAGGAGGCCGCTCTGGCCGTGCACACCCTGCAGAGCATGGGAG TGGACGTGGTGCTGATCACCGGCGATAACCGGAAGACAGCCCGCGCTATCGC TACCCAAGTGGGCATCAACAAGGTGTTCGCTGAGGTGCTGCCTTCCCACAAG GTGGCCAAGGTGCAGGAGCTGCAGAACAAGGGAAAGAAGGTGGCTATGGTGG GAGACGGCGTGAACGATTCTCCAGCTCTGGCCCAGGCTGACATGGGCGTGGC TATCGGAACAGGCACCGACGTGGCTATCGAGGCCGCTGATGTGGTGCTGATC CGGAACGACCTGCTGGATGTGGTGGCTTCTATCCACCTGAGCAAGAGGACAG TGCGGCGCATCAGAATCAACCTGGTGCTGGCCCTGATCTACAACCTGGTGGG CATCCCAATCGCCGCTGGAGTGTTTATGCCAATCGGCATCGTGCTGCAGCCC TGGATGGGCTCTGCCGCTATGGCCGCTTCTAGCGTGAGCGTGGTGCTGTCCT CTCTGCAGCTGAAGTGTTACAAGAAGCCCGACCTGGAGAGGTACGAGGCTCA GGCTCACGGACACATGAAGCCCCTGACCGCTTCTCAGGTGAGCGTGCACATC GGAATGGACGATCGGTGGAGGGACAGCCCACGGGCCACACCCTGGGATCAGG TGTCCTACGTGTCTCAGGTGAGCCTGAGCAGCCTGACCAGCGACAAGCCCTC CCGCCACTCTGCCGCTGCCGATGACGATGGTGACAAGTGGAGTCTGCTGCTG AACGGGCGAGATGAGGAACAGTACATTTGA | |
| **7** | ATGCCCGAACAGGAGCGCCAGATTACTGCCAGAGAGGGAGCATCCAGAAAAA TCCTGAGCAAACTGTCACTGCCCACACGAGCTTGGGAACCCGCAATGAAGAA AAGCTTCGCCTTTGACAACGTGGGATACGAGGGAGGACTGGATGGACTGGGA CCTAGCTCCCAGGTGGCCACCTCTACAGTCCGAATCCTGGGCATGACTTGCC AGAGTTGCGTGAAATCAATTGAAGACCGGATCAGTAATCTGAAGGGAATCAT | **Codon optimized ATP7B variant (SEQ ID** |
| | TAGCATGAAAGTGTCCCTGGAGCAGGGCTCAGCCACCGTGAAGTATGTCCCT AGCGTGGTCTGCCTGCAGCAGGTGTGCCACCAGATCGGCGATATGGGGTTCG AGGCCTCCATTGCTGAAGGGAAAGCCGCTTCTTGGCCTAGCCGGTCCCTGCC AGCACAGGAAGCAGTGGTCAAGCTGAGAGTGGAGGGAATGACATGCCAGAGC TGCGTGAGCAGTATCGAAGGAAAGGTCCGAAAACTGCAGGGCGTGGTCCGGG TGAAGGTCTCTCTGAGTAACCAGGAGGCCGTGATTACCTACCAGCCCTATCT GATCCAGCCTGAAGACCTGAGGGATCACGTGAATGACATGGGCTTCGAGGCA GCCATCAAGTCCAAAGTGGCCCCACTGTCTCTGGGGCCCATTGACATCGAAA GACTGCAGTCCACCAACCCAAAGAGGCCCCTGTCAAGCGCCAACCAGAACTT CAACAATAGTGAGACCCTGGGACACCAGGGCTCACATGTGGTCACACTGCAG CTGAGGATTGACGGCATGCACTGCAAGTCTTGCGTGCTGAACATTGAGGAAA ATATCGGCCAGCTGCTGGGGGTGCAGTCTATCCAGGTCAGTCTGGAGAACAA GACTGCTCAGGTGAAATACGATCCTTCATGCACCAGCCCAGTGGCACTGCAG CGCGCTATCGAAGCACTGCCCCCTGGAAATTTCAAGGTGAGCCTGCCTGACG GAGCAGAGGGATCCGGAACCGATCACAGGTCCTCTAGTTCACATTCCCCAGG ATCTCCACCACGAAACCAGGTGCAGGGAACATGTTCCACCACACTGATTGCA ATCGCCGGCATGACTTGCGCCTCATGCGTGCACAGCATTGAAGGGATGATCT CTCAGCTGGAGGGAGTGCAGCAGATCTCAGTCAGCCTGGCCGAGGGCACTGC TACCGTGCTGTACAATCCCAGTGTCATCTCACCTGAGGAACTGCGGGCTGCA ATTGAGGACATGGGGTTCGAAGCTTCCGTGGTCTCCGAATCTTGCAGTACCA ACCCCCTGGGGAATCATTCCGCCGGAAACTCTATGGTGCAGACTACCGACGG GACACCTACTTCTGTGCAGGAGGTCGCACCACACACAGGACGCCTGCCAGCC AATCATGCTCCCGACATCCTGGCCAAAAGCCCCCAGTCCACCCGGGCTGTGG CACCTCAGAAGTGTTTTCTGCAGATCAAAGGCATGACCTGCGCCTCTTGCGT GAGCAACATTGAGCGGAATCTGCAGAAGGAAGCTGGGGTGCTGAGCGTGCTG GTCGCACTGATGGCCGGAAAGGCTGAGATCAAGTACGACCCTGAAGTGATCC AGCCACTGGAGATTGCCCAGTTCATCCAGGATCTGGGCTTTGAGGCCGCTGT GATGGAAGACTATGCTGGGAGCGATGGAAACATTGAACTGACCATCACAGGC ATGACTTGTGCCTCTTGCGTGCACAACATCGAGAGTAAACTGACTAGAACCA ATGGGATTACCTACGCCAGTGTGGCCCTGGCTACATCAAAGGCTCTGGTGAA ATTCGACCCCGAGATCATTGGACCTAGGGACATCATCAAGATCATTGAGGAA ATCGGCTTTCACGCAAGCCTGGCCCAGCGCAACCCAAATGCCCACCATCTGG ACCATAAGATGGAGATCAAGCAGTGGAAGAAAAGTTTCCTGTGCTCACTGGT GTTTGGAATCCCCGTCATGGCCCTGATGATCTACATGCTGATCCCTAGCAAC GAGCCACACCAGTCCATGGTGCTGGATCATAACATCATTCCTGGCCTGTCCA TCCTGAATCTGATTTTCTTTATCCTGTGCACATTCGTGCAGCTGCTGGGAGG CTGGTACTTTTATGTGCAGGCATATAAATCACTGCGACACCGGAGCGCCAAT ATGGACGTGCTGATTGTCCTGGCAACCTCTATCGCCTACGTGTATAGTCTGG TCATCCTGGTGGTCGCAGTGGCAGAGAAGGCAGAACGGAGCCCCGTGACTTT CTTTGATACCCCTCCAATGCTGTTCGTGTTTATCGCTCTGGGCAGATGGCTG GAACATCTGGCAAAGTCAAAAACCAGCGAGGCTCTGGCAAAGCTGATGAGCC TGCAGGCTACCGAAGCAACAGTGGTCACTCTGGGAGAGGACAACCTGATCAT TCGCGAGGAACAGGTGCCTATGGAACTGGTCCAGCGAGGCGACATCGTGAAG GTGGTCCCAGGGGGAAAATTCCCCGTGGACGGCAAGGTCCTGGAGGGGAATA CTATGGCCGATGAATCCCTGATCACCGGCGAGGCTATGCCTGTGACAAAGAA ACCAGGATCAACTGTCATTGCTGGCAGCATCAACGCACACGGGTCCGTGCTG ATCAAGGCCACACATGTCGGGAATGACACAACTCTGGCTCAGATTGTGAAAC TGGTCGAGGAAGCCCAGATGTCCAAGGCTCCTATCCAGCAGCTGGCCGATCG GTTCTCCGGCTACTTCGTGCCCTTCATCATTATCATGTCTACACTGACTCTG GTGGTCTGGATTGTGATCGGATTCATTGACTTTGGCGTGGTCCAGAGATATT TTCCCAACCCTAATAAGCACATCAGCCAGACCGAAGTGATCATCAGGTTCGC ATTTCAGACCAGTATTACAGTGCTGTGCATCGCCTGCCCATGTTCACTGGGG | **NO: 1 from** WO 2017/1036 24**)** |
| | CTGGCTACCCCCACAGCAGTGATGGTCGGAACAGGGGTGGCAGCACAGAACG GCATCCTGATCAAGGGCGGGAAACCCCTGGAGATGGCCCACAAGATCAAAAC TGTGATGTTTGACAAAACTGGGACCATTACACATGGAGTGCCACGCGTCATG CGAGTGCTGCTGCTGGGCGATGTGGCAACCCTGCCTCTGAGAAAGGTCCTGG CAGTGGTCGGAACAGCAGAGGCTAGCTCCGAACACCCACTGGGGGTGGCCGT CACAAAGTACTGCAAAGAGGAACTGGGCACTGAGACCCTGGGGTATTGTACT GACTTCCAGGCAGTGCCAGGATGCGGAATCGGATGTAAAGTCTCTAACGTGG AAGGGATTCTGGCTCACAGTGAGCGGCCCCTGAGCGCACCTGCATCCCATCT GAATGAAGCAGGAAGCCTGCCAGCAGAGAAGGACGCTGTGCCTCAGACCTTT TCCGTCCTGATCGGCAACAGAGAATGGCTGCGGAGAAATGGGCTGACAATTT CTAGTGACGTGTCCGATGCCATGACAGATCACGAGATGAAAGGCCAGACTGC AATTCTGGTGGCCATCGACGGAGTCCTGTGCGGCATGATTGCTATCGCAGAT GCCGTGAAGCAGGAGGCTGCACTGGCCGTCCATACCCTGCAGTCTATGGGCG TGGACGTGGTCCTGATCACCGGGGATAACCGGAAAACAGCTAGAGCAATTGC CACTCAAGTGGGCATCAATAAGGTGTTCGCTGAAGTCCTGCCTAGCCACAAG GTCGCAAAAGTGCAGGAGCTGCAGAACAAGGGCAAGAAAGTCGCCATGGTGG GAGACGGCGTGAATGATAGCCCAGCTCTGGCACAGGCAGACATGGGAGTCGC TATTGGGACAGGAACTGACGTGGCAATCGAGGCCGCTGATGTGGTCCTGATT AGGAATGACCTGCTGGATGTGGTCGCTTCTATTCATCTGAGTAAGAGGACAG TGAGGCGCATTCGCATCAACCTGGTGCTGGCCCTGATCTACAATCTGGTGGG CATCCCCATCGCAGCAGGCGTGTTTATGCCAATTGGGATCGTCCTGCAGCCC TGGATGGGCTCAGCTGCAATGGCCGCTTCAAGCGTGAGCGTGGTCCTGTCCT CTCTGCAGCTGAAATGCTACAAGAAACCAGATCTGGAGCGGTACGAAGCTCA GGCACACGGACATATGAAGCCCCTGACCGCTTCCCAGGTGTCTGTCCACATC GGCATGGACGATAGATGGAGGGACAGCCCAAGGGCAACTCCATGGGATCAGG TCAGTTACGTGAGCCAGGTCAGCCTGAGTTCACTGACCAGCGACAAGCCCTC CCGCCATTCTGCAGCCGCTGATGACGACGGGGACAAGTGGAGCCTGCTGCTG AACGGGAGGGACGAAGAACAGTACATTTGA | |
| **8** | ATGCCCGAGCAGGAAAGACAGATCACCGCCAGAGAGGGCGCCAGCCGGAAGA TCCTGAGCAAGCTGAGCCTGCCCACCAGAGCCTGGGAGCCCGCCATGAAGAA GTCCTTCGCCTTCGACAACGTGGGCTACGAGGGCGGCCTGGACGGACTGGGA CCATCTTCTCAGGTGGCCACAAGCACCGTGCGCATCCTGGGCATGACCTGCC AGAGCTGCGTGAAGTCCATCGAGGACCGGATCAGCAACCTGAAGGGCATCAT CAGCATGAAGGTGTCCCTGGAACAGGGCAGCGCCACCGTGAAATACGTGCCC TCTGTCGTGTGCCTGCAGCAGGTGTGCCACCAGATCGGCGACATGGGCTTCG AGGCCTCTATCGCCGAGGGAAAGGCCGCCTCTTGGCCCTCTAGAAGCCTGCC TGCTCAGGAAGCCGTCGTGAAGCTGCGGGTGGAAGGGATGACCTGTCAGTCC TGCGTGTCCAGCATCGAGGGCAAAGTGCGGAAGCTGCAGGGCGTGGTGCGCG TGAAAGTGTCTCTGAGCAACCAGGAAGCTGTGATCACCTACCAGCCCTACCT GATCCAGCCCGAGGACCTGAGGGACCACGTGAACGATATGGGATTTGAGGCC GCCATCAAGAGCAAGGTGGCCCCTCTGTCTCTGGGCCCCATCGACATCGAGA GACTGCAGAGCACCAACCCCAAGCGGCCTCTGAGCAGCGCCAACCAGAACTT CAACAACAGCGAGACACTGGGCCACCAGGGCAGCCACGTCGTGACACTGCAG CTGCGGATCGACGGAATGCACTGCAAGAGCTGTGTGCTGAACATCGAGGAAA ACATCGGCCAGCTGCTGGGAGTGCAGAGCATCCAGGTGTCACTGGAAAACAA GACCGCCCAGGTGAAGTACGACCCCAGCTGCACAAGCCCCGTGGCCCTGCAG AGAGCCATTGAAGCTCTGCCCCCTGGCAACTTCAAAGTGTCCCTGCCTGACG GAGCCGAGGGCTCCGGAACAGATCACAGAAGCAGCAGCAGCCACAGCCCTGG CAGCCCCCCTAGAAATCAGGTGCAGGGCACCTGTAGCACCACCCTGATCGCC ATTGCCGGCATGACATGCGCCAGCTGCGTGCACTCTATTGAGGGCATGATCT CCCAGCTGGAAGGCGTGCAGCAGATCAGTGTGTCTCTGGCCGAGGGCACCGC CACAGTGCTGTACAACCCTAGCGTGATCAGCCCCGAAGAACTGAGAGCCGCC | **Codon optimized ATP7B variant (SEQ ID NO: 1 from** WO 2018/1261 16**)** |
| | ATTGAGGACATGGGATTCGAAGCCAGCGTGGTGTCCGAGAGCTGCTCCACCA ACCCTCTGGGCAATCACAGCGCCGGCAACAGCATGGTGCAGACCACCGACGG CACCCCCACCAGCGTGCAGGAAGTGGCCCCACATACAGGCAGACTGCCCGCC AATCACGCCCCCGATATCCTGGCCAAGAGCCCCCAGAGTACAAGAGCCGTGG CCCCCCAGAAGTGCTTCCTGCAGATCAAGGGCATGACTTGTGCCTCTTGTGT GTCCAATATCGAGCGGAACCTGCAGAAAGAGGCCGGCGTGCTGTCTGTGCTG GTGGCTCTGATGGCCGGCAAGGCCGAGATCAAATACGACCCCGAAGTGATTC AGCCCCTGGAAATCGCCCAGTTTATCCAGGACCTGGGCTTTGAAGCCGCCGT GATGGAAGATTACGCCGGCTCCGACGGCAACATCGAGCTGACCATCACCGGA ATGACCTGCGCCTCCTGTGTGCACAACATTGAGTCCAAGCTGACCCGGACCA ACGGCATCACCTACGCCTCTGTGGCTCTGGCCACCTCCAAGGCCCTCGTGAA GTTCGATCCCGAGATCATCGGCCCCAGGGACATCATCAAGATCATCGAAGAG ATCGGCTTCCACGCCAGCCTGGCCCAGAGGAACCCTAACGCCCACCACCTGG ACCACAAGATGGAAATCAAGCAGTGGAAGAAAAGCTTCCTGTGCAGCCTGGT GTTCGGCATCCCCGTGATGGCCCTGATGATCTACATGCTGATCCCCAGCAAC GAGCCCCACCAGTCCATGGTGCTGGATCACAACATCATCCCCGGCCTGTCTA TCCTGAACCTGATCTTCTTCATCCTGTGCACCTTCGTGCAGCTGCTGGGCGG CTGGTACTTCTACGTGCAGGCCTACAAGTCCCTGCGGCACAGATCCGCCAAC ATGGACGTGCTGATCGTGCTGGCCACATCTATCGCCTACGTGTACTCCCTCG TGATCCTGGTGGTGGCCGTGGCCGAGAAAGCCGAGAGAAGCCCTGTGACCTT CTTCGACACCCCCCCTATGCTGTTCGTGTTTATCGCCCTGGGCCGGTGGCTG GAACACCTGGCCAAAAGCAAGACCAGCGAGGCCCTGGCTAAGCTGATGAGTC TGCAGGCCACCGAGGCCACAGTCGTGACCCTGGGCGAGGACAACCTGATCAT CCGCGAGGAACAGGTGCCAATGGAACTGGTGCAGCGGGGCGACATCGTGAAG GTGGTGCCTGGCGGCAAGTTCCCCGTGGACGGAAAAGTGCTGGAAGGGAATA CCATGGCCGACGAGAGCCTGATCACAGGCGAGGCCATGCCCGTGACCAAGAA ACCTGGCAGCACAGTGATCGCCGGCAGCATCAATGCCCACGGCAGCGTGCTG ATTAAGGCCACACACGTGGGCAACGATACCACCCTGGCTCAGATTGTGAAGC TGGTGGAAGAGGCCCAGATGAGCAAGGCCCCCATTCAGCAGCTGGCTGACCG GTTCAGCGGCTACTTCGTGCCCTTTATCATCATCATGAGCACCCTGACACTG GTCGTGTGGATCGTGATCGGCTTTATCGACTTCGGAGTGGTGCAGAGATACT TCCCCAACCCTAACAAGCACATCAGCCAGACAGAAGTGATCATCAGATTCGC CTTTCAGACCAGCATCACCGTGCTGTGTATCGCCTGCCCCTGTAGCCTGGGA CTGGCCACACCTACCGCTGTGATGGTGGGAACAGGCGTGGCCGCTCAGAACG GCATCCTGATCAAGGGGGGCAAGCCTCTGGAAATGGCTCACAAGATCAAGAC CGTGATGTTCGACAAGACCGGCACCATCACCCACGGCGTGCCCAGAGTGATG AGAGTGCTGCTGCTGGGGGATGTGGCCACCCTGCCTCTGAGAAAGGTGCTGG CTGTCGTGGGCACAGCCGAGGCTAGCTCTGAACACCCACTGGGAGTGGCCGT GACAAAGTACTGCAAAGAGGAACTGGGCACCGAAACCCTGGGCTACTGCACC GACTTTCAGGCCGTGCCTGGCTGTGGCATCGGCTGCAAGGTGTCCAACGTGG AAGGCATCCTGGCCCACAGCGAGAGGCCACTGTCTGCCCCTGCCAGCCACCT GAACGAGGCCGGATCTCTGCCCGCCGAAAAGGACGCTGTGCCCCAGACCTTC TCTGTGCTGATTGGCAACAGAGAGTGGCTGCGGCGGAACGGCCTGACCATCT CCTCCGATGTGTCCGACGCCATGACCGACCACGAGATGAAGGGCCAGACCGC CATTCTGGTGGCCATTGACGGGGTGCTGTGCGGCATGATCGCAATCGCCGAT GCCGTGAAACAGGAAGCAGCACTGGCCGTGCACACCCTGCAGTCTATGGGAG TGGATGTGGTGCTGATCACCGGCGACAACAGAAAGACCGCCAGGGCCATTGC CACCCAGGTGGGCATCAACAAGGTGTTCGCCGAGGTGCTGCCCAGCCACAAA GTGGCCAAGGTGCAGGAACTGCAGAACAAAGGCAAAAAGGTGGCCATGGTGG GAGATGGCGTGAACGACTCTCCTGCTCTGGCCCAGGCAGATATGGGCGTGGC CATCGGCACAGGCACCGACGTGGCAATTGAGGCTGCTGACGTGGTGCTGATT CGGAACGACCTGCTGGACGTGGTGGCCTCCATCCACCTGTCCAAGAGAACCG | |
| | TGCGGCGGATCAGAATCAACCTGGTGCTGGCACTGATCTATAACCTCGTGGG CATCCCTATCGCCGCTGGCGTGTTCATGCCTATCGGAATCGTGCTGCAGCCC TGGATGGGCTCTGCCGCCATGGCTGCAAGCTCCGTGTCTGTGGTGCTGTCCA GCCTGCAGCTGAAGTGCTACAAGAAGCCCGACCTGGAAAGATACGAGGCCCA GGCCCACGGACACATGAAGCCTCTGACAGCCTCCCAGGTGTCCGTGCACATC GGCATGGACGACAGATGGCGGGACAGCCCTAGAGCCACCCCTTGGGATCAGG TGTCATACGTGTCACAGGTGTCCCTGAGCAGCCTGACCAGCGACAAGCCCAG CAGACATAGCGCCGCTGCCGACGACGATGGGGACAAGTGGTCCCTGCTGCTG AACGGCCGGGATGAGGAACAGTACATC | |
| **9** | ATGCCCGAGCAGGAAAGACAGATCACCGCCAGAGAGGGCGCCAGCCGGAAGA TCCTGAGCAAGCTGAGCCTGCCCACCAGAGCCTGGGAGCCCGCCATGAAGAA GTCCTTCGCCTTCGACAACGTGGGCTACGAGGGCGGCCTGGACGGACTGGGA CCATCTTCTCAGGTGGCCACAAGCACCGTGCGCGAGGCCGCCATCAAGAGCA AGGTGGCCCCTCTGTCTCTGGGCCCCATCGACATCGAGAGACTGCAGAGCAC CAACCCCAAGCGGCCTCTGAGCAGCGCCAACCAGAACTTCAACAACAGCGAG ACACTGGGCCACCAGGGCAGCCACGTCGTGACACTGCAGCTGCGGATCGACG GAATGCACTGCAAGAGCTGTGTGCTGAACATCGAGGAAAACATCGGCCAGCT GCTGGGAGTGCAGAGCATCCAGGTGTCACTGGAAAACAAGACCGCCCAGGTG AAGTACGACCCCAGCTGCACAAGCCCCGTGGCCCTGCAGAGAGCCATTGAAG CTCTGCCCCCTGGCAACTTCAAAGTGTCCCTGCCTGACGGAGCCGAGGGCTC CGGAACAGATCACAGAAGCAGCAGCAGCCACAGCCCTGGCAGCCCCCCTAGA AATCAGGTGCAGGGCACCTGTAGCACCACCCTGATCGCCATTGCCGGCATGA CATGCGCCAGCTGCGTGCACTCTATTGAGGGCATGATCTCCCAGCTGGAAGG CGTGCAGCAGATCAGTGTGTCTCTGGCCGAGGGCACCGCCACAGTGCTGTAC AACCCTAGCGTGATCAGCCCCGAAGAACTGAGAGCCGCCATTGAGGACATGG GATTCGAAGCCAGCGTGGTGTCCGAGAGCTGCTCCACCAACCCTCTGGGCAA TCACAGCGCCGGCAACAGCATGGTGCAGACCACCGACGGCACCCCCACCAGC GTGCAGGAAGTGGCCCCACATACAGGCAGACTGCCCGCCAATCACGCCCCCG ATATCCTGGCCAAGAGCCCCCAGAGTACAAGAGCCGTGGCCCCCCAGAAGTG CTTCCTGCAGATCAAGGGCATGACTTGTGCCTCTTGTGTGTCCAATATCGAG CGGAACCTGCAGAAAGAGGCCGGCGTGCTGTCTGTGCTGGTGGCTCTGATGG CCGGCAAGGCCGAGATCAAATACGACCCCGAAGTGATTCAGCCCCTGGAAAT CGCCCAGTTTATCCAGGACCTGGGCTTTGAAGCCGCCGTGATGGAAGATTAC GCCGGCTCCGACGGCAACATCGAGCTGACCATCACCGGAATGACCTGCGCCT CCTGTGTGCACAACATTGAGTCCAAGCTGACCCGGACCAACGGCATCACCTA CGCCTCTGTGGCTCTGGCCACCTCCAAGGCCCTCGTGAAGTTCGATCCCGAG ATCATCGGCCCCAGGGACATCATCAAGATCATCGAAGAGATCGGCTTCCACG CCAGCCTGGCCCAGAGGAACCCTAACGCCCACCACCTGGACCACAAGATGGA AATCAAGCAGTGGAAGAAAAGCTTCCTGTGCAGCCTGGTGTTCGGCATCCCC GTGATGGCCCTGATGATCTACATGCTGATCCCCAGCAACGAGCCCCACCAGT CCATGGTGCTGGATCACAACATCATCCCCGGCCTGTCTATCCTGAACCTGAT CTTCTTCATCCTGTGCACCTTCGTGCAGCTGCTGGGCGGCTGGTACTTCTAC GTGCAGGCCTACAAGTCCCTGCGGCACAGATCCGCCAACATGGACGTGCTGA TCGTGCTGGCCACATCTATCGCCTACGTGTACTCCCTCGTGATCCTGGTGGT GGCCGTGGCCGAGAAAGCCGAGAGAAGCCCTGTGACCTTCTTCGACACCCCC CCTATGCTGTTCGTGTTTATCGCCCTGGGCCGGTGGCTGGAACACCTGGCCA AAAGCAAGACCAGCGAGGCCCTGGCTAAGCTGATGAGTCTGCAGGCCACCGA GGCCACAGTCGTGACCCTGGGCGAGGACAACCTGATCATCCGCGAGGAACAG GTGCCAATGGAACTGGTGCAGCGGGGCGACATCGTGAAGGTGGTGCCTGGCG GCAAGTTCCCCGTGGACGGAAAAGTGCTGGAAGGGAATACCATGGCCGACGA GAGCCTGATCACAGGCGAGGCCATGCCCGTGACCAAGAAACCTGGCAGCACA GTGATCGCCGGCAGCATCAATGCCCACGGCAGCGTGCTGATTAAGGCCACAC | **Truncated ATP7B variant (SEQ ID NO: 17 from** WO 2018/1261 16**)** |
| | ACGTGGGCAACGATACCACCCTGGCTCAGATTGTGAAGCTGGTGGAAGAGGC CCAGATGAGCAAGGCCCCCATTCAGCAGCTGGCTGACCGGTTCAGCGGCTAC TTCGTGCCCTTTATCATCATCATGAGCACCCTGACACTGGTCGTGTGGATCG TGATCGGCTTTATCGACTTCGGAGTGGTGCAGAGATACTTCCCCAACCCTAA CAAGCACATCAGCCAGACAGAAGTGATCATCAGATTCGCCTTTCAGACCAGC ATCACCGTGCTGTGTATCGCCTGCCCCTGTAGCCTGGGACTGGCCACACCTA CCGCTGTGATGGTGGGAACAGGCGTGGCCGCTCAGAACGGCATCCTGATCAA GGGGGGCAAGCCTCTGGAAATGGCTCACAAGATCAAGACCGTGATGTTCGAC AAGACCGGCACCATCACCCACGGCGTGCCCAGAGTGATGAGAGTGCTGCTGC TGGGGGATGTGGCCACCCTGCCTCTGAGAAAGGTGCTGGCTGTCGTGGGCAC AGCCGAGGCTAGCTCTGAACACCCACTGGGAGTGGCCGTGACAAAGTACTGC AAAGAGGAACTGGGCACCGAAACCCTGGGCTACTGCACCGACTTTCAGGCCG TGCCTGGCTGTGGCATCGGCTGCAAGGTGTCCAACGTGGAAGGCATCCTGGC CCACAGCGAGAGGCCACTGTCTGCCCCTGCCAGCCACCTGAACGAGGCCGGA TCTCTGCCCGCCGAAAAGGACGCTGTGCCCCAGACCTTCTCTGTGCTGATTG GCAACAGAGAGTGGCTGCGGCGGAACGGCCTGACCATCTCCTCCGATGTGTC CGACGCCATGACCGACCACGAGATGAAGGGCCAGACCGCCATTCTGGTGGCC ATTGACGGGGTGCTGTGCGGCATGATCGCAATCGCCGATGCCGTGAAACAGG AAGCAGCACTGGCCGTGCACACCCTGCAGTCTATGGGAGTGGATGTGGTGCT GATCACCGGCGACAACAGAAAGACCGCCAGGGCCATTGCCACCCAGGTGGGC ATCAACAAGGTGTTCGCCGAGGTGCTGCCCAGCCACAAAGTGGCCAAGGTGC AGGAACTGCAGAACAAAGGCAAAAAGGTGGCCATGGTGGGAGATGGCGTGAA CGACTCTCCTGCTCTGGCCCAGGCAGATATGGGCGTGGCCATCGGCACAGGC ACCGACGTGGCAATTGAGGCTGCTGACGTGGTGCTGATTCGGAACGACCTGC TGGACGTGGTGGCCTCCATCCACCTGTCCAAGAGAACCGTGCGGCGGATCAG AATCAACCTGGTGCTGGCACTGATCTATAACCTCGTGGGCATCCCTATCGCC GCTGGCGTGTTCATGCCTATCGGAATCGTGCTGCAGCCCTGGATGGGCTCTG CCGCCATGGCTGCAAGCTCCGTGTCTGTGGTGCTGTCCAGCCTGCAGCTGAA GTGCTACAAGAAGCCCGACCTGGAAAGATACGAGGCCCAGGCCCACGGACAC ATGAAGCCTCTGACAGCCTCCCAGGTGTCCGTGCACATCGGCATGGACGACA GATGGCGGGACAGCCCTAGAGCCACCCCTTGGGATCAGGTGTCATACGTGTC ACAGGTGTCCCTGAGCAGCCTGACCAGCGACAAGCCCAGCAGACATAGCGCC GCTGCCGACGACGATGGGGACAAGTGGTCCCTGCTGCTGAACGGCCGGGATG AGGAACAGTACATC | |
| **10** | ATGCCCGAGCAGGAAAGACAGATCACCGCCAGAGAGGGCGCCAGCCGGAAGA TCCTGAGCAAGCTGAGCCTGCCCACCAGAGCCTGGGAGCCCGCCATGAAGAA GTCCTTCGCCTTCGACAACGTGGGCTACGAGGGCGGCCTGGACGGACTGGGA CCATCTTCTCAGGTGGCCACAAGCACCGTGCGCAGCCCCGAAGAACTGAGAG CCGCCATTGAGGACATGGGATTCGAAGCCAGCGTGGTGTCCGAGAGCTGCTC CACCAACCCTCTGGGCAATCACAGCGCCGGCAACAGCATGGTGCAGACCACC GACGGCACCCCCACCAGCGTGCAGGAAGTGGCCCCACATACAGGCAGACTGC CCGCCAATCACGCCCCCGATATCCTGGCCAAGAGCCCCCAGAGTACAAGAGC CGTGGCCCCCCAGAAGTGCTTCCTGCAGATCAAGGGCATGACTTGTGCCTCT TGTGTGTCCAATATCGAGCGGAACCTGCAGAAAGAGGCCGGCGTGCTGTCTG TGCTGGTGGCTCTGATGGCCGGCAAGGCCGAGATCAAATACGACCCCGAAGT GATTCAGCCCCTGGAAATCGCCCAGTTTATCCAGGACCTGGGCTTTGAAGCC GCCGTGATGGAAGATTACGCCGGCTCCGACGGCAACATCGAGCTGACCATCA CCGGAATGACCTGCGCCTCCTGTGTGCACAACATTGAGTCCAAGCTGACCCG GACCAACGGCATCACCTACGCCTCTGTGGCTCTGGCCACCTCCAAGGCCCTC GTGAAGTTCGATCCCGAGATCATCGGCCCCAGGGACATCATCAAGATCATCG AAGAGATCGGCTTCCACGCCAGCCTGGCCCAGAGGAACCCTAACGCCCACCA CCTGGACCACAAGATGGAAATCAAGCAGTGGAAGAAAAGCTTCCTGTGCAGC | **Truncated ATP7B variant (SEQ ID NO: 18 from** WO 2018/1261 16**)** |
| | CTGGTGTTCGGCATCCCCGTGATGGCCCTGATGATCTACATGCTGATCCCCA GCAACGAGCCCCACCAGTCCATGGTGCTGGATCACAACATCATCCCCGGCCT GTCTATCCTGAACCTGATCTTCTTCATCCTGTGCACCTTCGTGCAGCTGCTG GGCGGCTGGTACTTCTACGTGCAGGCCTACAAGTCCCTGCGGCACAGATCCG CCAACATGGACGTGCTGATCGTGCTGGCCACATCTATCGCCTACGTGTACTC CCTCGTGATCCTGGTGGTGGCCGTGGCCGAGAAAGCCGAGAGAAGCCCTGTG ACCTTCTTCGACACCCCCCCTATGCTGTTCGTGTTTATCGCCCTGGGCCGGT GGCTGGAACACCTGGCCAAAAGCAAGACCAGCGAGGCCCTGGCTAAGCTGAT GAGTCTGCAGGCCACCGAGGCCACAGTCGTGACCCTGGGCGAGGACAACCTG ATCATCCGCGAGGAACAGGTGCCAATGGAACTGGTGCAGCGGGGCGACATCG TGAAGGTGGTGCCTGGCGGCAAGTTCCCCGTGGACGGAAAAGTGCTGGAAGG GAATACCATGGCCGACGAGAGCCTGATCACAGGCGAGGCCATGCCCGTGACC AAGAAACCTGGCAGCACAGTGATCGCCGGCAGCATCAATGCCCACGGCAGCG TGCTGATTAAGGCCACACACGTGGGCAACGATACCACCCTGGCTCAGATTGT GAAGCTGGTGGAAGAGGCCCAGATGAGCAAGGCCCCCATTCAGCAGCTGGCT GACCGGTTCAGCGGCTACTTCGTGCCCTTTATCATCATCATGAGCACCCTGA CACTGGTCGTGTGGATCGTGATCGGCTTTATCGACTTCGGAGTGGTGCAGAG ATACTTCCCCAACCCTAACAAGCACATCAGCCAGACAGAAGTGATCATCAGA TTCGCCTTTCAGACCAGCATCACCGTGCTGTGTATCGCCTGCCCCTGTAGCC TGGGACTGGCCACACCTACCGCTGTGATGGTGGGAACAGGCGTGGCCGCTCA GAACGGCATCCTGATCAAGGGGGGCAAGCCTCTGGAAATGGCTCACAAGATC AAGACCGTGATGTTCGACAAGACCGGCACCATCACCCACGGCGTGCCCAGAG TGATGAGAGTGCTGCTGCTGGGGGATGTGGCCACCCTGCCTCTGAGAAAGGT GCTGGCTGTCGTGGGCACAGCCGAGGCTAGCTCTGAACACCCACTGGGAGTG GCCGTGACAAAGTACTGCAAAGAGGAACTGGGCACCGAAACCCTGGGCTACT GCACCGACTTTCAGGCCGTGCCTGGCTGTGGCATCGGCTGCAAGGTGTCCAA CGTGGAAGGCATCCTGGCCCACAGCGAGAGGCCACTGTCTGCCCCTGCCAGC CACCTGAACGAGGCCGGATCTCTGCCCGCCGAAAAGGACGCTGTGCCCCAGA CCTTCTCTGTGCTGATTGGCAACAGAGAGTGGCTGCGGCGGAACGGCCTGAC CATCTCCTCCGATGTGTCCGACGCCATGACCGACCACGAGATGAAGGGCCAG ACCGCCATTCTGGTGGCCATTGACGGGGTGCTGTGCGGCATGATCGCAATCG CCGATGCCGTGAAACAGGAAGCAGCACTGGCCGTGCACACCCTGCAGTCTAT GGGAGTGGATGTGGTGCTGATCACCGGCGACAACAGAAAGACCGCCAGGGCC ATTGCCACCCAGGTGGGCATCAACAAGGTGTTCGCCGAGGTGCTGCCCAGCC ACAAAGTGGCCAAGGTGCAGGAACTGCAGAACAAAGGCAAAAAGGTGGCCAT GGTGGGAGATGGCGTGAACGACTCTCCTGCTCTGGCCCAGGCAGATATGGGC GTGGCCATCGGCACAGGCACCGACGTGGCAATTGAGGCTGCTGACGTGGTGC TGATTCGGAACGACCTGCTGGACGTGGTGGCCTCCATCCACCTGTCCAAGAG AACCGTGCGGCGGATCAGAATCAACCTGGTGCTGGCACTGATCTATAACCTC GTGGGCATCCCTATCGCCGCTGGCGTGTTCATGCCTATCGGAATCGTGCTGC AGCCCTGGATGGGCTCTGCCGCCATGGCTGCAAGCTCCGTGTCTGTGGTGCT GTCCAGCCTGCAGCTGAAGTGCTACAAGAAGCCCGACCTGGAAAGATACGAG GCCCAGGCCCACGGACACATGAAGCCTCTGACAGCCTCCCAGGTGTCCGTGC ACATCGGCATGGACGACAGATGGCGGGACAGCCCTAGAGCCACCCCTTGGGA TCAGGTGTCATACGTGTCACAGGTGTCCCTGAGCAGCCTGACCAGCGACAAG CCCAGCAGACATAGCGCCGCTGCCGACGACGATGGGGACAAGTGGTCCCTGC TGCTGAACGGCCGGGATGAGGAACAGTACATC | |
| **11** | ATGCCCGAGCAGGAAAGACAGATCACCGCCAGAGAGGGCGCCAGCCGGAAGA TCCTGAGCAAGCTGAGCCTGCCCACCAGAGCCTGGGAGCCCGCCATGAAGAA GTCCTTCGCCTTCGACAACGTGGGCTACGAGGGCGGCCTGGACGGACTGGGA CCATCTTCTCAGGTGGCCACAAGCACCGTGCGCCTGGAAATCGCCCAGTTTA TCCAGGACCTGGGCTTTGAAGCCGCCGTGATGGAAGATTACGCCGGCTCCGA | **Truncated ATP7B variant (SEQ ID** |
| | CGGCAACATCGAGCTGACCATCACCGGAATGACCTGCGCCTCCTGTGTGCAC AACATTGAGTCCAAGCTGACCCGGACCAACGGCATCACCTACGCCTCTGTGG CTCTGGCCACCTCCAAGGCCCTCGTGAAGTTCGATCCCGAGATCATCGGCCC CAGGGACATCATCAAGATCATCGAAGAGATCGGCTTCCACGCCAGCCTGGCC CAGAGGAACCCTAACGCCCACCACCTGGACCACAAGATGGAAATCAAGCAGT GGAAGAAAAGCTTCCTGTGCAGCCTGGTGTTCGGCATCCCCGTGATGGCCCT GATGATCTACATGCTGATCCCCAGCAACGAGCCCCACCAGTCCATGGTGCTG GATCACAACATCATCCCCGGCCTGTCTATCCTGAACCTGATCTTCTTCATCC TGTGCACCTTCGTGCAGCTGCTGGGCGGCTGGTACTTCTACGTGCAGGCCTA CAAGTCCCTGCGGCACAGATCCGCCAACATGGACGTGCTGATCGTGCTGGCC ACATCTATCGCCTACGTGTACTCCCTCGTGATCCTGGTGGTGGCCGTGGCCG AGAAAGCCGAGAGAAGCCCTGTGACCTTCTTCGACACCCCCCCTATGCTGTT CGTGTTTATCGCCCTGGGCCGGTGGCTGGAACACCTGGCCAAAAGCAAGACC AGCGAGGCCCTGGCTAAGCTGATGAGTCTGCAGGCCACCGAGGCCACAGTCG TGACCCTGGGCGAGGACAACCTGATCATCCGCGAGGAACAGGTGCCAATGGA ACTGGTGCAGCGGGGCGACATCGTGAAGGTGGTGCCTGGCGGCAAGTTCCCC GTGGACGGAAAAGTGCTGGAAGGGAATACCATGGCCGACGAGAGCCTGATCA CAGGCGAGGCCATGCCCGTGACCAAGAAACCTGGCAGCACAGTGATCGCCGG CAGCATCAATGCCCACGGCAGCGTGCTGATTAAGGCCACACACGTGGGCAAC GATACCACCCTGGCTCAGATTGTGAAGCTGGTGGAAGAGGCCCAGATGAGCA AGGCCCCCATTCAGCAGCTGGCTGACCGGTTCAGCGGCTACTTCGTGCCCTT TATCATCATCATGAGCACCCTGACACTGGTCGTGTGGATCGTGATCGGCTTT ATCGACTTCGGAGTGGTGCAGAGATACTTCCCCAACCCTAACAAGCACATCA GCCAGACAGAAGTGATCATCAGATTCGCCTTTCAGACCAGCATCACCGTGCT GTGTATCGCCTGCCCCTGTAGCCTGGGACTGGCCACACCTACCGCTGTGATG GTGGGAACAGGCGTGGCCGCTCAGAACGGCATCCTGATCAAGGGGGGCAAGC CTCTGGAAATGGCTCACAAGATCAAGACCGTGATGTTCGACAAGACCGGCAC CATCACCCACGGCGTGCCCAGAGTGATGAGAGTGCTGCTGCTGGGGGATGTG GCCACCCTGCCTCTGAGAAAGGTGCTGGCTGTCGTGGGCACAGCCGAGGCTA GCTCTGAACACCCACTGGGAGTGGCCGTGACAAAGTACTGCAAAGAGGAACT GGGCACCGAAACCCTGGGCTACTGCACCGACTTTCAGGCCGTGCCTGGCTGT GGCATCGGCTGCAAGGTGTCCAACGTGGAAGGCATCCTGGCCCACAGCGAGA GGCCACTGTCTGCCCCTGCCAGCCACCTGAACGAGGCCGGATCTCTGCCCGC CGAAAAGGACGCTGTGCCCCAGACCTTCTCTGTGCTGATTGGCAACAGAGAG TGGCTGCGGCGGAACGGCCTGACCATCTCCTCCGATGTGTCCGACGCCATGA CCGACCACGAGATGAAGGGCCAGACCGCCATTCTGGTGGCCATTGACGGGGT GCTGTGCGGCATGATCGCAATCGCCGATGCCGTGAAACAGGAAGCAGCACTG GCCGTGCACACCCTGCAGTCTATGGGAGTGGATGTGGTGCTGATCACCGGCG ACAACAGAAAGACCGCCAGGGCCATTGCCACCCAGGTGGGCATCAACAAGGT GTTCGCCGAGGTGCTGCCCAGCCACAAAGTGGCCAAGGTGCAGGAACTGCAG AACAAAGGCAAAAAGGTGGCCATGGTGGGAGATGGCGTGAACGACTCTCCTG CTCTGGCCCAGGCAGATATGGGCGTGGCCATCGGCACAGGCACCGACGTGGC AATTGAGGCTGCTGACGTGGTGCTGATTCGGAACGACCTGCTGGACGTGGTG GCCTCCATCCACCTGTCCAAGAGAACCGTGCGGCGGATCAGAATCAACCTGG TGCTGGCACTGATCTATAACCTCGTGGGCATCCCTATCGCCGCTGGCGTGTT CATGCCTATCGGAATCGTGCTGCAGCCCTGGATGGGCTCTGCCGCCATGGCT GCAAGCTCCGTGTCTGTGGTGCTGTCCAGCCTGCAGCTGAAGTGCTACAAGA AGCCCGACCTGGAAAGATACGAGGCCCAGGCCCACGGACACATGAAGCCTCT GACAGCCTCCCAGGTGTCCGTGCACATCGGCATGGACGACAGATGGCGGGAC AGCCCTAGAGCCACCCCTTGGGATCAGGTGTCATACGTGTCACAGGTGTCCC TGAGCAGCCTGACCAGCGACAAGCCCAGCAGACATAGCGCCGCTGCCGACGA | **NO: 19 from** WO 2018/1261 16**)** |
| | CGATGGGGACAAGTGGTCCCTGCTGCTGAACGGCCGGGATGAGGAACAGTAC ATC | |
| **12** | ATGCCCGAGCAGGAAAGACAGATCACCGCCAGAGAGGGCGCCAGCCGGAAGA TCCTGAGCAAGCTGAGCCTGCCCACCAGAGCCTGGGAGCCCGCCATGAAGAA GTCCTTCGCCTTCGACAACGTGGGCTACGAGGGCGGCCTGGACGGACTGGGA CCATCTTCTCAGGTGGCCACAAGCACCGTGCGCAAATACGTGCCCTCTGTCG TGTGCCTGCAGCAGGTGTGCCACCAGATCGGCGACATGGGCTTCGAGGCCTC TATCGCCGAGGGAAAGGCCGCCTCTTGGCCCTCTAGAAGCCTGCCTGCTCAG GAAGCCGTCGTGAAGCTGCGGGTGGAAGGGATGACCTGTCAGTCCTGCGTGT CCAGCATCGAGGGCAAAGTGCGGAAGCTGCAGGGCGTGGTGCGCGTGAAAGT GTCTCTGAGCAACCAGGAAGCTGTGATCACCTACCAGCCCTACCTGATCCAG CCCGAGGACCTGAGGGACCACGTGAACGATATGGGATTTGAGGCCGCCATCA AGAGCAAGGTGGCCCCTCTGTCTCTGGGCCCCATCGACATCGAGAGACTGCA GAGCACCAACCCCAAGCGGCCTCTGAGCAGCGCCAACCAGAACTTCAACAAC AGCGAGACACTGGGCCACCAGGGCAGCCACGTCGTGACACTGCAGCTGCGGA TCGACGGAATGCACTGCAAGAGCTGTGTGCTGAACATCGAGGAAAACATCGG CCAGCTGCTGGGAGTGCAGAGCATCCAGGTGTCACTGGAAAACAAGACCGCC CAGGTGAAGTACGACCCCAGCTGCACAAGCCCCGTGGCCCTGCAGAGAGCCA TTGAAGCTCTGCCCCCTGGCAACTTCAAAGTGTCCCTGCCTGACGGAGCCGA GGGCTCCGGAACAGATCACAGAAGCAGCAGCAGCCACAGCCCTGGCAGCCCC CCTAGAAATCAGGTGCAGGGCACCTGTAGCACCACCCTGATCGCCATTGCCG GCATGACATGCGCCAGCTGCGTGCACTCTATTGAGGGCATGATCTCCCAGCT GGAAGGCGTGCAGCAGATCAGTGTGTCTCTGGCCGAGGGCACCGCCACAGTG CTGTACAACCCTAGCGTGATCAGCCCCGAAGAACTGAGAGCCGCCATTGAGG ACATGGGATTCGAAGCCAGCGTGGTGTCCGAGAGCTGCTCCACCAACCCTCT GGGCAATCACAGCGCCGGCAACAGCATGGTGCAGACCACCGACGGCACCCCC ACCAGCGTGCAGGAAGTGGCCCCACATACAGGCAGACTGCCCGCCAATCACG CCCCCGATATCCTGGCCAAGAGCCCCCAGAGTACAAGAGCCGTGGCCCCCCA GAAGTGCTTCCTGCAGATCAAGGGCATGACTTGTGCCTCTTGTGTGTCCAAT ATCGAGCGGAACCTGCAGAAAGAGGCCGGCGTGCTGTCTGTGCTGGTGGCTC TGATGGCCGGCAAGGCCGAGATCAAATACGACCCCGAAGTGATTCAGCCCCT GGAAATCGCCCAGTTTATCCAGGACCTGGGCTTTGAAGCCGCCGTGATGGAA GATTACGCCGGCTCCGACGGCAACATCGAGCTGACCATCACCGGAATGACCT GCGCCTCCTGTGTGCACAACATTGAGTCCAAGCTGACCCGGACCAACGGCAT CACCTACGCCTCTGTGGCTCTGGCCACCTCCAAGGCCCTCGTGAAGTTCGAT CCCGAGATCATCGGCCCCAGGGACATCATCAAGATCATCGAAGAGATCGGCT TCCACGCCAGCCTGGCCCAGAGGAACCCTAACGCCCACCACCTGGACCACAA GATGGAAATCAAGCAGTGGAAGAAAAGCTTCCTGTGCAGCCTGGTGTTCGGC ATCCCCGTGATGGCCCTGATGATCTACATGCTGATCCCCAGCAACGAGCCCC ACCAGTCCATGGTGCTGGATCACAACATCATCCCCGGCCTGTCTATCCTGAA CCTGATCTTCTTCATCCTGTGCACCTTCGTGCAGCTGCTGGGCGGCTGGTAC TTCTACGTGCAGGCCTACAAGTCCCTGCGGCACAGATCCGCCAACATGGACG TGCTGATCGTGCTGGCCACATCTATCGCCTACGTGTACTCCCTCGTGATCCT GGTGGTGGCCGTGGCCGAGAAAGCCGAGAGAAGCCCTGTGACCTTCTTCGAC ACCCCCCCTATGCTGTTCGTGTTTATCGCCCTGGGCCGGTGGCTGGAACACC TGGCCAAAAGCAAGACCAGCGAGGCCCTGGCTAAGCTGATGAGTCTGCAGGC CACCGAGGCCACAGTCGTGACCCTGGGCGAGGACAACCTGATCATCCGCGAG GAACAGGTGCCAATGGAACTGGTGCAGCGGGGCGACATCGTGAAGGTGGTGC CTGGCGGCAAGTTCCCCGTGGACGGAAAAGTGCTGGAAGGGAATACCATGGC CGACGAGAGCCTGATCACAGGCGAGGCCATGCCCGTGACCAAGAAACCTGGC AGCACAGTGATCGCCGGCAGCATCAATGCCCACGGCAGCGTGCTGATTAAGG CCACACACGTGGGCAACGATACCACCCTGGCTCAGATTGTGAAGCTGGTGGA | **Truncated ATP7B variant (SEQ ID NO: 20 from** WO 2018/1261 16**)** |
| | AGAGGCCCAGATGAGCAAGGCCCCCATTCAGCAGCTGGCTGACCGGTTCAGC GGCTACTTCGTGCCCTTTATCATCATCATGAGCACCCTGACACTGGTCGTGT GGATCGTGATCGGCTTTATCGACTTCGGAGTGGTGCAGAGATACTTCCCCAA CCCTAACAAGCACATCAGCCAGACAGAAGTGATCATCAGATTCGCCTTTCAG ACCAGCATCACCGTGCTGTGTATCGCCTGCCCCTGTAGCCTGGGACTGGCCA CACCTACCGCTGTGATGGTGGGAACAGGCGTGGCCGCTCAGAACGGCATCCT GATCAAGGGGGGCAAGCCTCTGGAAATGGCTCACAAGATCAAGACCGTGATG TTCGACAAGACCGGCACCATCACCCACGGCGTGCCCAGAGTGATGAGAGTGC TGCTGCTGGGGGATGTGGCCACCCTGCCTCTGAGAAAGGTGCTGGCTGTCGT GGGCACAGCCGAGGCTAGCTCTGAACACCCACTGGGAGTGGCCGTGACAAAG TACTGCAAAGAGGAACTGGGCACCGAAACCCTGGGCTACTGCACCGACTTTC AGGCCGTGCCTGGCTGTGGCATCGGCTGCAAGGTGTCCAACGTGGAAGGCAT CCTGGCCCACAGCGAGAGGCCACTGTCTGCCCCTGCCAGCCACCTGAACGAG GCCGGATCTCTGCCCGCCGAAAAGGACGCTGTGCCCCAGACCTTCTCTGTGC TGATTGGCAACAGAGAGTGGCTGCGGCGGAACGGCCTGACCATCTCCTCCGA TGTGTCCGACGCCATGACCGACCACGAGATGAAGGGCCAGACCGCCATTCTG GTGGCCATTGACGGGGTGCTGTGCGGCATGATCGCAATCGCCGATGCCGTGA AACAGGAAGCAGCACTGGCCGTGCACACCCTGCAGTCTATGGGAGTGGATGT GGTGCTGATCACCGGCGACAACAGAAAGACCGCCAGGGCCATTGCCACCCAG GTGGGCATCAACAAGGTGTTCGCCGAGGTGCTGCCCAGCCACAAAGTGGCCA AGGTGCAGGAACTGCAGAACAAAGGCAAAAAGGTGGCCATGGTGGGAGATGG CGTGAACGACTCTCCTGCTCTGGCCCAGGCAGATATGGGCGTGGCCATCGGC ACAGGCACCGACGTGGCAATTGAGGCTGCTGACGTGGTGCTGATTCGGAACG ACCTGCTGGACGTGGTGGCCTCCATCCACCTGTCCAAGAGAACCGTGCGGCG GATCAGAATCAACCTGGTGCTGGCACTGATCTATAACCTCGTGGGCATCCCT ATCGCCGCTGGCGTGTTCATGCCTATCGGAATCGTGCTGCAGCCCTGGATGG GCTCTGCCGCCATGGCTGCAAGCTCCGTGTCTGTGGTGCTGTCCAGCCTGCA GCTGAAGTGCTACAAGAAGCCCGACCTGGAAAGATACGAGGCCCAGGCCCAC GGACACATGAAGCCTCTGACAGCCTCCCAGGTGTCCGTGCACATCGGCATGG ACGACAGATGGCGGGACAGCCCTAGAGCCACCCCTTGGGATCAGGTGTCATA CGTGTCACAGGTGTCCCTGAGCAGCCTGACCAGCGACAAGCCCAGCAGACAT AGCGCCGCTGCCGACGACGATGGGGACAAGTGGTCCCTGCTGCTGAACGGCC GGGATGAGGAACAGTACATC | |
| **13** | ATGCCCGAGCAGGAAAGACAGATCACCGCCAGAGAGGGCGCCAGCCGGAAGA TCCTGAGCAAGCTGAGCCTGCCCACCAGAGCCTGGGAGCCCGCCATGAAGAA GTCCTTCGCCTTCGACAACGTGGGCTACGAGGGCGGCCTGGACGGACTGGGA CCATCTTCTCAGGTGGCCACAAGCACCGTGCGCATCCTGGGCATGACCTGCC AGAGCTGCGTGAAGTCCATCGAGGACCGGATCAGCAACCTGAAGGGCATCAT CAGCATGAAGGTGTCCCTGGAACAGGGCAGCGCCACCGTGAAATACGTGCCC TCTGTCGTGTGCCTGCAGCAGGTGTGCCACCAGATCGGCGACATGGGCTTCG AGGCCTCTATCGCCGAGGGAAAGGCCGCCTCTTGGCCCTCTAGAAGCCTGCC TGCTCAGGAAGCCGTCGTGAAGGAGGCCGCCATCAAGAGCAAGGTGGCCCCT CTGTCTCTGGGCCCCATCGACATCGAGAGACTGCAGAGCACCAACCCCAAGC GGCCTCTGAGCAGCGCCAACCAGAACTTCAACAACAGCGAGACACTGGGCCA CCAGGGCAGCCACGTCGTGACACTGCAGCTGCGGATCGACGGAATGCACTGC AAGAGCTGTGTGCTGAACATCGAGGAAAACATCGGCCAGCTGCTGGGAGTGC AGAGCATCCAGGTGTCACTGGAAAACAAGACCGCCCAGGTGAAGTACGACCC CAGCTGCACAAGCCCCGTGGCCCTGCAGAGAGCCATTGAAGCTCTGCCCCCT GGCAACTTCAAAGTGTCCCTGCCTGACGGAGCCGAGGGCTCCGGAACAGATC ACAGAAGCAGCAGCAGCCACAGCCCTGGCAGCCCCCCTAGAAATCAGGTGCA GGGCACCTGTAGCACCACCCTGATCGCCATTGCCGGCATGACATGCGCCAGC TGCGTGCACTCTATTGAGGGCATGATCTCCCAGCTGGAAGGCGTGCAGCAGA | **Truncated ATP7B variant (SEQ ID NO: 21 from** WO 2018/1261 16**)** |
| | TCAGTGTGTCTCTGGCCGAGGGCACCGCCACAGTGCTGTACAACCCTAGCGT GATCAGCCCCGAAGAACTGAGAGCCGCCATTGAGGACATGGGATTCGAAGCC AGCGTGGTGTCCGAGAGCTGCTCCACCAACCCTCTGGGCAATCACAGCGCCG GCAACAGCATGGTGCAGACCACCGACGGCACCCCCACCAGCGTGCAGGAAGT GGCCCCACATACAGGCAGACTGCCCGCCAATCACGCCCCCGATATCCTGGCC AAGAGCCCCCAGAGTACAAGAGCCGTGGCCCCCCAGAAGTGCTTCCTGCAGA TCAAGGGCATGACTTGTGCCTCTTGTGTGTCCAATATCGAGCGGAACCTGCA GAAAGAGGCCGGCGTGCTGTCTGTGCTGGTGGCTCTGATGGCCGGCAAGGCC GAGATCAAATACGACCCCGAAGTGATTCAGCCCCTGGAAATCGCCCAGTTTA TCCAGGACCTGGGCTTTGAAGCCGCCGTGATGGAAGATTACGCCGGCTCCGA CGGCAACATCGAGCTGACCATCACCGGAATGACCTGCGCCTCCTGTGTGCAC AACATTGAGTCCAAGCTGACCCGGACCAACGGCATCACCTACGCCTCTGTGG CTCTGGCCACCTCCAAGGCCCTCGTGAAGTTCGATCCCGAGATCATCGGCCC CAGGGACATCATCAAGATCATCGAAGAGATCGGCTTCCACGCCAGCCTGGCC CAGAGGAACCCTAACGCCCACCACCTGGACCACAAGATGGAAATCAAGCAGT GGAAGAAAAGCTTCCTGTGCAGCCTGGTGTTCGGCATCCCCGTGATGGCCCT GATGATCTACATGCTGATCCCCAGCAACGAGCCCCACCAGTCCATGGTGCTG GATCACAACATCATCCCCGGCCTGTCTATCCTGAACCTGATCTTCTTCATCC TGTGCACCTTCGTGCAGCTGCTGGGCGGCTGGTACTTCTACGTGCAGGCCTA CAAGTCCCTGCGGCACAGATCCGCCAACATGGACGTGCTGATCGTGCTGGCC ACATCTATCGCCTACGTGTACTCCCTCGTGATCCTGGTGGTGGCCGTGGCCG AGAAAGCCGAGAGAAGCCCTGTGACCTTCTTCGACACCCCCCCTATGCTGTT CGTGTTTATCGCCCTGGGCCGGTGGCTGGAACACCTGGCCAAAAGCAAGACC AGCGAGGCCCTGGCTAAGCTGATGAGTCTGCAGGCCACCGAGGCCACAGTCG TGACCCTGGGCGAGGACAACCTGATCATCCGCGAGGAACAGGTGCCAATGGA ACTGGTGCAGCGGGGCGACATCGTGAAGGTGGTGCCTGGCGGCAAGTTCCCC GTGGACGGAAAAGTGCTGGAAGGGAATACCATGGCCGACGAGAGCCTGATCA CAGGCGAGGCCATGCCCGTGACCAAGAAACCTGGCAGCACAGTGATCGCCGG CAGCATCAATGCCCACGGCAGCGTGCTGATTAAGGCCACACACGTGGGCAAC GATACCACCCTGGCTCAGATTGTGAAGCTGGTGGAAGAGGCCCAGATGAGCA AGGCCCCCATTCAGCAGCTGGCTGACCGGTTCAGCGGCTACTTCGTGCCCTT TATCATCATCATGAGCACCCTGACACTGGTCGTGTGGATCGTGATCGGCTTT ATCGACTTCGGAGTGGTGCAGAGATACTTCCCCAACCCTAACAAGCACATCA GCCAGACAGAAGTGATCATCAGATTCGCCTTTCAGACCAGCATCACCGTGCT GTGTATCGCCTGCCCCTGTAGCCTGGGACTGGCCACACCTACCGCTGTGATG GTGGGAACAGGCGTGGCCGCTCAGAACGGCATCCTGATCAAGGGGGGCAAGC CTCTGGAAATGGCTCACAAGATCAAGACCGTGATGTTCGACAAGACCGGCAC CATCACCCACGGCGTGCCCAGAGTGATGAGAGTGCTGCTGCTGGGGGATGTG GCCACCCTGCCTCTGAGAAAGGTGCTGGCTGTCGTGGGCACAGCCGAGGCTA GCTCTGAACACCCACTGGGAGTGGCCGTGACAAAGTACTGCAAAGAGGAACT GGGCACCGAAACCCTGGGCTACTGCACCGACTTTCAGGCCGTGCCTGGCTGT GGCATCGGCTGCAAGGTGTCCAACGTGGAAGGCATCCTGGCCCACAGCGAGA GGCCACTGTCTGCCCCTGCCAGCCACCTGAACGAGGCCGGATCTCTGCCCGC CGAAAAGGACGCTGTGCCCCAGACCTTCTCTGTGCTGATTGGCAACAGAGAG TGGCTGCGGCGGAACGGCCTGACCATCTCCTCCGATGTGTCCGACGCCATGA CCGACCACGAGATGAAGGGCCAGACCGCCATTCTGGTGGCCATTGACGGGGT GCTGTGCGGCATGATCGCAATCGCCGATGCCGTGAAACAGGAAGCAGCACTG GCCGTGCACACCCTGCAGTCTATGGGAGTGGATGTGGTGCTGATCACCGGCG ACAACAGAAAGACCGCCAGGGCCATTGCCACCCAGGTGGGCATCAACAAGGT GTTCGCCGAGGTGCTGCCCAGCCACAAAGTGGCCAAGGTGCAGGAACTGCAG AACAAAGGCAAAAAGGTGGCCATGGTGGGAGATGGCGTGAACGACTCTCCTG CTCTGGCCCAGGCAGATATGGGCGTGGCCATCGGCACAGGCACCGACGTGGC | |
| | AATTGAGGCTGCTGACGTGGTGCTGATTCGGAACGACCTGCTGGACGTGGTG GCCTCCATCCACCTGTCCAAGAGAACCGTGCGGCGGATCAGAATCAACCTGG TGCTGGCACTGATCTATAACCTCGTGGGCATCCCTATCGCCGCTGGCGTGTT CATGCCTATCGGAATCGTGCTGCAGCCCTGGATGGGCTCTGCCGCCATGGCT GCAAGCTCCGTGTCTGTGGTGCTGTCCAGCCTGCAGCTGAAGTGCTACAAGA AGCCCGACCTGGAAAGATACGAGGCCCAGGCCCACGGACACATGAAGCCTCT GACAGCCTCCCAGGTGTCCGTGCACATCGGCATGGACGACAGATGGCGGGAC AGCCCTAGAGCCACCCCTTGGGATCAGGTGTCATACGTGTCACAGGTGTCCC TGAGCAGCCTGACCAGCGACAAGCCCAGCAGACATAGCGCCGCTGCCGACGA CGATGGGGACAAGTGGTCCCTGCTGCTGAACGGCCGGGATGAGGAACAGTAC ATC | |
| **14** | ATGCCCGAGCAGGAAAGACAGATCACCGCCAGAGAGGGCGCCAGCCGGAAGA TCCTGAGCAAGCTGAGCCTGCCCACCAGAGCCTGGGAGCCCGCCATGAAGAA GTCCTTCGCCTTCGACAACGTGGGCTACGAGGGCGGCCTGGACGGACTGGGA CCATCTTCTCAGGTGGCCACAAGCACCGTGCGCATCCTGGGCATGACCTGCC AGAGCTGCGTGAAGTCCATCGAGGACCGGATCAGCAACCTGAAGGGCATCAT CAGCATGAAGGTGTCCCTGGAACAGGGCAGCGCCACCGTGAAATACGTGCCC TCTGTCGTGTGCCTGCAGCAGGTGTGCCACCAGATCGGCGACATGGGCTTCG AGGCCTCTATCGCCGAGGGAAAGGCCGCCTCTTGGCCCTCTAGAAGCCTGCC TGCTCAGGAAGCCGTCGTGAAGCTGCGGGTGGAAGGGATGACCTGTCAGTCC TGCGTGTCCAGCATCGAGGGCAAAGTGCGGAAGCTGCAGGGCGTGGTGCGCG TGAAAGTGTCTCTGAGCAACCAGGAAGCTGTGATCACCTACCAGCCCTACCT GATCCAGCCCGAGGACCTGAGGGACCACGTGAACGATATGGGATTTGAGGCC GCCATCAAGAGCAAGGTGGCCCCTCTGTCTCTGGGCCCCATCGACATCGAGA GACTGCAGAGCACCAACCCCAAGCGGCCTCTGAGCAGCGCCAACCAGAACTT CAACAACAGCGAGACACTGGGCCACCAGGGCAGCCACGTCGTGACACTGCAG AGCATCCAGGTGTCACTGGAAAACAAGACCGCCCAGGTGAAGTACGACCCCA GCTGCACAAGCCCCGTGGCCCTGCAGAGAGCCATTGAAGCTCTGCCCCCTGG CAACTTCAAAGTGTCCCTGCCTGACGGAGCCGAGGGCTCCGGAACAGATCAC AGAAGCAGCAGCAGCCACAGCCCTGGCAGCCCCCCTAGAAATCAGGTGCAGG GCACCTGTAGCACCACCCTGATCGCCATTGCCGGCATGACATGCGCCAGCTG CGTGCACTCTATTGAGGGCATGATCTCCCAGCTGGAAGGCGTGCAGCAGATC AGTGTGTCTCTGGCCGAGGGCACCGCCACAGTGCTGTACAACCCTAGCGTGA TCAGCCCCGAAGAACTGAGAGCCGCCATTGAGGACATGGGATTCGAAGCCAG CGTGGTGTCCGAGAGCTGCTCCACCAACCCTCTGGGCAATCACAGCGCCGGC AACAGCATGGTGCAGACCACCGACGGCACCCCCACCAGCGTGCAGGAAGTGG CCCCACATACAGGCAGACTGCCCGCCAATCACGCCCCCGATATCCTGGCCAA GAGCCCCCAGAGTACAAGAGCCGTGGCCCCCCAGAAGTGCTTCCTGCAGATC AAGGGCATGACTTGTGCCTCTTGTGTGTCCAATATCGAGCGGAACCTGCAGA AAGAGGCCGGCGTGCTGTCTGTGCTGGTGGCTCTGATGGCCGGCAAGGCCGA GATCAAATACGACCCCGAAGTGATTCAGCCCCTGGAAATCGCCCAGTTTATC CAGGACCTGGGCTTTGAAGCCGCCGTGATGGAAGATTACGCCGGCTCCGACG GCAACATCGAGCTGACCATCACCGGAATGACCTGCGCCTCCTGTGTGCACAA CATTGAGTCCAAGCTGACCCGGACCAACGGCATCACCTACGCCTCTGTGGCT CTGGCCACCTCCAAGGCCCTCGTGAAGTTCGATCCCGAGATCATCGGCCCCA GGGACATCATCAAGATCATCGAAGAGATCGGCTTCCACGCCAGCCTGGCCCA GAGGAACCCTAACGCCCACCACCTGGACCACAAGATGGAAATCAAGCAGTGG AAGAAAAGCTTCCTGTGCAGCCTGGTGTTCGGCATCCCCGTGATGGCCCTGA TGATCTACATGCTGATCCCCAGCAACGAGCCCCACCAGTCCATGGTGCTGGA TCACAACATCATCCCCGGCCTGTCTATCCTGAACCTGATCTTCTTCATCCTG TGCACCTTCGTGCAGCTGCTGGGCGGCTGGTACTTCTACGTGCAGGCCTACA AGTCCCTGCGGCACAGATCCGCCAACATGGACGTGCTGATCGTGCTGGCCAC | **Truncated ATP7B variant (SEQ ID NO: 22 from** WO 2018/1261 16**)** |
| | ATCTATCGCCTACGTGTACTCCCTCGTGATCCTGGTGGTGGCCGTGGCCGAG AAAGCCGAGAGAAGCCCTGTGACCTTCTTCGACACCCCCCCTATGCTGTTCG TGTTTATCGCCCTGGGCCGGTGGCTGGAACACCTGGCCAAAAGCAAGACCAG CGAGGCCCTGGCTAAGCTGATGAGTCTGCAGGCCACCGAGGCCACAGTCGTG ACCCTGGGCGAGGACAACCTGATCATCCGCGAGGAACAGGTGCCAATGGAAC TGGTGCAGCGGGGCGACATCGTGAAGGTGGTGCCTGGCGGCAAGTTCCCCGT GGACGGAAAAGTGCTGGAAGGGAATACCATGGCCGACGAGAGCCTGATCACA GGCGAGGCCATGCCCGTGACCAAGAAACCTGGCAGCACAGTGATCGCCGGCA GCATCAATGCCCACGGCAGCGTGCTGATTAAGGCCACACACGTGGGCAACGA TACCACCCTGGCTCAGATTGTGAAGCTGGTGGAAGAGGCCCAGATGAGCAAG GCCCCCATTCAGCAGCTGGCTGACCGGTTCAGCGGCTACTTCGTGCCCTTTA TCATCATCATGAGCACCCTGACACTGGTCGTGTGGATCGTGATCGGCTTTAT CGACTTCGGAGTGGTGCAGAGATACTTCCCCAACCCTAACAAGCACATCAGC CAGACAGAAGTGATCATCAGATTCGCCTTTCAGACCAGCATCACCGTGCTGT GTATCGCCTGCCCCTGTAGCCTGGGACTGGCCACACCTACCGCTGTGATGGT GGGAACAGGCGTGGCCGCTCAGAACGGCATCCTGATCAAGGGGGGCAAGCCT CTGGAAATGGCTCACAAGATCAAGACCGTGATGTTCGACAAGACCGGCACCA TCACCCACGGCGTGCCCAGAGTGATGAGAGTGCTGCTGCTGGGGGATGTGGC CACCCTGCCTCTGAGAAAGGTGCTGGCTGTCGTGGGCACAGCCGAGGCTAGC TCTGAACACCCACTGGGAGTGGCCGTGACAAAGTACTGCAAAGAGGAACTGG GCACCGAAACCCTGGGCTACTGCACCGACTTTCAGGCCGTGCCTGGCTGTGG CATCGGCTGCAAGGTGTCCAACGTGGAAGGCATCCTGGCCCACAGCGAGAGG CCACTGTCTGCCCCTGCCAGCCACCTGAACGAGGCCGGATCTCTGCCCGCCG AAAAGGACGCTGTGCCCCAGACCTTCTCTGTGCTGATTGGCAACAGAGAGTG GCTGCGGCGGAACGGCCTGACCATCTCCTCCGATGTGTCCGACGCCATGACC GACCACGAGATGAAGGGCCAGACCGCCATTCTGGTGGCCATTGACGGGGTGC TGTGCGGCATGATCGCAATCGCCGATGCCGTGAAACAGGAAGCAGCACTGGC CGTGCACACCCTGCAGTCTATGGGAGTGGATGTGGTGCTGATCACCGGCGAC AACAGAAAGACCGCCAGGGCCATTGCCACCCAGGTGGGCATCAACAAGGTGT TCGCCGAGGTGCTGCCCAGCCACAAAGTGGCCAAGGTGCAGGAACTGCAGAA CAAAGGCAAAAAGGTGGCCATGGTGGGAGATGGCGTGAACGACTCTCCTGCT CTGGCCCAGGCAGATATGGGCGTGGCCATCGGCACAGGCACCGACGTGGCAA TTGAGGCTGCTGACGTGGTGCTGATTCGGAACGACCTGCTGGACGTGGTGGC CTCCATCCACCTGTCCAAGAGAACCGTGCGGCGGATCAGAATCAACCTGGTG CTGGCACTGATCTATAACCTCGTGGGCATCCCTATCGCCGCTGGCGTGTTCA TGCCTATCGGAATCGTGCTGCAGCCCTGGATGGGCTCTGCCGCCATGGCTGC AAGCTCCGTGTCTGTGGTGCTGTCCAGCCTGCAGCTGAAGTGCTACAAGAAG CCCGACCTGGAAAGATACGAGGCCCAGGCCCACGGACACATGAAGCCTCTGA CAGCCTCCCAGGTGTCCGTGCACATCGGCATGGACGACAGATGGCGGGACAG CCCTAGAGCCACCCCTTGGGATCAGGTGTCATACGTGTCACAGGTGTCCCTG AGCAGCCTGACCAGCGACAAGCCCAGCAGACATAGCGCCGCTGCCGACGACG ATGGGGACAAGTGGTCCCTGCTGCTGAACGGCCGGGATGAGGAACAGTACAT C | |
| **15** | ATGCCAGAACAGGAACGCCAGATCACAGCAAGAGAGGGAGCAAGTCGGAAAA TCCTGAGCAAACTGAGCCTGCCAACCAGAGCATGGGAACCCGCAATGAAGAA AAGCTTCGCCTTTGACAACGTGGGATACGAGGGAGGGCTGGATGGACTGGGA CCTAGCTCCCAGGTGGCCACCTCTACAGTCCGAATCCTGGGCATGACTTGCC AGAGTTGCGTGAAATCAATTGAAGACCGGATCAGTAATCTGAAGGGAATCAT TAGCATGAAAGTGTCCCTGGAGCAGGGCTCAGCCACCGTGAAGTATGTCCCT AGCGTGGTCTGCCTGCAGCAGGTGTGCCACCAGATCGGCGATATGGGGTTCG AGGCCTCCATTGCTGAAGGGAAAGCCGCTTCTTGGCCTAGCCGGTCCCTGCC AGCACAGGAAGCAGTGGTCAAGCTGAGAGTGGAGGGAATGACATGCCAGAGC | **Codon optimized ATP7B variant (SEQ ID NO: 3 from WO** |
| | TGCGTGAGCAGTATCGAAGGAAAGGTCCGAAAACTGCAGGGCGTGGTCCGGG TGAAGGTCTCTCTGAGTAACCAGGAGGCCGTGATTACCTACCAGCCCTATCT GATCCAGCCTGAAGACCTGAGGGATCACGTGAATGACATGGGCTTCGAGGCA GCCATCAAGTCCAAAGTGGCCCCACTGTCTCTGGGGCCCATTGATATCGAAA GACTGCAGTCCACCAACCCAAAGAGGCCCCTGTCAAGCGCCAACCAGAACTT CAACAATAGTGAGACCCTGGGACACCAGGGCTCACATGTGGTCACACTGCAG CTGAGGATTGACGGCATGCACTGCAAGTCTTGCGTGCTGAACATTGAGGAAA ATATCGGCCAGCTGCTGGGGGTGCAGTCTATCCAGGTCAGTCTGGAGAACAA GACTGCTCAGGTGAAATACGATCCTTCATGCACCAGCCCAGTGGCACTGCAG CGCGCTATCGAAGCACTGCCCCCTGGAAATTTCAAGGTGAGCCTGCCTGACG GAGCAGAGGGATCCGGAACCGATCACAGGTCCTCTAGTTCACATTCCCCAGG GTCTCCACCACGAAACCAGGTGCAGGGAACATGTTCCACCACACTGATTGCA ATCGCCGGCATGACTTGCGCCTCATGCGTGCACAGCATTGAAGGGATGATCT CTCAGCTGGAGGGAGTGCAGCAGATCTCAGTCAGCCTGGCCGAGGGCACTGC TACCGTGCTGTACAATCCCAGTGTCATCTCACCTGAGGAACTGCGGGCTGCA ATTGAGGACATGGGGTTCGAAGCTTCCGTGGTCTCCGAATCTTGCAGTACCA ACCCCCTGGGGAATCATTCCGCCGGAAACTCTATGGTGCAGACTACCGACGG GACACCTACTTCTGTGCAGGAGGTCGCACCACACACAGGACGCCTGCCAGCC AATCATGCTCCCGATATCCTGGCCAAAAGCCCCCAGTCCACTCGAGCTGTGG CACCTCAGAAGTGTTTTCTGCAGATCAAAGGCATGACCTGCGCCTCTTGCGT GAGCAACATTGAGCGGAATCTGCAGAAGGAAGCTGGGGTGCTGAGCGTGCTG GTCGCACTGATGGCCGGAAAGGCTGAGATCAAGTACGACCCTGAAGTGATCC AGCCACTGGAGATTGCCCAGTTCATCCAGGATCTGGGCTTTGAGGCCGCTGT GATGGAAGACTATGCTGGGAGCGATGGAAACATTGAACTGACCATCACCGGA ATGACTTGTGCCTCTTGCGTGCACAACATCGAGAGTAAACTGACTAGAACCA ATGGGATTACCTACGCCAGTGTGGCCCTGGCTACATCAAAGGCTCTGGTGAA ATTCGACCCCGAGATCATTGGACCTAGGGATATCATTAAGATCATTGAGGAA ATCGGCTTTCACGCAAGCCTGGCCCAGCGCAACCCAAATGCCCACCATCTGG ACCATAAGATGGAGATCAAGCAGTGGAAGAAAAGTTTCCTGTGCTCACTGGT GTTTGGAATCCCCGTCATGGCCCTGATGATCTACATGCTGATCCCTAGCAAC GAGCCACACCAGTCCATGGTGCTGGATCATAACATCATTCCTGGCCTGTCCA TCCTGAATCTGATTTTCTTTATCCTGTGCACATTCGTGCAGCTGCTGGGAGG CTGGTACTTTTATGTGCAGGCATATAAATCACTGCGACACCGGAGCGCCAAT ATGGACGTGCTGATTGTCCTGGCAACCTCTATCGCCTACGTGTATAGTCTGG TCATCCTGGTGGTCGCAGTGGCAGAGAAGGCAGAACGGAGCCCAGTGACTTT CTTTGATACCCCTCCAATGCTGTTCGTGTTTATCGCTCTGGGCAGATGGCTG GAACATCTGGCAAAGTCAAAAACCAGCGAGGCTCTGGCAAAGCTGATGAGCC TGCAGGCTACCGAAGCAACAGTGGTCACTCTGGGAGAGGACAACCTGATCAT TCGCGAGGAACAGGTGCCTATGGAACTGGTCCAGCGAGGCGATATCGTGAAG GTGGTCCCAGGGGGAAAATTCCCCGTGGACGGCAAGGTCCTGGAGGGGAATA CTATGGCCGATGAATCCCTGATCACCGGCGAGGCTATGCCTGTGACAAAGAA ACCAGGATCAACTGTCATTGCTGGCAGCATCAACGCACACGGGTCCGTGCTG ATCAAGGCCACACATGTCGGGAATGACACAACTCTGGCTCAGATTGTGAAAC TGGTCGAGGAAGCCCAGATGTCCAAGGCTCCTATCCAGCAGCTGGCCGATCG GTTCTCCGGCTACTTCGTGCCCTTCATCATTATCATGTCTACACTGACTCTG GTGGTCTGGATTGTGATCGGATTCATTGACTTTGGCGTGGTCCAGAGATATT TTCCCAACCCTAATAAGCACATCAGCCAGACCGAAGTGATCATCAGGTTCGC ATTTCAGACCAGTATTACAGTGCTGTGCATCGCCTGCCCATGTTCACTGGGG CTGGCTACCCCCACAGCAGTGATGGTCGGAACAGGAGTGGCAGCACAGAACG GAATTCTGATCAAGGGCGGGAAACCCCTGGAGATGGCCCACAAGATCAAAAC TGTGATGTTTGACAAAACTGGGACCATTACACATGGAGTGCCCCGCGTCATG CGAGTGCTGCTGCTGGGCGATGTGGCAACCCTGCCTCTGAGAAAGGTCCTGG | **2016/0972 19)** |
| | CAGTGGTCGGAACAGCAGAGGCTAGCTCCGAACACCCACTGGGGGTGGCCGT CACAAAGTACTGCAAAGAGGAACTGGGCACTGAGACCCTGGGGTATTGTACT GACTTCCAGGCAGTGCCCGGATGCGGAATCGGATGTAAAGTCTCTAACGTGG AAGGGATTCTGGCTCACAGTGAGCGGCCCCTGAGCGCACCTGCATCCCATCT GAATGAAGCAGGAAGCCTGCCAGCAGAGAAGGACGCTGTGCCTCAGACCTTT TCCGTCCTGATCGGCAACAGAGAATGGCTGCGGAGAAATGGGCTGACAATTT CTAGTGACGTGTCCGATGCCATGACAGATCACGAGATGAAAGGCCAGACTGC AATTCTGGTGGCCATCGACGGAGTCCTGTGCGGCATGATTGCTATCGCAGAT GCCGTGAAGCAGGAGGCTGCACTGGCCGTCCATACCCTGCAGTCTATGGGCG TGGACGTGGTCCTGATCACCGGGGATAACCGGAAAACAGCTAGAGCAATTGC CACTCAAGTGGGCATCAATAAGGTGTTCGCTGAAGTCCTGCCTAGCCACAAG GTCGCAAAAGTGCAGGAGCTGCAGAACAAGGGCAAGAAAGTCGCCATGGTGG GAGACGGCGTGAATGATAGCCCAGCTCTGGCACAGGCAGACATGGGAGTCGC TATTGGGACAGGAACTGACGTGGCAATCGAGGCCGCTGATGTGGTCCTGATT AGGAATGACCTGCTGGATGTGGTCGCTTCTATTCATCTGAGTAAGAGGACAG TGAGGCGCATTCGCATCAACCTGGTGCTGGCCCTGATCTACAATCTGGTGGG AATTCCAATCGCAGCCGGCGTGTTTATGCCAATTGGGATCGTCCTGCAGCCC TGGATGGGCTCAGCTGCAATGGCCGCTTCAAGCGTGAGCGTGGTCCTGTCCT CTCTGCAGCTGAAATGCTACAAGAAACCAGACCTGGAGCGGTACGAAGCTCA GGCACACGGACATATGAAGCCCCTGACCGCTTCCCAGGTGTCTGTCCACATC GGCATGGACGATAGATGGAGGGACAGCCCAAGGGCCACTCCATGGGATCAGG TCAGTTACGTGAGCCAGGTCAGCCTGAGTTCACTGACCAGCGACAAGCCCTC CCGCCATTCTGCAGCCGCTGATGACGACGGGGACAAGTGGAGCCTGCTGCTG AACGGAAGGGACGAAGAACAGTATATC | |
| **16** | ATGCCTGAGCAGGAGAGACAGATCACAGCCAGAGAAGGGGCCAGTCGGAAAA TCTTATCTAAGCTTTCTTTGCCTACCCGTGCCTGGGAACCAGCAATGAAGAA GAGTTTTGCTTTTGACAATGTTGGCTATGAAGGTGGTCTGGATGGCCTGGGC CCTTCTTCTCAGCCGCAGAAGTGCTTCTTACAGATCAAAGGCATGACCTGTG CATCCTGTGTGTCTAACATAGAAAGGAATCTGCAGAAAGAAGCTGGTGTTCT CTCCGTGTTGGTTGCCTTGATGGCAGGAAAGGCAGAGATCAAGTATGACCCA GAGGTCATCCAGCCCCTCGAGATAGCTCAGTTCATCCAGGACCTGGGTTTTG AGGCAGCAGTCATGGAGGACTACGCAGGCTCCGATGGCAACATTGAGCTGAC AATCACAGGGATGACCTGCGCGTCCTGTGTCCACAACATAGAGTCCAAACTC ACGAGGACAAATGGCATCACTTATGCCTCCGTTGCCCTTGCCACCAGCAAAG CCCTTGTTAAGTTTGACCCGGAAATTATCGGTCCACGGGATATTATCAAAAT TATTGAGGAAATTGGCTTTCATGCTTCCCTGGCCCAGAGAAACCCCAACGCT CATCACTTGGACCACAAGATGGAAATAAAGCAGTGGAAGAAGTCTTTCCTGT GCAGCCTGGTGTTTGGCATCCCTGTCATGGCCTTAATGATCTATATGCTGAT ACCCAGCAACGAGCCCCACCAGTCCATGGTCCTGGACCACAACATCATTCCA GGACTGTCCATTCTAAATCTCATCTTCTTTATCTTGTGTACCTTTGTCCAGC TCCTCGGTGGGTGGTACTTCTACGTTCAGGCCTACAAATCTCTGAGACACAG GTCAGCCAACATGGACGTGCTCATCGTCCTGGCCACAAGCATTGCTTATGTT TATTCTCTGGTCATCCTGGTGGTTGCTGTGGCTGAGAAGGCGGAGAGGAGCC CTGTGACATTCTTCGACACGCCCCCCATGCTCTTTGTGTTCATTGCCCTGGG CCGGTGGCTGGAACACTTGGCAMETLEUPHEVALPHEILEALALEUGLYARG TRPLEUGLUHISLEUALAAAGAGCAAAACCTCAGAAGCCCTGGCTAAACTCA TGTCTCTCCAAGCCACAGAAGCCACCGTTGTGACCCTTGGTGAGGACAATTT AATCATCAGGGAGGAGCAAGTCCCCATGGAGCTGGTGCAGCGGGGCGATATC GTCAAGGTGGTCCCTGGGGGAAAGTTTCCAGTGGATGGGAAAGTCCTGGAAG GCAATACCATGGCTGATGAGTCCCTCATCACAGGAGAAGCCATGCCAGTCAC TAAGAAACCCGGAAGCACTGTAATTGCGGGGTCTATAAATGCACATGGCTCT GTGCTCATTAAAGCTACCCACGTGGGCAATGACACCACTTTGGCTCAGATTG | **Truncated ATP7B variant (SEQ ID NO: 6 from** WO 2016/0972 19**)** |
| | TGAAACTGGTGGAAGAGGCTCAGATGTCAAAGGCACCCATTCAGCAGCTGGC TGACCGGTTTAGTGGATATTTTGTCCCATTTATCATCATCATGTCAACTTTG ACGTTGGTGGTATGGATTGTAATCGGTTTTATCGATTTTGGTGTTGTTCAGA GATACTTTCCTAACCCCAACAAGCACATCTCCCAGACAGAGGTGATCATCCG GTTTGCTTTCCAGACGTCCATCACGGTGCTGTGCATTGCCTGCCCCTGCTCC CTGGGGCTGGCCACGCCCACGGCTGTCATGGTGGGCACCGGGGTGGCCGCGC AGAACGGCATCCTCATCAAGGGAGGCAAGCCCCTGGAGATGGCGCACAAGAT AAAGACTGTGATGTTTGACAAGACTGGCACCATTACCCATGGCGTCCCCAGG GTCATGCGGGTGCTCCTGCTGGGGGATGTGGCCACACTGCCCCTCAGGAAGG TTCTGGCTGTGGTGGGGACTGCGGAGGCCAGCAGTGAACACCCCTTGGGCGT GGCAGTCACCAAATACTGTAAAGAGGAACTTGGAACAGAGACCTTGGGATAC TGCACGGACTTCCAGGCAGTGCCAGGCTGTGGAATTGGGTGCAAAGTCAGCA ACGTGGAAGGCATCCTGGCCCACAGTGAGCGCCCTTTGAGTGCACCGGCCAG TCACCTGAATGAGGCTGGCAGCCTTCCCGCAGAAAAAGATGCAGTCCCCCAG ACCTTCTCTGTGCTGATTGGAAACCGTGAGTGGCTGAGGCGCAACGGTTTAA CCATTTCTAGCGATGTCAGTGACGCTATGACAGACCACGAGATGAAAGGACA GACAGCCATCCTGGTGGCTATTGACGGTGTGCTCTGTGGGATGATCGCAATC GCAGACGCTGTCAAGCAGGAGGCTGCCCTGGCTGTGCACACGCTGCAGAGCA TGGGTGTGGACGTGGTTCTGATCACGGGGGACAACCGGAAGACAGCCAGAGC TATTGCCACCCAGGTTGGCATCAACAAAGTCTTTGCAGAGGTGCTGCCTTCG CACAAGGTGGCCAAGGTCCAGGAGCTCCAGAATAAAGGGAAGAAAGTCGCCA TGGTGGGGGATGGGGTCAATGACTCCCCGGCCTTGGCCCAGGCAGACATGGG TGTGGCCATTGGCACCGGCACGGATGTGGCCATCGAGGCAGCCGACGTCGTC CTTATCAGAAATGATTTGCTGGATGTGGTGGCTAGCATTCACCTTTCCAAGA GGACTGTCCGAAGGATACGCATCAACCTGGTCCTGGCACTGATTTATAACCT GGTTGGGATACCCATTGCAGCAGGTGTCTTCATGCCCATCGGCATTGTGCTG CAGCCCTGGATGGGCTCAGCGGCCATGGCAGCCTCCTCTGTGTCTGTGGTGC TCTCATCCCTGCAGCTCAAGTGCTATAAGAAGCCTGACCTGGAGAGGTATGA GGCACAGGCGCATGGCCACATGAAGCCCCTGACGGCATCCCAGGTCAGTGTG CACATAGGCATGGATGACAGGTGGCGGGACTCCCCCAGGGCCACACCATGGG ACCAGGTCAGCTATGTCAGCCAGGTGTCGCTGTCCTCCCTGACGTCCGACAA GCCATCTCGGCACAGCGCTGCAGCAGACGATGATGGGGACAAGTGGTCTCTG CTCCTGAATGGCAGGGATGAGGAGCAGTACATC | |
| **17** | ATGCCAGAACAGGAACGCCAGATCACAGCAAGAGAGGGAGCAAGTCGGAAAA TCCTGAGCAAACTGAGCCTGCCAACCAGAGCATGGGAACCCGCAATGAAGAA AAGCTTCGCCTTTGACAACGTGGGATACGAGGGAGGGCTGGATGGACTGGGA CCTAGCTCCCAGCCTCAGAAGTGTTTTCTGCAGATCAAAGGCATGACCTGCG CCTCTTGCGTGAGCAACATTGAGCGGAATCTGCAGAAGGAAGCTGGGGTGCT GAGCGTGCTGGTCGCACTGATGGCCGGAAAGGCTGAGATCAAGTACGACCCT GAAGTGATCCAGCCACTGGAGATTGCCCAGTTCATCCAGGATCTGGGCTTTG AGGCCGCTGTGATGGAAGACTATGCTGGGAGCGATGGAAACATTGAACTGAC CATCACCGGAATGACTTGTGCCTCTTGCGTGCACAACATCGAGAGTAAACTG ACTAGAACCAATGGGATTACCTACGCCAGTGTGGCCCTGGCTACATCAAAGG CTCTGGTGAAATTCGACCCCGAGATCATTGGACCTAGGGATATCATTAAGAT CATTGAGGAAATCGGCTTTCACGCAAGCCTGGCCCAGCGCAACCCAAATGCC CACCATCTGGACCATAAGATGGAGATCAAGCAGTGGAAGAAAAGTTTCCTGT GCTCACTGGTGTTTGGAATCCCCGTCATGGCCCTGATGATCTACATGCTGAT CCCTAGCAACGAGCCACACCAGTCCATGGTGCTGGATCATAACATCATTCCT GGCCTGTCCATCCTGAATCTGATTTTCTTTATCCTGTGCACATTCGTGCAGC TGCTGGGAGGCTGGTACTTTTATGTGCAGGCATATAAATCACTGCGACACCG GAGCGCCAATATGGACGTGCTGATTGTCCTGGCAACCTCTATCGCCTACGTG TATAGTCTGGTCATCCTGGTGGTCGCAGTGGCAGAGAAGGCAGAACGGAGCC | **Truncated ATP7B variant (SEQ ID NO: 8 from** WO 2016/0972 19**)** |
| | CAGTGACTTTCTTTGATACCCCTCCAATGCTGTTCGTGTTTATCGCTCTGGG CAGATGGCTGGAACATCTGGCAAAGTCAAAAACCAGCGAGGCTCTGGCAAAG CTGATGAGCCTGCAGGCTACCGAAGCAACAGTGGTCACTCTGGGAGAGGACA ACCTGATCATTCGCGAGGAACAGGTGCCTATGGAACTGGTCCAGCGAGGCGA TATCGTGAAGGTGGTCCCAGGGGGAAAATTCCCCGTGGACGGCAAGGTCCTG GAGGGGAATACTATGGCCGATGAATCCCTGATCACCGGCGAGGCTATGCCTG TGACAAAGAAACCAGGATCAACTGTCATTGCTGGCAGCATCAACGCACACGG GTCCGTGCTGATCAAGGCCACACATGTCGGGAATGACACAACTCTGGCTCAG ATTGTGAAACTGGTCGAGGAAGCCCAGATGTCCAAGGCTCCTATCCAGCAGC TGGCCGATCGGTTCTCCGGCTACTTCGTGCCCTTCATCATTATCATGTCTAC ACTGACTCTGGTGGTCTGGATTGTGATCGGATTCATTGACTTTGGCGTGGTC CAGAGATATTTTCCCAACCCTAATAAGCACATCAGCCAGACCGAAGTGATCA TCAGGTTCGCATTTCAGACCAGTATTACAGTGCTGTGCATCGCCTGCCCATG TTCACTGGGGCTGGCTACCCCCACAGCAGTGATGGTCGGAACAGGAGTGGCA GCACAGAACGGAATTCTGATCAAGGGCGGGAAACCCCTGGAGATGGCCCACA AGATCAAAACTGTGATGTTTGACAAAACTGGGACCATTACACATGGAGTGCC CCGCGTCATGCGAGTGCTGCTGCTGGGCGATGTGGCAACCCTGCCTCTGAGA AAGGTCCTGGCAGTGGTCGGAACAGCAGAGGCTAGCTCCGAACACCCACTGG GGGTGGCCGTCACAAAGTACTGCAAAGAGGAACTGGGCACTGAGACCCTGGG GTATTGTACTGACTTCCAGGCAGTGCCCGGATGCGGAATCGGATGTAAAGTC TCTAACGTGGAAGGGATTCTGGCTCACAGTGAGCGGCCCCTGAGCGCACCTG CATCCCATCTGAATGAAGCAGGAAGCCTGCCAGCAGAGAAGGACGCTGTGCC TCAGACCTTTTCCGTCCTGATCGGCAACAGAGAATGGCTGCGGAGAAATGGG CTGACAATTTCTAGTGACGTGTCCGATGCCATGACAGATCACGAGATGAAAG GCCAGACTGCAATTCTGGTGGCCATCGACGGAGTCCTGTGCGGCATGATTGC TATCGCAGATGCCGTGAAGCAGGAGGCTGCACTGGCCGTCCATACCCTGCAG TCTATGGGCGTGGACGTGGTCCTGATCACCGGGGATAACCGGAAAACAGCTA GAGCAATTGCCACTCAAGTGGGCATCAATAAGGTGTTCGCTGAAGTCCTGCC TAGCCACAAGGTCGCAAAAGTGCAGGAGCTGCAGAACAAGGGCAAGAAAGTC GCCATGGTGGGAGACGGCGTGAATGATAGCCCAGCTCTGGCACAGGCAGACA TGGGAGTCGCTATTGGGACAGGAACTGACGTGGCAATCGAGGCCGCTGATGT GGTCCTGATTAGGAATGACCTGCTGGATGTGGTCGCTTCTATTCATCTGAGT AAGAGGACAGTGAGGCGCATTCGCATCAACCTGGTGCTGGCCCTGATCTACA ATCTGGTGGGAATTCCAATCGCAGCCGGCGTGTTTATGCCAATTGGGATCGT CCTGCAGCCCTGGATGGGCTCAGCTGCAATGGCCGCTTCAAGCGTGAGCGTG GTCCTGTCCTCTCTGCAGCTGAAATGCTACAAGAAACCAGACCTGGAGCGGT ACGAAGCTCAGGCACACGGACATATGAAGCCCCTGACCGCTTCCCAGGTGTC TGTCCACATCGGCATGGACGATAGATGGAGGGACAGCCCAAGGGCCACTCCA TGGGATCAGGTCAGTTACGTGAGCCAGGTCAGCCTGAGTTCACTGACCAGCG ACAAGCCCTCCCGCCATTCTGCAGCCGCTGATGACGACGGGGACAAGTGGAG CCTGCTGCTGAACGGAAGGGACGAAGAACAGTATATC | |
| **18** | ATGCCTGAGCAGGAGAGACAGATCACAGCCAGAGAAGGGGCCAGTCGGAAAA TCTTATCTAAGCTTTCTTTGCCTACCCGTGCCTGGGAACCAGCAATGAAGAA GAGTTTTGCTTTTGACAATGTTGGCTATGAAGGTGGTCTGGATGGCCTGGGC CCTTCTTCTCAGGTGGCCACCAGCACAGTCAGGATCTTGGGCATGACTTGCC AGTCATGTGTGAAGTCCATTGAGGACAGGATTTCCAATTTGAAAGGCATCAT CAGCATGAAGGTTTCCCTGGAACAAGGCAGTGCCACTGTGAAATATGTGCCA TCGGTTGTGTGCCTGCAACAGGTTTGCCATCAAATTGGGGACATGGGCTTCG AGGCCAGCATTGCAGAAGGAAAGGCAGCCTCCTGGCCCTCAAGGTCCTTGCC TGCCCAGGAGGCTGTGGTCAAGCTCCGGGTGGAGGGCATGACCTGCCAGTCC TGTGTCAGCTCCATTGAAGGCAAGGTCCGGAAACTGCAAGGAGTAGTGAGAG TCAAAGTCTCACTCAGCAACCAAGAGGCCGTCATCACTTATCAGCCTTATCT | **Truncated ATP7B variant (SEQ ID NO: 12 from** WO 2016/0972 19**)** |
| | CATTCAGCCCGAAGACCTCAGGGACCATGTAAATGACATGGGATTTGAAGCT GCCATCAAGAGCAAAGTGGCTCCCTTAAGCCTGGGACCAATTGGCATGACCT GTGCATCCTGTGTCCATTCCATTGAAGGCATGATCTCCCAACTGGAAGGGGT GCAGCAAATATCGGTGTCTTTGGCCGAAGGGACTGCAACAGTTCTTTATAAT CCCTCTGTAATTAGCCCAGAAGAACTCAGAGCTGCTATAGAAGACATGGGAT TTGAGGCTTCAGTCGTTTCTGAAAGCTGTTCTACTAACCCTCTTGGAAACCA CAGTGCTGGGAATTCCATGGTGCAAACTACAGATGGTACACCTACATCTGTG CAGGAAGTGGCTCCCCACACTGGGAGGCTCCCTGCAAACCATGCCCCGGACA TCTTGGCAAAGTCCCCACAATCAACCAGAGCAGTGGCACCGCAGAAGTGCTT CTTACAGATCAAAGGCATGACCTGTGCATCCTGTGTGTCTAACATAGAAAGG AATCTGCAGAAAGAAGCTGGTGTTCTCTCCGTGTTGGTTGCCTTGATGGCAG GAAAGGCAGAGATCAAGTATGACCCAGAGGTCATCCAGCCCCTCGAGATAGC TCAGTTCATCCAGGACCTGGGTTTTGAGGCAGCAGTCATGGAGGACTACGCA GGCTCCGATGGCAACATTGAGCTGACAATCACAGGGATGACCTGCGCGTCCT GTGTCCACAACATAGAGTCCAAACTCACGAGGACAAATGGCATCACTTATGC CTCCGTTGCCCTTGCCACCAGCAAAGCCCTTGTTAAGTTTGACCCGGAAATT ATCGGTCCACGGGATATTATCAAAATTATTGAGGAAATTGGCTTTCATGCTT CCCTGGCCCAGAGAAACCCCAACGCTCATCACTTGGACCACAAGATGGAAAT AAAGCAGTGGAAGAAGTCTTTCCTGTGCAGCCTGGTGTTTGGCATCCCTGTC ATGGCCTTAATGATCTATATGCTGATACCCAGCAACGAGCCCCACCAGTCCA TGGTCCTGGACCACAACATCATTCCAGGACTGTCCATTCTAAATCTCATCTT CTTTATCTTGTGTACCTTTGTCCAGCTCCTCGGTGGGTGGTACTTCTACGTT CAGGCCTACAAATCTCTGAGACACAGGTCAGCCAACATGGACGTGCTCATCG TCCTGGCCACAAGCATTGCTTATGTTTATTCTCTGGTCATCCTGGTGGTTGC TGTGGCTGAGAAGGCGGAGAGGAGCCCTGTGACATTCTTCGACACGCCCCCC ATGCTCTTTGTGTTCATTGCCCTGGGCCGGTGGCTGGAACACTTGGCAAAGA GCAAAACCTCAGAAGCCCTGGCTAAACTCATGTCTCTCCAAGCCACAGAAGC CACCGTTGTGACCCTTGGTGAGGACAATTTAATCATCAGGGAGGAGCAAGTC CCCATGGAGCTGGTGCAGCGGGGCGATATCGTCAAGGTGGTCCCTGGGGGAA AGTTTCCAGTGGATGGGAAAGTCCTGGAAGGCAATACCATGGCTGATGAGTC CCTCATCACAGGAGAAGCCATGCCAGTCACTAAGAAACCCGGAAGCACTGTA ATTGCGGGGTCTATAAATGCACATGGCTCTGTGCTCATTAAAGCTACCCACG TGGGCAATGACACCACTTTGGCTCAGATTGTGAAACTGGTGGAAGAGGCTCA GATGTCAAAGGCACCCATTCAGCAGCTGGCTGACCGGTTTAGTGGATATTTT GTCCCATTTATCATCATCATGTCAACTTTGACGTTGGTGGTATGGATTGTAA TCGGTTTTATCGATTTTGGTGTTGTTCAGAGATACTTTCCTAACCCCAACAA GCACATCTCCCAGACAGAGGTGATCATCCGGTTTGCTTTCCAGACGTCCATC ACGGTGCTGTGCATTGCCTGCCCCTGCTCCCTGGGGCTGGCCACGCCCACGG CTGTCATGGTGGGCACCGGGGTGGCCGCGCAGAACGGCATCCTCATCAAGGG AGGCAAGCCCCTGGAGATGGCGCACAAGATAAAGACTGTGATGTTTGACAAG ACTGGCACCATTACCCATGGCGTCCCCAGGGTCATGCGGGTGCTCCTGCTGG GGGATGTGGCCACACTGCCCCTCAGGAAGGTTCTGGCTGTGGTGGGGACTGC GGAGGCCAGCAGTGAACACCCCTTGGGCGTGGCAGTCACCAAATACTGTAAA GAGGAACTTGGAACAGAGACCTTGGGATACTGCACGGACTTCCAGGCAGTGC CAGGCTGTGGAATTGGGTGCAAAGTCAGCAACGTGGAAGGCATCCTGGCCCA CAGTGAGCGCCCTTTGAGTGCACCGGCCAGTCACCTGAATGAGGCTGGCAGC CTTCCCGCAGAAAAAGATGCAGTCCCCCAGACCTTCTCTGTGCTGATTGGAA ACCGTGAGTGGCTGAGGCGCAACGGTTTAACCATTTCTAGCGATGTCAGTGA CGCTATGACAGACCACGAGATGAAAGGACAGACAGCCATCCTGGTGGCTATT GACGGTGTGCTCTGTGGGATGATCGCAATCGCAGACGCTGTCAAGCAGGAGG CTGCCCTGGCTGTGCACACGCTGCAGAGCATGGGTGTGGACGTGGTTCTGAT CACGGGGGACAACCGGAAGACAGCCAGAGCTATTGCCACCCAGGTTGGCATC | |
| | AACAAAGTCTTTGCAGAGGTGCTGCCTTCGCACAAGGTGGCCAAGGTCCAGG AGCTCCAGAATAAAGGGAAGAAAGTCGCCATGGTGGGGGATGGGGTCAATGA CTCCCCGGCCTTGGCCCAGGCAGACATGGGTGTGGCCATTGGCACCGGCACG GATGTGGCCATCGAGGCAGCCGACGTCGTCCTTATCAGAAATGATTTGCTGG ATGTGGTGGCTAGCATTCACCTTTCCAAGAGGACTGTCCGAAGGATACGCAT CAACCTGGTCCTGGCACTGATTTATAACCTGGTTGGGATACCCATTGCAGCA GGTGTCTTCATGCCCATCGGCATTGTGCTGCAGCCCTGGATGGGCTCAGCGG CCATGGCAGCCTCCTCTGTGTCTGTGGTGCTCTCATCCCTGCAGCTCAAGTG CTATAAGAAGCCTGACCTGGAGAGGTATGAGGCACAGGCGCATGGCCACATG AAGCCCCTGACGGCATCCCAGGTCAGTGTGCACATAGGCATGGATGACAGGT GGCGGGACTCCCCCAGGGCCACACCATGGGACCAGGTCAGCTATGTCAGCCA GGTGTCGCTGTCCTCCCTGACGTCCGACAAGCCATCTCGGCACAGCGCTGCA GCAGACGATGATGGGGACAAGTGGTCTCTGCTCCTGAATGGCAGGGATGAGG AGCAGTACATC | |

**TABLE 2: Regulatory Element Sequences**

| **SEQ ID NO:** | **Sequence (5'-to-3')** | **Length** |
|---|---|---|
| **19** | GTAAGGTAAGAATTGAATTTCTCAGTTGAAGGATGCTTACACTCTTGTCCAT CTAG | 56bp |
| **20** | GTGTGTATGCTCAGGGGCTGGGAAAGGAGGGGAGGGAGCTCCGGCTCAG | 49bp |
| **21** | GTGATGCGGTTTTGGCAGTACATCAATGGGCGTGGATAGCGGTTTGACTCAC GGGGATTTCCAAGTCTCCACCCCATTGACGTCAATGGGAGTTTGTTTTGGCA CCAAAATCAACGGGACTTTCCAAAATGTCGTAACAACTCCGCCCCATTGACG CAAATGGGCGGTAGGCGTGTACGGTGGGAGGTCTATATAAGCAGAGCTGGTA CCGTAAGGTAAGAATTGAATTTCTCAGTTGAAGGATGCTTACACTCTTGTCC ATCTAG | 266bp |
| **22** | GTGATGCGGTTTTGGCAGTACATCAATGGGCGTGGATAGCGGTTTGACTCAC GGGGATTTCCAAGTCTCCACCCCATTGACGTCAATGGGAGTTTGTTTTGGCA CCAAAATCAACGGGACTTTCCAAAATGTCGTAACAACTCCGCCCCATTGACG CAAATGGGCGGTAGGCGTGTACGGTGGGAGGTCTATATAAGCAGAGCTGGTA CCGTGTGTATGCTCAGGGGCTGGGAAAGGAGGGGAGGGAGCTCCGGCTCAG | 259bp |
| **23** | GTGATGACGTGTCCCATAAGGCCCCTCGGTCTAAGGCTTCCCTATTTCCTGG TTCGCCGGCGGCCATTTTGGGTGGAAGCGATAGCTGAGTGGCGGCGGCTGCT GATTGTGTTCTAG | 117bp |
| **24** | GTGATGACGTGTCCCATACTTCCGGGTCAGGTGGGCCGGCTGTCTTGACCTT CTTTGCGGCTCGGCCATTTTGTCCCAGTCAGTCCGGAGGCTGCGGCTGCAGA AGTACCGCCTGCG | 117bp |
| **25** | GTGATGACGTGTCCCATATTTTCATCTCGCGAGACTTGTGAGCGGCCATCTT GGTCCTGCCCTGACAGATTCTCCTATCGGGGTCACAGGGACGCTAAGATTGC TACCTGGACTTTC | 117bp |
| **26** | GTGATGACGTGTCCCATGGCCTCATTGGATGAGAGGTCCCACCTCACGGCCC GAGGCGGGGCTTCTTTGCGCTTAAAAGCCGAGCCGGGCCAATGTTCAAATGC GCAGCTCTTAGTC | 117bp |
| **27** | GTGATGACGTGTCCCATCCCCCCTCCACCCCCTAGCCCGCGGAGCACGCTGG GATTTGGCGCCCCCCTCCTCGGTGCAACCTATATAAGGCTCACAGTCTGCGC TCCTGGTACACGC | 117bp |
| **28** | CCCCCCTCCACCCCCTAGCCCGCGGAGCACGCTGGGATTTGGCGCCCCCCTC CTCGGTGCAACCTATATAAGGCTCACAGTCTGCGCTCCTGGTACACGC | 100bp |
| **29** | GGCCTCATTGGATGAGAGGTCCCACCTCACGGCCCGAGGCGGGGCTTCTTTG CGCTTAAAAGCCGAGCCGGGCCAATGTTCAAATGCGCAGCTCTTAGTC | 100bp |
| **30** | GGGTGGGGCCCGCGCGTATAAAGGGGGCGCAGGCGGGCTGGGCGTTCCACAG GCCAAGTGCGCTGTGCTCGAGGGGTGCCGGCCAGGCCTGAGCGAGCGA | 100bp |
| **31** | GGTGCGATATTCGGATTGGCTGGAGTCGGCCATCACGCTCCAGCTACGCCAC TTCCTTTTCGTGGCACTATAAAGGGTGCTGCACGGCGCTTGCATCTCT | 100bp |
| **32** | ACTTCCGGGTCAGGTGGGCCGGCTGTCTTGACCTTCTTTGCGGCTCGGCCAT TTTGTCCCAGTCAGTCCGGAGGCTGCGGCTGCAGAAGTACCGCCTGCG | 100bp |
| **33** | GCTGAGCGCGCGCGATGGGGCGGGAGGTTTGGGGTCAAGGAGCAAACTCTGC ACAAGATGGCGGCGGTAGCGGCAGTGGCGGCGCGTAGGAGGCGGTGAG | 100bp |
| **34** | ATTTTCATCTCGCGAGACTTGTGAGCGGCCATCTTGGTCCTGCCCTGACAGA TTCTCCTATCGGGGTCACAGGGACGCTAAGATTGCTACCTGGACTTTC | 100bp |
| **35** | TGGGACCCCCGGAAGGCGGAAGTTCTAGGGCGGAAGTGGCCGAGAGGAGAGG AGAATGGCGGCGGAAGGCTGGATTTGGCGTTGGGGCTGGGGCCGGCGG | 100bp |
| **36** | AAGGCCCCTCGGTCTAAGGCTTCCCTATTTCCTGGTTCGCCGGCGGCCATTT TGGGTGGAAGCGATAGCTGAGTGGCGGCGGCTGCTGATTGTGTTCTAG | 100bp |
| **37** | AGTGACCCGGAAGTAGAAGTGGCCCTTGCAGGCAAGAGTGCTGGAGGGCGGC AGCGGCGACCGGAGCGGTAGGAGCAGCAATTTATCCGTGTGCAGCCCC | 100bp |
| **38** | GGGAGGGGCGCGCTGGGGAGCTTCGGCGCATGCGCGCTGAGGCCTGCCTGAC CGACCTTCAGCAGGGCTGTGGCTACCATGTTCTCTCGCGCGGGTGTCG | 100bp |
| **39** | ACTGCGCACGCGCGCGGTCGCACCGATTCACGCCCCCTTCCGGCGCCTAGAG CACCGCTGCCGCCATGTTGAGGGGGGGACCGCGACCAGCTGGGCCCCT | 100bp |
| **40** | CCCTCGAGGGGCGGAGCAAAAAGTGAGGCAGCAACGCCTCCTTATCCTCGCT CCCGCTTTCAGTTCTCAATAAGGTCCGATGTTCGTGTATAAATGCTCG | 100bp |
| **41** | CTTGGTGACCAAATTTGAAAAAAAAAAAAAACCGCGCCAACTCATGTTGTTT TCAATCAGGTCCGCCAAGTTTGTATTTAAGGAACTGTTTCAGTTCATA | 100bp |
| **42** | GGCTGAGCTATCCTATTGGCTATCGGGACAAAATTTGCTTGAGCCAATCAAA GTGCTCCGTGGACAATCGCCGTTCTGTCTATAAAAAGGTGAAGCAGCG | 100bp |
| **43** | GGAAGTGCCAGACCGGAGGTGCGTCATTCACCGGCGACGCCGATACGGTTCC TCCACCGAGGCCCATGCGAAGCTTTCCACTATGGCTTCCAGCACTGTC | 100bp |
| **66** | TAATCAGTAACCGTCCCACAGGGGATCATACTTTGGAATAGCAAATATTTGC TGAAGGTTCTGGGCTGCAAAGCTGAAGCTTTGGTTTCTGCTCTAAATG | 100bp |
| **67** | TGCAGAGGCGGAACCAGGATGTGACGTAATAACCGCGCGCGGCGCTCGGCGT TCCCGCAAGGTCGCTTTGCAGAGCGGGAGCGCGCTTAAGTAACTAGTC | 100bp |
| **68** | GTGATGACGTGTCCCATGCTGAGCGCGCGCGATGGGGCGGGAGGTTTGGGGT CAAGGAGCAAACTCTGCACAAGATGGCGGCGGTAGCGGCAGTGGCGGCGCGT AGGAGGCGGTGAG | 117bp |

**TABLE 3: Additional Promoter Sequences**

| **SEQ ID NO.** | **Sequence (5'-to-3')** | **Name** |
|---|---|---|
| **48** | GTACTTATATAAGGGGGTGGGGGCGCGTTCGTCCTCAGTCGCGATCGAACAC TCGAGCCGAGCAGACGTGCCTACGGACC | SCP |
| **49** | GGGGAGGCTGCTGGTGAATATTAACCAAGGTCACCCCAGTTATCGGAGGAGC AAACAGGGGCTAAGTCCACGCTAGCGTCTGTCTGCACATTTCGTAGAGCGAG TGTTCCGATACTCTAATCTCCCTAGGCAAGGTTCATATTTGTGTAGGTTACT TATTCTCCTTTTGTTGACTAAGTCAATAATCAGAATCAGCAGGTTTGGAGTC AGCTTGGCAGGGATCAGCAGCCTGGGTTGGAAGGAGGGGGTATAAAAGCCCC TTCACCAGGAGAAGCCGTC | SerpE_TTR |
| **50** | GTTTGCTGCTTGCAATGTTTGCCCATTTTAGGGTGGACACAGGACGCTGTGG TTTCTGAGCCAGGGCTAGCGGGCGACTCAGATCCCAGCCAGTGGACTTAGCC CCTGTTTGCTCCTCCGATAACTGGGGTGACCTTGGTTAATATTCACCAGCAG CCTCCCCCGTTGCCCCTCTGGATCCACTGCTTAAATACGGACGAGGACAGGG CCCTGTCTCCTCAGCTTCAGGCACCACCACTGACCTGGGACAGTGAATCGCC AC | Proto 1 |
| **51** | TGATGCGGTTTTGGCAGTACATCAATGGGCGTGGATAGCGGTTTGACTCACG GGGATTTCCAAGTCTCCACCCCATTGACGTCAATGGGAGTTTGTTTTGGCAC CAAAATCAACGGGACTTTCCAAAATGTCGTAACAACTCCGCCCCATTGACGC AAATGGGCGGTAGGCGTGTACGGTGGGAGGTCTATATAAGCAGAGCT | minCMV |
| **52** | GTTTGCTGCTTGCAATGTTTGCCCATTTTAGGGTGGACACAGGACGCTGTGG TTTCTGAGCCAGGGGGCGACTCAGATCCCAGCCAGTGGACTTAGCCCCTGTT TGCTCCTCCGATAACTGGGGTGACCTTGGTTAATATTCACCAGCAGCCTCCC CCGTTGCCCCTCTGGATCCACTGCTTAAATACGGACGAGGACAGGGCCCTGT CTCCTCAGCTTCAGGCACCACCACTGACCTGGGACAGTGAATC | UCL-HLP |
| **53** | CGTTACATAACTTACGGTAAATGGCCCGCCTGGCTGACCGCCCAACGACCCC CGCCCATTGACGTCAATAATGACGTATGTTCCCATAGTAACGCCAATAGGGA CTTTCCATTGACGTCAATGGGTGGAGTATTTACGGTAAACTGCCCACTTGGC AGTACATCAAGTGTATCATATGCCAAGTACGCCCCCTATTGACGTCAATGAC GGTAAATGGCCCGCCTGGCATTATGCCCAGTACATGACCTTATGGGACTTTC CTACTTGGCAGTACATCTACGTATTAGTCATCGCTATTACCATG | CMVe |
| **54** | GTTACATAACTTACGGTAAATGGCCCGCCTGGCTGACCGCCCAACGACCCCC GCCCATTGACGTCAATAATGACGTATGTTCCCATAGTAACGCCAATAGGGAC TTTCCATTGACGTCAATGGGTGGAGTATTTACGGTAAACTGCCCACTTGGCA GTACATCAAGTGTATCATATGCCAAGTACGCCCCCTATTGACGTCAATGACG GTAAATGGCCCGCCTGGCATTATGCCCAGTACATGACCTTATGGGACTTTCC TACTTGGCAGTACATCTACGTATTAGTCATCGCTATTACCATGGGTCGAGGT GAGCCCCACGTTCTGCTTCACTCTCCCCATCTCCCCCCCCTCCCCACCCCCA ATTTTGTATTTATTTATTTTTTAATTATTTTGTGCAGCGATGGGGGCGGGGG GGGGGGGGGCGCGCGCCAGGCGGGGCGGGGCGGGGCGAGGGGCGGGGCGGGG CGAGGCGGAGAGGTGCGGCGGCAGCCAATCAGAGCGGCGCGCTCCGAAAGTT TCCTTTTATGGCGAGGCGGCGGCGGCGGCGGCCCTATAAAAAGCGAAGCGCG CGGCGGGCGGGAGTCGCTGCGTTGCCTTCGCCCCGTGCCCCGCTCCGCGCCG CCTCGCGCCGCCCGCCCCGGCTCTGACTGACCGCGTTACTCCCACAGGTGAG CGGGCGGGACGGCCCTTCTCCTCCGGGCTGTAATTAGCGCTTGGTTTAATGA CGGCTCGTTTCTTTTCTGTGGCTGCGTGAAAGCCTTAAAGGGCTCCGGGAGG GCCCTTTGTGCGGGGGGGAGCGGCTCGGGGGGTGCGTGCGTGTGTGTGTGCG TGGGGAGCGCCGCGTGCGGCCCGCGCTGCCCGGCGGCTGTGAGCGCTGCGGG CGCGGCGCGGGGCTTTGTGCGCTCCGCGTGTGCGCGAGGGGAGCGCGGCCGG GGGCGGTGCCCCGCGGTGCGGGGGGGCTGCGAGGGGAACAAAGGCTGCGTGC GGGGTGTGTGCGTGGGGGGGTGAGCAGGGGGTGTGGGCGCGGCGGTCGGGCT GTAACCCCCCCCTGCACCCCCCTCCCCGAGTTGCTGAGCACGGCCCGGCTTC | CAG |
| | GGGTGCGGGGCTCCGTGCGGGGCGTGGCGCGGGGCTCGCCGTGCCGGGCGGG GGGTGGCGGCAGGTGGGGGTGCCGGGCGGGGCGGGGCCGCCTCGGGCCGGGG AGGGCTCGGGGGAGGGGCGCGGCGGCCCCGGAGCGCCGGCGGCTGTCGAGGC GCGGCGAGCCGCAGCCATTGCCTTTTATGGTAATCGTGCGAGAGGGCGCAGG GACTTCCTTTGTCCCAAATCTGGCGGAGCCGAAATCTGGGAGGCGCCGCCGC ACCCCCTCTAGCGGGCGCGGGCGAAGCGGTGCGGCGCCGGCAGGAAGGAAAT GGGCGGGGAGGGCCTTCGTGCGTCGCCGCGCCGCCGTCCCCTTCTCCATCTC CAGCCTCGGGGCTGCCGCAGGGGGACGGCTGCCTTCGGGGGGGACGGGGCAG GGCGGGGTTCGGCTTCTGGCGTGTGACCGGCGGCTCTAGAGCCTCTGCTAAC CATGTTCATGCCTTCTTCTTTTTCCTACAGCTCCTGGGCAACGTGCTGGTTG TTGTGCTGTCTCATCATTTTGGCAAAGAATT | |
| **55** | GCTCCGGTGCCCGTCAGTGGGCAGAGCGCACATCGCCCACAGTCCCCGAGAA GTTGGGGGGAGGGGTCGGCAATTGAACCGGTGCCTAGAGAAGGTGGCGCGGG GTAAACTGGGAAAGTGATGTCGTGTACTGGCTCCGCCTTTTTCCCGAGGGTG GGGGAGAACCGTATATAAGTGCAGTAGTCGCCGTGAACGTTCTTTTTCGCAA CGGGTTTGCCGCCAGAACACAGG | EFS |
| **69** | GTCTGTCTGCACATTTCGTAGAGCGAGTGTTCCGATACTCTAATCTCCCTAG GCAAGGTTCATATTTGTGTAGGTTACTTATTCTCCTTTTGTTGACTAAGTCA ATAATCAGAATCAGCAGGTTTGGAGTCAGCTTGGCAGGGATCAGCAGCCTGG GTTGGAAGGAGGGGGTATAAAAGCCCCTTCACCAGGAGAAGCCGTC | Minimal Transthyretin (mTTR) |
| **70** | CGCCACCCCCTCCACCTTGGACACAGGACGCTGTGGTTTCTGAGCCAGGTAC AATGACTCCTTTCGGTAAGTGCAGTGGAAGCTGTACACTGCCCAGGCAAAGC GTCCGGGCAGCGTAGGCGGGCGACTCAGATCCCAGCCAGTGGACTTAGCCCC TGTTTGCTCCTCCGATAACTGGGGTGACCTTGGTTAATATTCACCAGCAGCC TCCCCCGTTGCCCCTCTGGATCCACTGCTTAAATACGGACGAGGACAGGGCC CTGTCTCCTCAGCTTCAGGCACCACCACTGACCTGGGACAGTGAA | Alpha 1 antitrypsin |

### EXAMPLES

These examples are provided for illustrative purposes only and not to limit the scope of the claims provided herein.

### EXAMPLE 1

### Expression of ATP7B Codon Optimized Variants in HEK293 Cells

HEK293 cells were cultured per standard methods, and transfected (PEI) with 3ug plasmid per well of a 6-well plate. 72h following transfection, cells were collected and RNA was isolated using an RNeasy Mini kit (Qiagen) in accordance with manufacturer's protocols, including on-column DNase treatment. RNA (3ug) was reverse transcribed Superscript IV (Invitrogen), using OligoDT primers (65°C 5m, 55°C 10m, 85°C 10m). Primers specific to codon optimized variants of ATP7B were tested by standard curves to test reliability, and cDNA was used for qPCR (Phusion, SYBR Green) under the following conditions: (98°C 2m, 40X[98°C 10s, 58°C 30s, 92°C 15s]).

**TABLE 4. Sequences of Forward and Reverse Primers for ATP7B Constructs and GAPDH.**

| Construct | Forward Primer | SEQ ID NO | Reverse Primer | SEQ ID NO |
|---|---|---|---|---|
| WT ATP7B | 5'-GACCTTTATCTTCTGCTAACC-3' | 56 | 5'-CAAGACGCAAGACTTACAA-3' | 57 |
| ATP7B Variant 2 | 5'-AGGGTACATGCAGTACCAC-3' | 58 | 5'-GGCTTATAACGGATGGATTG-3' | 59 |
| ATP7B Variant 3 | 5'-CACCAGCACCGTGAGAATC-3' | 60 | 5'-CCTTCATAGAGATGATGCCC-3' | 61 |
| ATP7B Variant 4 | 5'-CACCAGCACCGTGAGAATC-3' | 62 | 5'-CACCTTCATAGAGATGATTCC-3' | 63 |
| GAPDH | 5'-ACCACAGTCCATGCCATCAC-3' | 44 | 5'-TCCACCACCCTGTTGCTGTA-3' | 45 |

Primers against WT ATP7B (SEQ ID NO: 1), ATP7B Variant No. 2 (SEQ ID NO: 4), and ATP7B Variant No. 3 (SEQ ID NO: 5) showed comparable amplification efficiencies near 100% (data not shown). ATP7B expression was normalized to GAPDH, and presented as fold change over baseline using the delta-delta Ct method.

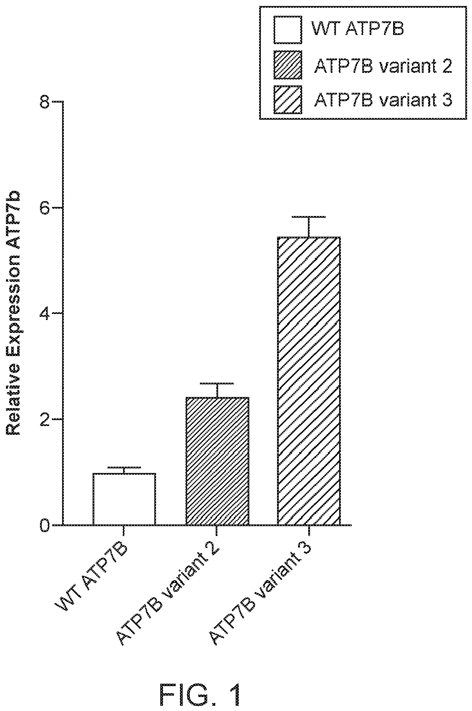

Results are shown in **FIG. 1****.** The level of expression of ATP7B Variant 2 (SEQ ID NO: 4) in HEK293 cells was about 2.5 fold higher than the level of expression of wild-type ATP7B (SEQ ID NO: 1), and the level of expression of ATP7B Variant 3 (SEQ ID NO: 5) in HEK293 cells was about 5.5 fold higher than the level of expression of wild-type ATP7B (SEQ ID NO: 1).

### EXAMPLE 2

### Expression of ATP7B Codon Optimized Variants in Mouse Liver

C57BL/6 mice (n=10 mice per/condition) were injected via tail vein with AAV8 vectors (1E12 gc/mouse) containing wild-type ATP7B (SEQ ID NO: 1) or codon optimized ATP7B variants 2 or 3 (SEQ ID NO: 4 and 5, respectively) under the control of the promoter having SEQ ID NO: 24. Two weeks after viral delivery, livers were harvested. Left liver lobes were stored in RNA later (Invitrogen, AM7020M) overnight and transferred to -80°C after RNA later removal for human ATP7B RNA analysis by RT-qPCR.

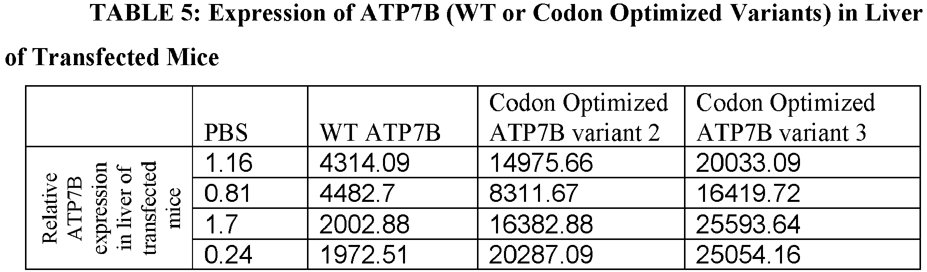

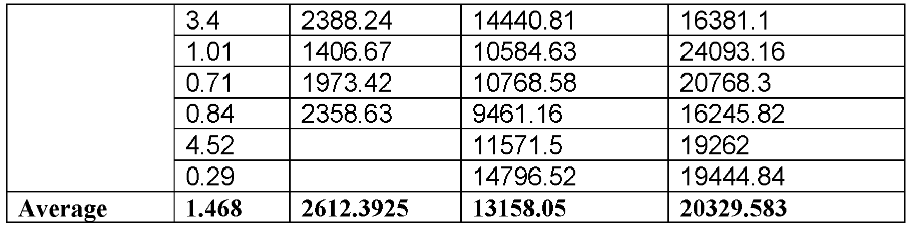

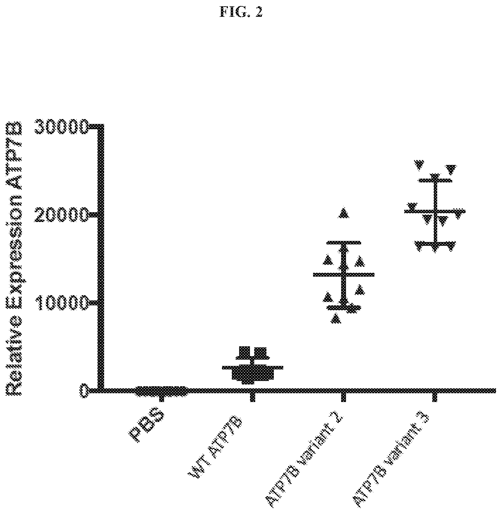

As shown in Table 5 and **FIG. 2****,** The level of expression of ATP7B Variant 2 (SEQ ID NO: 4) in mouse liver was about 5 fold higher than the level of expression of wild-type ATP7B (SEQ ID NO: 1), and the level of expression of ATP7B Variant 3 (SEQ ID NO: 5) in mouse liver was about 7.8 fold higher than the level of expression of wild-type ATP7B (SEQ ID NO: 1).

### EXAMPLE 3

### High Expression From Short Promoters in HEK293T Cells

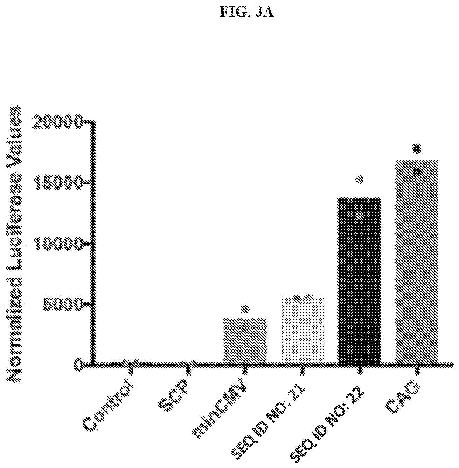

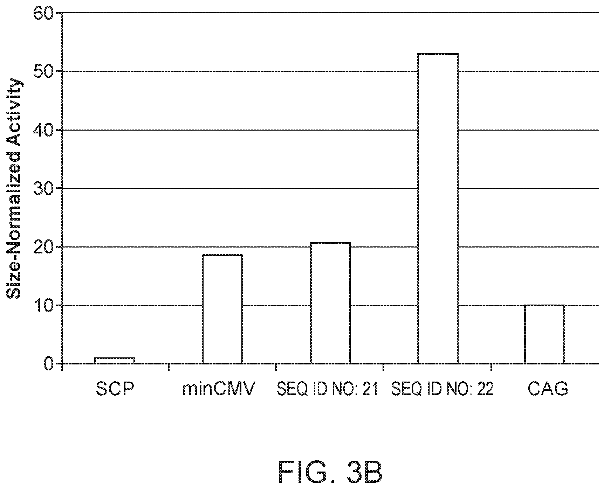

HEK293T cells were transfected with plasmid DNA containing a luciferase gene under the control of one of several different regulatory elements, i.e., no promoter control, SCP, CMV, SEQ ID NO: 21 (SEQ ID NO: 19 operably linked to minCMV), SEQ ID NO: 22 (SEQ ID NO: 20 operably linked to minCMV), and CAG. The normalized luciferase values from each construct are illustrated in **FIG. 3A****.** The size-normalized activity values from each construct are illustrated in **FIG. 3B****.** The sequences of the regulatory elements and promoters used herein are provided in **TABLE** 2 and **TABLE** 3 above. Regulatory element SEQ ID NO: 19 linked to a minCMV promoter and regulatory element SEQ ID NO: 20 linked to a minCMV promoter drove higher levels of luciferase expression than minCMV alone and SCP alone. Both SEQ ID NO: 19 and SEQ ID NO: 20, when linked to a minCMV promoter, drove high expression of luciferase in HEK293T kidney cells as compared to the control, SCP, or minCMV promoter without the regulatory element (i.e., SEQ ID NO: 19 or 20).

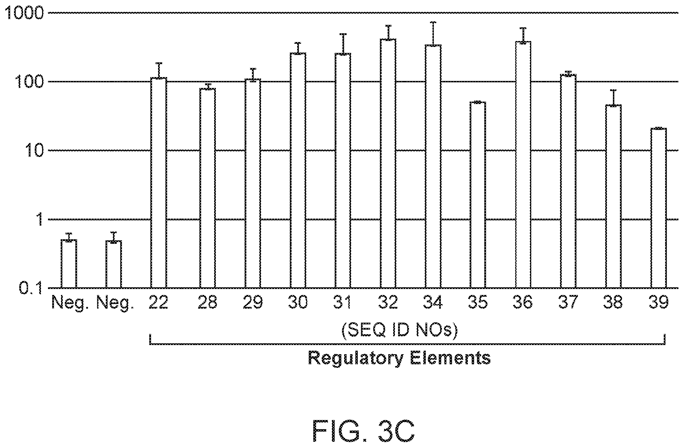

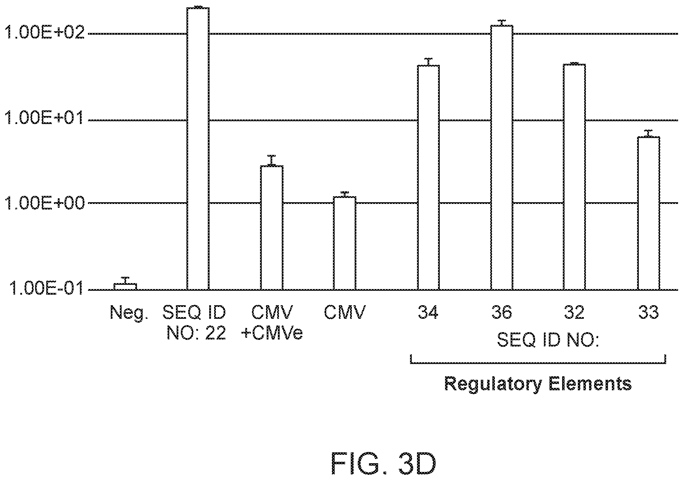

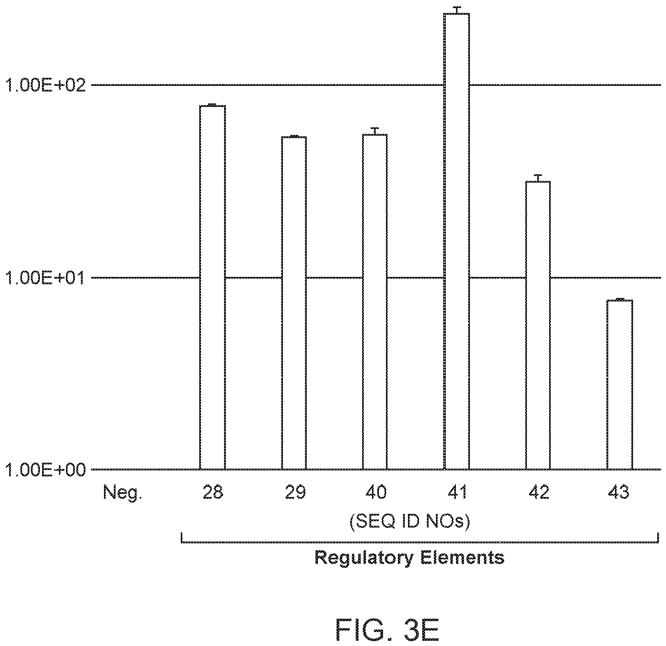

Similar normalized luciferase expression experiments were done with additional controls and regulatory sequences, as illustrated in **FIG. 3C****,** **FIG. 3D****,** and **FIG. 3E****.** Firefly expression was also assayed to ensure similar transfection efficiency in all the samples tested. In **FIG. 3C****,** normalized luciferase expression of regulatory elements SEQ ID NO: 22, SEQ ID NO: 28, SEQ ID NO: 29, SEQ ID NO: 30, SEQ ID NO: 31, SEQ ID NO: 32, SEQ ID NO: 34, SEQ ID NO: 35, SEQ ID NO: 36, SEQ ID NO: 37, SEQ ID NO: 38, and SEQ ID NO: 39 were compared with two negative controls (i.e., sequences known to not drive any expression). Each of the regulatory elements tested resulted in high levels of luciferase expression, e.g., by at least 10, 50, or 100 or more fold as compared to the negative controls.

In **FIG. 3D****,** normalized luciferase expression from plasmids comprising regulatory elements SEQ ID NO: 22, SEQ ID NO: 34, SEQ ID NO: 36, SEQ ID NO: 32, or SEQ ID NO: 33 were compared to a negative control, and a similar plasmid comprising either CMV linked to CMVe or CMV operably linked to luciferase. Each regulatory element tested (i.e., SEQ ID NO: 22, SEQ ID NO: 34, SEQ ID NO: 36, SEQ ID NO: 32, and SEQ ID NO: 33) resulted in higher luciferase expression than the negative control, CMV alone, and CMV+CMVe. The relatively short regulatory elements tested showed at least 10 fold, at least 15 fold, at least 20 fold, at least 30 fold, at least 40 fold, or more than 50 fold normalized luciferase expression as compared to a plasmid comprising a CMV promoter or CMV+CMVe linked to luciferase.

In **FIG. 3E****,** normalized luciferase expression from plasmids comprising regulatory elements SEQ ID NO: 28, SEQ ID NO: 29, SEQ ID NO: 40, SEQ ID NO: 41, SEQ ID NO: 42, or SEQ ID NO: 43, operably linked to luciferase were compared to a negative control (i.e., sequence known to have no expression activity). Each regulatory element (i.e., SEQ ID NOs: 28, 29, and 40-43) drove higher luciferase expression than the negative control, while SEQ ID NO: 41 drove a normalized luciferase expression higher than 10², or at least 100 fold higher luciferase expression than the negative control.

### EXAMPLE 4

### Wild-Type ATP7B Expression in Liver from Short Promoter

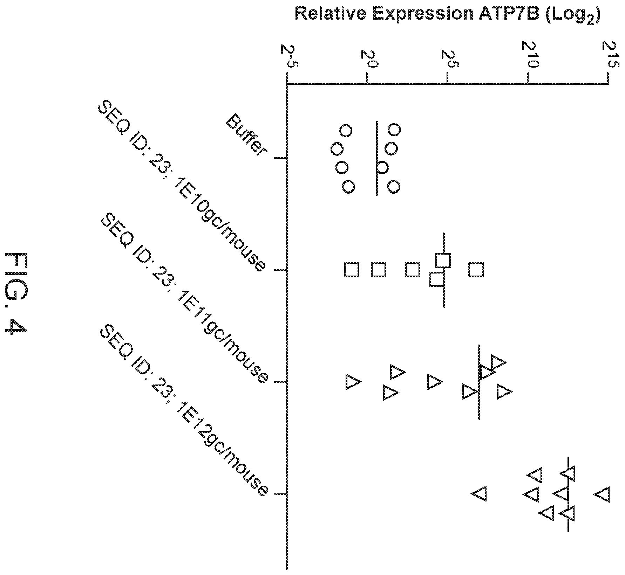

C57BL6/J mice were intravenously injected with AAV8 vectors containing wild-type ATP7B under the control of SEQ ID NO: 23. Two weeks after viral delivery, livers were harvested. Liver punches were disrupted and homogenized in 600ul of RLT supplemented with 10ul/mL beta-mercaptoethanol, and RNA was extracted using an RNeasy Mini kit (Qiagen) in accordance with manufacturer's protocols, including on-column DNase treatment. For each sample, RNA (3ug) was reverse transcribed with Superscript IV (Invitrogen), using OligoDT primers (65°C 5m, 55°C 10m, 85°C 10m). Primers against human ATP7B (5'-CATTCCAGGACTGTCCATTCT-3'(SEQ ID NO: 64); 5'-GGCCTGAACGTAGAAGTACCA-3' (SEQ ID NO: 65)), and GAPDH (5'ACCACAGTCCATGCCATCAC-3' (SEQ ID NO: 44); 5'-TCCACCACCCTGTTGCTGTA-3' (SEQ ID NO: 45)) were verified with standard curves to ensure reliability, and used for qPCR (Phusion, SYBR Green) under the following amplification conditions: (98°C 2m, 40X[98°C 10s, 67°C 30s, 92°C 15s]). ATP7B expression was normalized to GAPDH and presented as fold change over baseline using the delta-delta Ct method. As illustrated in **FIG. 4****,** and **TABLE 6,** SEQ ID NO: 23 drove expression of ATP7B in the liver at three different doses, and showed a dose response with the highest viral dose resulting in the highest ATP7B expression.

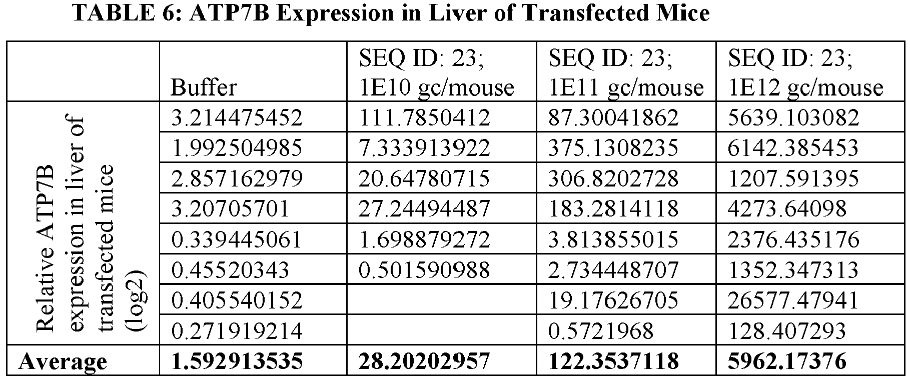

Example 4 illustrates expression cassettes comprising one or more regulatory elements disclosed herein operably linked to a ATP7B transgene can be used to treat a disorder or condition associated with ATP7B deficiency, e.g., Wilson's disease, in an animal, a mammal, or a human subject in need thereof. Further, such expression cassettes can be delivered systemically *in vivo* using rAAV8, e.g., via intravenous injection or infusion.

### EXAMPLE 5

### High EGFP Transcription in Liver

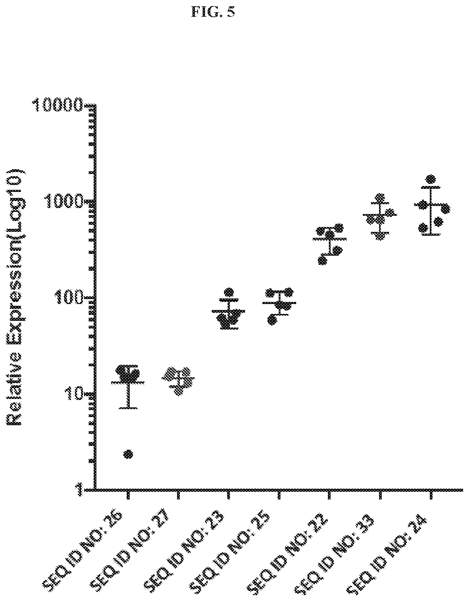

C57BL6/J mice were intravenously injected with AAV8 vectors containing EGFP under the control of various regulatory elements: SEQ ID NO: 22, 23, 24, 25, 26, 27, and 33. Each AAV8 vector was administered at a dose of 5x10¹¹ gc/mouse. Two weeks after viral delivery, livers were harvested. Liver punches were disrupted and homogenized in 600ul of RLT supplemented with 10ul/mL beta-mercaptoethanol, and RNA was extracted using an RNeasy Mini kit (Qiagen) in accordance with manufacturer's protocols, including on-column DNase treatment. For each sample, RNA (3ug) was reverse transcribed with Superscript IV (Invitrogen), using OligoDT primers (65 °C 5m, 55 °C 10m, 85 °C 10m). Primers against EGFP (5'- GCTACCCCGACCACATGAAG -3' (SEQ ID NO: 46); 5'-TCTTGTAGTTGCCGTCGTCC -3' (SEQ ID NO: 47), and GAPDH (SEQ ID NO: 44 and SEQ ID NO: 45) were used for qPCR (Phusion, SYBR Green) under the following amplification conditions: (98 °C 2m, 40X[98 °C 10s, 65 °C 30s, 92 °C 15s]). EGFP expression was normalized to GAPDH and presented as fold change over baseline using the delta-delta Ct method (**FIG. 5**). As illustrated in **FIG. 5****,** each of the regulatory elements tested increased EGFP expression, as measured by RNA transcripts, in the liver. Further, SEQ ID NO: 22, SEQ ID NO: 33, and SEQ ID NO: 24 resulted in comparable levels of EGFP.

Example 5 illustrates that the relatively short regulatory elements of this disclosure can result in high levels of transgene expression in the liver, as measured by RNA transcripts.

### EXAMPLE 6

### High Percentage of Hepatocytes Express EGFP

Wildtype (C57B16/J) mice were injected intravenously (tail vein) with AAV 8 (5x10¹¹ gc/mouse) containing EGFP fused to a KASH nuclear tethering domain (EGFP-KASH) under the control of candidate regulatory elements, or PBS control. Four weeks post injection, animals were sacrificed and 4mm liver punches were collected from right liver lateral lobes and stored in RNAlater.

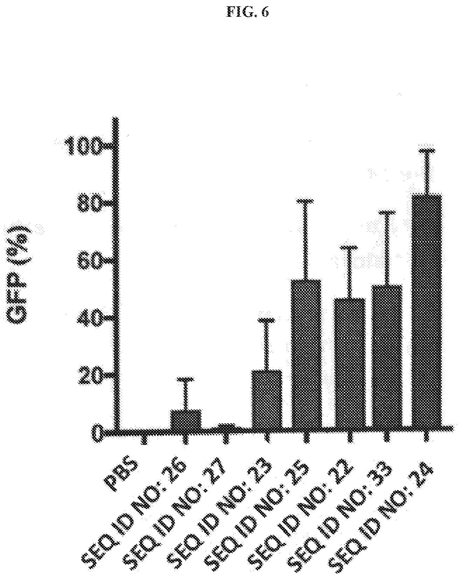

Liver punches (N=5, 1 per animal) were dounced in lysis buffer to release nuclei. Crude nuclei preparations were obtained by centrifugation and stained with DAPI to confirm nuclei integrity. Nuclei were analyzed for GFP expression by flow cytometry (LSRII flow cytometer), using the PBS-injected control samples to define the gating strategy. For every sample, 10,000 events were recorded and data were analyzed using FlowJo software. GFP+ and GFP- nuclei were determined such that <99.5% of control samples were considered GFP positive. Data are presented as the percentage of total DAPI+ events detected as GFP+ (FIG. **6**). As illustrated in **FIG. 6****,** many of the regulatory elements tested were expressed in a high percentage of hepatocytes in mice, with SEQ ID NO: 24 being expressed in greater than 80% of hepatocytes.

### EXAMPLE 7

### Treatment of Wilson's Disease in Mouse Models

Treatment of Wilson's Disease and/or symptoms thereof using the nucleic acid constructs described herein can be tested in various mouse lines, such as the Long Evans Cinnamon rat, which has a 300 bp deletion in the ATP7B gene resulting in loss of the protein (K. Terada et al. Pediatr Int. 41(4):414-8(1999)); the Jackson's toxic milk mouse (*tx^{j}*), which has a Gly712Asp missense mutation in the ATP7B coding sequence that corresponds to the second putative membrane-spanning domain of the encoded protein and results in a dysfunctional ATP7B protein (E.A. Roberts et al. Mol Genet Metab. 93(1):54-65 (2008)); ATP7B knockout mice (ATP7B KO, ATP7B^{-/-} or WD mice), created by introducing an early termination codon in the mouse ATP7B mRNA by engineering the substitution of a portion of ATP7B exon 2 with a neomycin cassette oriented in the opposite transcriptional frame (Buikova O.I. et al. Human Molecular Genetics 8(9): 1665-1671 (1999)); and an ATP7B mouse (D. Huster D et al. Am J Pathol. 168(2):423-34 (2006)) lines. ATP7B knockout mice show no ATP7B expression in the liver and high Cu excretion in the urine, low holoceruloplasmin levels in serum, high transaminase levels, high Cu concentration in the liver and a pathologic liver histology. These mice exhibit the typical biochemical characteristics of human Wilson's disease except for the neurological affectation (Lutsenko S. Biochemical Society Transactions 36(Pt 6): 1233-1238 (2008)).

To test the ATP7B variant compositions described herein, including AAV gene therapy and treatment using such gene therapy, Wilson's Disease model mice of each of the mouse strains described above and control mice (e.g., mice with a wild-type ATP7B gene) are injected (e.g., administered by intravenous injection) with a control (e.g., either PBS, an empty AAV, or an AAV expressing either eGFP or another reporter gene) or an expression cassette comprising a variant ATP7B sequence (e.g., any one of the variant ATP7B sequences described in **TABLE 1** above). Some AAVs can further comprise one or more regulatory element sequences described in **TABLE 2** above.

Following AAV injections, one or more of the following measurements are regularly monitored over time: serum alanine aminotransferase (ALT), serum aspartate aminotransferase (AST), and total serum bilirubin levels, serum Cu levels, serum ceruloplasmin activity, and/or urine Cu levels.

Serum ALT, AST and/or bilirubin levels may be determined, e.g., by collecting blood samples and analyzing by Antech Diagnostics (Irvine, CA, USA) or by the DGKC method (Roche Diagnostics, Mannheim, Germany) using a Hitachi 747 Clinical Analyzer (Hitachi, Tokyo, Japan). Serum ceruloplasmin activity may be determined e.g., with o-dianisidine dihydro chloride (4, 4'-diamino-3,3'-dimethoxy-biphenyl) as substrate (Sigma-Aldrich, San Louis, MO, United States) and measuring absorbance at 540 nm in a spectrophotometer as described by Schosinsky (Clinical Chemistry 1974; 20(12): 1556-1563). Serum and urine copper content may be determined e.g., by atomic absorption spectroscopy (SIMAA 6000, from Perkin-Elmer GmbH, Bodenseewerk), by inductively coupled plasma-mass spectrometry, or by Exova (Edinburgh, UK). In addition, blood samples may be evaluated for the levels of both copper bound and non-copper-bound forms of ceruloplasmin via Western blot.

In addition, after treatment of the mice with AAV, the expression levels of ATP7B can be monitored over time using various PCR and/or sequencing methods to show treatment with a ATP7B AAV can result in an increase in ATP7B expression. Northern blot analysis and *in situ* hybridization can also be used to analyze ATP7B expression *in vivo.* The level of ATP7B protein can also be monitored after treatment to show an increase in ATP7B mRNA expression correlates with an increase in ATP7B protein. ATP7B protein can be assayed using various methods, including, but not limited to, Western blot analysis, immunohistochemistry, immunofluorescence histochemistry, and/or ELISA assays.

Animals are sacrificed at 6-7 months after vector administration and the livers may be excised for histological analyses. Hepatic copper content may be determined in dry liver tissue e.g., by atomic absorption spectroscopy (SIMAA 6000, from Perkin-Elmer GmbH, Bodenseewerk), and by Timm's sulfide silver staining (Danscher G. and Zimmer J. Histochemistry 1978; 55(1): 27-40). Liver structure may be assessed e.g., in sections stained with hematoxylin and eosin. Immunohistochemistry with anti-mouse CD45 antibody (BioLegend, San Diego, USA; Catalog Number 103102) may be performed to detect inflammatory infiltration in the liver. In addition, Immunohistochemistry with anti-mouse PanCk antibody (Invitrogen/Life Technologies, 18-0132, clon AE1/AE3) may also be performed to detect biliary cells. Liver fibrosis may be determined using conventional Sirius Red staining as a method for collagen determination.

Different doses of AAV comprising an expression cassette can also be administered to mice to determine the safety and efficacy profile of each gene therapy treatment. These preclinical studies can also inform the optimal dose(s) of the gene therapy to use for treating Wilson's Disease.

### EXAMPLE 8

### Liver Expression of AAV8 Delivered ATP7B

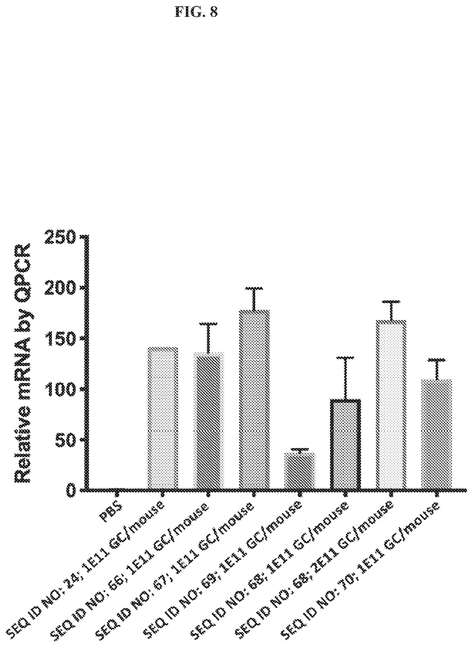

ATP7B null mice were generated by inserting a stop codon into exon 2 of the coding region of mouse ATP7B (ATP7B KO). AAV8 viruses were generated and pseudotyped with AAV5 capsid proteins by transient transfection of adherent HEK293T cells using triple transfection. Recombinant vectors were purified by ultracentrifugation and column chromatography, then sterile filtered prior to vialing. C57BL6 male mice (approx. 8 weeks old) were injected intravenously via the tail vein with AAV8 at a concentration of 1x10¹¹ gc/mouse or 2x10¹¹ gc/mouse, containing ATP7B variant 3 (SEQ ID NO: 5) under the control of various regulatory elements (SEQ ID NO: 24, 66, 67, 68, 69 and 70). Four weeks after viral delivery, livers were harvested. Four millimeter liver punches were collected from right liver lateral lobes and RNA samples were collected from these samples. QPCR analysis was performed using the RNA samples to establish the level of ATP7B transgene expression. Briefly, RNA was extracted from liver samples using RNeasy kit, and SuperScript IV first-strand synthesis system was used to generate cDNA from input RNA samples. QPCR reaction was set up using KAPA SYBR Fast qPCR master mix according to manufacturer's protocol, and was performed on the Roche LightCycler 96 system. Expression levels were normalized using GAPDH as an internal control. It was observed that promoter candidates having SEQ ID NO: 24 and SEQ ID NO: 67 candidates showed the highest levels of ATP7B expression among all tested candidates, when normalized for concentration of injected virus (**FIG. 8**). No detectable level of hATP7B RNA was observed using liver samples from PBS-treated animals.

**TABLE 7. Sequences of Forward and Reverse Primers for ATP7B Constructs and GAPDH.**

| Construct | Forward Primer | SEQ ID NO | Reverse Primer | SEQ ID NO |
|---|---|---|---|---|
| ATP7B Variant 3 | 5' -AACATCATCCCTGGCCTG-3' | 71 | 5'-CCAGGCTATACACGTAGGCG-3' | 72 |
| GAPDH | 5'-ACCACAGTCCATGCCATCAC-3' | 44 | 5'-TCCACCACCCTGTTGCTGTA-3' | 45 |

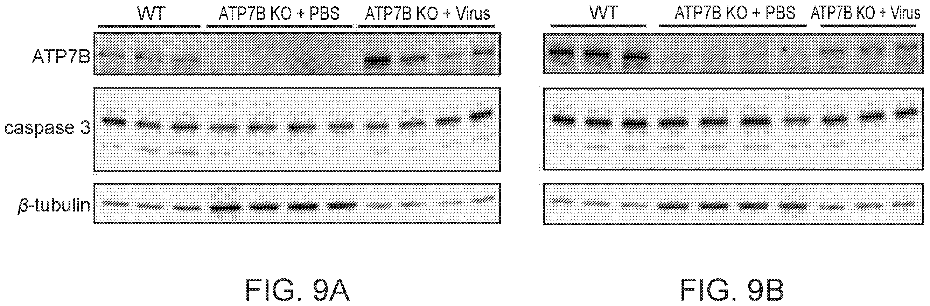

ATP7B protein expression was also measured by Western Blot analysis. ATP7B KO mice (approx. 4-6 weeks old) were injected intravenously via the tail vein with AAV8 at a concentration of 1×10¹¹ gc/mouse, containing ATP7B variant 3 (SEQ ID NO: 5) under the control of the promoter having SEQ ID NO: 24, or PBS control. Five months after viral delivery, livers were harvested. Four millimeter liver punches were collected from right liver lateral lobes and frozen in liquid nitrogen. Liver punches (1 per animal) were submerged in lysis buffer to release and solubilize the proteins. After centrifugation to clear the lysates, western blotting analysis was carried out to measure ATP7B expression. Approximately 15 micrograms of total protein was loaded onto the gel for western blotting, and Abcam recombinant anti-ATP7B antibody (EPR6793, catalog # ab 131208) was used for detection of ATP7B protein. Virus treatment led to robust and durable ATP7B expression in both ATP7B KO males and females (**FIG. 9**). No clear difference in caspase 3 protein abundance or cleavage was observed. Each lane represented a sample from an individual animal in the labeled group.

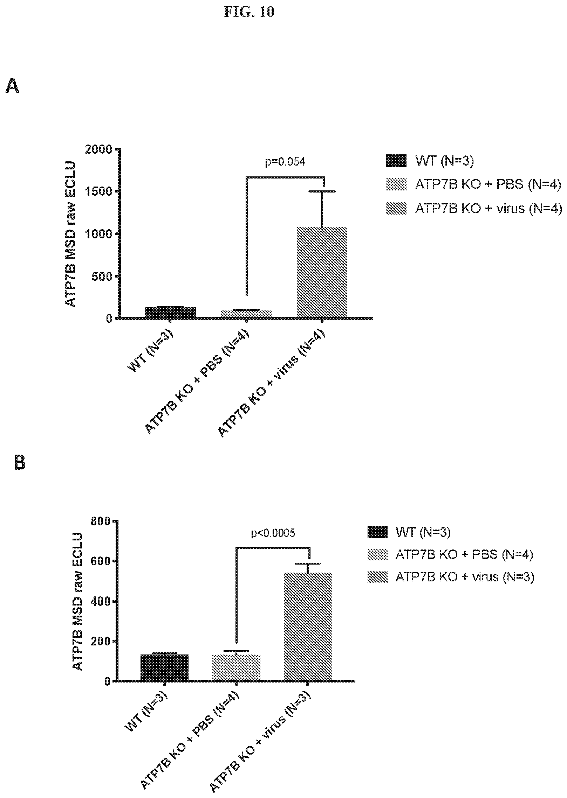

ATP7B protein expression was also measured by MSD-ELISA analysis. ATP7B KO mice (approx. 4-6 weeks old) were injected intravenously via the tail vein with AAV8 at a concentration of 1×10¹¹ gc/mouse, containing ATP7B variant 3 (SEQ ID NO: 5) under the control of the promoter having SEQ ID NO: 24, or PBS control. Five months after viral delivery, livers were harvested. Four millimeter liver punches were collected from right liver lateral lobes and frozen in liquid nitrogen. Liver punches (1 per animal) were submerged in lysis buffer to release and solubilize the proteins. After centrifugation to clear the lysates, MSD-ELISA analysis was carried out to measure ATP7B expression. Study samples were plated alongside a standard curve on an anti-ATP7B mAb (Sigma-Aldrich, St. Louis, MO) coated MSD plate and detected using a secondary anti-ATP7B pAb (Sigma-Aldrich, St. Louis, MO) in a sandwich ELISA format. Afterwards, a SULFOTAG reporter antibody (Meso Scale Diagnostics, Rockville, MD) was added to the plate. Readout occured when 1X Read Buffer was added, and the plate was read on the MSD machine. Sample concentrations of ATP7B were obtained by back-calculating the electrochemiliuminescence signals against the standard curve. Virus treatment led to robust and durable ATP7B expression in both male and female ATP7B KO mice (**FIG. 10A and 10B**).

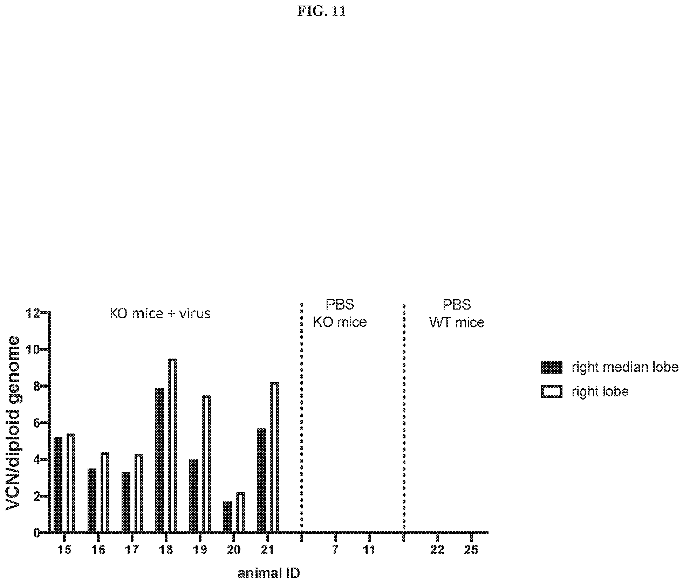

Finally, *in vivo* liver vector copy number was measured. ATP7B KO mice (approx. 4-6 weeks old) were injected intravenously via the tail vein with AAV8 at a concentration of 1×10¹¹ gc/mouse, containing ATP7B variant 3 (SEQ ID NO: 5) under the control of the promoter having SEQ ID NO: 24, or PBS control. Five months after viral delivery, livers were harvested and liver punches collected and processed as described above to create genomic DNA preps. Viral DNA content was quantified in the resulting genomic DNA preps. Mouse liver DNA was isolated with DNeasy Blood & Tissues kits (Qiagen). DNA quantity was determined and normalized using UV spectrophotometer. 20 ng of liver DNA was added to a 20µl reaction along with ddPCR Super Mix for Probes (no dUTP) (Bio-Rad) and TaqMan primers and probes directed against regions of ATP7B codon-optimized sequence. Droplets were generated and templates were amplified using Automated Droplet Generator (Bio-Rad) and thermo cycler (Bio-Rad). After PCR, the samples were loaded and read by QX2000 Droplet Reader to determine vector copy number in livers. Mouse genomic Tfrc (*Tfrc*) sequence served as an internal control (Thermo Fisher Scientific) for normalizing genomic DNA content and was amplified in the same reaction. Virus treatment led to robust and durable viral content in the liver of both ATP7B KO male and female animals (**FIG. 11**). The TaqMan^{™} Copy Number Reference Assay, mouse, Tfrc; Cat#: 4458367 (Thermo Fisher Scientific) was used for copy number analysis.

**TABLE 8. Sequences of Forward and Reverse Primers and Probes for ATP7B Constructs.**

| Construct | Primer/Probe Sequence | SEQ ID NO |
|---|---|---|
| ATP7B Variant 3 Forward Primer | 5'-GTGATGGAGGACTATGCCGG-3' | 73 |
| ATP7B Variant 3 Reverse Primer | 5' - TTCTGGTCAGCTTGCTCTCG-3' | 74 |
| Probe | 5'-TGGCAACATCGAGCTGACCATCACAGGCA-3' | 75 |

### EXAMPLE 9

### Viral Administration of ATP7B Attenuates Liver Toxicity in ATP7B KO Animals

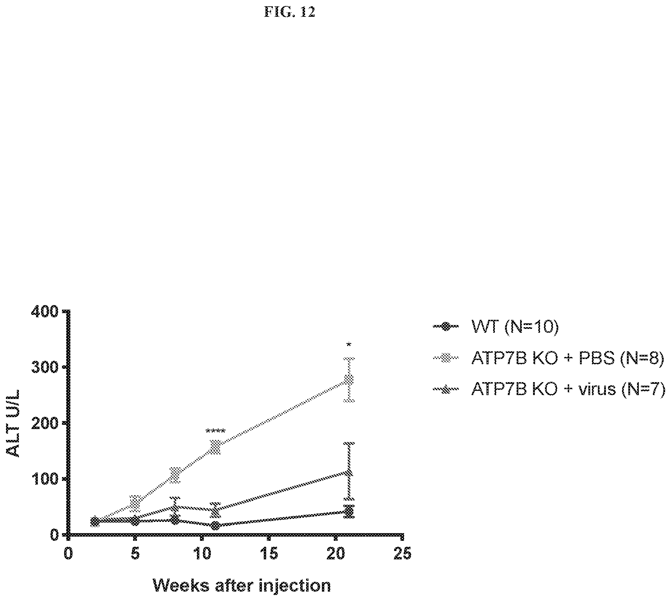

Alanine transaminase (ALT) activity was measured to assess liver damage in ATP7B animals. ALT activity is a commonly used serum biomarker for liver injury, where increased ALT activity suggests ongoing liver damage. ATP7B KO mice (approx. 4-6 weeks old) were injected intravenously via the tail vein with AAV8 at a concentration of 1×10¹¹ gc/mouse, containing ATP7B variant 3 (SEQ ID NO: 5) under the control of the promoter having SEQ ID NO: 24, or PBS control. Serum samples were collected every 3 weeks after virus injection for the first 3 months following injection. Terminal serum samples were also collected during necropsy procedures. ALT measurements were carried out with the serum samples. ALT activity was measured using a clinical analyzer according to manufacturer's manual. Virus treatment significantly attenuated the induction of ALT in ATP7B KO animals, suggesting a normalization of liver injury in the ATP7B KO model (**FIG. 12**).

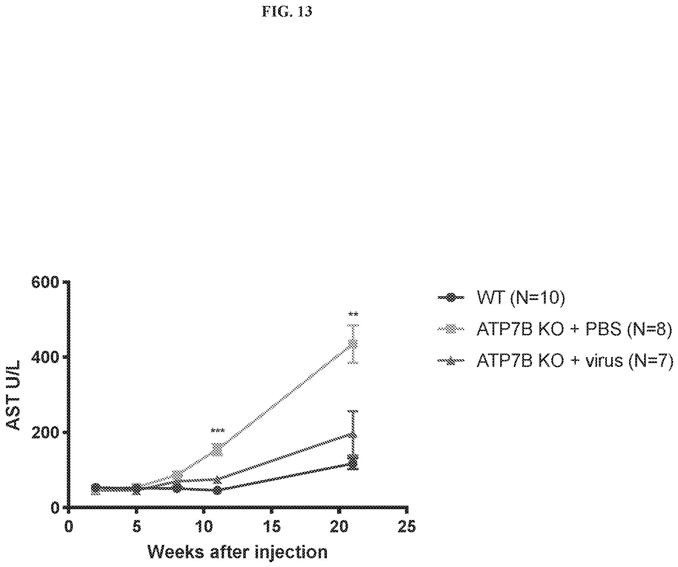

Aspartate aminotransferase (AST) is another commonly used serum biomarker for liver injury in animals, where increased AST activity suggests ongoing liver damage. AST activity was measured to assess liver damage in ATP7B animals. ATP7B KO mice (approx. 4-6 weeks old) were injected intravenously via the tail vein with AAV8 at a concentration of 1×10¹¹ gc/mouse, containing ATP7B variant 3 (SEQ ID NO: 5) under the control of the promoter having SEQ ID NO: 24, or PBS control. Serum samples were collected every 3 weeks after virus injection for the first 3 months following injection. Terminal serum samples were also collected during necropsy procedures. AST measurements were carried out with the serum samples. ALT activity was measured using a clinical analyzer according to manufacturer's manual. Virus treatment significantly attenuated the induction of AST in ATP7B KO animals, suggesting a normalization of liver injury in the ATP7B KO model (**FIG. 13**).

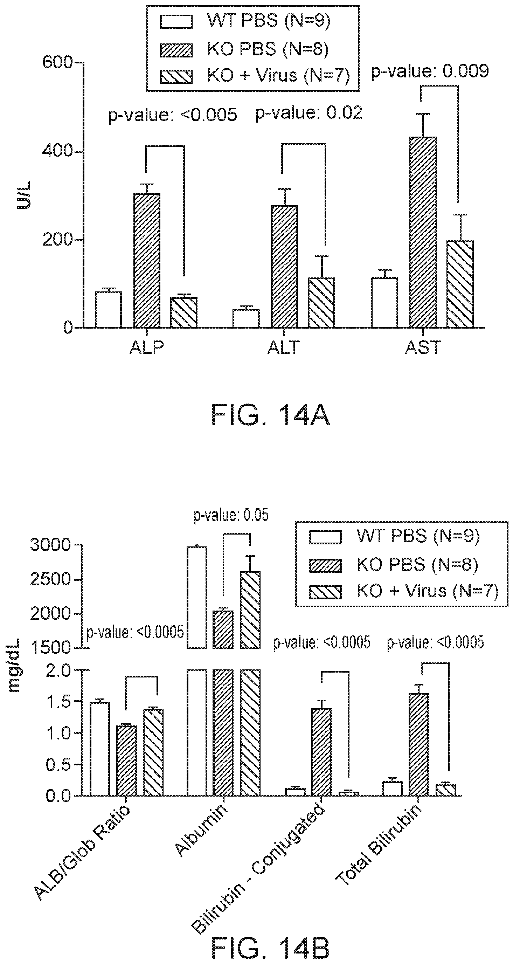

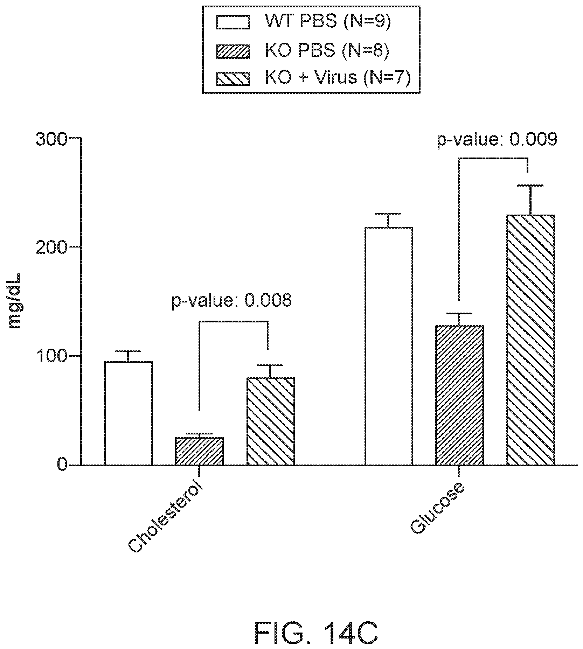

Wild-type and ATP7B KO mouse livers were also evaluated for inflammation, fibrosis, and large cell change. Mice were treated with AAV8 containing ATP7B variant 3 (SEQ ID NO: 5) under the control of the promoter having SEQ ID NO: 24, or PBS control as described above. Terminal serum samples were collected five months after virus injection. Terminal serum samples were subjected to a comprehensive serum chemistry panel analysis to measure liver injury and metabolic functions, including ALP, ALT, AST, albumin, bilirubin, cholesterol and glucose level measurement. Serum chemistry panel analyses were performed using a clinical analyzer following manufacturer's manual. A broad array of biochemical changes were observed in ATP7B KO animals. Treatment with AAV8 containing ATP7B variant 3 completely normalized these changes back to WT levels (**FIG. 14**).

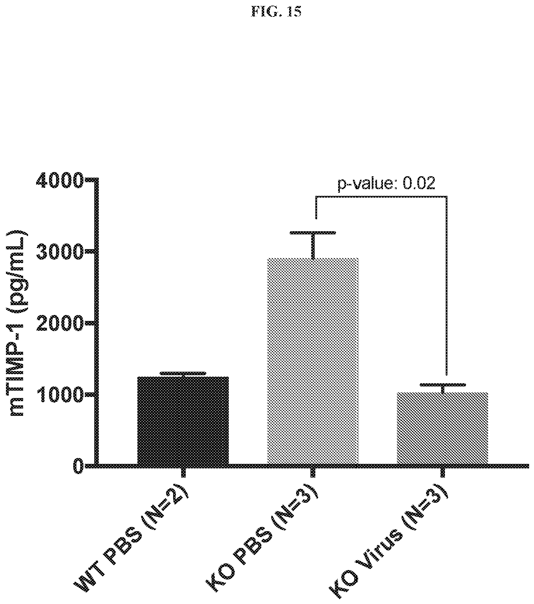

ELISA analysis for TIMP1 protein abundance was also performed on terminal serum sample using the mouse TIMP1 ELISA kit (Sigma RAB0468-1KT) based on manufacturer's instructions. TIMP1 is a serum-based biomarker for liver fibrosis. Significantly increased serum TIMP1 was observed in PBS-treated ATP7B KO animals, consistent with ongoing liver fibrosis. Virus treatment completely normalized serum TEMPI levels in ATP7B KO animals back to WT levels (**FIG. 15**).

### EXAMPLE 10

### Viral Administration of ATP7B Attenuates Increased Copper Concentrations in ATP7B KO Animals

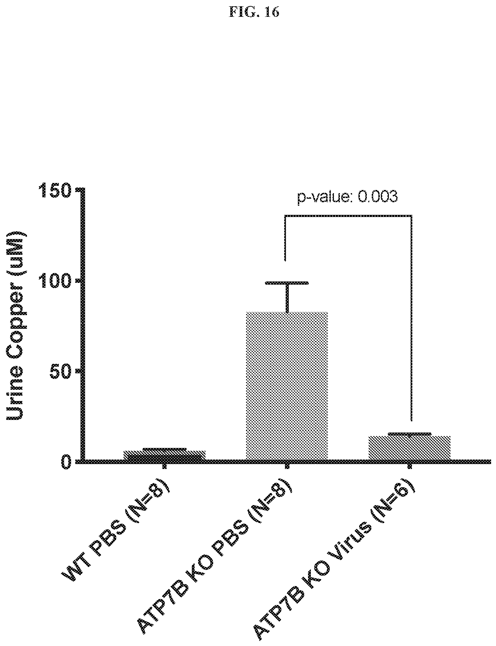

ATP7B KO mice (approx. 4-6 weeks old) were injected intravenously via the tail vein with AAV8 at a concentration of 1×10¹¹ gc/mouse, containing ATP7B variant 3 (SEQ ID NO: 5) under the control of the promoter having SEQ ID NO: 24, or PBS control. Urine samples were collected 21 weeks following virus injection. Copper concentrations were measured by inductively coupled plasma mass spectrometry, using PerkinElmer Sciex Elan 6000 ICP mass spectrometer according to manufacturer's manual. Urine copper concentrations from ATP7B KO animals were significantly increased compared to WT animals. However, virus treatment substantially attenuated the increased urinary copper concentrations observed in ATP7B KO mice (**FIG. 16**).

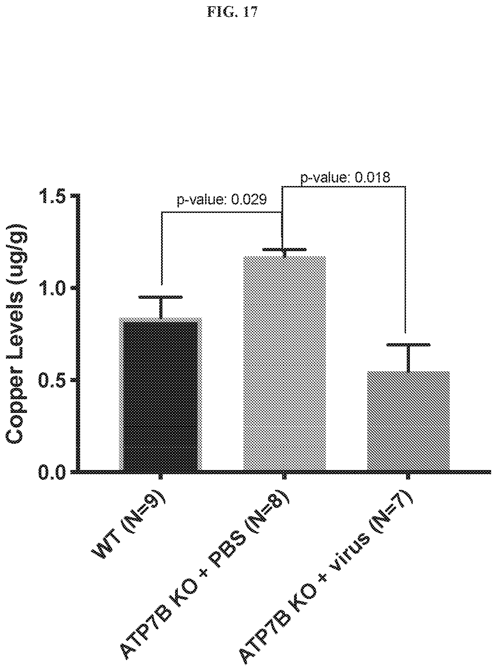

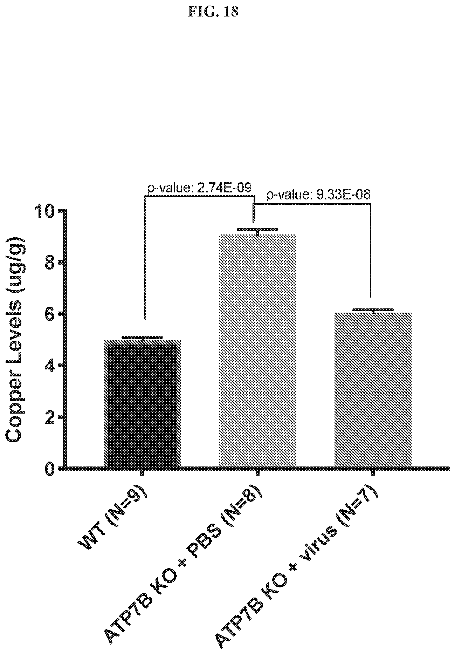

Copper concentration was also measured in terminal serum and brain samples of ATP7B KO mice injected with AAV8 containing ATP7B variant 3 (SEQ ID NO: 5) under the control of the promoter having SEQ ID NO: 24, or PBS control. Terminal serum and brain samples were collected five months after virus injection, and copper measurement performed. ATP7B KO animals showed increased copper levels in both serum and the brain. However, virus treatment normalized the increased copper content observed in ATP7B KO animals (**FIGs. 17** **and** **18**).

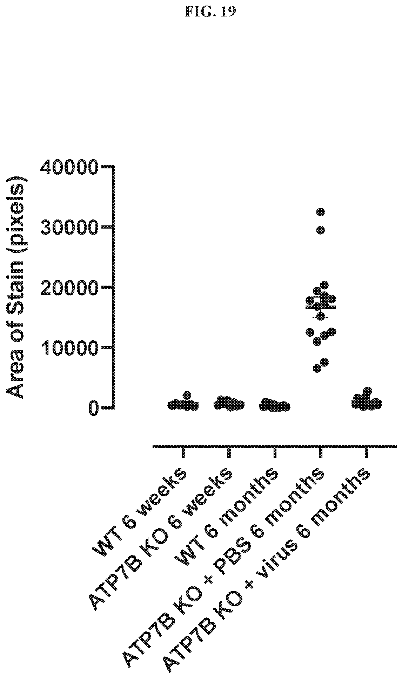

Liver tissues were also stained with rhodanine stain to assess copper deposition levels in WT and ATB7B KO mice. Rhodanine is a chelating reagent with a strong affinity for proteinaceous copper deposits in tissue sections, and is commonly used to detect copper deposits in Wilson disease patient samples. Liver tissues in WT and ATP7B KO mice were processed for histology analysis five months after virus injection. Tissue was harvested, formalin-fixed for 24 hrs then paraffin-embedded and cut in 5 um thick sections. Paraffin sections were deparaffinized and stained with hematoxylin and eosin (H&E), picro sirius red (PSR), rhodanine and anti-alphaSMA. Routine histology was performed following procedures in J.A. Keirnan, Histological and Histochemical Methods 5th Edition. H&E page 149-50. PSR page 190-1 and rhodanine page 331. For immunohistochemistry, tissue was heat retrieved at 95C in pH 6 Citrate buffer after deparaffin and then stained with anti-alpha SMA (abcam ab5694) which was detected using anti-rabbit-HRP (Thermo A16110) followed by Akoya TSA-Fitc. Tissue was imaged on an Akoya Polaris at 20x magnification. DAPI staining allowed for nuclei segmentation and counting in CellProfiler and the area of alpha SMA staining was quantified and normalized to nuclei count. Rhodanine staining showed that, compared to liver tissues from WT animals, liver tissue from PBS-treated ATP7B KO animals showed significantly increased copper deposition, consistent with the fact that copper accumulation is a main feature of Wilson disease liver. However, treatment with AAV8 virus containing ATP7B variant 3 (SEQ ID NO: 5) under the control of the promoter having SEQ ID NO: 24 attenuated the copper deposition in the livers of ATP7b KO mice (**FIG. 19**).

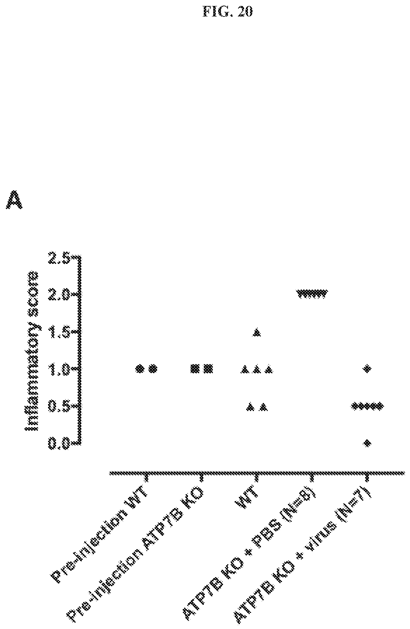

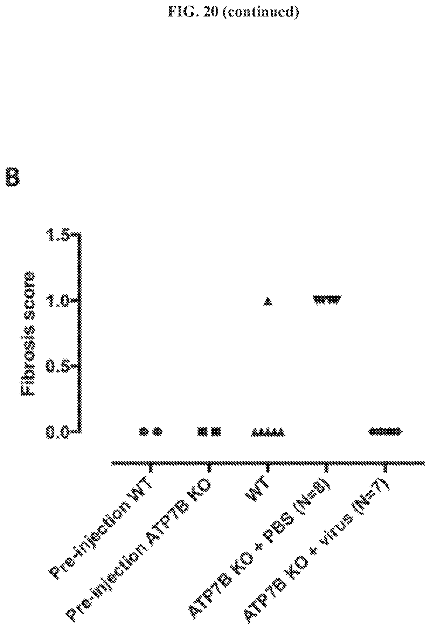

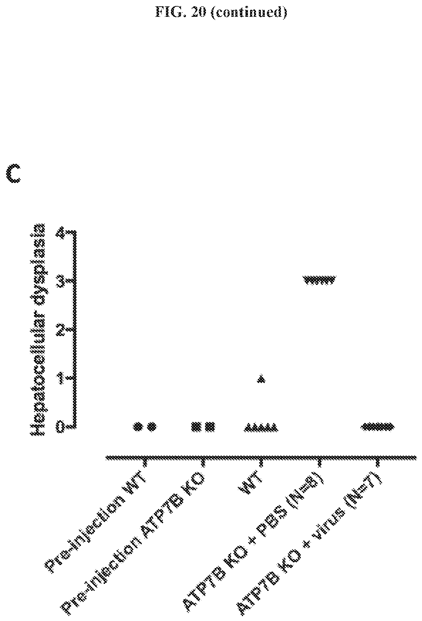

Wild-type and ATP7B KO mouse livers were also evaluated for inflammation, fibrosis, and large cell change. Mice were treated with AAV8 containing ATP7B variant 3 (SEQ ID NO: 5) under the control of the promoter having SEQ ID NO: 24, or PBS control as described above. Liver tissues in WT and ATP7B KO mice were processed for histology analysis five months after virus injection. Blinded pathology evaluation by an independent pathologist highlighted increased liver inflammation, increased fibrosis and large cell change in the PBS-treated ATP7B KO animals. Virus treatment completely normalized these changes back to WT levels. The scoring of liver inflammation follows the scoring system based on this study (Brunt, Hepatology 31(1): 241-246 (2000)), using an ascending scale from 0 to 4,: 0 (absent), 1 (mild), 2 (moderate), 3 (marked), and 4 (severe). The scoring of liver fibrosis follows the scoring system based on this study (T. Gilat et al., Hepatology 38(2): 436-442 (2003)), using an ascending scale from 0 to 4. The scoring of large cell change follows the scoring system based on this study (B. Thoolen et al., Toxicologic Pathology 38(7): 5S-81S (2010)), using an ascending scale from 0 to 4. Each dot in the figures represents scoring for an individual animal (**FIG. 20**).

### EXAMPLE 11

### Viral Administration of ATP7B Attenuates Liver Injury in ATP7B KO Animals

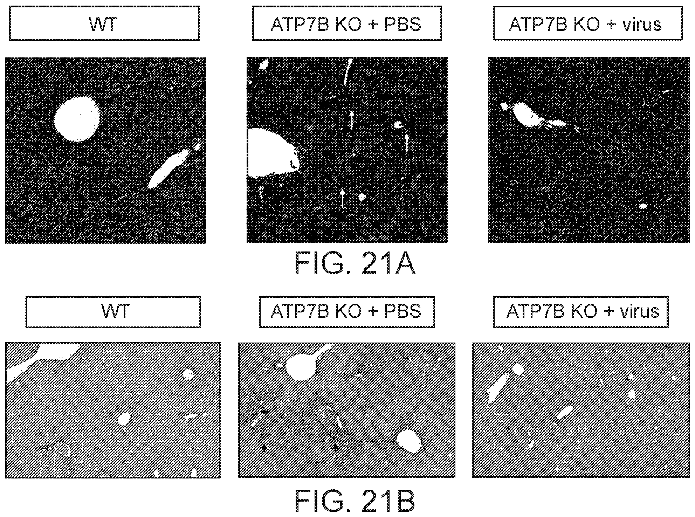

ATP7B KO mice (approx. 4-6 weeks old) were injected intravenously via the tail vein with AAV8 at a concentration of 1×10¹¹ gc/mouse, containing ATP7B variant 3 (SEQ ID NO: 5) under the control of the promoter having SEQ ID NO: 24, or PBS control. Five months following injection, liver tissues were processed for histology analysis as described above. Virus treatment significantly attenuated the induction of immune cell infiltration in ATP7B KO animals (**FIG. 21A**).

Liver tissues were also stained Picro Sirius Red (PSR) using standard staining procedures described in the PSR stain kit (Abcam ab150681). PSR staining showed that compared to liver tissues from WT animals, liver tissue from PBS-treated ATP7B KO animals showed significantly increased fibrosis (black arrows). However, virus treatment with AAV8 virus containing ATP7B variant 3 (SEQ ID NO: 5) under the control of the promoter having SEQ ID NO: 24 normalized the induction of immune cell infiltration in ATP7B KO animals (**FIG. 21B**).

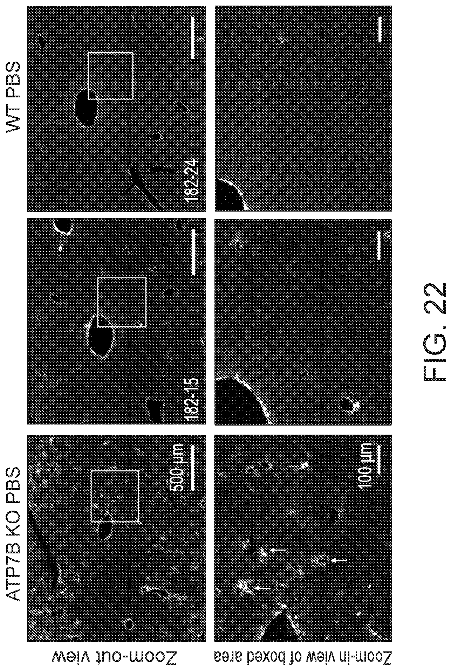

Liver tissues were further subjected to alpha-SMA staining analysis. Alpha-SMA is a marker for activated hepatic stellate cells, and positive signal suggests ongoing liver injury/fibrotic response. Significant activation of hepatic stellate cells was observed in PBS-treated ATP7B KO animals (shown by white arrows), consistent with ongoing liver fibrosis and immune infiltration. Virus treatment with AAV8 virus containing ATP7B variant 3 (SEQ ID NO: 5) under the control of the promoter having SEQ ID NO: 24 completely normalized these changes back to WT levels (**FIG. 22**).

### EXAMPLE 12

### Transcriptome Analysis in ATP7B KO Animals

ATP7B KO mice (approx. 4-6 weeks old) were injected intravenously via the tail vein with AAV8 at a concentration of 1×10¹¹ gc/mouse, containing ATP7B variant 3 (SEQ ID NO: 5) under the control of the promoter having SEQ ID NO: 24, or PBS control. RNA samples were collected from liver samples and whole transcriptome analysis was performed. Poly-adenylated RNA sequencing libraries were generated using the Illumina TruSeq stranded mRNA sample preparation kit according to the manufacturer's manual, and were sequenced on a NextSeq 500 system. ~20 million reads were generated for each sample. Read mapping, transcriptome assembly and gene expression analysis were performed with Hisat2, StringTie and DEseq2 according to their respective manuals. Significant changes were identified in multiple biological pathways comparing WT and PBS-treated ATP7B KO animals. Virus treatment significantly normalized the transcriptome-level changes of the innate immune and liver metabolism pathways in ATP7B KO animals back to WT levels.

Principle component analysis (PCA) was also performed with the transcriptome dataset. PCA analysis is used to describe similarities and variance of high-dimensional datasets, and is a commonly used method to define the similarity of RNA-sequencing samples, i.e. to identify subgroups or outliers. PCA analysis was performed using the DESeq2 package in R, according to the user manuals. Major changes were identified in the transcriptome signature for PBS-treated ATP7B KO animals compared to WT animals. Virus treatment normalized the transcriptome signature in ATP7B KO animals back to WT levels.

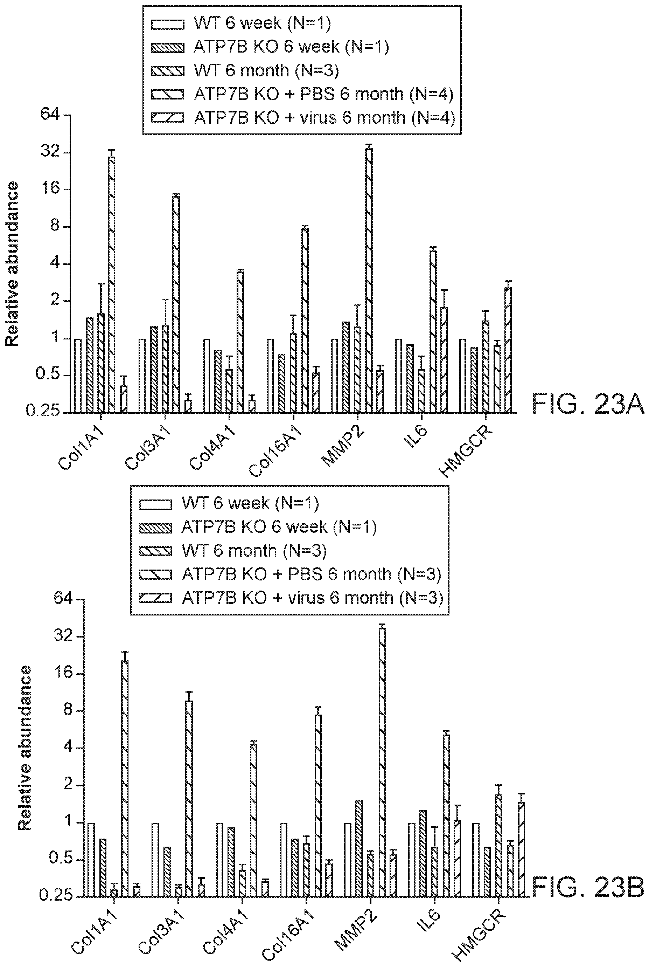

RNA samples were further subjected to QPCR analysis in order to validate key genes showing differential expression in the transcriptome analysis. Examples include Col1A1, Col3A1, Col4A1, Col16A1, all of which are genes encoding components important for collagen synthesis and liver fibrosis. MMP2 is a metalloproteinase important for extracellular matrix remodeling and has a profibrogenic function in liver fibrosis. IL6 is a cytokine that regulates inflammation, and HMGCR is a key enzyme involved in cholesterol biosynthesis in the liver. High consistency was observed between the QPCR validation and transcriptome profiling data, where ATP7B KO animals showed significant induction of collagen and pro-inflammatory genes involved in liver fibrosis, and reduction of metabolic genes such as HMGCR. Virus treatment normalized the gene expression profile in ATP7B KO animals back to WT levels (**FIG. 23**).

**TABLE 9. Sequences of Forward and Reverse Primers used for QPCR for Col1A1, Col3A1, Col4A1, Col16A1, MMP2, IL-6 and HMGCR.**

| Construct | Forward Primer | SEQ ID NO | Reverse Primer | SEQ ID NO |
|---|---|---|---|---|
| Col1A1 | 5'-TGGTGCAGCTGGTCTT-3' | 76 | 5'-ACCAGCTTCACCCTTGTC-3' | 77 |
| Col3A1 | 5'-CTACACCTGCTCCTGTG-3' | 78 | 5'-CCGGATAGCCACCCATT-3' | 79 |
| Col4A1 | 5'-CAGCTTCCAAGGACCAA-3' | 80 | 5'-CTTGCTTGCCAGGTTCA-3' | 81 |
| Col16A1 | 5'-TATTTCTGGGCTTGGACG-3' | 82 | 5'-CCCTCCCGGTAAGATCT-3' | 83 |
| MMP2 | 5'-CGAATCCATGATGGGGAG-3' | 84 | 5'-CAGTACTCGCCATCAGC-3' | 85 |
| IL-6 | 5'-CTCTGGGAAATCGTGGAA-3' | 86 | 5'-CCAGGTAGCTATGGTACTCC-3' | 87 |
| HMGCR | 5'-CATGTTCACCGGCAACAA-3' | 88 | 5'-GCCGTAGGTTCTGGAACT-3' | 89 |

### EXAMPLE 13

### Liver Expression of ATP7B in AAV5 and AAV8 Mice

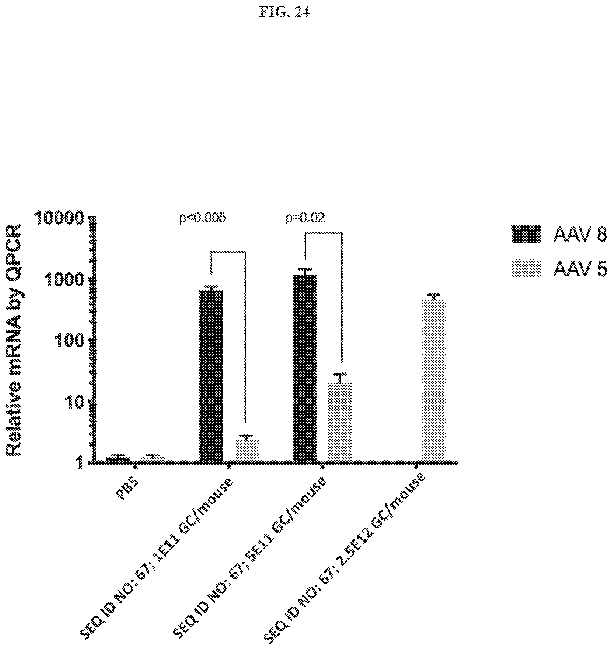

C57BL6 male mice (approx. 8 weeks old) were injected intravenously via the tail vein with AAV8 or AAV5 at a concentration of 1×10¹¹ gc/mouse, 5×10¹¹ gc/mouse, or 2.5×10¹² gc/mouse, containing ATP7B variant 3 (SEQ ID NO: 5) under the control of a promoter having SEQ ID NO: 67. Four weeks after viral delivery, livers were harvested. Four millimeter liver punches were collected from right liver lateral lobes and RNA samples were collected from these samples. QPCR analysis was performed using the RNA samples to establish the level of ATP7B transgene expression. Briefly, RNA was extracted from liver samples using RNeasy kit, and SuperScrit IV first-strand synthesis system was used to generate cDNA from input RNA samples. QPCR reaction was set up using KAPA SYBR Fast qPCR master mix according to manual, and was performed on Roche LightCycler 96 system. Expression levels were normalized using GAPDH as an internal control. We observed that AAV8 virus administration led to the highest levels of ATP7B expression, while AAV5 virus administration also showed increased ATP7B expression (**FIG. 24**). No detectable level of hATP7B RNA was observed using liver samples from PBS-treated animals. Forward and reverse primers used in this study for ATP7B Constructs and GAPDH are listed in Table 7 above.

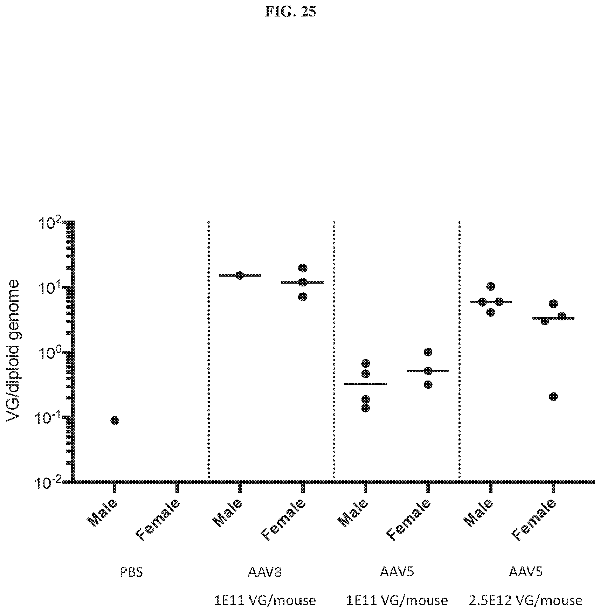

*In vivo* liver vector copy number was measured in mice injected with AAV8 or AAV5 viruses containing ATP7B variant #3 (SEQ ID NO: 5) under the control of a promoter having SEQ ID NO: 67 at a concentration of 1×10¹¹ gc/mouse, 5×10¹¹ gc/mouse, or 2.5×10¹² gc/mouse. Four weeks after viral delivery, livers were harvested and processed as described above to create genomic DNA preps. Viral DNA content was quantified in the resulting genomic DNA preps. Mouse liver DNA was isolated with DNeasy Blood & Tissues kits (Qiagen). DNA quantity was determined and normalized using UV spectrophotometer. 20 ng of liver DNA was added to a 20 µl reaction along with ddPCR Super Mix for Probes (no dUTP) (Bio-Rad) and TaqMan primers and probes directed against regions of ATP7B codon-optimized sequence. Droplets were generated and templates were amplified using Automated Droplet Generator (Bio-Rad) and thermo cycler (Bio-Rad). After PCR, the samples were loaded and read by QX2000 Droplet Reader to determine vector copy number in livers. Mouse genomic Tfrc (*Tfrc*) sequence served as an internal control (Thermo Fisher Scientific) for normalizing genomic DNA content and was amplified in the same reaction. Virus treatment led to robust and durable viral content in the liver of WT animals (**FIG. 25**). The TaqMan^{™} Copy Number Reference Assay, mouse, Tfrc; Cat#: 4458367 (Thermo Fisher Scientific) was used for copy number analysis.

**TABLE 10. Sequences of Forward and Reverse Primers and Probes for ATP7B Constructs.**

| Construct | Primer/Probe Sequence | SEQ ID NO |
|---|---|---|
| ATP7B Variant 3 Forward Primer | 5'-GTGATGGAGGACTATGCCGG-3' | 73 |
| ATP7B Variant 3 Reverse Primer | 5' - TTCTGGTCAGCTTGCTCTCG-3' | 74 |
| Probe | 5'-TGGCAACATCGAGCTGACCATCACAGGCA-3' | 75 |

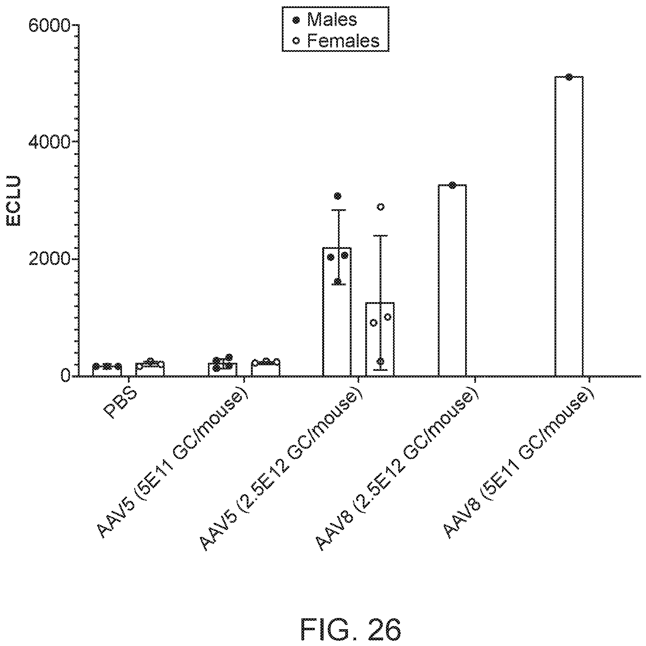

ATP7B protein expression was also measured by MSD-ELISA analysis as described above. C57BL6 male mice (approx. 8 weeks old) were injected intravenously at different concentrations via the tail vein with AAV8 or AAV5, containing ATP7B variant 3 (SEQ ID NO: 5) under the control of the promoter having SEQ ID NO: 67, or PBS control. Four weeks after viral delivery, livers were processed and MSD-ELISA analysis was carried out to measure ATP7B expression. Virus treatment led to dose-dependent ATP7B expression in WT animals (**FIG. 26**).

### EXAMPLE 14

### Ceruloplasmin Activity in ATP7 KO Animals

Ceruloplasmin activity is a biomarker for liver ATP7B activity, and is highly dependent on normal copper transport/metabolism in the liver. ATP7B KO mice (approx. 4-6 weeks old) were injected intravenously via the tail vein with AAV8 at a concentration of 5×10¹¹ gc/mouse, containing ATP7B variant 3 (SEQ ID NO: 5) under the control of the promoter having SEQ ID NO: 67, or PBS control. Serum samples were collected every 3 weeks after virus injection. Ceruloplasmin activity was measured using the ceruloplasmin colorimetric activity kit (Arbor assays K035-H1) according to manufacturer's manual. PBS-treated ATP7B KO animals showed significantly lower ceruloplasmin activity compared to WT animals. Virus treatment normalized the ceruloplasmin activity deficit in ATP7B KO animals, indicating a normalization of copper metabolism in the ATP7B KO model (**FIG**. **27**).

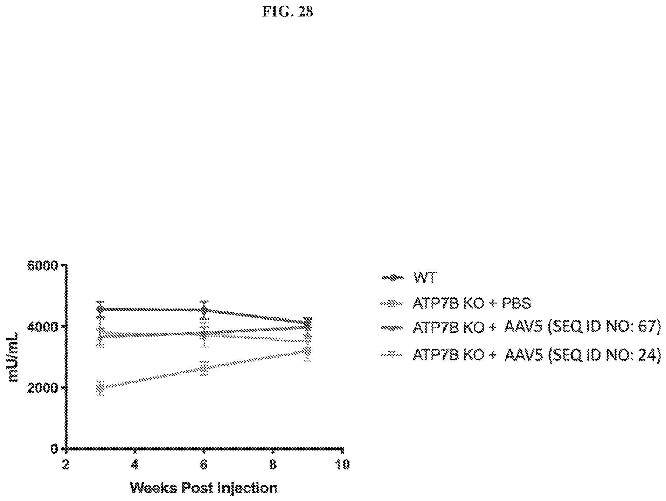

Ceruloplasmin activity was similarly measured in 4-6 week old ATP7B KO mice injected intravenously (tail vein) with AAV5 (5×10¹² gc/mouse) containing ATP7B variant 3 under the control of the promoter having SEQ ID NO: 24, SEQ ID NO: 67, or PBS control. Serum samples were collected every 3 weeks after virus injection and ceruloplasmin activity measured as described above. PBS-treated ATP7B KO animals showed significantly lower ceruloplasmin activity compared to WT animal, while virus treatment normalized the ceruloplasmin activity deficit in ATP7B KO animals. This confirmed the normalization of copper metabolism for ATP7B KO mice administered with AAV5 virus (**FIG. 28**).

### EXAMPLE 15

### Viral Administration of ATP7B Attenuates Liver Toxicity in ATP7B KO Animals

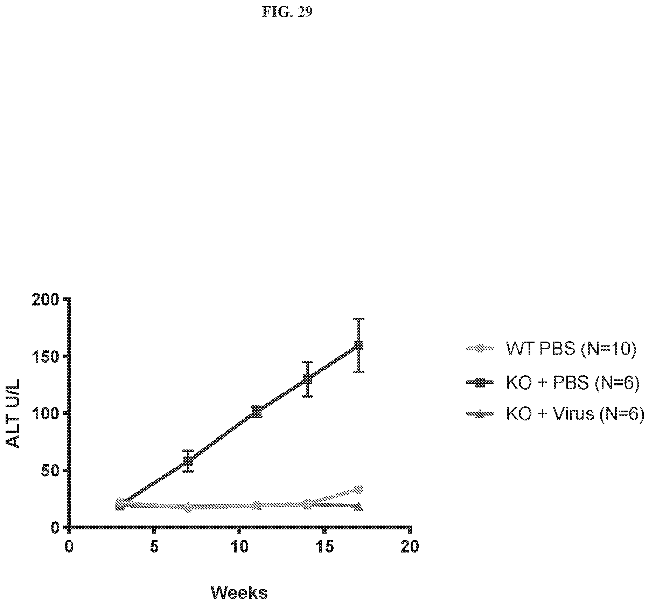

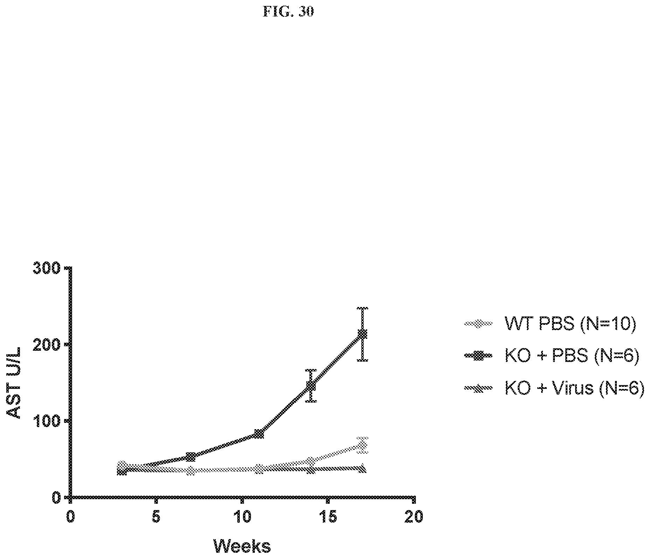

ALT and AST activity was measured in 4-6 week old ATP7B KO mice injected intravenously (tail vein) with AAV8 (5×10¹¹ gc/mouse) containing ATP7B variant 3 under the control of the promoter having SEQ ID NO: 67, or PBS control. Serum samples were collected every 3 weeks after virus injection for the first 3 months following injection. Terminal serum samples were also collected during necropsy procedures. ALT and AST measurements were carried out with the serum samples as discussed above. Virus treatment significantly attenuated the induction of ALT and AST in ATP7B KO animals, suggesting a normalization of liver injury in the ATP7B KO model (**FIG. 29** **and** **30**).

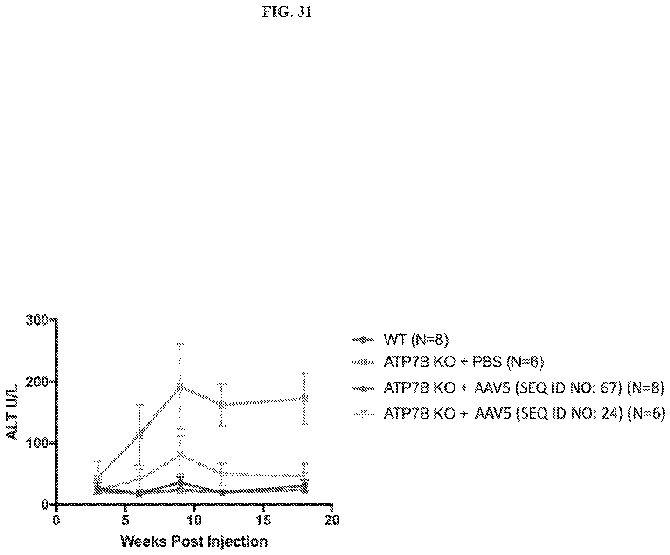

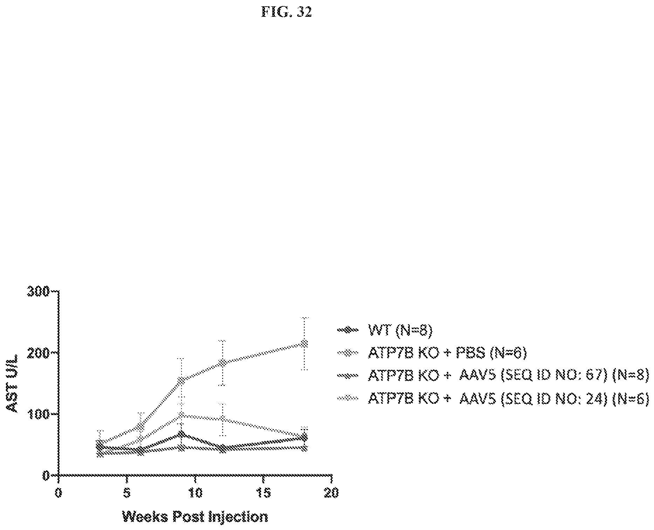

ALT and AST activity was similarly measured in 4-6 week old ATP7B KO mice injected intravenously (tail vein) with AAV5 (5×10¹² gc/mouse) containing ATP7B variant 3 under the control of the promoter having SEQ ID NO: 24, SEQ ID NO: 67, or PBS control. Serum samples were collected every 3 weeks after virus injection for the first 3 months following injection. Terminal serum samples were also collected during necropsy procedures. ALT and AST measurements were carried out with the serum samples as discussed above. Virus treatment significantly attenuated the induction of ALT and AST in ATP7B KO animals, suggesting a normalization of liver injury in the ATP7B KO model (**FIG. 31** **and** **32**).

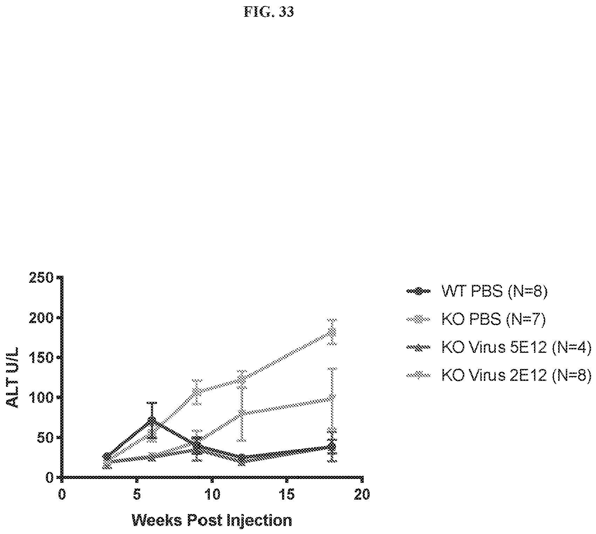

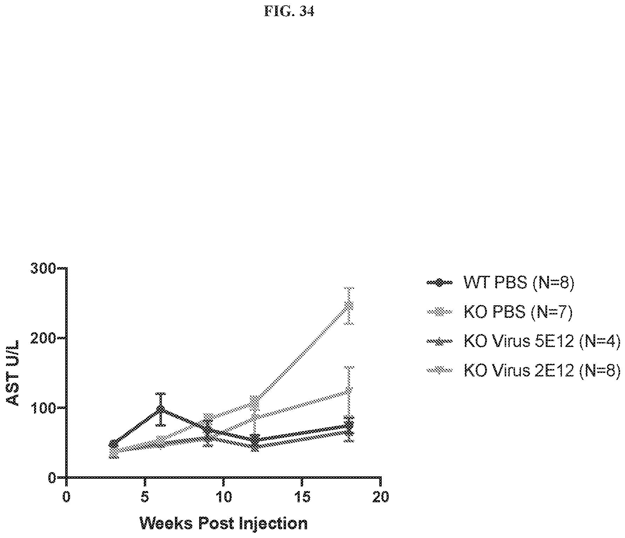

ALT and AST activity was next measured in 4-6 week old ATP7B KO mice injected intravenously (tail vein) with AAV5 (2×10¹² gc/mouse or 5×10¹² gc/mouse) containing ATP7B variant 3 under the control of the promoter having SEQ ID NO: 67, or PBS control. Serum samples were collected every 3-4 weeks after virus injection. ALT and AST measurements were carried out with the serum samples as discussed above. Virus treatment significantly attenuated the induction of ALT and AST in ATP7B KO animals, further confirming the normalization of liver injury in the ATP7B KO model (**FIG. 33** **and** **34**).

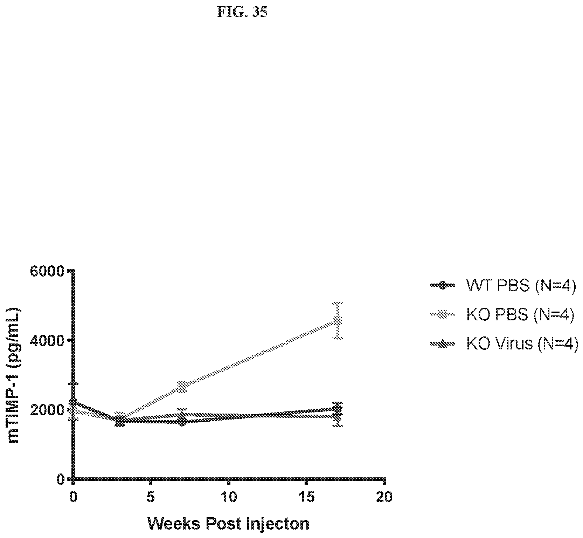

ELISA analysis for TIMP1 protein abundance was also performed on serum samples from ATP7B KO mice injected intravenously (tail vein) with AAV8 (5×10¹¹ gc/mouse) containing ATP7B variant 3 under the control of the promoter having SEQ ID NO: 67, or PBS control. The mouse TIMP1 ELISA kit (Sigma RAB0468-1KT) was used according to the manufacturer's manual. TIMP1 is a serum-based biomarker for liver fibrosis. Significantly increased serum TIMP1 was observed in PBS-treated ATP7B KO animals, consistent with ongoing liver fibrosis. Virus treatment completely normalized serum TIMP1 levels in ATP7B KO animals back to WT levels (**FIG. 35**).

### EXAMPLE 16

### Viral Administration of ATP7B Attenuates Increased Copper Concentrations in ATP7B KO Animals

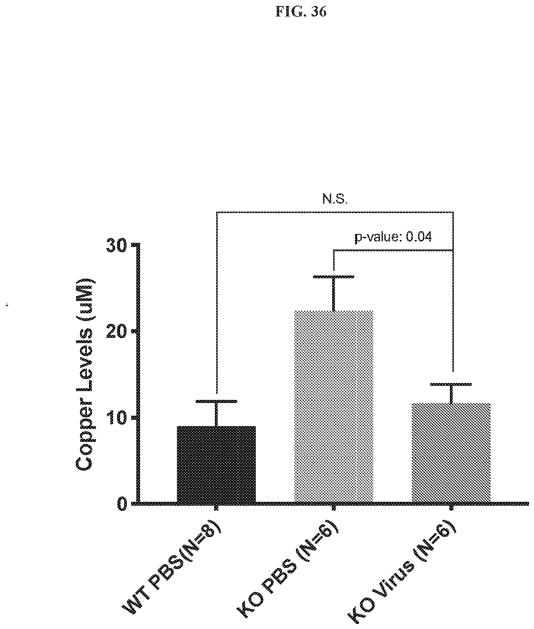

ATP7B KO mice (approx. 4-6 weeks old) were injected intravenously via the tail vein with AAV8 at a concentration of 5×10¹¹ gc/mouse, containing ATP7B variant 3 (SEQ ID NO: 5) under the control of the promoter having SEQ ID NO: 67, or PBS control. Urine samples were collected 3 weeks following virus injection. Copper concentrations were measured by inductively coupled plasma mass spectrometry, using PerkinElmer Sciex Elan 6000 ICP mass spectrometer according to manufacturer's manual. Urine copper concentrations from ATP7B KO animals were significantly increased compared to WT animals. However, virus treatment substantially attenuated the increased urinary copper concentrations observed in ATP7B KO mice (**FIG. 36**).

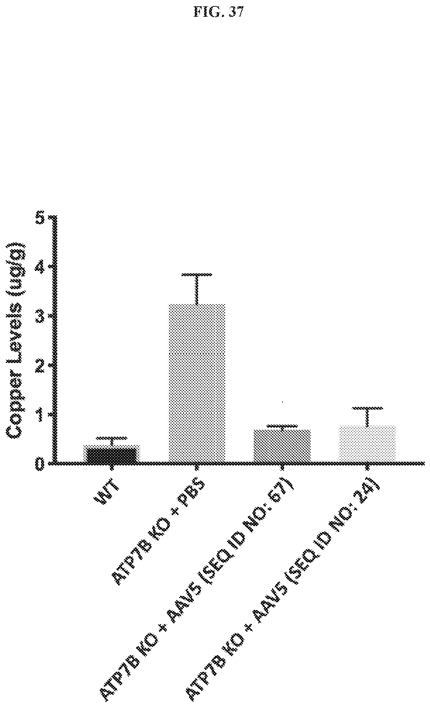

Urine copper concentration was also measured in mice injected with AAV5 virus. ATP7B KO mice (approx. 4-6 weeks old) were injected intravenously via the tail vein with AAV5 at a concentration of 5×10¹² gc/mouse, containing ATP7B variant 3 (SEQ ID NO: 5) under the control of the promoter having SEQ ID NO: 24, SEQ ID NO: 67, or PBS control. Urine samples were collected 16 weeks following injection. Copper concentrations were measured as described above. Urine copper concentrations from ATP7B KO animals were significantly increased compared to WT animals, and virus treatment significantly normalized the urinary copper concentrations (**FIG. 37**).

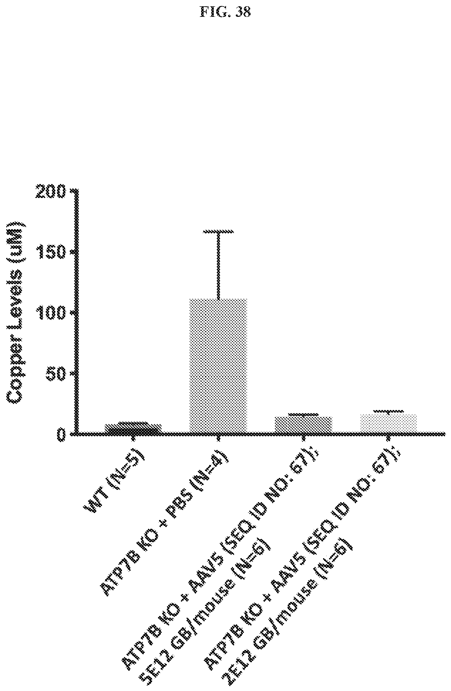

ATP7B KO mice (approx. 4-6 weeks old) were then injected intravenously via the tail vein with AAV5 at a concentration of 2×10¹² gc/mouse or 5×10¹² gc/mouse, containing ATP7B variant 3 (SEQ ID NO: 5) under the control of the promoter having SEQ ID NO: 67, or PBS control. Urine samples were collected 4 weeks following injection. Copper concentrations were measured as described above. Urine copper concentrations from ATP7B KO animals were significantly increased compared to WT animals, and virus treatment significantly normalized the urinary copper concentrations (**FIG. 38**).

### EXAMPLE 17

### Liver Expression of ATP7B in AAV5 Mice

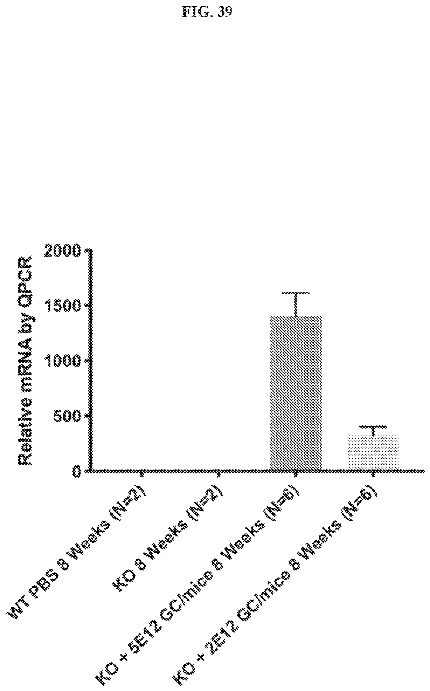

ATP7B knockout (KO) mice (approx. 4-6 weeks old) were injected intravenously (tail vein) with AAV5 (2×10¹² gc/mouse or 5×10¹² gc/mouse) containing ATP7B variant 3 (SEQ ID NO: 5) under the control of the promoter having SEQ ID NO: 67, or PBS control. Livers were processed eight weeks after viral delivery and stored in RNAlater reagent. RNA samples were purified from the preserved liver samples, and transcript abundance for ATP7B variant 3 was quantified by QPCR as described above. Virus treatment led to dose-dependent ATP7B expression in ATP7B KO animals (**FIG. 39**).

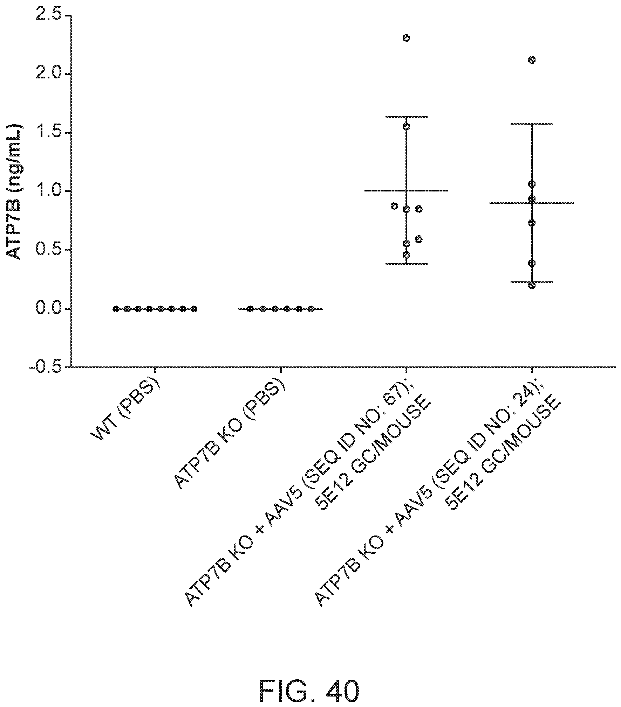

ATP7B protein expression was measured by MSD-ELISA analysis as described above. ATP7B KO mice (approx. 4-6 weeks old) were injected intravenously via the tail vein with AAV5 at a concentration of 5×10¹² gc/mouse, containing ATP7B variant 3 (SEQ ID NO: 5) under the control of the promoter having SEQ ID NO: 24, SEQ ID NO: 67, or PBS control. Five months after viral delivery, livers were processed as described above and MSD-ELISA analysis was carried out to measure ATP7B expression. Virus treatment led to robust and durable ATP7B expression in ATP7B KO animals (**FIG. 40**).

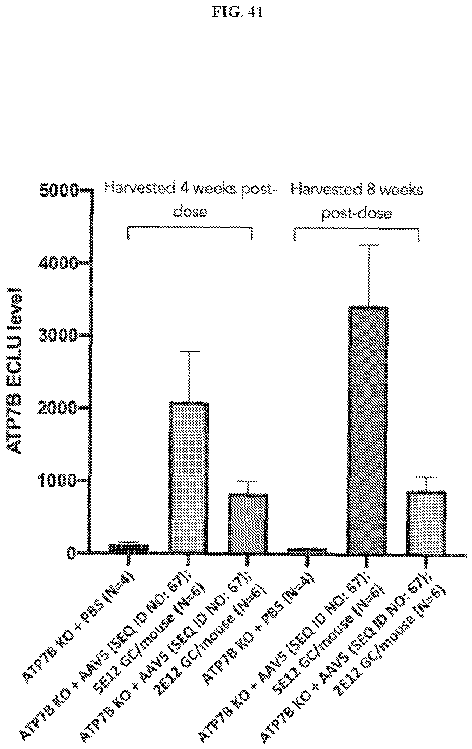

ATP7B protein expression was also measured by MSD-ELISA analysis in mice administered with AAV5 at a concentration of 5×10¹² gc/mouse, or 2×10¹² gc/mouse. ATP7B knockout (KO) mice (approx. 4-6 weeks old) were injected intravenously (tail vein) with AAV5 containing ATP7B variant 3 (SEQ ID NO: 5) under the control of the promoter having SEQ ID NO: 67, or PBS control. Four or eight weeks after viral delivery, livers were processed as described above and MSD-ELISA analysis was carried out as previously described to measure ATP7B expression. Virus treatment led to dose-dependent ATP7B expression in ATP7B KO animals (**FIG. 41**).

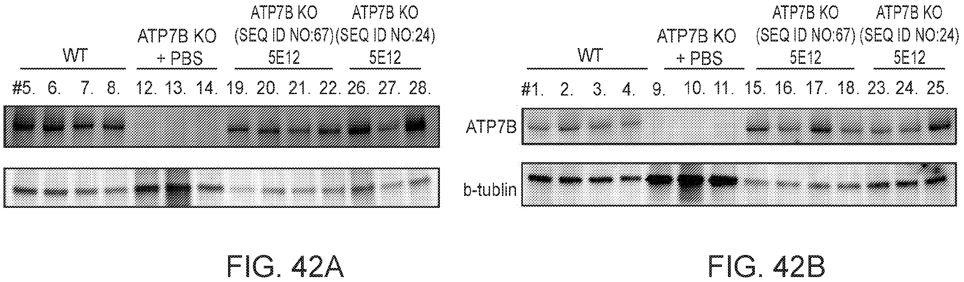

ATP7B protein expression was also measured by Western Blot analysis. ATP7B KO mice (approx. 4-6 weeks old) were injected intravenously via the tail vein with AAV5 at a concentration of 5×10¹² gc/mouse, containing ATP7B variant 3 (SEQ ID NO: 5) under the control of the promoter having SEQ ID NO: 24, SEQ ID NO: 67, or PBS control. Five months after viral delivery, livers were harvested. Four millimeter liver punches were collected from right liver lateral lobes and frozen in liquid nitrogen. Liver punches (1 per animal) were submerged in lysis buffer to release and solubilize the proteins. After centrifugation to clear the lysates, western blotting analysis was carried out to measure ATP7B expression. ~15ug of total protein was loaded onto the gel for western blotting, and Abcam recombinant anti-ATP7B antibody (EPR6793, catalog # ab131208) was used for detection of ATP7B protein. Virus treatment led to robust and durable ATP7B expression in both ATP7B KO males and females (**FIG. 42**).

While preferred embodiments of the present invention have been shown and described herein, it will be obvious to those skilled in the art that such embodiments are provided by way of example only. Numerous variations, changes, and substitutions will now occur to those skilled in the art without departing from the invention. It should be understood that various alternatives to the embodiments of the invention described herein may be employed in practicing the invention. It is intended that the following claims define the scope of the invention and that methods and structures within the scope of these claims and their equivalents be covered thereby.

### EMBODIMENTS

The invention further provides the following non-limiting embodiments:
1. A nucleic acid molecule comprising a nucleotide sequence having (i) at least 80% sequence identity to SEQ ID NO: 3 or 4, or (ii) at least 92% sequence identity to SEQ ID NO: 5 or 6, wherein said nucleic acid molecule encodes a functional ATP7B protein.
2. The nucleic acid molecule of embodiment 1, comprising a nucleotide sequence having at least 85% sequence identity to SEQ ID NO: 3 or 4.
3. The nucleic acid molecule of embodiment 1, comprising a nucleotide sequence having at least 90% sequence identity to SEQ ID NO: 3 or 4.
4. The nucleic acid molecule of embodiment 1, comprising a nucleotide sequence having at least 95% sequence identity to any one of SEQ ID NOs: 3-6.
5. The nucleic acid molecule of embodiment 1, comprising a nucleotide sequence having any one of SEQ ID NOs: 3-6.
6. The nucleic acid molecule of embodiment 1, wherein the nucleic acid molecule encodes a functional fragment of ATP7B.
7. The nucleic acid molecule of embodiment 1, wherein the nucleic acid molecule encodes an ATP7B protein having SEQ ID NO: 2.
8. The nucleic acid molecule of any one of embodiments 1-7, wherein the level of expression of the functional ATP7B protein from the nucleic acid molecule in the liver is at least 3-fold greater relative to the level of expression of ATP7B protein in the liver from a nucleic acid molecule comprising SEQ ID NO: 1.
9. The nucleic acid molecule of embodiment 8, wherein the level of expression of the functional ATP7B protein from the nucleic acid molecule in the liver is at least 5-fold greater relative to the level of expression of ATP7B protein in the liver from a nucleic acid molecule comprising SEQ ID NO: 1.
10. The nucleic acid molecule of embodiment 9, wherein the level of expression of the functional ATP7B protein from the nucleic acid molecule in the liver is at least 7-fold greater relative to the level of expression of ATP7B protein in the liver from a nucleic acid molecule comprising SEQ ID NO: 1.
11. The nucleic acid molecule of any one of embodiments 1-10, further comprising a regulatory element.
12. The nucleic acid molecule of embodiment 11, wherein the regulatory element comprises a sequence having at least 85% sequence identity to any one of SEQ ID NOs: 19-43.
13. The nucleic acid molecule of embodiment 12, wherein the regulatory element comprises a sequence having at least 90% sequence identity to any one of SEQ ID NOs: 19-43.
14. The nucleic acid molecule of embodiment 13, wherein the regulatory element comprises a sequence having at least 95% sequence identity to any one of SEQ ID NOs: 19-43.
15. The nucleic acid molecule of embodiment 14, wherein the regulatory element comprises a sequence having any one of SEQ ID NOs: 19-43.
16. The nucleic acid molecule of any one of embodiments 11-15, wherein the regulatory element comprises a promoter sequence.
17. The nucleic acid molecule of embodiment 16, wherein the promoter sequence produces at least 10-fold greater expression in a mammalian cell relative to the CMV promoter.
18. The nucleic acid molecule of embodiment 16, wherein the promoter sequence produces at least 50-fold greater expression in a mammalian cell relative to the CMV promoter.
19. The nucleic acid molecule of any one of embodiments 11-18, wherein the regulatory element further comprises an enhancer sequence.
20. The nucleic acid molecule of any one of embodiments 11-19, wherein the regulatory element has less than 150 bp.
21. The nucleic acid molecule of any one of embodiments 11-19, wherein the regulatory element has less than 120 bp.
22. The nucleic acid molecule of any one of embodiments 11-19, wherein the regulatory element has less than 105 bp.
23. The nucleic acid molecule of any one of embodiments 1-22, further comprising a 5' ITR and 3' ITR sequences of a virus.
24. The nucleic acid molecule of embodiment 23, wherein the 5' ITR and 3' ITR sequences are adeno-associated virus (AAV) sequences.
25. The nucleic acid molecule of embodiment 24, wherein the AAV is an AAV2, AAV5, AAV8 or AAV9.
26. An expression vector comprising the nucleic acid molecule of any one of embodiments 1-25.
27. The expression vector of embodiment 26, wherein the expression vector is a viral vector.
28. The expression vector of embodiment 27, wherein the viral vector is an AAV vector.
29. A viral particle comprising the nucleic acid molecule of any one of embodiments 1-25 or the expression vector of any one of embodiments 26-28.
30. The viral particle of embodiment 29, wherein the viral particle comprises capsid proteins of an AAV.
31. The viral particle of embodiment 30, wherein the AAV is an AAV5, AAV8 or AAV9.
32. A host cell comprising the nucleic acid molecule of any one of embodiments 1-25 or the expression vector of any one of embodiments 26-28.
33. A pharmaceutical composition comprising the nucleic acid molecule of any one of embodiments 1-25, the expression vector of any one of embodiments 26-28, or the viral particle of any one of embodiments 29-31, and one or more pharmaceutically acceptable excipients.
34. A method for increasing expression of functional ATP7B protein in a subject comprising administering to said subject the nucleic acid molecule of any one of embodiments 1-25, the expression vector of any one of embodiments 26-28, the viral particle of any one of embodiments 29-31, or the pharmaceutical composition of embodiment 33.
35. A method for treating a disorder associated with an ATP7B deficiency comprising administering to a subject in need thereof a therapeutically effective amount of the nucleic acid molecule of any one of embodiments 1-25, the expression vector of any one of embodiments 26-28, the viral particle of any one of embodiments 29-31, or the pharmaceutical composition of embodiment 33.
36. A method for treating Wilson's disease comprising administering to a subject in need thereof a therapeutically effective amount of the nucleic acid molecule of any one of embodiments 1-25, the expression vector of any one of embodiments 26-28, the viral particle of any one of embodiments 29-31, or the pharmaceutical composition of embodiment 33.
37. The nucleic acid molecule of any one of embodiments 1-25, the expression vector of any one of embodiments 26-28, the viral particle of any one of embodiments 29-31, or the pharmaceutical composition of embodiment 33 for use in increasing the expression of functional ATP7B in a subject.
38. The nucleic acid molecule of any one of embodiments 1-25, the expression vector of any one of embodiments 26-28, the viral particle of any one of embodiments 29-31, or the pharmaceutical composition of embodiment 33 for use in increasing treating a disorder associated with an ATP7B deficiency.
39. The nucleic acid molecule of any one of embodiments 1-25, the expression vector of any one of embodiments 26-28, the viral particle of any one of embodiments 29-31, or the pharmaceutical composition of embodiment 33 for use in treating Wilson's disease.
40. A process of producing a viral particle according to any of embodiments 29-31, comprising: (i) culturing a host cell according to embodiment 32 in a culture medium, and (ii) harvesting the viral particles from the cell culture supernatant or the host cells.
41. The process of embodiment 40, wherein the host cell further comprises (a) a nucleic acid molecule encoding AAV rep and/or cap genes, and/or (b) a nucleic acid molecule comprising viral helper genes.
42. Use of the nucleic acid molecule of any of embodiments 1-25 or the expression vector of any one of embodiments 26-28 for the production of viral particles.
43. A nucleic acid molecule comprising:
   (i) a regulatory element comprising a sequence having at least 80% sequence identity to any one of SEQ ID NOs: 19-43, and
   (ii) a variant nucleotide sequence encoding a functional ATP7B protein, wherein the variant nucleotide sequence has been optimized for expression in liver from a viral vector.
44. The nucleic acid molecule of embodiment 43, wherein the regulatory element comprises a sequence having at least 85% sequence identity to any one of SEQ ID NOs: 19-43.
45. The nucleic acid molecule of embodiment 44, wherein the regulatory element comprises a sequence having at least 90% sequence identity to any one of SEQ ID NOs: 19-43.
46. The nucleic acid molecule of embodiment 45, wherein the regulatory element comprises a sequence having at least 95% sequence identity to any one of SEQ ID NOs: 19-43.
47. The nucleic acid molecule of embodiment 46, wherein the regulatory element comprises a sequence having any one of SEQ ID NOs: 19-43.
48. The nucleic acid molecule of any one of embodiments 43-47, wherein the variant nucleotide sequence has been codon optimized for expression in the liver.
49. The nucleic acid molecule of any one of embodiments 43-48, wherein the variant nucleotide sequence has been optimized for expression from an AAV vector.
50. The nucleic acid molecule of any one of embodiments 43-49, wherein the variant nucleotide sequence has least 80% sequence identity to any one of SEQ ID NOs: 3-18.
51. The nucleic acid molecule of embodiment 50, wherein the variant nucleotide sequence has at least 85% sequence identity to any one of SEQ ID NOs: 3-18.
52. The nucleic acid molecule of embodiment 51, wherein the variant nucleotide sequence has at least 90% sequence identity to any one of SEQ ID NOs: 3-18.
53. The nucleic acid molecule of embodiment 52, wherein the variant nucleotide sequence has at least 95% sequence identity to any one of SEQ ID NOs: 3-18.
54. The nucleic acid molecule of embodiment 53, wherein the variant nucleotide sequence comprises any one of SEQ ID NOs: 3-18.
55. The nucleic acid molecule of any one of embodiments 43-54, wherein the regulatory element is a promoter sequence.
56. The nucleic acid molecule of any one of embodiments 43-55, further comprising an enhancer sequence.
57. The nucleic acid molecule of any one of embodiments 43-56, wherein the regulatory element has less than 150 bp.
58. The nucleic acid molecule of any one of embodiments 43-56, wherein the regulatory element has less than 120 bp.
59. The nucleic acid molecule of any one of embodiments 43-56, wherein the regulatory element has less than 105 bp.
60. The nucleic acid molecule of any one of embodiments 43-59, wherein the regulatory element produces at least 10-fold greater expression in a mammalian cell relative to the CMV promoter.
61. The nucleic acid molecule of any one of embodiments 43-60, wherein the regulatory element produces at least 50-fold greater expression in a mammalian cell relative to the CMV promoter.
62. The nucleic acid molecule of any one of embodiments 43-61, wherein the nucleic acid molecule encodes a functional fragment of ATP7B.
63. The nucleic acid molecule of any one of embodiments 43-61, wherein the nucleic acid molecule encodes an ATP7B protein having SEQ ID NO: 2.
64. The nucleic acid molecule of any one of embodiments 43-63, wherein the level of expression of the functional ATP7B protein from the variant nucleotide sequence in the liver is at least 5-fold greater relative to the level of expression of ATP7B protein in the liver from a nucleic acid molecule comprising SEQ ID NO: 1.
65. The nucleic acid molecule of any one of embodiments 43-63, wherein the level of expression of the functional ATP7B protein from the variant nucleotide sequence in the liver is at least 7-fold greater relative to the level of expression of ATP7B protein in the liver from a nucleic acid molecule comprising SEQ ID NO: 1.
66. The nucleic acid molecule of any one of embodiments 43-65, further comprising a 5' ITR and 3' ITR sequences of a virus.
67. The nucleic acid molecule of embodiment 66, wherein the 5' ITR and 3' ITR sequences are adeno-associated virus (AAV) sequences.
68. The nucleic acid molecule of embodiment 67, wherein the AAV is an AAV2, AAV5, AAV8 or AAV9.
69. An expression vector comprising the nucleic acid molecule of any one of embodiments 43-68.
70. The expression vector of embodiment 69, wherein the expression vector is a viral vector.
71. The expression vector of embodiment 70, wherein the viral vector is an AAV vector.
72. A viral particle comprising the nucleic acid molecule of any one of embodiments 43-68 or the expression vector of any one of embodiments 69-71.
73. The viral particle of embodiment 72, wherein the viral particle comprises capsid proteins of an AAV.
74. The viral particle of embodiment 73, wherein the AAV is an AAV5, AAV8 or AAV9.
75. A host cell comprising the nucleic acid molecule of any one of embodiments 43-68 or the expression vector of any one of embodiments 69-71.
76. A pharmaceutical composition comprising the nucleic acid molecule of any one of embodiments 43-68, the expression vector of any one of embodiments 69-71, or the viral particle of any one of embodiments 72-74, and one or more pharmaceutically acceptable excipients.
77. A method for increasing expression of functional ATP7B protein in a subject comprising administering to said subject the nucleic acid molecule of any one of embodiments 43-68, the expression vector of any one of embodiments 68-70, the viral particle of any one of embodiments 72-74, or the pharmaceutical composition of embodiment 76.
78. A method for treating a disorder associated with an ATP7B deficiency comprising administering to a subject in need thereof a therapeutically effective amount of the nucleic acid molecule of any one of embodiments 43-68, the expression vector of any one of embodiments 69-71, the viral particle of any one of embodiments 72-74, or the pharmaceutical composition of embodiment 76.
79. A method for treating Wilson's disease comprising administering to a subject in need thereof a therapeutically effective amount of the nucleic acid molecule of any one of embodiments 43-68, the expression vector of any one of embodiments 69-71, the viral particle of any one of embodiments 72-74, or the pharmaceutical composition of embodiment 76.
80. The nucleic acid molecule of any one of embodiments 43-68, the expression vector of any one of embodiments 69-71, the viral particle of any one of embodiments 72-74, or the pharmaceutical composition of embodiment 76 for use in increasing the expression of functional ATP7B in a subject.
81. The nucleic acid molecule of any one of embodiments 41-68, the expression vector of any one of embodiments 69-71, the viral particle of any one of embodiments 72-74, or the pharmaceutical composition of embodiment 76 for use in increasing treating a disorder associated with an ATP7B deficiency.
82. The nucleic acid molecule of any one of embodiments 43-68, the expression vector of any one of embodiments 69-71, the viral particle of any one of embodiments 72-74, or the pharmaceutical composition of embodiment 76 for use in treating Wilson's disease.
83. A process of producing a viral particle according to any one of embodiments 72-74, comprising: (i) culturing a host cell according to embodiment 75 in a culture medium, and (ii) harvesting the viral particles from the cell culture supernatant or the host cells.
84. The process of embodiment 83, wherein the host cell further comprises (a) a nucleic acid molecule encoding AAV rep and/or cap genes, and/or (b) a nucleic acid molecule comprising viral helper genes.
85. Use of the nucleic acid molecule of any one of embodiments 43-68 or the expression vector of any one of embodiments 69-71 for the production of viral particles.
86. A nucleic acid molecule comprising:
   (i) a regulatory element comprising a sequence having at least 80% sequence identity to any one of SEQ ID NOs: 66-68, and
   (ii) a nucleotide sequence encoding a therapeutic transgene,
   wherein the therapeutic transgene is operably linked to the regulatory element.
87. The nucleic acid molecule of embodiment 86, wherein the regulatory element comprises a sequence having at least 85% sequence identity to any one of SEQ ID NOs: 66-68.
88. The nucleic acid molecule of embodiment 87, wherein the regulatory element comprises a sequence having at least 90% sequence identity to any one of SEQ ID NOs: 66-68.
89. The nucleic acid molecule of embodiment 88, wherein the regulatory element comprises a sequence having at least 95% sequence identity to any one of SEQ ID NOs: 66-68.
90. The nucleic acid molecule of embodiment 89, wherein the regulatory element comprises a sequence having any one of SEQ ID NOs: 66-68.
91. The nucleic acid molecule of embodiment 90, wherein the regulatory element comprises SEQ ID NO: 67.
92. The nucleic acid molecule of any one of embodiments 86-91, wherein the therapeutic transgene encodes any one of ATP7A, ATP7B, ATP8B1, ABCB4, ABCB11, CDKL5, CNTNAP2, ZEB2, Factor V, Factor VII, Factor VIII, Factor IX, Factor X, Factor XI or Factor XII, or a variant or functional fragment thereof.
93. The nucleic acid molecule of embodiment 92, wherein the therapeutic transgene encodes Factor VIII or a variant or functional fragment thereof.
94. The nucleic acid molecule of embodiment 90, wherein the therapeutic transgene encodes ATP7B or a variant or functional fragment thereof.
95. The nucleic acid molecule of any one of embodiments 86-94, wherein the therapeutic transgene comprises a variant nucleotide sequence that has been codon optimized for expression in the liver.
96. The nucleic acid molecule of any one of embodiments 86-95, wherein the therapeutic transgene comprises a variant nucleotide sequence has been optimized for expression from an AAV vector.
97. The nucleic acid molecule of any one of embodiments 86-96, wherein the therapeutic transgene comprises a variant nucleotide sequence having least 80% sequence identity to any one of SEQ ID NOs: 3-18.
98. The nucleic acid molecule of embodiment 97, wherein the therapeutic transgene sequence comprises a variant nucleotide sequence having at least 85% sequence identity to any one of SEQ ID NOs: 3-18.
99. The nucleic acid molecule of embodiment 98, wherein the therapeutic transgene sequence comprises a variant nucleotide sequence having at least 90% sequence identity to any one of SEQ ID NOs: 3-18.
100. The nucleic acid molecule of embodiment 99, wherein the therapeutic transgene sequence comprises a variant nucleotide sequence having at least 95% sequence identity to any one of SEQ ID NOs: 3-18.
101. The nucleic acid molecule of embodiment 100, wherein the therapeutic transgene sequence comprises any one of SEQ ID NOs: 3-18.
102. The nucleic acid molecule of embodiment 101, wherein the therapeutic transgene sequence comprises SEQ ID NO: 5.
103. The nucleic acid molecule of any one of embodiments 86-102, wherein the regulatory element is a promoter sequence.
104. The nucleic acid molecule of any one of embodiments 86-103, further comprising an enhancer sequence.
105. The nucleic acid molecule of any one of embodiments 86-104, wherein the regulatory element has less than 150 bp.
106. The nucleic acid molecule of any one of embodiments 86-104, wherein the regulatory element has less than 120 bp.
107. The nucleic acid molecule of any one of embodiments 86-104, wherein the regulatory element has less than 105 bp.
108. The nucleic acid molecule of any one of embodiments 86-107, wherein the regulatory element produces at least 10-fold greater expression in a mammalian cell relative to the CMV promoter.
109. The nucleic acid molecule of any one of embodiments 86-108, wherein the regulatory element produces at least 50-fold greater expression in a mammalian cell relative to the CMV promoter.
110. The nucleic acid molecule of any one of embodiments 86-109, wherein the therapeutic transgene encodes a functional fragment of ATP7B.
111. The nucleic acid molecule of any one of embodiments 86-109, wherein the therapeutic transgene encodes an ATP7B protein having SEQ ID NO: 2.
112. The nucleic acid molecule of any one of embodiments 86-111, wherein the level of expression of the functional ATP7B protein from the therapeutic transgene sequence in the liver is at least 5-fold greater relative to the level of expression of ATP7B protein in the liver from a nucleic acid molecule comprising SEQ ID NO: 1.
113. The nucleic acid molecule of embodiment 112, wherein the level of expression of the functional ATP7B protein from the therapeutic transgene sequence in the liver is at least 7-fold greater relative to the level of expression of ATP7B protein in the liver from a nucleic acid molecule comprising SEQ ID NO: 1.
114. The nucleic acid molecule of any one of embodiments 86-113, further comprising a 5' ITR and 3' ITR sequences of a virus.
115. The nucleic acid molecule of embodiment 114, wherein the 5' ITR and 3' ITR sequences are adeno-associated virus (AAV) sequences.
116. The nucleic acid molecule of embodiment 115, wherein the AAV is an AAV2, AAV5, AAV8 or AAV9.
117. An expression vector comprising the nucleic acid molecule of any one of embodiments 86-116.
118. The expression vector of embodiment 117, wherein the expression vector is a viral vector.
119. The expression vector of embodiment 118, wherein the viral vector is an AAV vector.
120. A viral particle comprising the nucleic acid molecule of any one of embodiments 86-116 or the expression vector of any one of embodiments 117-119.
121. The viral particle of embodiment 120, wherein the viral particle comprises capsid proteins of an AAV.
122. The viral particle of embodiment 121, wherein the AAV is an AAV5, AAV8 or AAV9.
123. A host cell comprising the nucleic acid molecule of any one of embodiments 86-116 or the expression vector of any one of embodiments 117-119.
124. A pharmaceutical composition comprising the nucleic acid molecule of any one of embodiments 86-116, the expression vector of any one of embodiments 117-119, or the viral particle of any one of embodiments 120-122, and one or more pharmaceutically acceptable excipients.
125. A method for increasing expression of functional ATP7B protein in a subject comprising administering to said subject the nucleic acid molecule of any one of embodiments 86-116, the expression vector of any one of embodiments 117-119, the viral particle of any one of embodiments 120-122, or the pharmaceutical composition of embodiment 124.
126. A method for treating a disorder associated with an ATP7B deficiency comprising administering to a subject in need thereof a therapeutically effective amount of the nucleic acid molecule of any one of embodiments 86-116, the expression vector of any one of embodiments 117-119, the viral particle of any one of embodiments 120-122, or the pharmaceutical composition of embodiment 124.
127. A method for treating Wilson's disease comprising administering to a subject in need thereof a therapeutically effective amount of the nucleic acid molecule of any one of embodiments 86-116, the expression vector of any one of embodiments 117-119, the viral particle of any one of embodiments 120-122, or the pharmaceutical composition of embodiment 124.
128. The nucleic acid molecule of any one of embodiments 86-116, the expression vector of any one of embodiments 117-119, the viral particle of any one of embodiments 120-122, or the pharmaceutical composition of embodiment 124 for use in increasing the expression of functional ATP7B in a subject.
129. The nucleic acid molecule of any one of embodiments 86-116, the expression vector of any one of embodiments 117-119, the viral particle of any one of embodiments 120-122, or the pharmaceutical composition of embodiment 124 for use in increasing treating a disorder associated with an ATP7B deficiency.
130. The nucleic acid molecule of any one of embodiments 86-116, the expression vector of any one of embodiments 117-119, the viral particle of any one of embodiments 120-122, or the pharmaceutical composition of embodiment 124 for use in treating Wilson's disease.
131. A process of producing a viral particle according to any one of embodiments 120-122, comprising: (i) culturing a host cell according to embodiment 123 in a culture medium, and (ii) harvesting the viral particles from the cell culture supernatant or the host cells.
132. The process of embodiment 131, wherein the host cell further comprises (a) a nucleic acid molecule encoding AAV rep and/or cap genes, and/or (b) a nucleic acid molecule comprising viral helper genes.
133. Use of the nucleic acid molecule of any one of embodiments 86-116 or the expression vector of any one of embodiments 117-119 for the production of viral particles.
134. A nucleic acid molecule comprising:
   (i) a regulatory element comprising a sequence having at least 80% sequence identity to SEQ ID NO: 24, and
   (ii) a variant nucleotide sequence having at least 80% sequence identity to SEQ ID NO: 5, wherein said variant nucleotide sequence encodes a functional ATP7B protein.
135. The nucleic acid molecule of embodiment 134, wherein the regulatory element comprises a sequence having at least 85% sequence identity to SEQ ID NO: 24.
136. The nucleic acid molecule of embodiment 135, wherein the regulatory element comprises a sequence having at least 90% sequence identity to SEQ ID NO: 24.
137. The nucleic acid molecule of embodiment 136, wherein the regulatory element comprises a sequence having at least 95% sequence identity to SEQ ID NOs: 24.
138. The nucleic acid molecule of embodiment 137, wherein the regulatory element comprises a sequence having SEQ ID NO: 24.
139. The nucleic acid molecule of any one of embodiments 134-138, wherein the variant nucleotide sequence comprises a sequence having at least 85% sequence identity to SEQ ID NO: 5.
140. The nucleic acid molecule of embodiment 139, wherein the variant nucleotide sequence comprises a sequence having at least 90% sequence identity to SEQ ID NO: 5.
141. The nucleic acid molecule of embodiment 140, wherein the variant nucleotide sequence comprises a sequence having at least 95% sequence identity to SEQ ID NOs: 5.
142. The nucleic acid molecule of embodiment 141, wherein the variant nucleotide sequence comprises a sequence having SEQ ID NO: 5.
143. A nucleic acid molecule comprising:
   (i) a regulatory element comprising a sequence having at least 80% sequence identity to SEQ ID NO: 67, and
   (ii) a variant nucleotide sequence having at least 80% sequence identity to SEQ ID NO: 5, wherein said variant nucleotide sequence encodes a functional ATP7B protein.
144. The nucleic acid molecule of embodiment 143, wherein the regulatory element comprises a sequence having at least 85% sequence identity to SEQ ID NO: 67.
145. The nucleic acid molecule of embodiment 144, wherein the regulatory element comprises a sequence having at least 90% sequence identity to SEQ ID NO: 67.
146. The nucleic acid molecule of embodiment 145, wherein the regulatory element comprises a sequence having at least 95% sequence identity to SEQ ID NOs: 67.
147. The nucleic acid molecule of embodiment 146, wherein the regulatory element comprises a sequence having SEQ ID NO: 67.
148. The nucleic acid molecule of any one of embodiments 143-147, wherein the variant nucleotide sequence comprises a sequence having at least 85% sequence identity to SEQ ID NO: 5.
149. The nucleic acid molecule of embodiment 148, wherein the variant nucleotide sequence comprises a sequence having at least 90% sequence identity to SEQ ID NO: 5.
150. The nucleic acid molecule of embodiment 149, wherein the variant nucleotide sequence comprises a sequence having at least 95% sequence identity to SEQ ID NOs: 5.
151. The nucleic acid molecule of embodiment 150, wherein the variant nucleotide sequence comprises a sequence having SEQ ID NO: 5.
152. The nucleic acid molecule of any one of embodiments 134-151, wherein the variant nucleotide sequence encodes a functional fragment of ATP7B.
153. The nucleic acid molecule of any one of embodiments 134-152, wherein the variant nucleotide sequence encodes an ATP7B protein having SEQ ID NO: 2.
154. The nucleic acid molecule of any one of embodiments 134-153, further comprising a 5' ITR and 3' ITR sequences of a virus.
155. The nucleic acid molecule of embodiment 154, wherein the 5' ITR and 3' ITR sequences are adeno-associated virus (AAV) sequences.
156. The nucleic acid molecule of embodiment 155, wherein the AAV is an AAV2, AAV5, AAV8 or AAV9.
157. An expression vector comprising the nucleic acid molecule of any one of embodiments 134-156.
158. The expression vector of embodiment 157, wherein the expression vector is a viral vector.
159. The expression vector of embodiment 158, wherein the viral vector is an AAV vector.
160. A viral particle comprising the nucleic acid molecule of any one of embodiments 134-156 or the expression vector of any one of embodiments 157-159.
161. The viral particle of embodiment 160, wherein the viral particle comprises capsid proteins of an AAV.
162. The viral particle of embodiment 161, wherein the AAV is an AAV5, AAV8 or AAV9.
163. A host cell comprising the nucleic acid molecule of any one of embodiments 134-156 or the expression vector of any one of embodiments 157-159.
164. A pharmaceutical composition comprising the nucleic acid molecule of any one of embodiments 134-156, the expression vector of any one of embodiments 157-159, or the viral particle of any one of embodiments 160-162, and one or more pharmaceutically acceptable excipients.
165. A method for increasing expression of functional ATP7B protein in a subject comprising administering to said subject the nucleic acid molecule of any one of embodiments 134-156, the expression vector of any one of embodiments 157-159, the viral particle of any one of embodiments 160-162, or the pharmaceutical composition of embodiment 164.
166. A method for treating a disorder associated with an ATP7B deficiency comprising administering to a subject in need thereof a therapeutically effective amount of the nucleic acid molecule of any one of embodiments 134-156, the expression vector of any one of embodiments 157-159, the viral particle of any one of embodiments 160-162, or the pharmaceutical composition of embodiment 164.
167. A method for treating Wilson's disease comprising administering to a subject in need thereof a therapeutically effective amount of the nucleic acid molecule of any one of embodiments 134-156, the expression vector of any one of embodiments 157-159, the viral particle of any one of embodiments 160-162, or the pharmaceutical composition of embodiment 164.
168. The nucleic acid molecule of any one of embodiments 134-156, the expression vector of any one of embodiments 157-159, the viral particle of any one of embodiments 160-162, or the pharmaceutical composition of embodiment 164 for use in increasing the expression of functional ATP7B in a subject.
169. The nucleic acid molecule of any one of embodiments 134-156, the expression vector of any one of embodiments 157-159, the viral particle of any one of embodiments 160-162, or the pharmaceutical composition of embodiment 164 for use in increasing treating a disorder associated with an ATP7B deficiency.
170. The nucleic acid molecule of any one of embodiments 134-156, the expression vector of any one of embodiments 157-159, the viral particle of any one of embodiments 160-162, or the pharmaceutical composition of embodiment 164 for use in treating Wilson's disease.
171. A process of producing a viral particle according to any one of embodiments 160-162, comprising: (i) culturing a host cell according to embodiment 163 in a culture medium, and (ii) harvesting the viral particles from the cell culture supernatant or the host cells.
172. The process of embodiment 171, wherein the host cell further comprises (a) a nucleic acid molecule encoding AAV rep and/or cap genes, and/or (b) a nucleic acid molecule comprising viral helper genes.
173. Use of the nucleic acid molecule of any one of embodiments 134-156 or the expression vector of any one of embodiments 157-159 for the production of viral particles.

## Claims

1. A nucleic acid molecule comprising:
a regulatory element comprising a sequence having at least 80%, at least 85%, at least 90%, or at least 95%, or at least 100% sequence identity to any one of SEQ ID NOs: 66-68, and
a nucleotide sequence encoding a therapeutic transgene, wherein the therapeutic transgene is operably linked to the regulatory element.

2. The nucleic acid molecule of claim 1, wherein the therapeutic transgene encodes any one of ATP7A, ATP7B, ATP8B1, ABCB4, ABCB11, CDKL5, CNTNAP2, ZEB2, Factor V, Factor VII, Factor VIII, Factor IX, Factor X, Factor XI or Factor XII, or a variant or functional fragment thereof.

3. The nucleic acid molecule of claim 1 or claim 2, wherein the therapeutic transgene comprises a variant nucleotide sequence that has been codon optimized for expression in the liver, and/or wherein the therapeutic transgene comprises a variant nucleotide sequence has been optimized for expression from an AAV vector.

4. The nucleic acid molecule of any one of claims 1-3, wherein the regulatory element is a promoter sequence, optionally wherein the regulatory element has less than 150 bp, less than 120 bp, or less than 105 bp.

5. The nucleic acid molecule of any one of claims 1-4, further comprising an enhancer sequence.

6. The nucleic acid molecule of any one of claims 1-5, wherein the regulatory element produces at least 10-fold, or at least 50-fold, greater expression in a mammalian cell relative to the CMV promoter.

7. The nucleic acid molecule of any one of claims 1-6, further comprising a 5' ITR and 3' ITR sequences of a virus.

8. The nucleic acid molecule of claim 7, wherein the 5' ITR and 3' ITR sequences are adeno-associated virus (AAV) sequences, optionally AAV2, AAV5, AAV8 or AAV9.

9. An expression vector comprising the nucleic acid molecule of any one of claims 1-8.

10. The expression vector of claim 9, wherein the expression vector is a viral vector, optionally an AAV vector.

11. A viral particle comprising the nucleic acid molecule of any one of claims 1-8 or the expression vector of any one of claims 9-10, optionally wherein the viral particle comprises capsid proteins of an AAV, optionally AAV5, AAV8 or AAV9.

12. A host cell comprising the nucleic acid molecule of any one of claims 1-8 or the expression vector of any one of claims 9-10.

13. A pharmaceutical composition comprising the nucleic acid molecule of any one of claims 1-8, the expression vector of any one of claims 9-10, or the viral particle of claim 11, and one or more pharmaceutically acceptable excipients.

14. A process of producing a viral particle according to claim 11, comprising:
(i) culturing a host cell according to claim 12 in a culture medium, and
(ii) harvesting the viral particles from the cell culture supernatant or the host cells,
optionally wherein the host cell further comprises:
(a) a nucleic acid molecule encoding AAV rep and/or cap genes, and/or
(b) a nucleic acid molecule comprising viral helper genes.

15. Use of the nucleic acid molecule of any one of claims 1-8 or the expression vector of any one of claims 9-10 for the production of viral particles.
